(19) Europäisches Patentamt / European Patent Office / Office européen des brevets

(11) **EP 4 261 211 A1**

(12) **EUROPEAN PATENT APPLICATION**
published in accordance with Art. 153(4) EPC


(43) Date of publication:
**18.10.2023 Bulletin 2023/42**

(21) Application number: **21902742.2**

(22) Date of filing: **10.12.2021**

(51) International Patent Classification (IPC):
***C07D 401/14*** (2006.01) ***C07D 413/14*** (2006.01)
***C07D 215/12*** (2006.01) ***A61K 31/47*** (2006.01)
***A61P 35/00*** (2006.01)

(52) Cooperative Patent Classification (CPC):
**A61K 31/47; A61P 35/00; C07D 215/12; C07D 401/14; C07D 413/14**

(86) International application number:
**PCT/CN2021/137254**

(87) International publication number:
**WO 2022/122037 (16.06.2022 Gazette 2022/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.12.2020 CN 202011459208**
**10.03.2021 CN 202110261282**
**29.07.2021 CN 202110861847**



(71) Applicant: **Etern Biopharma (Shanghai) Co., Ltd.**
**Shanghai 200135 (CN)**

(72) Inventor: **ZHENG, Qiangang**
**Shanghai 200135 (CN)**

(74) Representative: **Santoro, Sofia et al**
**Società Italiana Brevetti S.p.A.**
**Via Giosuè Carducci, 8**
**20123 Milano (IT)**


(54) **DIHYDROISOQUINOLINONE DERIVATIVE AND APPLICATION THEREOF**


(57) Provided are a dihydroisoquinolinone derivative and an application thereof. The dihydroisoquinolinone derivative is represented by formula I, and comprises a pharmaceutically acceptable salt thereof, or an enantiomer, a diastereomer, a tautomer, a solvate, an isotope substitute, a polymorph, a prodrug or a metabolite thereof. The compound of formula I has higher protein-protein interaction inhibition activity on WDR5, and therefore can be used to prevent or treat diseases associated with WDR5.


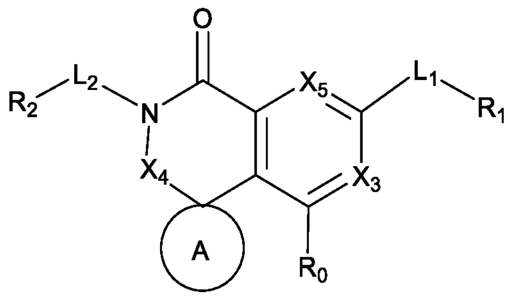

Formula I

EP 4 261 211 A1

## Description

### Technical field

The present disclosure relates to the field of pharmaceutical chemistry, in particular, to a novel heterocyclic compound, a pharmaceutically acceptable salt thereof, or an enantiomer, a diastereoisomer, a tautomer, a solvate, an isotope substitute, a prodrug or metabolite thereof that may be used for WDR5 protein-protein interaction inhibitors. Further, the present disclosure also relates to a method for preparing the compound, a pharmaceutical composition containing the compound and the use of the compound for the preparation of drugs related to diseases or conditions associated with abnormal WDR5 activity.

### Background

WDR5 belongs to the WD40-repeat protein family. WD40 is an important domain composed of about 40 amino acid residues. WDR domain contains a ring-shaped seven-bladed β-propeller domain, wherein each blade contains a conserved serine-histidine (SH) and tryptophan-aspartic acid (WD) sequence. WDR5 protein contains a central cavity formed from 7-bladed β-propeller that runs up and down. WDR5 protein mainly plays a role in regulation through a deep arginine-binding pocket, that is, a core catalytic complex composed of WIN site and MLL1, and forms MWRAD (MLL1, WDR5, RbBP5, ASH2L and DYP-30) core catalytic complex with other proteins. It catalyzes methylation of histone H3, activates gene transcription and participates in epiregulation.

The MLL family protein complex, which is widely present in eukaryotes, is an important class of histone methyltransferases, which catalyzes the methylation of histone H3K4, activates transcription by catalyzing H3K4 methylation and/or by recruiting other transcription-related proteins and mediators, and plays a crucial role in development. Mutations in these proteins, including genomic translocations that fuse them with other transcription factors, can cause a variety of diseases, such as mixed-lineage leukemia (MLL), lymphoma, Kabuki syndrome, etc. By analyzing multiple high-resolution electron microscopy structures of human MLL1 and MLL3 complex catalytic centers binding to nucleosomes, the researchers have found that the amino acid ($MLL1^{AS}$, activation segment) at positions 3775-3786 of MLL1 is inserted into the barrel structure center of WDR5, while the amino end of the SET domain of MLL3 is inserted into WDR5. Compounds designed for WIN pockets where WDR5 binds to histones and MLL1 by using WDR5 as a drug target can reduce gene expression downstream of WDR5 and treat MLL rearrangement-dependent leukemia.

TP53 gene is the most common variant in tumors, and TP53 mutations occur in 50% of human cancers. The high frequency of missense mutations and the high expression level of mutant p53 (mp53) protein in cancer cells promote the new function of mp53 protein ("gain-of" (GOF)), and also actively promote the development and progression of cancer. The growth of tumor cells driven by p53 gene mutation depends on the binding of WDR5 to MLL. MLL, MOZ and histone modifications are all regulated by the expression of GOF p53. Therefore, WDR5 protein interaction inhibitors can also be used to treat TP53-mutated tumors.

WIN site inhibitors of WDR5 can also inhibit the co-binding locus of MYC and WDR5. MYC is a class of oncoproteins with transcription factor function, which is overexpressed in most malignant tumors. MYC can bind to the regulatory sequence of thousands of target genes, regulate target gene expression, and affect the processes such as cell growth, proliferation, metabolism, genome stability and apoptosis. Early studies have found that the extensive binding of MYC to chromatin also relies on WDR5 interactions, and MYC plays an important role in the process of tumorigenesis in vivo through its evolutionarily conserved "MYC box IIIb" sequence binding to hydrophobic fissures on the surface of WDR5. Therefore, by blocking WDR5 protein-protein interaction, MYC function can be blocked and the ability of MYC to drive cancer can be inhibited

Therefore, there is an urgent need to develop WDR5 protein-protein interaction inhibitors having a novel structure, good biological activity and high druggability.

### Summary

An object of the present disclosure is to provide a compound of Formula I or a pharmaceutically acceptable salt thereof, a pharmaceutical composition comprising the compound or a pharmaceutically acceptable salt thereof, and use of the compound or its pharmaceutical composition in the prevention and treatment of diseases or conditions associated with abnormal WDR5 activity.

In one aspect of the present disclosure, a compound of Formula I, or a pharmaceutically acceptable salt thereof, or an enantiomer, a diastereoisomer, a tautomer, an isotope substitute, a solvate, a polymorph, a prodrug or metabolite thereof is provided:

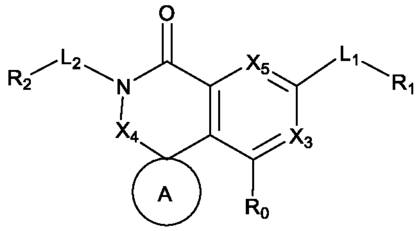

Formula I

wherein,

Ring A is a substituted or unsubstituted 3-8 membered saturated or unsaturated carboncycle or a substituted or unsubstituted 3-8 membered saturated or unsaturated heterocycle, wherein said heterocycle contains 1-2 hetero atoms selected from N, O and S;
$X_3$ is selected from $CR_5$ and N;
$X_4$ is optionally substituted $-(CH_2)_n-$, -C(=O)- and $NR_6$, wherein, n is 1, 2 or 3;
$X_5$ is selected from N and $CR_5$;
$L_1$ is selected from a bond, optionally substituted C1-C6 alkylene, optionally substituted C1-C6 alkylene-O-, optionally substituted C1-C6 alkylene-NH-, optionally substituted C1-C6 alkylene-S- and optionally substituted C2-C4 alkenylene;
$L_2$ is selected from a bond, optionally substituted C1-C6 alkylene, optionally substituted C1-C6 alkylene-O-, optionally substituted C1-C6 alkylene-NH-, optionally substituted C1-C6 alkylene-S- and optionally substituted C2-C4 alkenylene;
$R_0$ is selected from a bond, H, C1-C6 alkyl, optionally substituted C1-C6 alkoxy, optionally substituted C1-C6 haloalkyl, $-C(O)NR_3R_4$, $-CH_2OR_3$ and C1-C6 alkyl substituted with $-NR_3R_4$;
$R_1$ is selected from optionally substituted cycloalkyl, optionally substituted aryl, optionally substituted heteroaryl, optionally substituted heterocyclyl, optionally substituted amino and guanidyl;
$R_2$ is selected from optionally substituted cycloalkyl, optionally substituted aryl, optionally substituted heteroaryl and optionally substituted heterocyclyl;
$R_3$ and $R_4$ are each independently selected from H, optionally substituted C1-C6 alkyl, optionally substituted C1-C6 alkoxy, optionally substituted C1-C6 haloalkyl, optionally substituted 4-10 membered heterocyclyl and optionally substituted 5-10 membered heteroaryl; or $R_3$ and $R_4$ form an optionally substituted 4-10 membered heterocyclyl or 5-10 membered heteroaryl with the nitrogen atom to which they are attached;
$R_5$ is selected from H, halogen, C1-C6 alkyl, halogenated C1-C6 alkyl and C1-C6 alkoxy;
$R_6$ is selected from H, C1-C6 alkyl, halogenated C1-C6 alkyl and C1-C6 alkoxy.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is C3-C8 cycloalkyl or 3-7 membered heterocyclyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is selected from cyclopropyl, cyclobutyl, cyclopentyl, cyclohexyl, aziridinyl, azetidinyl, pyrrolidinyl, piperidinyl, piperazinyl, oxiranyl, oxetanyl, tetrahydrofuranyl, tetrahydropyranyl and morpholinyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is substituted with one, two or three substituents selected from halogen, hydroxyl, cyano, C1-C6 alkyl, halogenated C1-C6 alkyl, C1-C6 alkyl substituted with hydroxyl, C1-C6 alkoxy, halogenated C1-C6 alkoxy, $-NR_aR_b$, carbonyl substituted with $-NR_aR_b$, C1-C6 alkyl substituted with $-NR_aR_b$, C1-C6 alkylcarbonyl, C1-C6 alkoxycarbonyl, optionally substituted 6-14 membered aryl, optionally substituted 5-10 membered heteroaryl, optionally substituted 4-10 membered heterocyclyl, optionally substituted C3-C8 cycloalkyl, C1-C6 alkyl substituted with optionally substituted 6-14 membered aryl, C1-C6 alkyl substituted with optionally substituted 5-10 membered heteroaryl, C1-C6 alkyl substituted with optionally substituted 4-10 membered heterocyclyl, and C1-C6 alkyl substituted with optionally substituted C3-C8 cycloalkyl; wherein when said 6-14 membered aryl, 5-10 membered heteroaryl, 4-10 membered heterocyclyl and C3-C8 cycloalkyl is substituted, the substituent is 1, 2 or 3 substituents selected from halogen, C1-C6 alkyl, halogenated C1-C6 alkyl, C1-C6 alkoxy, halogenated C1-C6 alkoxy, $-NR_aR_b$, carbonyl substituted with $-NR_aR_b$, C1-C6 alkyl substituted with $-NR_aR_b$, C1-C6 alkylcarbonyl, C1-C6 alkoxycarbonyl; and wherein said $R_a$ and $R_b$ are each independently selected from Hand C1-C6 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is cyclopropyl, cyclobutyl, cyclopentyl, azetidinyl, oxetanyl, pyrrolidinyl or tetrahydrofuranyl,

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is optionally substituted with 1, 2 or 3 substituents selected from F, Cl, Br, $CF_3$, $CH_3$, -OH, $-OCH_3$, $-NH_2$, $-NHCH_3$ and - $NH_2(CH_3)_2$.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_0$ is C1-C6 alkyl substituted with $-NR_3R_4$; wherein $R_3$ and $R_4$ are each independently selected from H, C1-C6 alkyl optionally substituted with 1, 2, 3, 4, 5 or 6 substituents selected from halogen, cyano, C1-C6 alkoxy, halogenated C1-C6 alkoxy, $-NR_aR_b$ and hydroxyl, or $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

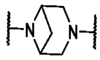 or 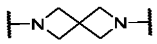

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

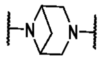 or 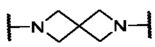

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, sulfo, C1-C6 alkylsulfonyl, C1-C6 alkoxysulfonyl, C1-C6 alkylcarbonyl, hydroxyl and halogenated C1-C6 alkylcarbonyl, wherein $R_a$ and $R_b$ are each independently H or C1-C4 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_0$ is C1-C6 alkyl substituted with $-NR_3R_4$, and wherein $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

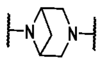 or 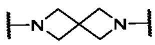

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

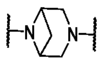 or 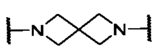

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, sulfo, C1-C6 alkylsulfonyl, C1-C6 alkoxysulfonyl, C1-C6 alkylcarbonyl, halogenated C1-C6 alkylcarbonyl, C1-C6 alkoxycarbonyl, halogenated C1-C6 alkoxycarbonyl, $-NR_aR_b$ and hydroxyl, wherein $R_a$ and $R_b$ are each independently H or C1-C4 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_0$ is C1-C6 alkyl substituted with $-NR_3R_4$, wherein $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl,

indolyl, isoindolyl, indazolyl, benzopyrrolyl, benzimidazolyl, benzotriazolyl, benzoxazolyl, benzisoxazolyl, quinolyl,

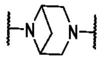 or 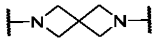

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

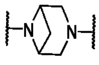 or 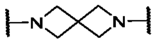

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, $-NR_aR_b$ and hydroxyl; wherein $R_a$ and $R_b$ are each independently H or C1-C4 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $X_3$ and/or $X_5$ is $CR_5$.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_5$ is selected from H and C1-C4 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $X_4$ is $-(CH_2)_n-$, n is 1 or 2; and wherein said $-(CH_2)_n-$ is optionally substituted with 1 or 2 substituents selected from halogen, hydroxyl, amino, C1-C6 alkyl and halogenated C1-C6 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $L_1$ is selected from C1-C6 alkylene, C1-C6 alkylene-O-, C1-C6 alkylene-NH-, C1-C6 alkylene-S- and C2-C4 alkenylene, wherein said C1-C6 alkylene, C1-C6 alkylene-O-, C1-C6 alkylene-NH-, C1-C6 alkylene-S- and C2-C4 alkenylene are optionally substituted with 1, 2 or 3 substituents selected from halogen, C1-C4 alkyl and hydroxyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $L_1$ is selected from C1-C6 alkylene-NH- or C1-C6 alkylene optionally substituted with 1, 2 or 3 substituents selected from halogen, C1-C4 alkyl and hydroxyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_1$ is selected from optionally substituted C3-C8 cycloalkyl, optionally substituted 6-14 membered aryl, optionally substituted 5-10 membered heteroaryl and optionally substituted 4-10 membered heterocyclyl, and said cycloalkyl, aryl, heteroaryl and heterocyclyl are each optionally substituted with 1, 2 or 3 substituents selected from optionally substituted amino, C1-C6 alkyl, halogen, hydroxyl, C1-C6 alkoxy, C1-C6 haloalkyl and cyano; wherein said optionally substituted amino is $-NR_aR_b$, wherein $R_a$ and $R_b$ are each independently H or C1-C6 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_1$ is selected from optionally substituted phenyl, naphthyl, pyridyl, pyrrolyl, pyrazolyl, imidazolyl, triazolyl, pyrimidinyl, pyrazinyl, triazinyl, indolyl, isoindolyl, indazolyl, benzimidazolyl, imidazopyridinyl, benzotriazolyl, benzoxazolyl, benzisoxazolyl, quinolyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, decahydroisoquinolyl, imidazolinyl, imidazolidinyl, quinazinyl, thiazolidinyl, isothiazolidinyl, isoxazolidinyl, dihydroindolyl, octahydroindolyl, octahydroisoindolyl, pyrrolidinyl, pyrazolindinyl, phthalimidinyl,

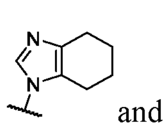 and 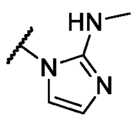.

In particular, in the compound of Formula I according to the present disclosure, or a pharmaceutically acceptable

salt thereof, or an enantiomer, a diastereoisomer, a tautomer, isotope substitute, solvate, a polymorph, a prodrug or metabolite thereof, said $L_1$ is C1-C4 alkylene or C1-C4 alkylene-NH-, $R_1$ is pyridyl, pyrrolyl, pyrazolyl, imidazolyl, triazolyl, pyrimidinyl, pyrazinyl, triazinyl or

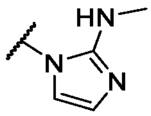,

said pyridyl, pyrrolyl, pyrazolyl, imidazolyl, triazolyl, pyrimidinyl, pyrazinyl, triazinyl or

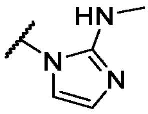

is optionally substituted with 1 or 2 substituents selected from halogen, C1-C4 alkyl and $-NR_aR_b$, wherein $R_a$ and $R_b$ are each independently H or C1-C4 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $L_2$ is selected from a bond and C1-C6 alkylene, wherein said C1-C6 alkylene is optionally substituted with 1, 2 or 3 substituents selected from halogen, C1-C3 alkyl, C3-C5 cycloalkyl and hydroxyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_2$ is optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano; wherein $R_a$ and $R_b$ are each independently H or C1-C6 alkyl; and wherein said heteroaryl is preferably a heteroaryl containing 1 or 2 nitrogen ring atoms, preferably selected from pyrazolyl, imidazolyl, pyridyl, pyrazinyl and piperazinyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_2$ is selected from 6-14 membered aryl, 5-10 membered heteroaryl and 4-10 membered heterocyclyl, wherein said 6-14 membered aryl, 5-10 membered heteroaryl and 4-10 membered heterocyclyl are optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_2$ is selected from phenyl, naphthyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl, azetidinyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, benzodihydrofuranyl, decahydroisoquinolyl, imidazolinyl, imidazolidinyl, 6,7-dihydro-5H-cyclopenta[c]pyridine, quinazinyl, thiazolidinyl, isothiazolidinyl, isoxazolidinyl, dihydroindolyl, octahydroindolyl, octahydroisoindolyl, pyrrolidinyl, pyrazolindinyl,

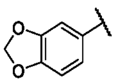 and 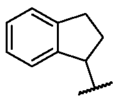,

wherein said phenyl, naphthyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl, azetidinyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, benzodihydrofuranyl, decahydroisoquinolyl, imidazolinyl, imidazolidinyl, 6,7-dihydro-5H-cyclopenta[c]pyridine, quinazinyl, thiazolidinyl, isothiazolidinyl, isoxazolidinyl, dihydroindolyl, octahydroindolyl, octahydroisoindolyl, pyrrolidinyl, pyrazolindinyl,

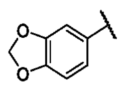

and

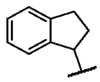

are optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $L_2$ is C1-C4 alkylene, $R_2$ is 6-14 membered aryl, 5-10 membered nitrogen-containing heteroaryl or 4-10 membered heterocyclyl substituted with 1 or 2 substituents selected from C1-C6 alkoxy, halogen, C1-C6 alkyl, cyano, C3-C8 cycloalkyl-O- and halogenated C1-C6 alkoxy, preferably phenyl, pyridyl, pyrazolyl, pyrrolopyridyl, dihydrofuropyridinyl, pyrido 1,3-dioxolyl or indolyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is C3-C6 cycloalkyl or 4-8 membered heterocyclyl, wherein said cycloalkyl or heterocyclyl is optionally substituted with 1, 2 or 3 substituents selected from halogen, C1-C4 alkyl, halogenated C1-C4 alkyl, C1-C4 alkoxy, halogenated C1-C4 alkoxy;

$X_3$ and $X_5$ are CH;
$X_4$ is $CH_2$;
$L_1$ is C1-C4 alkylene;
$R_1$ is a 5 or 6 membered nitrogen-containing heteroaryl optionally substituted with 1 or 2 substituents selected from halogen, C1-C4 alkyl and $-NR_aR_b$, wherein said 5 or 6 membered nitrogen-containing heteroaryl is preferably pyridyl, pyrrolyl, pyrazolyl, imidazolyl, triazolyl, pyrimidinyl, pyrazinyl, triazinyl or

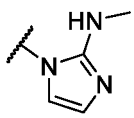
;

wherein $R_a$ and $R_b$ are each independently H or C1-C4 alkyl;
$L_2$ is C1-C4 alkylene;
$R_2$ is phenyl, 5-10 membered nitrogen-containing heteroaryl or 4-10 membered heterocyclyl, preferably pyridyl, pyrimidinyl, pyrazolyl, pyrrolyl, imidazolyl, pyrazinyl, pyrrolopyridyl, dihydrofuropyridinyl, pyrido 1,3-dioxolyl or indolyl, wherein said phenyl, 5-10 membered nitrogen-containing heteroaryl and 4-10 membered heterocyclyl are each optionally substituted with 1 or 2 substituents selected from C1-C6 alkoxy, halogen, C1-C6 alkyl, cyano, C3-C8 cycloalkyl-O- and halogenated C1-C6 alkoxy;
$R_0$ is C1-C4 alkyl substituted with $-NR_3R_4$; wherein $R_3$ and $R_4$ are each independently selected from H, C1-C6 alkyl optionally substituted with 1-6 substituents selected from hydroxyl, cyano and halogen, and 4-8 membered heterocyclyl optionally substituted with 1, 2 or 3 substituents selected from halogen, C1-C6 alkyl, halogenated C1-C6 alkyl, C1-C6 alkoxy, halogenated C1-C6 alkoxy, $-NR_aR_b$ and hydroxyl, wherein $R_a$ and $R_b$ are each independently H or C1-C4 alkyl; or $R_3$ and $R_4$ form a 4-8 membered heterocyclyl optionally substituted with 1, 2 or 3 substituents selected from halogen, C1-C6 alkyl, C3-C6 cycloalkyl, halogenated C1-C6 alkyl, C1-C6 alkoxy, halogenated C1-C6 alkoxy, $-NR_aR_b$ and hydroxyl with the nitrogen atom to which they are attached, wherein $R_a$ and $R_b$ are each independently H or C1-C4 alkyl; preferably, said 4-8 membered heterocyclyl is each independently selected from oxetanyl, azetidinyl, pyrrolidinyl, tetrahydrofuranyl, piperidinyl, piperazinyl, tetrahydropyranyl, morpholinyl,

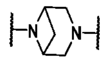

or

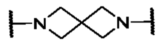.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is C3-C6 cycloalkyl, wherein said cycloalkyl is optionally substituted with 1, 2 or 3 substituents selected from halogen, C1-C4 alkyl and halogenated C1-C4 alkyl;

$X_3$ and $X_5$ are both CH;
$X_4$ is $CH_2$;
$L_1$ is C1-C4 alkylene;
$R_1$ is 5 or 6 membered nitrogen-containing heteroaryl substituted with 1 or 2 substituents selected from C1-C4 alkyl and $-NR_aR_b$, preferably pyridyl, pyrrolyl, pyrazolyl, imidazolyl, triazolyl, pyrimidinyl, pyrazinyl, triazinyl, or

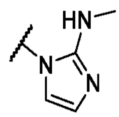;

wherein $R_a$ and $R_b$ are each independently H or C1-C4 alkyl;
$L_2$ is C1-C4 alkylene;
$R_2$ is a 5-10 membered nitrogen-containing heteroaryl or a 4-10 membered heterocyclyl, preferably pyridyl, pyrimidinyl, pyrazolyl, pyrrolyl, imidazolyl, pyrazinyl, pyrrolopyridyl, dihydrofuropyridinyl, pyrido 1,3-dioxolyl or indolyl, wherein said 5-10 membered nitrogen-containing heteroaryl or 4-10 membered heterocyclyl is each optionally substituted with 1 or 2 substituents selected from C1-C6 alkoxy, C1-C6 alkyl, C3-C8 cycloalkyl-O-, halogenated C1-C6 alkoxy, halogen and cyano;
$R_0$ is C1-C4 alkyl substituted with $-NR_3R_4$, wherein $R_3$ and $R_4$ are each independently selected from Hand C1-C6 alkyl optionally substituted with 1, 2, 3, 4, 5 or 6 substituents selected from hydroxyl, cyano and halogen, or $R_3$ and $R_4$ form a 4-8 membered heterocyclyl optionally substituted with 1, 2 or 3 substituents selected from halogen, hydroxyl, C1-C4 alkoxy, C3-C6 alkoxy and C1-C4 alkyl with the nitrogen atom to which they are attached, preferably, said 4-8 membered heterocyclyl is selected from azetidinyl, pyrrolidinyl, piperidinyl, piperazinyl, morpholinyl,

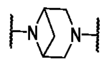 or 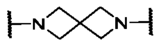.

In one aspect of the present disclosure, a compound of Formula II, or a pharmaceutically acceptable salt thereof, or an enantiomer, a diastereoisomer, a tautomer, an isotope substitute, a solvate, a polymorph, a prodrug or metabolite thereof is provided:

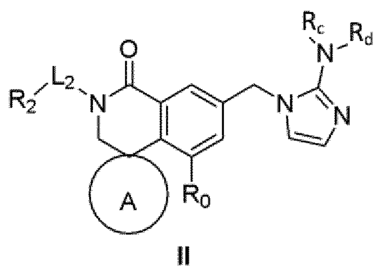

II

wherein

ring A is a substituted or unsubstituted 3-8 membered saturated or unsaturated carboncycle or a substituted or unsubstituted 3-8 membered saturated or unsaturated heterocycle, wherein said heterocycle contains 1-2 hetero atoms selected from N, O and S;
$L_2$ is selected from a bond, optionally substituted C1-C6 alkylene, optionally substituted C1-C6 alkylene-O-, optionally substituted C1-C6 alkylene-NH-, optionally substituted C1-C6 alkylene-S- and optionally substituted C2-C4 alkenylene;

$R_0$ is selected from H, C1-C6 alkyl, optionally substituted C1-C6 alkoxy, optionally substituted C1-C6 haloalkyl, $-C(O)NR_3R_4$, $-CH_2OR_3$, and C1-C6 alkyl substituted with $-NR_3R_4$,;

$R_2$ is selected from optionally substituted cycloalkyl, optionally substituted aryl, optionally substituted heteroaryl and optionally substituted heterocyclyl;

$R_3$ and $R_4$ are each independently selected from H, optionally substituted C1-C6 alkyl, optionally substituted C1-C6 alkoxy, optionally substituted C1-C6 haloalkyl, optionally substituted 4-10 membered heterocyclyl and optionally substituted 5-10 membered heteroaryl; or $R_3$ and $R_4$ form an optionally substituted 4-10 membered heterocyclyl or a 5-10 membered heteroaryl with the nitrogen atom to which they are attached; and

$R_c$ and $R_d$ are each independently H or C1-C4 alkyl.

In particular, in the compound of Formula II according to the present disclosure, or a pharmaceutically acceptable salt thereof, or an enantiomer, a diastereoisomer, a tautomer, an isotope substitute, a solvate, a polymorph, a prodrug or metabolite thereof, ring A is C3-C8 cycloalkyl or 3-7 membered heterocyclyl.

In particular, in the compound of Formula II according to the present disclosure, or a pharmaceutically acceptable salt thereof, or an enantiomer, a diastereoisomer, a tautomer, an isotope substitute, a solvate, a polymorph, a prodrug or metabolite thereof, ring A is selected from cyclopropyl, cyclobutyl, cyclopentyl, cyclohexyl, aziridinyl, azetidinyl, pyrrolidinyl, piperidinyl, piperazinyl, oxiranyl, oxetanyl, tetrahydrofuranyl, tetrahydropyranyl and morpholinyl.

In particular, in the compound of Formula II according to the present disclosure, or a pharmaceutically acceptable salt thereof, or an enantiomer, a diastereoisomer, a tautomer, an isotope substitute, a solvate, a polymorph, a prodrug or metabolite thereof, ring A is substituted with 1, 2 or 3 substituents selected from halogen, hydroxyl, cyano, C1-C6 alkyl, halogenated C1-C6 alkyl, hydroxyl-substituted C1-C6 alkyl, C1-C6 alkoxy, halogenated C1-C6 alkoxy, $-NR_aR_b$, C1-C6 alkylcarbonyl and C1-C6 alkoxycarbony, said $R_a$ and $R_b$ are each independently selected from Hand C1-C6 alkyl.

In particular, in the compound of Formula II according to the present disclosure, or a pharmaceutically acceptable salt thereof, or an enantiomer, a diastereoisomer, a tautomer, an isotope substitute, a solvate, a polymorph, a prodrug or metabolite thereof, ring A is cyclopropyl, cyclobutyl, cyclopentyl, cyclohexyl, azetidinyl, oxetanyl, pyrrolidinyl, tetrahydrofuranyl or tetrahydropyranyl.

In particular, in the compound of Formula II according to the present disclosure, or a pharmaceutically acceptable salt thereof, or an enantiomer, a diastereoisomer, a tautomer, an isotope substitute, a solvate, a polymorph, a prodrug or metabolite thereof, ring A is optionally substituted with 1, 2 or 3 substituents selected from F, Cl, Br, $CF_3$, $CH_3$, -OH, $-OCH_3$, $-NH_2$, - $NHCH_3$, and $-NH_2(CH_3)_2$.

In particular, in the compound of Formula II according to the present disclosure, or a pharmaceutically acceptable salt thereof, or an enantiomer, a diastereoisomer, a tautomer, an isotope substitute, a solvate, a polymorph, a prodrug or metabolite thereof, $R_0$ is C1-C6 alkyl substituted with $-NR_3R_4$; wherein $R_3$ and $R_4$ are each independently selected from H, C1-C6 alkyl optionally substituted with 1, 2, 3, 4, 5 or 6 substituents selected from halogen, cyano, C1-C6 alkoxy, halogenated C1-C6 alkoxy, $-NR_aR_b$ and hydroxyl, or $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

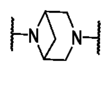

or

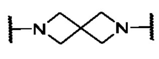

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

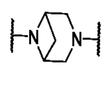

or

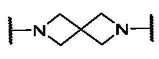

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, sulfo, C1-C6 alkylsulfonyl, C1-C6 alkoxysulfonyl, C1-C6 alkylcarbonyl, halogenated C1-C6 alkylcarbonyl, C1-C6 alkoxycarbonyl, halogenated C1-C6 alkoxycarbonyl, $-NR_aR_b$ and hydroxyl, wherein $R_a$ and $R_b$ are each independently H or C1-C4 alkyl.

In particular, in the compound of Formula II according to the present disclosure, or a pharmaceutically acceptable salt thereof, or an enantiomer, a diastereoisomer, a tautomer, an isotope substitute, a solvate, a polymorph, a prodrug or metabolite thereof, $R_0$ is C1-C6 alkyl substituted with $-NR_3R_4$, and wherein $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

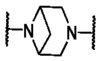 or 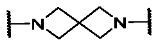

with the nitrogen atom to which they are attached, wherein these groups are each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, sulfo, C1-C6 alkylsulfonyl, C1-C6 alkoxysulfonyl, C1-C6 alkylcarbonyl, halogenated C1-C6 alkylcarbonyl, C1-C6 alkoxycarbonyl, halogenated C1-C6 alkoxycarbonyl, $-NR_aR_b$ and hydroxyl; wherein $R_a$ and $R_b$ are each independently H or C1-C4 alkyl.

In particular, in the compound of Formula II according to the present disclosure, or a pharmaceutically acceptable salt thereof, or an enantiomer, a diastereoisomer, a tautomer, an isotope substitute, a solvate, a polymorph, a prodrug or metabolite thereof, $R_0$ is C1-C6 alkyl substituted with $-NR_3R_4$, wherein $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

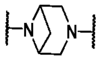 or 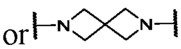

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

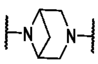 or 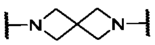

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, $-NR_aR_b$ and hydroxyl; wherein $R_a$ and $R_b$ are each independently H or C1-C4 alkyl.

In particular, in the compound of Formula II according to the present disclosure, or a pharmaceutically acceptable salt thereof, or an enantiomer, a diastereoisomer, a tautomer, an isotope substitute, a solvate, a polymorph, a prodrug or metabolite thereof, $R_c$ and $R_d$ are each independently H or methyl.

In particular, in the compound of Formula II according to the present disclosure, or a pharmaceutically acceptable salt thereof, or an enantiomer, a diastereoisomer, a tautomer, an isotope substitute, a solvate, a polymorph, a prodrug or metabolite thereof, $L_2$ is selected from a bond and C1-C6 alkylene, wherein said C1-C6 alkylene is optionally substituted with 1, 2 or 3 substituents selected from halogen, C1-C3 alkyl, C3-C5 cycloalkyl and hydroxyl.

In particular, in the compound of Formula II according to the present disclosure, or a pharmaceutically acceptable salt thereof, or an enantiomer, a diastereoisomer, a tautomer, an isotope substitute, a solvate, a polymorph, a prodrug or metabolite thereof, $R_2$ is optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano; wherein $R_a$ and $R_b$ are each independently H or C1-C6 alkyl; and wherein said heteroaryl is preferably a heteroaryl containing 1 or 2 nitrogen ring atoms, preferably selected from pyrazolyl, imidazolyl, pyridyl, pyrazinyl and piperazinyl.

In particular, in the compound of Formula II according to the present disclosure, or a pharmaceutically acceptable salt thereof, or an enantiomer, a diastereoisomer, a tautomer, an isotope substitute, a solvate, a polymorph, a prodrug or metabolite thereof, $R_2$ is selected from 6-14 membered aryl, 5-10 membered heteroaryl and 4-10 membered heterocyclyl, wherein said 6-14 membered aryl, 5-10 membered heteroaryl and 4-10 membered heterocyclyl may be optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano.

In particular, in the compound of Formula II according to the present disclosure, or a pharmaceutically acceptable salt thereof, or an enantiomer, a diastereoisomer, a tautomer, an isotope substitute, a solvate, a polymorph, a prodrug or metabolite thereof, $R_2$ is selected from phenyl, naphthyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl, azetidinyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, benzodihydrofuranyl, decahydroisoquinolyl, imidazolinyl, imidazolidinyl, 6,7-dihydro-5H-cyclopenta[c]pyridine, quinazinyl, thiazolidinyl, isothiazolidinyl, isoxazolidinyl, dihydroindolyl, octahydroindolyl, octahydroisoindolyl, pyrrolidinyl, pyrazolindinyl,

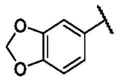 and 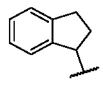,

wherein said phenyl, naphthyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl, azetidinyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, benzodihydrofuranyl, decahydroisoquinolyl, imidazolinyl, imidazolidinyl, 6,7-dihydro-5H-cyclopenta[c]pyridine, quinazinyl, thiazolidinyl, isothiazolidinyl, isoxazolidinyl, dihydroindolyl, octahydroindolyl, octahydroisoindolyl, pyrrolidinyl, pyrazolindinyl,

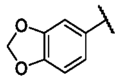 and 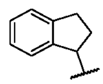

are optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano.

In particular, in the compound of Formula II according to the present disclosure, or a pharmaceutically acceptable salt thereof, or an enantiomer, a diastereoisomer, a tautomer, an isotope substitute, a solvate, a polymorph, a prodrug or metabolite thereof, $L_2$ is C1-C4 alkylene, $R_2$ is phenyl, pyridyl, pyrazolyl, pyrrolopyridyl, dihydrofuropyridinyl, pyrido 1,3-dioxolyl or indolyl, wherein said phenyl, pyridyl, pyrazolyl, pyrrolopyridyl, dihydrofuropyridinyl, pyrido 1,3-dioxolyl or indolyl is optionally substituted with 1 or 2 substituents selected from C1-C6 alkoxy, halogen, C1-C6 alkyl, cyano, C3-C8 cycloalkyl-O- and halogenated C1-C6 alkoxy.

In particular, in the compound of Formula II according to the present disclosure, or a pharmaceutically acceptable salt thereof, or an enantiomer, a diastereoisomer, a tautomer, an isotope substitute, a solvate, a polymorph, a prodrug or metabolite thereof, ring A is C3-C6 cycloalkyl optionally substituted with substituents selected from F, Cl, hydroxyl or C1-C4 alkyl or C4-C6 heterocyclyl containing one nitrogen or one O atom, preferably cyclopropyl, cyclobutyl, oxetanyl or azetidinyl optionally substituted with F or C1-C4 alkyl;

$R_0$ is C1-C4 alkyl substituted with H or $-NR_3R_4$, wherein $R_3$ and $R_4$ are each independently selected from H, 4-6 membered heterocyclyl and C1-C6 alkyl optionally substituted with 1, 2, 3, 4, 5 or 6 substituents selected from hydroxyl, cyano and halogen, or $R_3$ and $R_4$ form 4-6 membered heterocyclyl optionally substituted with 1, 2 or 3 substituents selected from halogen, hydroxyl, C1-C4 alkoxy, C3-C6 cycloalkyl and C1-C4 alkyl with the nitrogen atom to which they are attached; wherein said 4-6 membered heterocyclyl is preferably an oxygen and/or nitrogen-containing heterocyclyl, preferably selected from the group consisting of oxetanyl, azetidinyl, pyrrolidinyl, tetrahydrofuranyl, piperidinyl, piperazinyl, 2-oxopiperazinyl, tetrahydropyranyl, morpholinyl,

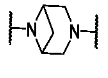 or 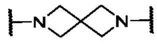;

$L_2$ is C1-C4 alkylene;

$R_2$ is a 6-14 membered aryl, a 5-10 membered nitrogen-containing heteroaryl or a 4-10 membered heterocyclyl, preferably phenyl, pyridyl, pyrimidinyl, pyrazolyl, pyrrolyl, imidazolyl, pyrazinyl, indolyl, pyrrolopyridyl, dihydrofuropyridinyl, pyrido 1,3-dioxolyl,

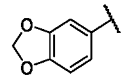

or

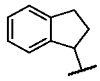,

wherein said 6-14 membered aryl, 5-10 membered nitrogen-containing heteroaryl or 4-10 membered heterocyclyl is each optionally substituted with 1 or 2 substituents selected from C1-C6 alkoxy, C1-C4 alkyl, halogen, C3-C6 cycloalkyl-oxy, halogenated C1-C4 alkoxy and cyano;
$R_c$ and $R_d$ are each independently H or C1-C4 alkyl.

In particular, in the compound of Formula II according to the present disclosure, or a pharmaceutically acceptable salt thereof, or an enantiomer, a diastereoisomer, a tautomer, an isotope substitute, a solvate, a polymorph, a prodrug or metabolite thereof, ring A is C3-C6 cycloalkyl or a 4-8 membered heterocyclyl, wherein said cycloalkyl or heterocyclyl is optionally substituted with 1, 2 or 3 substituents selected from halogen, C1-C4 alkyl, halogenated C1-C4 alkyl, C1-C4 alkoxy, halogenated C1-C4 alkoxy;

$L_2$ is C1-C4 alkylene;
$R_2$ is phenyl, 5-10 membered nitrogen-containing heteroaryl or a 4-10 membered heterocyclyl, preferably pyridyl, pyrimidinyl, pyrazolyl, pyrrolyl, imidazolyl, pyrazinyl, pyrrolopyridyl, dihydrofuropyridinyl or pyrido 1,3-dioxolyl or indolyl, wherein said phenyl, 5-10 membered nitrogen-containing heteroaryl and 4-10 membered heterocyclyl are each optionally substituted with 1 or 2 substituents selected from C1-C6 alkoxy, halogen, C1-C6 alkyl, cyano, C3-C8 cycloalkyl-O- and halogenated C1-C6 alkoxy;
$R_0$ is C1-C4 alkyl substituted with $-NR_3R_4$; wherein $R_3$ and $R_4$ are each independently selected from H, C1-C6 alkyl optionally substituted with 1-6 substituents selected from hydroxyl, cyano and halogen, and a 4-8 membered heterocyclyl optionally substituted with 1, 2 or 3 substituents selected from halogen, C1-C6 alkyl, halogenated C1-C6 alkyl, C1-C6 alkoxy, halogenated C1-C6 alkoxy, $-NR_aR_b$ and hydroxyl, wherein $R_a$ and $R_b$ are each independently H or C1-C4 alkyl; or $R_3$ and $R_4$ form a 4-8 membered heterocyclyl optionally substituted with 1, 2 or 3 substituents selected from halogen, C1-C6 alkyl, C3-C6 cycloalkyl, halogenated C1-C6 alkyl, C1-C6 alkoxy, halogenated C1-C6 alkoxy, $-NR_aR_b$ and hydroxyl with the nitrogen atom to which they are attached, wherein $R_a$ and $R_b$ are each independently H or C1-C4 alkyl; preferably, said 4-8 membered heterocyclyl is each independently selected from the group consisting of oxetanyl, azetidinyl, pyrrolidinyl, tetrahydrofuranyl, piperidinyl, piperazinyl, 2-oxopiperazinyl, tetrahydropyranyl, morpholinyl,

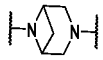 or 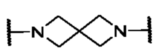;

and
$R_c$ and $R_d$ are each independently H or C1-C4 alkyl.

In particular, in the compound of Formula II according to the present disclosure, or a pharmaceutically acceptable salt thereof, or an enantiomer, a diastereoisomer, a tautomer, an isotope substitute, a solvate, a polymorph, a prodrug or metabolite thereof, ring A is C3-C6 cycloalkyl, wherein said cycloalkyl is optionally substituted with 1, 2 or 3 substituents selected from halogen, C1-C4 alkyl and halogenated C1-C4 alkyl;

$L_2$ is C1-C4 alkylene;
$R_2$ is 5-10 membered nitrogen-containing heteroaryl or a 4-10 membered heterocyclyl, preferably pyridyl, pyrimidinyl, pyrazolyl, pyrrolyl, imidazolyl, pyrazinyl, pyrrolopyridyl, dihydrofuropyridinyl or pyrido 1,3-dioxolyl or indolyl, wherein said 5-10 membered nitrogen-containing heteroaryl or 4-10 membered heterocyclyl is each optionally substituted with 1 or 2 substituents selected from C1-C6 alkoxy, C1-C6 alkyl, C3-C8 cycloalkyl-O-, halogenated C1-C6 alkoxy, halo and cyano;

$R_0$ is C1-C4 alkyl substituted with $-NR_3R_4$, wherein $R_3$ and $R_4$ are each independently selected from Hand C1-C6 alkyl optionally substituted with 1, 2, 3, 4, 5 or 6 substituents selected from hydroxyl, cyano and halogen, or $R_3$ and $R_4$ form a 4-8 membered heterocyclyl optionally substituted with 1, 2 or 3 substituents selected from halogen, hydroxyl, C1-C4 alkoxy and C1-C4 alky with the nitrogen atom to which they are attached, preferably, said 4-8 membered heterocyclyl is selected from azetidinyl, pyrrolidinyl, piperidinyl, piperazinyl, 2-oxopiperazinyl, morpholinyl,

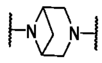 or 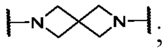;

and

$R_c$ and $R_d$ are each independently H or C1-C4 alkyl.

In another aspect according to the present disclosure, a compound of Formula III, or a pharmaceutically acceptable salt thereof, or an enantiomer, a diastereoisomer, a tautomer, an isotope substitute, a solvate, a polymorph, a prodrug or metabolite thereof is provided,

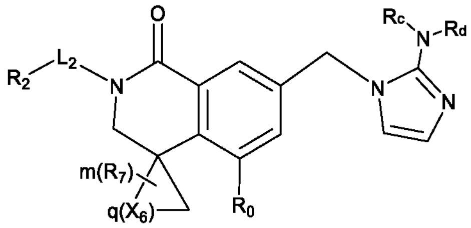

III

wherein

$L_2$ is selected from a bond, optionally substituted C1-C6 alkylene, optionally substituted C1-C6 alkylene-O-, optionally substituted C1-C6 alkylene-NH-, optionally substituted C1-C6 alkylene-S- and optionally substituted C2-C4 alkenylene;

$R_0$ is selected from H, C1-C6 alkyl, optionally substituted C1-C6 alkoxy, optionally substituted C1-C6 haloalkyl, $-C(O)NR_3R_4$, $-CH_2OR_3$, C1-C6 alkyl substituted with $-NR_3R_4$;

$R_2$ is selected from optionally substituted cycloalkyl, optionally substituted aryl, optionally substituted heteroaryl and optionally substituted heterocyclyl;

$R_3$ and $R_4$ are each independently selected from H, optionally substituted C1-C6 alkyl, optionally substituted C1-C6 alkoxy, optionally substituted C1-C6 haloalkyl, optionally substituted 4-10 membered heterocyclyl and optionally substituted 5-10 membered heteroaryl; or $R_3$ and $R_4$ form an optionally substituted 4-10 membered heterocyclyl or 5-10 membered heteroaryl with the nitrogen atom to which they are attached;

$X_6$ are each independently selected from the group consisting of C, O, N, S,

q is an integer of 1, 2 or 3,

$R_7$ is selected from F, Cl, cyano, hydroxyl, C1-C6 alkyl, fluorinated or chlorinated C1-C6 alkyl, hydroxyl-substituted C1-C6 alkyl, C1-C6 alkoxy, fluorinated or chlorinated C1-C6 alkoxy, hydroxyl-substituted C1-C6 alkyl and $-NR_aR_b$; wherein said $R_a$ and $R_b$ are each independently selected from H and C1-C6 alkyl,

m is an integer of 0, 1, 2 or 3; and

$R_c$ and $R_d$ are each independently H or C1-C4 alkyl.

In particular, in the compound of Formula III according to the present disclosure, or a pharmaceutically acceptable salt thereof, or an enantiomer, a diastereoisomer, a tautomer, an isotope substitute, a solvate, a polymorph, a prodrug or metabolite thereof, $R_0$ is C1-C6 alkyl substituted with $-NR_3R_4$; wherein $R_3$ and $R_4$ are each independently selected from the group consisting of H, C1-C6 alkyl optionally substituted with 1, 2, 3, 4, 5 or 6 substituents selected from halogen, cyano, C1-C6 alkoxy, halogenated C1-C6 alkoxy, $-NR_aR_b$ and hydroxyl, or $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

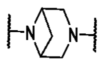 or 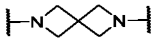

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

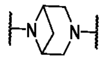

or

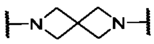

is each optionally substituted with 1, 2 or 3 substituents selected from halogen, cyano, C1-C6 alkyl, halogenated C1-C6 alkyl, C3-C6 cycloalkyl, C1-C6 alkoxy, halogenated C1-C6 alkoxy, sulfo, C1-C6 alkylsulfonyl, C1-C6 alkoxysulfonyl, C1-C6 alkylcarbonyl, halogenated C1-C6 alkylcarbonyl, C1-C6 alkoxycarbonyl, halogenated C1-C6 alkoxycarbonyl, $-NR_aR_b$ and hydroxyl, wherein $R_a$ and $R_b$ are each independently H or C1-C4 alkyl.

In particular, in the compound of Formula III according to the present disclosure, or a pharmaceutically acceptable salt thereof, or an enantiomer, a diastereoisomer, a tautomer, an isotope substitute, a solvate, a polymorph, a prodrug or metabolite thereof, $R_0$ is C1-C6 alkyl substituted with $-NR_3R_4$, and wherein $R_3$ and $R_4$ form azetidinyl, pyrrolidinyl, piperidinyl, piperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

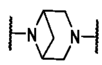 or 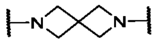

with the nitrogen atom to which they are attached, wherein said azetidinyl, pyrrolidinyl, piperidinyl, piperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

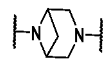 or 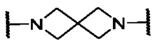

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, sulfo, C1-C6 alkylsulfonyl, C1-C6 alkoxysulfonyl, C1-C6 alkylcarbonyl, halogenated C1-C6 alkylcarbonyl, C1-C6 alkoxycarbonyl, halogenated C1-C6 alkoxycarbonyl, $-NR_aR_b$ and hydroxyl; wherein $R_a$ and $R_b$ are each independently H or C1-C4 alkyl.

In particular, in the compound of Formula III according to the present disclosure, or a pharmaceutically acceptable salt thereof, or an enantiomer, a diastereoisomer, a tautomer, an isotope substitute, a solvate, a polymorph, a prodrug or metabolite thereof, $R_0$ is C1-C6 alkyl substituted with $-NR_3R_4$, wherein $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

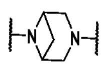 or 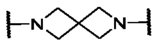

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

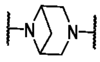 or 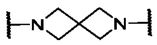

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, $-NR_aR_b$ and hydroxyl; wherein $R_a$ and $R_b$ are each independently H or C1-C4 alkyl.

In particular, in the compound of Formula III according to the present disclosure, or a pharmaceutically acceptable salt thereof, or an enantiomer, a diastereoisomer, a tautomer, an isotope substitute, a solvate, a polymorph, a prodrug or metabolite thereof, $R_c$ and $R_d$ are each independently H or methyl.

In particular, in the compound of Formula III according to the present disclosure, or a pharmaceutically acceptable salt thereof, or an enantiomer, a diastereoisomer, a tautomer, an isotope substitute, a solvate, a polymorph, a prodrug or metabolite thereof, $L_2$ is selected from a bond and C1-C6 alkylene, wherein said C1-C6 alkylene is optionally substituted with 1, 2 or 3 substituents selected from halogen, C1-C3 alkyl, C3-C5 cycloalkyl and hydroxyl.

In particular, in the compound of Formula III according to the present disclosure, or a pharmaceutically acceptable salt thereof, or an enantiomer, a diastereoisomer, a tautomer, an isotope substitute, a solvate, a polymorph, a prodrug or metabolite thereof, $R_2$ is optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano; wherein $R_a$ and $R_b$ are each independently H or C1-C6 alkyl; and wherein said heteroaryl is preferably a heteroaryl containing 1 or 2 nitrogen ring atoms, preferably selected from pyrazolyl, imidazolyl, pyridyl, pyrazinyl and piperazinyl.

In particular, in the compound of Formula III according to the present disclosure, or a pharmaceutically acceptable salt thereof, or an enantiomer, a diastereoisomer, a tautomer, an isotope substitute, a solvate, a polymorph, a prodrug or metabolite thereof, $R_2$ is selected from 6-14 membered aryl, 5-10 membered heteroaryl and 4-10 membered heterocyclyl, wherein said 6-14 membered aryl, 5-10 membered heteroaryl and 4-10 membered heterocyclyl are optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano.

In particular, in the compound of Formula III according to the present disclosure, or a pharmaceutically acceptable salt thereof, or an enantiomer, a diastereoisomer, a tautomer, an isotope substitute, a solvate, a polymorph, a prodrug or metabolite thereof, $R_2$ is selected from phenyl, naphthyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl, azetidinyl, pyrrolidinyl, morpholinyl, piperazinyl, homopiperazinyl, piperidinyl, pyranyl, dihydrofuropyridinyl, pyrido 1,3-dioxolyl, 2-oxopiperazinyl, tetrahydropyranyl, tetrahydrofuranyl, oxazinyl, tetrahydroisoquinolyl, benzodihydrofuranyl, decahydroisoquinolyl, imidazolinyl, imidazolidinyl, 6,7-dihydro-5H-cyclopenta[c]pyridine, quinazinyl, thiazolidinyl, isothiazolidinyl, isoxazolidinyl, dihydroindolyl, octahydroindolyl, octahydroisoindolyl, pyrrolidinyl, pyrazolindinyl,

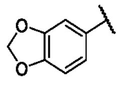 and 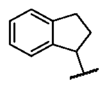,

wherein said phenyl, naphthyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl, azetidinyl, pyrrolidinyl, morpholinyl, piperazinyl, homopiperazinyl, piperidinyl, pyranyl, dihydrofuropyridinyl, pyrido 1,3-dioxolyl, 2-oxopiperazinyl, tetrahydropyranyl, tetrahydrofuranyl, oxazinyl, tetrahydroisoquinolyl, benzodihydrofuranyl, decahydroisoquinolyl, imidazolinyl, imidazolidinyl, 6,7-dihydro-5H-cyclopenta[c]pyridine, quinazinyl, thiazolidinyl, isothiazolidinyl, isoxazolidinyl, dihydroindolyl, octahydroindolyl, octahydroisoindolyl, pyrrolidinyl, pyrazolindinyl,

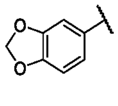 and 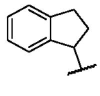

are optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano.

In particular, in the compound of Formula III according to the present disclosure, or a pharmaceutically acceptable salt thereof, or an enantiomer, a diastereoisomer, a tautomer, an isotope substitute, a solvate, a polymorph, a

prodrug or metabolite thereof, $L_2$ is C1-C4 alkylene, $R_2$ is 6-14 membered aryl, 5-10 membered nitrogen-containing heteroaryl or 4-10 membered heterocyclyl substituted with 1 or 2 substituents selected from C1-C6 alkoxy, halogen, C1-C6 alkyl, cyano, C3-C8 cycloalkyl-O- and halogenated C1-C6 alkoxy, preferably phenyl, pyridyl, pyrazolyl, pyrrolopyridyl, dihydrofuropyridinyl, pyrido 1,3-dioxolyl or indolyl.

In particular, in the compound of Formula III according to the present disclosure, or a pharmaceutically acceptable salt thereof, or an enantiomer, a diastereoisomer, a tautomer, an isotope substitute, a solvate, a polymorph, a prodrug or metabolite thereof, $R_0$ is H or C1-C4 alkyl substituted with $-NR_3R_4$, wherein $R_3$ and $R_4$ are each independently selected from H, 4-6 membered heterocyclyl and C1-C6 alkyl optionally substituted with 1, 2, 3, 4, 5 or 6 substituents selected from hydroxyl, cyano and halogen, or $R_3$ and $R_4$ form a 4-6 membered heterocyclyl optionally substituted with 1, 2 or 3 substituents selected from halogen, hydroxyl, C1-C4 alkoxy and C1-C4 alkyl with the nitrogen atom to which they are attached; wherein said 4-6 membered heterocyclyl is preferably an oxygen and/or nitrogen-containing heterocyclyl, preferably selected from oxetanyl, azetidinyl, pyrrolidinyl, tetrahydrofuranyl, piperidinyl, piperazinyl, 2-oxopiperazinyl, tetrahydropyranyl, morpholinyl,

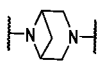 or 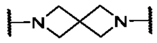;

$L_2$ is C1-C4 alkylene;
$R_2$ is 6-14 membered aryl, 5-10 membered nitrogen-containing heteroaryl or 4-10 membered heterocyclyl, preferably phenyl, pyridyl, pyrimidinyl, pyrazolyl, pyrrolyl, imidazolyl, pyrazinyl, indolyl, pyrrolopyridyl, dihydrofuropyridinyl or pyrido 1,3-dioxolyl, wherein said 6-14 membered aryl, 5-10 membered nitrogen-containing heteroaryl or 4-10 membered heterocyclyl is each optionally substituted with 1 or 2 substituents selected from C1-C6 alkoxy, C1-C4 alkyl, halogen, C3-C6 cycloalkyl-oxy, halogenated C1-C4 alkoxy and cyano;
$R_c$ and $R_d$ are each independently H or C1-C4 alkyl;
$X_6$ is C or O;
q is 1 or 2; and
$R_7$ is selected from F, Cl, cyano, hydroxyl, C1-C6 alkyl, fluorinated or chlorinated C1-C6 alkyl, hydroxyl-substituted C1-C6 alkyl, C1-C6 alkoxy, fluorinated or chlorinated C1-C6 alkoxy and hydroxyl-substituted C1-C6 alkyl.

In particular, in the compound of Formula III according to the present disclosure, or a pharmaceutically acceptable salt thereof, or an enantiomer, a diastereoisomer, a tautomer, an isotope substitute, a solvate, a polymorph, a prodrug or metabolite thereof, $L_2$ is C1-C4 alkylene;

$R_2$ is phenyl, 5-10 membered nitrogen-containing heteroaryl or 4-10 membered heterocyclyl, preferably pyridyl, pyrimidinyl, pyrazolyl, pyrrolyl, imidazolyl, pyrazinyl, pyrrolopyridyl, dihydrofuropyridinyl or pyrido 1,3-dioxolyl or indolyl, wherein said phenyl, 5-10 membered nitrogen-containing heteroaryl and 4-10 membered heterocyclyl are each optionally substituted with 1 or 2 substituents selected from C1-C6 alkoxy, halogen, C1-C6 alkyl, cyano, C3-C8 cycloalkyl-O- and halogenated C1-C6 alkoxy;
$R_0$ is C1-C4 alkyl substituted with $-NR_3R_4$; wherein $R_3$ and $R_4$ are each independently selected from H, C1-C6 alkyl optionally substituted with 1-6 substituents selected from hydroxyl, cyano and halogen, and a 4-8 membered heterocyclyl optionally substituted with 1, 2 or 3 substituents selected from halogen, C1-C6 alkyl, halogenated C1-C6 alkyl, C1-C6 alkoxy, halogenated C1-C6 alkoxy, $-NR_aR_b$ and hydroxyl, wherein $R_a$ and $R_b$ are each independently H or C1-C4 alkyl; or $R_3$ and $R_4$ form a 4-8 membered heterocyclyl optionally substituted with 1, 2 or 3 substituents selected from halogen, C1-C6 alkyl, halogenated C1-C6 alkyl, C1-C6 alkoxy, halogenated C1-C6 alkoxy, $-NR_aR_b$ and hydroxyl with the nitrogen atom to which they are attached, wherein $R_a$ and $R_b$ are each independently H or C1-C4 alkyl; preferably, said 4-8 membered heterocyclyl is each independently selected from the group consisting of oxetanyl, azetidinyl, pyrrolidinyl, tetrahydrofuranyl, piperidinyl, piperazinyl, 2-oxopiperazinyl, tetrahydropyranyl, morpholinyl,

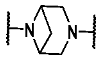 or 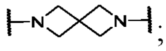;

$R_c$ and $R_d$ are each independently H or C1-C4 alkyl;
$X_6$ is C or O;
q is 1 or 2; and
$R_7$ is selected from the group consisting of F, Cl, cyano, hydroxyl, C1-C6 alkyl, fluorinated or chlorinated C1-C6

alkyl, hydroxyl-substituted C1-C6 alkyl, C1-C6 alkoxy, fluorinated or chlorinated C1-C6 alkoxy and hydroxyl-substituted C1-C6 alkyl.

In particular, in the compound of Formula III according to the present disclosure, or a pharmaceutically acceptable salt thereof, or an enantiomer, a diastereoisomer, a tautomer, an isotope substitute, a solvate, a polymorph, a prodrug or metabolite thereof, $L_2$ is C1-C4 alkylene;

$R_2$ is 5-10 membered nitrogen-containing heteroaryl or 4-10 membered heterocyclyl, preferably pyridyl, pyrimidinyl, pyrazolyl, pyrrolyl, imidazolyl, pyrazinyl, pyrrolopyridyl, dihydrofuropyridinyl or pyrido 1,3-dioxolyl or indolyl, wherein said 5-10 membered nitrogen-containing heteroaryl or 4-10 membered heterocyclyl is each optionally substituted with 1 or 2 substituents selected from C1-C6 alkoxy, C1-C6 alkyl, C3-C8 cycloalkyl-O-, halogenated C1-C6 alkoxy, halogen and cyano;

$R_0$ is C1-C4 alkyl substituted with $-NR_3R_4$, wherein $R_3$ and $R_4$ are each independently selected from Hand C1-C6 alkyl optionally substituted with 1, 2, 3, 4, 5 or 6 substituents selected from hydroxyl, cyano and halogen, or $R_3$ and $R_4$ form a 4-8 membered heterocyclyl optionally substituted with 1, 2 or 3 substituents selected from halogen, hydroxyl and C1-C4 alkyl with the nitrogen atom to which they are attached, preferably, said 4-8 membered heterocyclyl is selected from azetidinyl, pyrrolidinyl, piperidinyl, piperazinyl, morpholinyl,

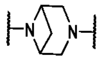 or 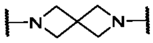;

$R_c$ and $R_d$ are each independently H or C1-C4 alkyl;

$X_6$ is C or O;

q is 1 or 2; and

$R_7$ is selected from F, Cl, cyano, hydroxyl, C1-C6 alkyl, fluorinated or chlorinated C1-C6 alkyl, hydroxyl-substituted C1-C6 alkyl, C1-C6 alkoxy, fluorinated or chlorinated C1-C6 alkoxy and hydroxyl-substituted C1-C6 alkyl.

In another aspect according to the present disclosure, a compound of Formula I, or a pharmaceutically acceptable salt thereof, or an enantiomer, a diastereoisomer, a tautomer, an isotope substitute, a solvate, a polymorph, a prodrug or metabolite thereof is provided:

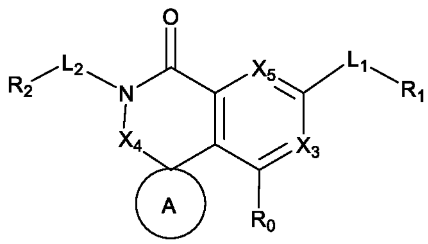

Formula I

wherein

ring A is substituted or unsubstituted 3-8 membered saturated carboncycle or substituted or unsubstituted 3-8 membered saturated heterocycle, wherein said heterocycle contains 1-2 hetero atoms selected from N, O and S;

$X_3$ is selected from $CR_5$ and N;

$X_4$ is selected from optionally substituted $-(CH_2)_n-$, -C(=O)- and $NR_6$, wherein n is 1, 2 or 3;

$X_5$ is selected from N and $CR_5$;

$L_1$ is selected from a bond, optionally substituted C1-C6 alkylene, optionally substituted C1-C6 alkylene-O-, optionally substituted C1-C6 alkylene-NH-, optionally substituted C1-C6 alkylene-S- and optionally substituted C2-C4 alkenylene;

$L_2$ is selected from a bond, optionally substituted C1-C6 alkylene, optionally substituted C1-C6 alkylene-O-, optionally substituted C1-C6 alkylene-NH-, optionally substituted C1-C6 alkylene-S- and optionally substituted C2-C4 alkenylene;

$R_0$ is selected from optionally substituted C1-C6 haloalkyl, $-C(O)NR_3R_4$, $-CH_2OR_3$ and C1-C6 alkyl substituted with $-NR_3R_4$,;

$R_1$ is selected from optionally substituted cycloalkyl, optionally substituted aryl, optionally substituted heteroaryl, optionally substituted heterocyclyl, optionally substituted amino and guanidyl;
$R_2$ is selected from optionally substituted cycloalkyl, optionally substituted aryl, optionally substituted heteroaryl and optionally substituted heterocyclyl;
$R_3$ and $R_4$ are each independently selected from H, optionally substituted C1-C6 alkyl, optionally substituted C1-C6 alkoxy, optionally substituted C1-C6 haloalkyl, optionally substituted 4-10 membered heterocyclyl and optionally substituted 5-10 membered heteroaryl; or $R_3$ and $R_4$ form an optionally substituted 4-10 membered heterocyclyl or 5-10 membered heteroaryl with the nitrogen atom to which they are attached;
$R_5$ is selected from H, halogen, C1-C6 alkyl, halogenated C1-C6 alkyl and C1-C6 alkoxy; and
$R_6$ is selected from H, C1-C6 alkyl, halogenated C1-C6 alkyl and C1-C6 alkoxy.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is C3-C8 cycloalkyl or 3-7 membered heterocyclyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is selected from cyclopropyl, cyclobutyl, cyclopentyl, cyclohexyl, aziridinyl, azetidinyl, pyrrolidinyl, piperidinyl, piperazinyl, oxiranyl, oxetanyl, tetrahydrofuranyl, tetrahydropyranyl and morpholinyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is substituted with 1, 2 or 3 substituents selected from halogen, hydroxyl, cyano, C1-C6 alkyl, halogenated C1-C6 alkyl, hydroxyl-substituted C1-C6 alkyl, C1-C6 alkoxy, halogenated C1-C6 alkoxy and $-NR_aR_b$; wherein said $R_a$ and $R_b$ are each independently selected from Hand C1-C6 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is cyclopropyl, cyclobutyl, cyclopentyl, azetidinyl, oxetanyl, pyrrolidinyl or tetrahydrofuranyl,

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is optionally substituted with 1, 2 or 3 substituents selected from F, Cl, Br, $CF_3$, $CH_3$, -OH, $-OCH_3$, $-NH_2$, $-NHCH_3$, and - $NH_2(CH_3)_2$.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_0$ is C1-C6 alkyl substituted with $-NR_3R_4$; wherein $R_3$ and $R_4$ are each independently selected from H, C1-C6 alkyl optionally substituted with 1, 2, 3, 4, 5 or 6 substituents selected from halogen, cyano, C1-C6 alkoxy, halogenated C1-C6 alkoxy, $-NR_aR_b$ and hydroxyl, or $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

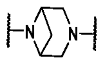 or 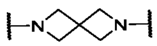

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

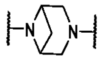 or 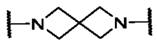

is optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, sulfo, C1-C6 alkylsulfonyl, C1-C6 alkoxysulfonyl, C1-C6 alkylcarbonyl, halogenated C1-C6 alkylcarbonyl, C1-C6 alkoxycarbonyl, halogenated C1-C6 alkoxycarbonyl, $-NR_aR_b$ and hydroxyl, wherein $R_a$ and $R_b$ are each independently H or C1-C4 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_0$ is C1-C6 alkyl substituted with $-NR_3R_4$, and wherein $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

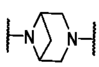 or 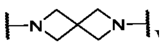,

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

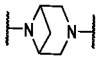 or 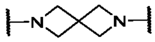

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, sulfo, C1-C6 alkylsulfonyl, C1-C6 alkoxysulfonyl, C1-C6 alkylcarbonyl, halogenated C1-C6 alkylcarbonyl, C1-C6 alkoxycarbonyl, halogenated C1-C6 alkoxycarbonyl, $-NR_aR_b$ and hydroxyl; wherein $R_a$ and $R_b$ are each independently H or C1-C4 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_0$ is C1-C6 alkyl substituted with $-NR_3R_4$, wherein $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

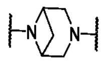 or 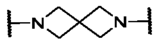.

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

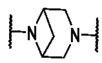 or 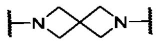

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, $-NR_aR_b$ and hydroxyl; wherein $R_a$ and $R_b$ are each independently H or C1-C4 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $X_3$ and/or $X_5$ is $CR_5$.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_5$ is selected from H and C1-C4 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $X_4$ is $-(CH_2)_n-$, n is 1 or 2; wherein said $-(CH_2)_n-$ is optionally substituted with 1 or 2 substituents selected from halogen, hydroxyl, amino, C1-C6 alkyl and halogenated C1-C6 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $X_3$ and $X_5$ are both $CR_5$; and $X_4$ is optionally substituted $-(CH_2)-$.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $L_1$ is selected from C1-C6 alkylene, C1-C6 alkylene-O-, C1-C6 alkylene-NH-, C1-C6 alkylene-S- and C2-C4 alkenylene, wherein said C1-C6 alkylene, C1-C6 alkylene-O-, C1-C6 alkylene-NH-, C1-C6 alkylene-S- and C2-C4 alkenylene may be optionally substituted with 1, 2 or 3 substituents selected from halogen, C1-C4 alkyl and hydroxyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $L_1$ is selected from C1-C6 alkylene and C1-C6 alkylene-NH-, wherein said C1-C6 alkylene and C1-C6 alkylene-NH- are optionally substituted with 1, 2 or 3 substituents selected from halogen, C1-C4 alkyl and hydroxyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_1$ is selected from optionally substituted C3-C8 cycloalkyl, optionally substituted 6-14 membered aryl, optionally substituted 5-10 membered heteroaryl and optionally substituted 4-10 membered heterocyclyl, wherein said cycloalkyl, aryl, heteroaryl and heterocyclyl are each optionally substituted with 1, 2 or 3 substituents selected from optionally substituted amino, C1-C6 alkyl, halogen, hydroxyl, C1-C6 alkoxy, C1-C6 haloalkyl and cyano; wherein said optionally substituted amino is $-NR_aR_b$, wherein $R_a$ and $R_b$ are each independently H or C1-C6 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_1$ is selected from optionally substituted phenyl, naphthyl pyridyl, pyrrolyl, pyrazolyl, imidazolyl, triazolyl, pyrimidinyl, pyrazinyl, triazinyl, imidazopyridinyl, indolyl, isoindolyl, indazolyl, benzimidazolyl, benzotriazolyl, benzoxazolyl, benzisoxazolyl, quinolyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, decahydroisoquinolyl, imidazolinyl, imidazolidinyl, quinazinyl, thiazolidinyl, isothiazolidinyl, isoxazolidinyl, dihydroindolyl, octahydroindolyl, octahydroisoindolyl, pyrrolidinyl, pyrazolindinyl, phthalimidinyl,

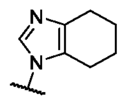 and 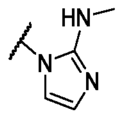.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, said $L_1$ is C1-C4 alkylene or C1-C4 alkylene-NH-, $R_1$ is pyridyl, pyrrolyl, pyrazolyl, imidazolyl, triazolyl, pyrimidinyl, pyrazinyl, triazinyl or

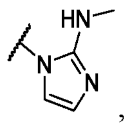,

wherein said pyridyl, pyrrolyl, pyrazolyl, imidazolyl, triazolyl, pyrimidinyl, pyrazinyl, triazinyl or

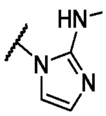

is optionally substituted with 1 or 2 substituents selected from halogen, C1-C4 alkyl and $-NR_aR_b$, wherein $R_a$ and $R_b$ are each independently H or C1-C4 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $L_2$ is selected from a bond and C1-C6 alkylene, wherein said C1-C6 alkylene is optionally substituted with 1, 2 or 3 substituents selected from halogen, C1-C3 alkyl, C3-C5 cycloalkyl and hydroxyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_2$ is optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano; wherein $R_a$ and $R_b$ are each independently H or C1-C6 alkyl; wherein said heteroaryl is preferably a heteroaryl containing 1 or 2 nitrogen ring atoms, preferably selected from pyrazolyl, imidazolyl, pyridyl, pyrazinyl and piperazinyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_2$ is selected from 6-14 membered aryl, 5-10 membered heteroaryl and 4-10 membered heterocyclyl,

wherein said 6-14 membered aryl, 5-10 membered heteroaryl and 4-10 membered heterocyclyl may be optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_2$ is selected from phenyl, naphthyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl, azetidinyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, benzodihydrofuranyl, decahydroisoquinolyl, imidazolinyl, imidazolidinyl, 6,7-dihydro-5H-cyclopenta[c]pyridine, quinazinyl, thiazolidinyl, isothiazolidinyl, isoxazolidinyl, dihydroindolyl, octahydroindolyl, octahydroisoindolyl, pyrrolidinyl, pyrazolindinyl,

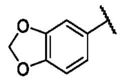 and 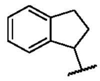,

wherein said phenyl, naphthyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl, azetidinyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, benzodihydrofuranyl, decahydroisoquinolyl, imidazolinyl, imidazolidinyl, 6,7-dihydro-5H-cyclopenta[c]pyridine, quinazinyl, thiazolidinyl, isothiazolidinyl, isoxazolidinyl, dihydroindolyl, octahydroindolyl, octahydroisoindolyl, pyrrolidinyl, pyrazolindinyl,

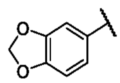

and

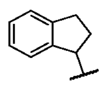

are optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $L_2$ is C1-C4 alkylene, $R_2$ is 6-14 membered aryl, 5-10 membered nitrogen-containing heteroaryl or 4-10 membered heterocyclyl, wherein said 6-14 membered aryl, 5-10 membered nitrogen-containing heteroaryl or 4-10 membered heterocyclyl is optionally substituted with 1 or 2 substituents selected from C1-C6 alkoxy, halogen, C1-C6 alkyl, cyano, C3-C8 cycloalkyl-O- and halogenated C1-C6 alkoxy, preferably phenyl, pyridyl, pyrazolyl, pyrrolopyridyl, dihydrofuropyridinyl, pyrido 1,3-dioxolyl or indolyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is C3-C6 cycloalkyl or 4-8 membered heterocyclyl, wherein said cycloalkyl or heterocyclyl is optionally substituted with 1, 2 or 3 substituents selected from halogen, C1-C4 alkyl, halogenated C1-C4 alkyl, C1-C4 alkoxy and halogenated C1-C4 alkoxy;

$X_3$ and $X_5$ are both CH;
$X_4$ is $CH_2$;
$L_1$ is C1-C4 alkylene;
$R_1$ is

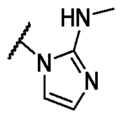

;

$L_2$ is C1-C4 alkylene;

$R_2$ is phenyl, 5-10 membered nitrogen-containing heteroaryl or 4-10 membered heterocyclyl, preferably pyridyl, pyrimidinyl, pyrazolyl, pyrrolyl, imidazolyl, pyrazinyl, pyrrolopyridyl, dihydrofuropyridinyl or pyrido 1,3-dioxolyl or indolyl, wherein said phenyl, 5-10 membered nitrogen-containing heteroaryl and 4-10 membered heterocyclyl are each optionally substituted with 1 or 2 substituents selected from C1-C6 alkoxy, halogen, C1-C6 alkyl, cyano, C3-C8 cycloalkyl-O- and halogenated C1-C6 alkoxy;

$R_0$ is C1-C4 alkyl substituted with $-NR_3R_4$; wherein $R_3$ and $R_4$ are each independently selected from H, C1-C6 alkyl optionally substituted with 1-6 substituents selected from hydroxyl, cyano and halo and a 4-8 membered heterocyclyl optionally substituted with 1, 2 or 3 substituents selected from halogen, C1-C6 alkyl, C3-C6 cycloalkyl, halogenated C1-C6 alkyl, C1-C6 alkoxy, halogenated C1-C6 alkoxy, $-NR_aR_b$ and hydroxyl, wherein $R_a$ and $R_b$ are each independently H or C1-C4 alkyl; or $R_3$ and $R_4$ form a 4-8 membered heterocyclyl optionally substituted with 1, 2 or 3 substituents selected from halogen, C1-C6 alkyl, halogenated C1-C6 alkyl, C1-C6 alkoxy, halogenated C1-C6 alkoxy, $-NR_aR_b$ and hydroxyl with the nitrogen atom to which they are attached, wherein $R_a$ and $R_b$ are each independently H or C1-C4 alkyl; preferably, said 4-8 membered heterocyclyl is each independently selected from the group consisting of oxetanyl, azetidinyl, pyrrolidinyl, tetrahydrofuranyl, piperidinyl, piperazinyl, tetrahydropyranyl, morpholinyl,

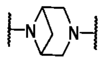 or 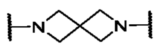.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is C3-C6 cycloalkyl, wherein said cycloalkyl is optionally substituted with 1, 2 or 3 substituents selected from halogen, C1-C4 alkyl and halogenated C1-C4 alkyl;

$X_3$ and $X_5$ are both CH;

$X_4$ is $CH_2$;

$L_1$ is C1-C4 alkylene;

$R_1$ is

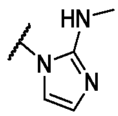

;

$L_2$ is C1-C4 alkylene;

$R_2$ is 5-10 membered nitrogen-containing heteroaryl or a 4-10 membered heterocyclyl, preferably pyridyl, pyrimidinyl, pyrazolyl, pyrrolyl, imidazolyl, pyrazinyl, pyrrolopyridyl, dihydrofuropyridinyl or pyrido 1,3-dioxolyl or indolyl, wherein said 5-10 membered nitrogen-containing heteroaryl or 4-10 membered heterocyclyl is each optionally substituted with 1 or 2 substituents selected from C1-C6 alkoxy, C1-C6 alkyl, C3-C8 cycloalkyl-O-, halogenated C1-C6 alkoxy, halogen and cyano;

$R_0$ is C1-C4 alkyl substituted with $-NR_3R_4$, wherein $R_3$ and $R_4$ are each independently selected from Hand C1-C6 alkyl optionally substituted with 1, 2, 3, 4, 5 or 6 substituents selected from hydroxyl, cyano and halogen, or $R_3$ and $R_4$ form a 4-8 membered heterocyclyl optionally substituted with 1, 2 or 3 substituents selected from halogen, hydroxyl, C1-C4 alkoxy and C1-C4 alky with the nitrogen atom to which they are attached, preferably, said 4-8 membered heterocyclyl is selected from the group consisting of azetidinyl, pyrrolidinyl, piperidinyl, piperazinyl, morpholinyl,

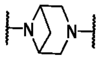 or 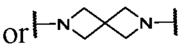.

In another aspect according to the present disclosure, the following compounds, or a pharmaceutically acceptable salt thereof, or an enantiomer, a diastereoisomer, a tautomer, an isotope substitute, a solvate, a polymorph, a prodrug or metabolite thereof are provided, wherein the compound is selected from the group consisting of:

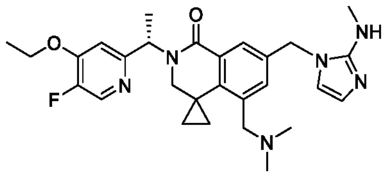 , 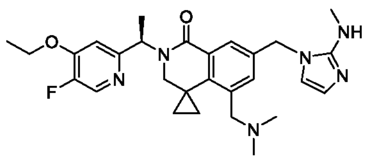 , 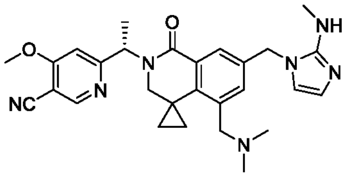 ,

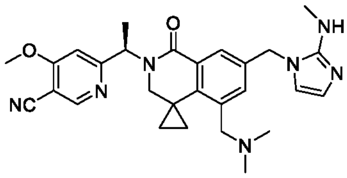 , 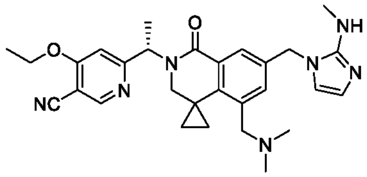 , 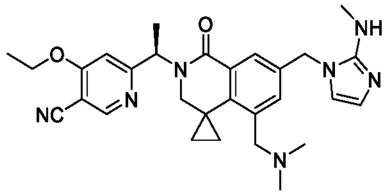 ,

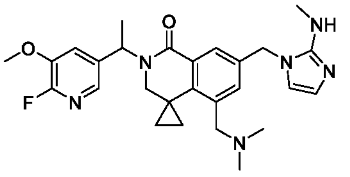 , 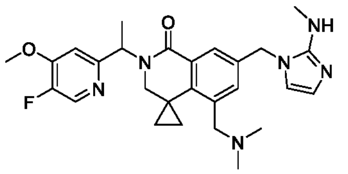 , 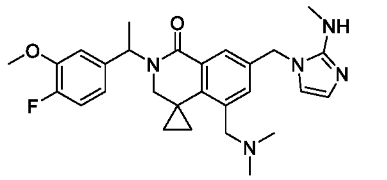 ,

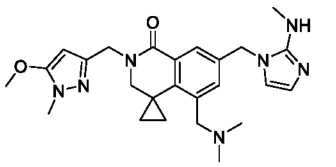 , 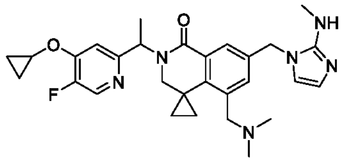 , 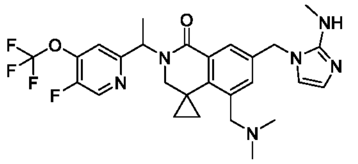 ,

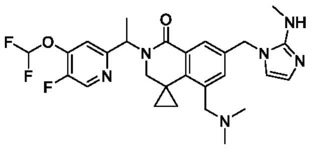 , 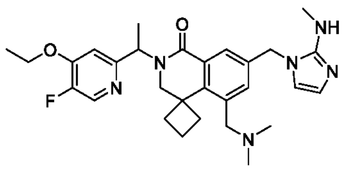 , 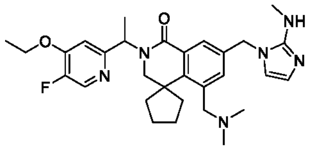 ,

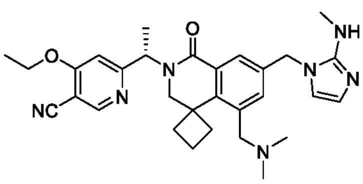 , 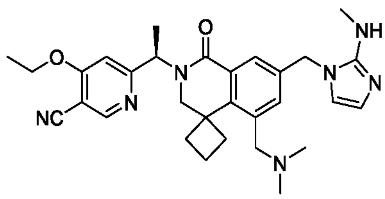 , 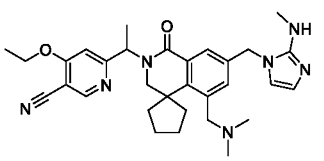 ,

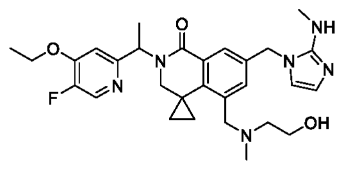 , 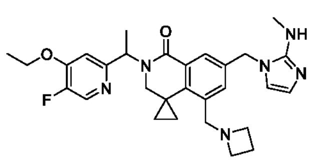 , 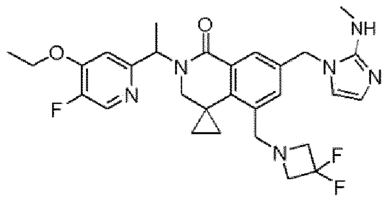 ,

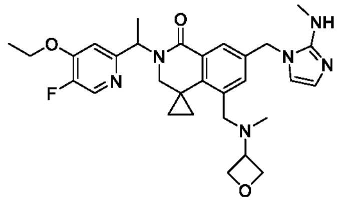 , 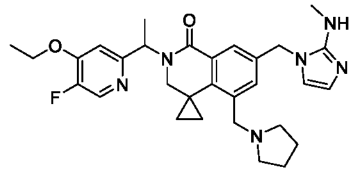 , 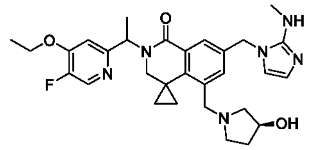 ,

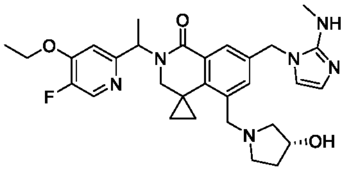 , 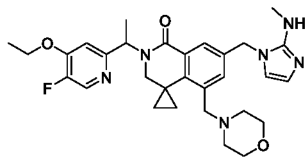 , 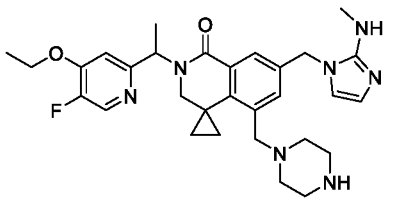 ,

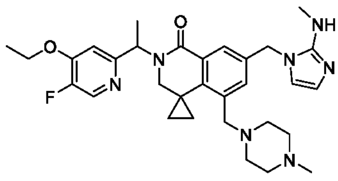 , 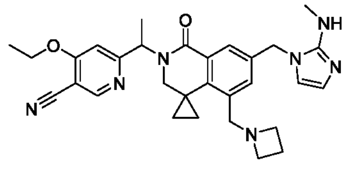 , 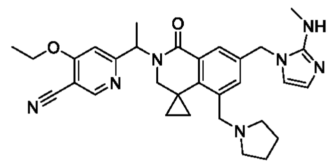 ,

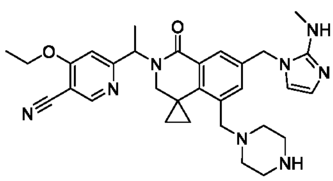 , 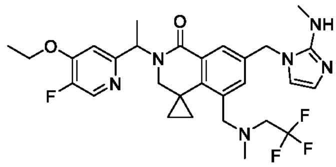 , 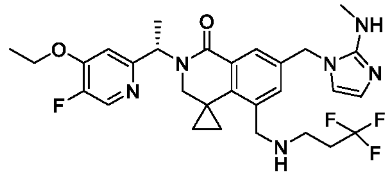 ,

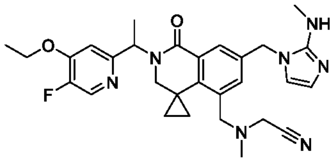 , 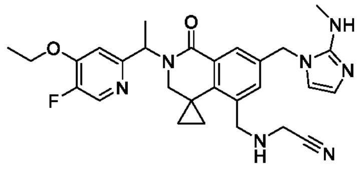 , 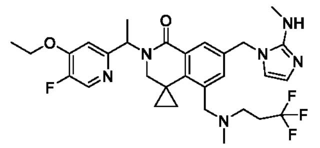 ,

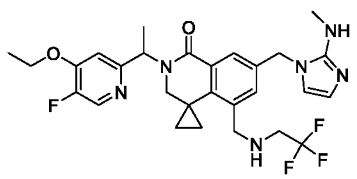 , 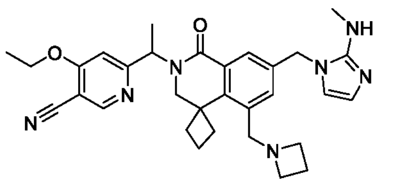 , 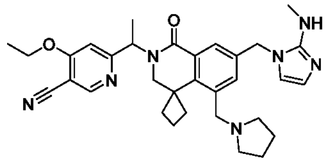 ,

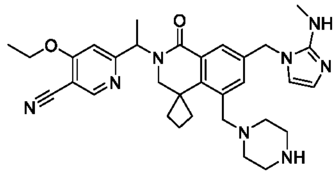 , 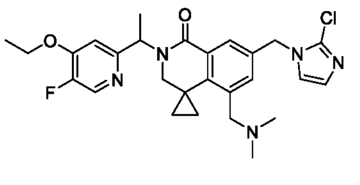 , 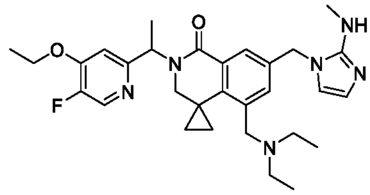 ,

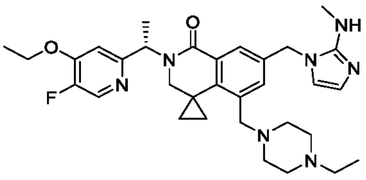 , 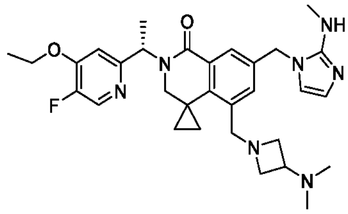 , 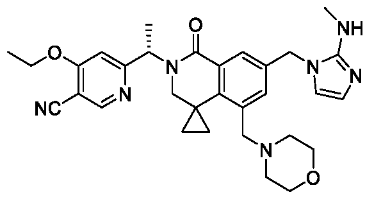 ,

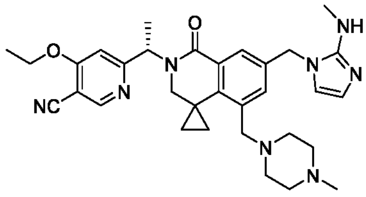 , 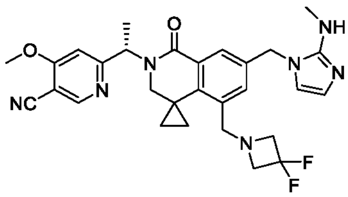 , 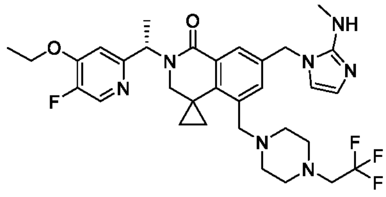 ,

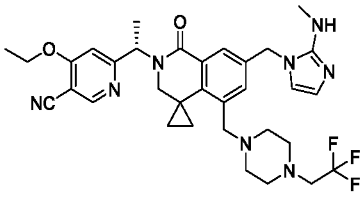 , 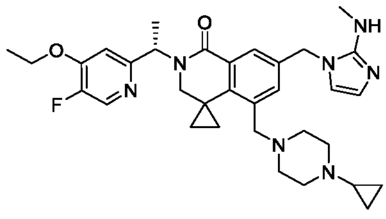 , 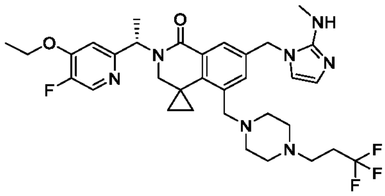 ,

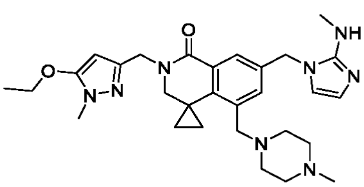 , 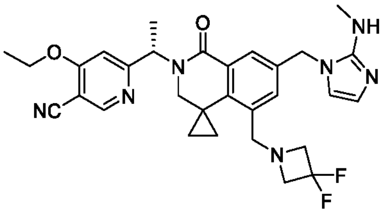 , 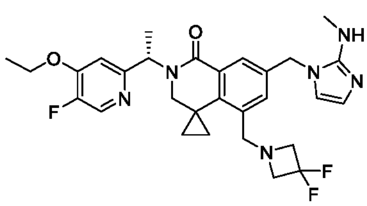 ,

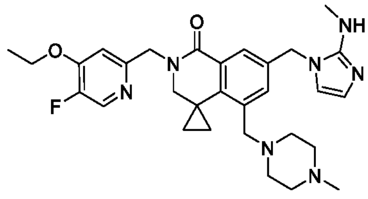,
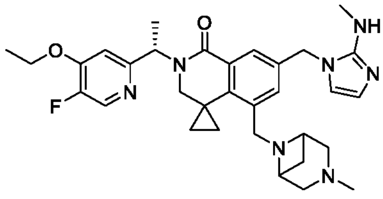,
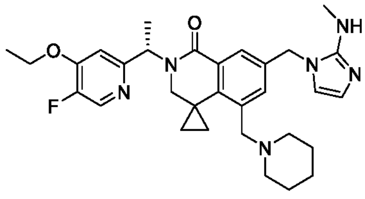,

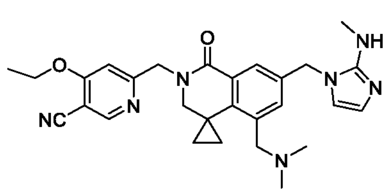,
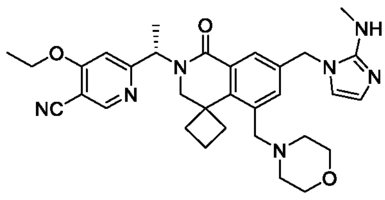,
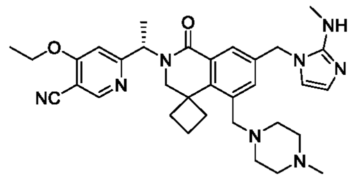,

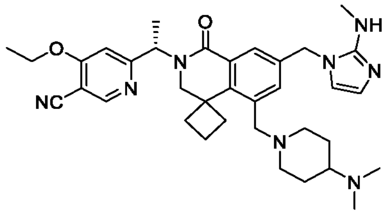,
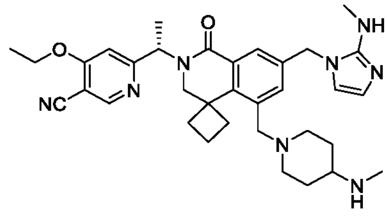,
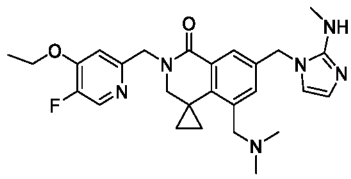,

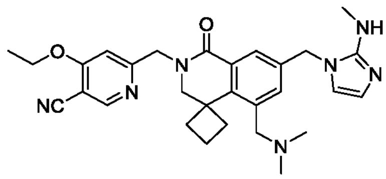,
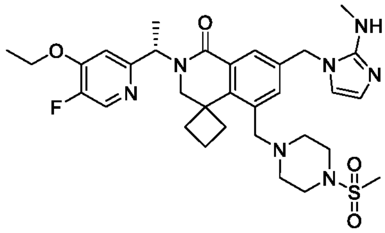,
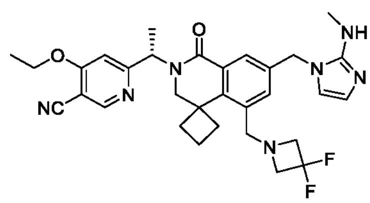,

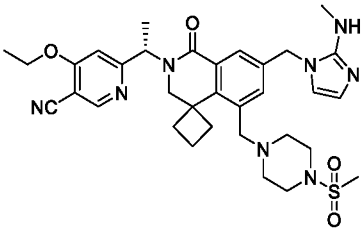,
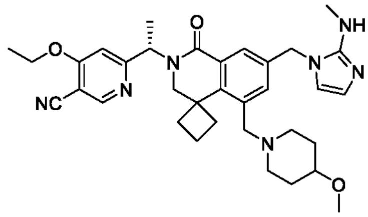,
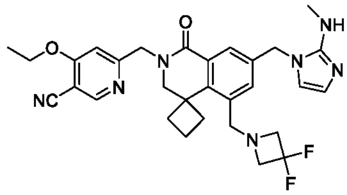,

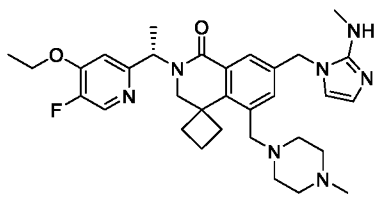,
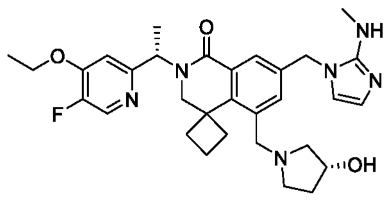,
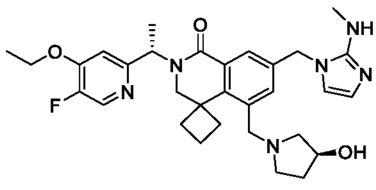,

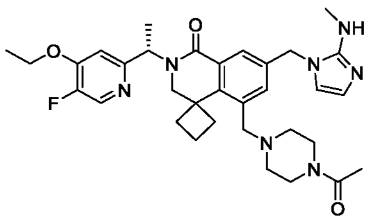, 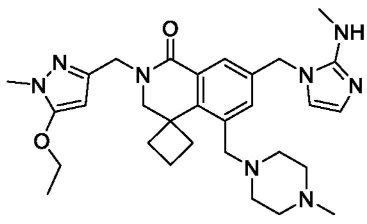, 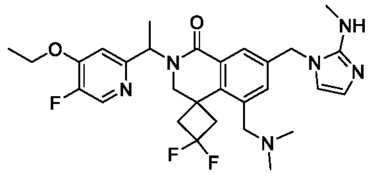,

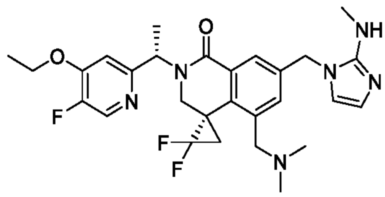, 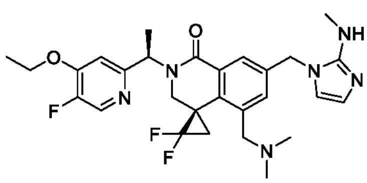, 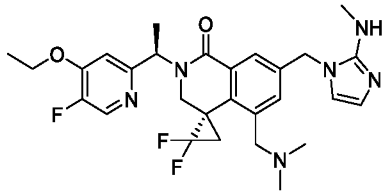,

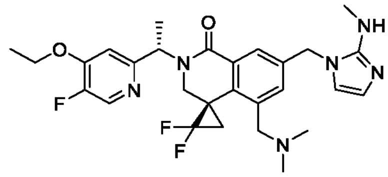, 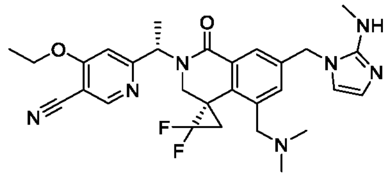, 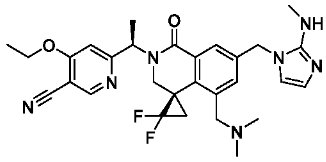,

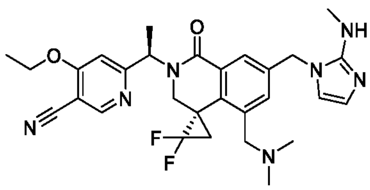, 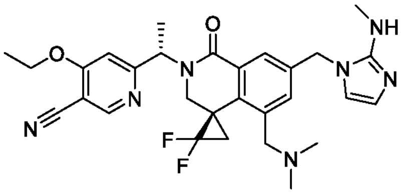, 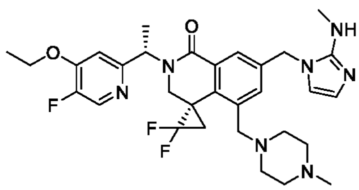,

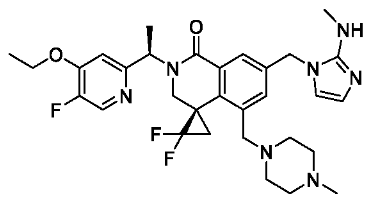, 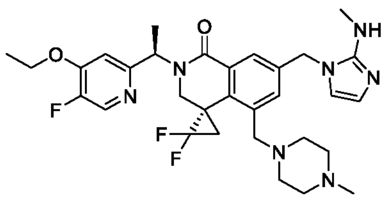, 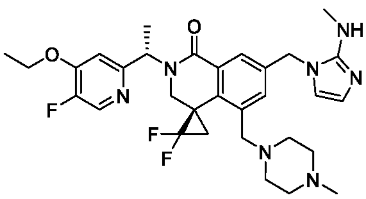,

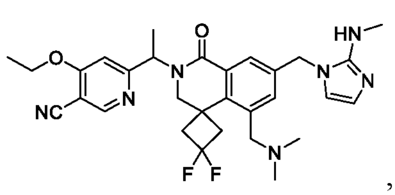, 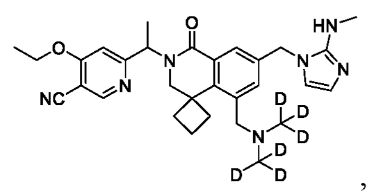, 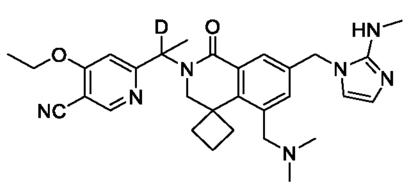,

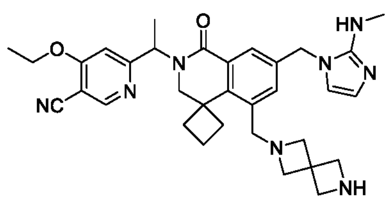, 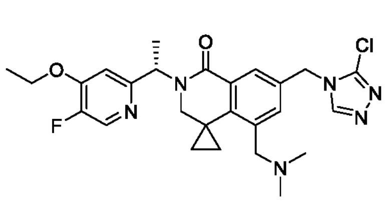, 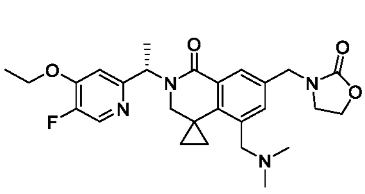,

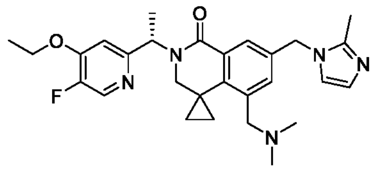,
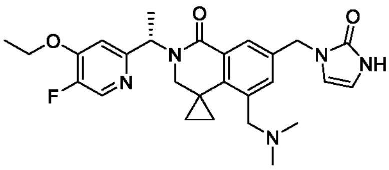,
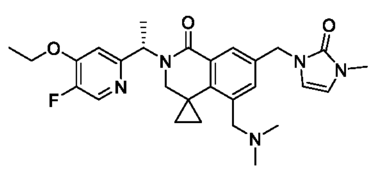,

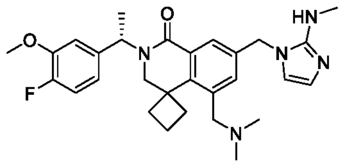,
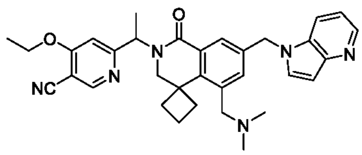,
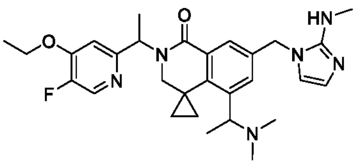,

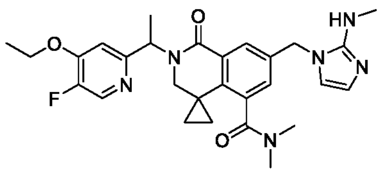,
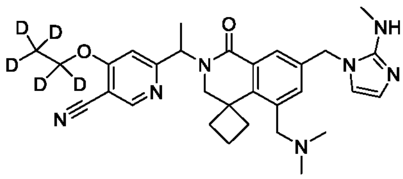,
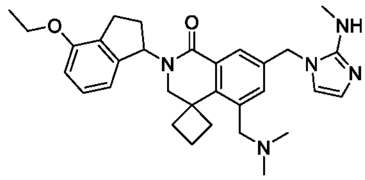,

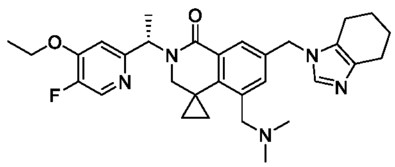,
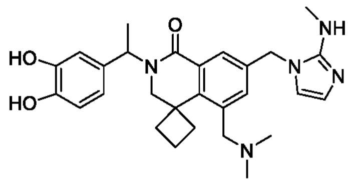,
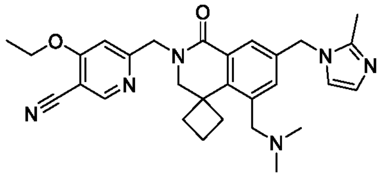,

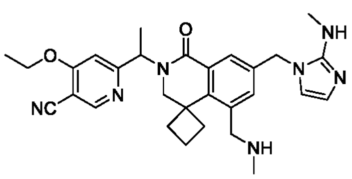,
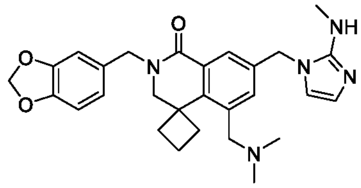,
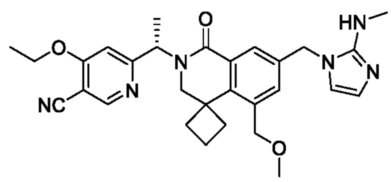,

and

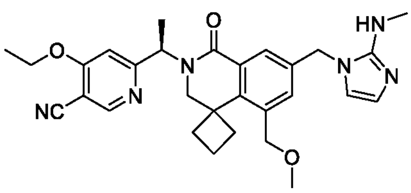.

In another aspect according to the present disclosure, a pharmaceutical composition comprising the compound according to the present disclosure, or a pharmaceutically acceptable salt thereof, or an enantiomer, a diastereoisomer, a tautomer, an isotope substitute, a solvate, a polymorph, a prodrug or metabolite thereof, and a pharmaceutically acceptable carriers or excipients is provided.

In another aspect according to the present disclosure, use of the compound according to the present disclosure, or a pharmaceutically acceptable salt thereof, or an enantiomer, a diastereoisomer, a tautomer, an isotope substitute, a solvate, a polymorph, a prodrug or metabolite thereof, in the manufacture of drugs for the treatment or prevention of WDR5-mediated diseases. In particular, said WDR5-mediated disease comprises Kabuki syndrome as well as various solid tumors and hematological tumors. In particular, said WDR5-mediated disease comprises Noonan syndrome, leopard syndrome, neuroblastoma, sarcoma, melanoma, articular chondroma, cholangioma, leukemia, breast cancer, gastrointestinal stromal tumor, histiocytic lymphoma, lung cancer, esophageal cancer, pancreatic cancer, breast cancer, prostate cancer, liver cancer, skin cancer, epithelial cell carcinoma, cervical cancer, ovarian cancer, bowel cancer, nasopharyngeal cancer, brain cancer, bone cancer, kidney cancer, oral cancer, head cancer, neuroblastoma, squamous cell carcinoma of the head and neck, anaplastic large cell lymphoma or glioblastoma, wherein leukemia is preferably juvenile myeloid monocytic leukemia, human myeloid monocytic leukemia, and lung cancer is preferably non-small cell lung cancer, small cell lung cancer, lung squamous cell carcinoma and lung adenocarcinoma.

**Detailed Description**

After long-term and in-depth research, the inventors have prepared a class of novel heterocyclic compounds represented by Formula I, which can inhibit the growth of tumor cells by blocking WDR5 protein-protein interaction. The compounds of the present disclosure exhibit good biological activity and druggability and have a good drug development prospect. Since it can inhibit WDR5 protein-protein interaction at very low concentrations (as low as ≤ 100nM/L) and the inhibitory activity is quite excellent, it can be used to treat WDR5-related diseases or conditions, such as tumors. Based on the above findings, the inventors have completed the invention.

Term

Unless otherwise defined, all technical terms herein have the same meanings as those generally understood by those skilled in the field that the subject matter of the claims belongs to. Unless otherwise indicated, all patents, patent applications, and public materials cited herein are entirely incorporated by reference.

It should be understood that the above brief description and the following details are exemplary and illustrative only, without any limitation on the subject matter of the present disclosure. In the present disclosure, unless otherwise indicated, the singular also includes the plural. It must be noted that, unless expressly stated otherwise, the singular form used in the specification and the claims includes the plural form of the referred subject matter. It should also be noted that the words "or", "alternatively" are used to indicate "and/or" unless otherwise indicated. In addition, the terms "including" and other forms used, such as "comprising", "comprised" and "containing", are not restrictive and may be open, semi-closed and closed. In other words, the term also includes "substantively composedof ... ", or "composed of ...".

Definitions of standard chemistry terms can be found in the references, including "ADVANCED ORGANIC CHEMISTRY 4TH ED. ", Carey and Sundberg, Vols. A (2000) and B (2001), Plenum Press, New York. Unless otherwise indicated, conventional methods in the art, such as mass spectrometry, NMR, IR and UV/VIS spectroscopy and pharmacological methods, are employed. Unless a specific definition is proposed, the terms used herein in relevant descriptions of analytical chemistry, synthetic organic chemistry, drugs and pharmaceutical chemistry are known in the art. Standard techniques can be used in chemical synthesis, chemical analysis, drug preparation, formulation and delivery, and in the treatment of patients. For example, the reaction and purification can be performed according to the manufacturer's instructions for use of the kit, or in a manner well known in the art or according to the description of the present disclosure. The above techniques and methods may generally be implemented in accordance with the conventional methods well known in the art according to a plurality of summary and more specific literature cited and discussed in the present specification. In the present specification, groups and their substituents may be selected by those skilled in the art to provide a stable structural moiety and compound.

When a substituent is described by a conventional chemical formula written from left to right, the substituent also includes the chemically equivalent substituent obtained when the structural formula is written from right to left. For example, $-CH_2O-$ is equivalent to $-OCH_2-$.

The section headings used herein are for the purpose of organizing the article only and should not be construed as limiting the subject matter. All documents or parts of documents cited in the present disclosure, including but not limited to patents, patent applications, articles, books, operating manuals and theses, are entirely incorporated herein by reference.

Some of the chemical groups defined herein are preceded by simplified symbols indicating the total number of carbon atoms present in that group. For example, C1-C6 alkyl groups refer to alkyl groups with a total of 1 to 6 carbon atoms as defined below. The total number of carbon atoms in the simplified symbol does not include carbon that may be present in the substituents of said group.

In addition to the foregoing, when used in the specification and claims of the present application, unless otherwise specifically specified, the following terms have the meanings set out below.

In the present disclosure, the term "halogen" refers to fluorine, chlorine, bromine or iodine.

"hydroxyl" refers to -OH.

"hydroxylalkyl" refers to alkyl substituted with hydroxyl(-OH) as defined below.

"carbonyl" refers to -C(=O)-.

"nitro" refers to $-NO_2$.
"cyano" refers to -CN.
"amino" refers to $-NH_2$.
"substituted amino" refers to amino substituted with 1 or 2 of alkyl, alkylcarbonyl, alkyl substituted aminocarbonyl, aralkyl, heteroaralkyl as defined below, for example, monoalkylamino, dialkylamino, alkylamido, aralkylamino, heteroaralkylamino, alkylaminocarbonylamino.
"carboxyl" refers to -COOH.

In the present disclosure, as a group or part of other groups (e.g., used in a group such as alkyl group substituted with halogen (e.g., fluorine, chlorine, bromine or iodine)), the term "alkyl" refers to a fully saturated linear or branched hydrocarbon chain group, which is composed only of carbon atoms and hydrogen atoms, having for example, 1 to 12 (preferably 1 to 8, more preferably 1 to 6) carbon atoms, and is connected to the rest of the molecule by a single bond. Alkyl groups include, but are not limited to, methyl, ethyl, n-propyl, isopropyl, n-butyl, isobutyl, sec-butyl, tert-butyl, n-pentyl, 2-methylbutyl, 2,2-dimethylpropyl, n-hexyl, heptyl, 2-methylhexyl, 3-methylhexyl, octyl, nonyl and decyl, etc. More preferably, alkyl group is C1-C4 alkyl. The term "alkylene" refers to a divalent alkyl group, such as - $(CH_2)_n$-, wherein n is an integer of 1-12, preferably an integer of 1-6, and more preferably an integer of 1-4. The divalent alkyl group may also be a linear or branched chain group, such as $-CH(CH_3)CH_2-$, $-CH_2CH(CH_3)CH_2-$, etc.

In the present disclosure, as a group or part of other groups, the term "alkenyl" refers to a hydrocarbon chain group composed of only carbon atoms and hydrogen atoms, which comprises at least one double bond and has for example, 2 to 20 (preferably 2 to 10, more preferably 2 to 6, more preferably 2 to 4) carbon atoms, and is connected to the rest of the molecule by a single bond. Alkenyl includes but is not limited to vinyl, propenyl, allyl, but-1-enyl, but-2-enyl, pent-1-enyl, pent-1,4-dienyl, etc. The term "alkenylene" refers to a divalent alkenyl group, such as - CH=CH-, - $CH_2$-CH=CH-, etc.

In the present disclosure, as a group or part of other groups, the term "alkynyl" refers to a hydrocarbon chain group composed of only carbon atoms and hydrogen atoms, which comprises at least one triple bond and has for example 2 to 20 (preferably 2 to 10, more preferably 2 to 6, more preferably 2 to 4) carbon atoms, and is connected to the rest of the molecule by a single bond. Alkynyl includes but is not limited to ethynyl, propynyl, etc.

In the present disclosure, as a group or part of other groups, the term "alkoxy" refers to "alkyl-O-", wherein the alkyl group is as defined herein. Preferably, alkoxy is C1-C6 alkoxy, more preferably C1-C4 alkoxy, including but not limited to methoxy, ethoxy, etc.

In the present disclosure, as a group or part of other groups, the term "cyclic hydrocarbon group" means a stable non-aromatic monocyclic or polycyclic hydrocarbon group (e.g., alkyl, alkenyl or alkynyl) composed of only carbon atoms and hydrogen atoms, which may include a fused ring system, a bridge ring system or a spiro ring system having 3 to 15 carbon atoms, preferably 3 to 10 carbon atoms, and more preferably 3 to 8 carbon atoms, such as 3, 4, 5, 6, 7 or 8 carbon atoms and is saturated or unsaturated and can be connected to the rest of the molecule by a single bond via any suitable carbon atom. Unless otherwise specified in the specification, carbon atoms in cyclic hydrocarbon groups may optionally be oxidized. Preferably, the term "cyclic hydrocarbon group" refers to a cycloalkyl group with a number of 3-8 ring carbon atoms. Examples of cyclic hydrocarbon groups include, but are not limited to, cyclopropyl, cyclobutyl, cyclopentyl, cyclopentenyl, cyclohexyl, cyclohexenyl, cyclohexadienyl, cycloheptyl, cyclooctyl, 1H-indenyl, 2,3-dihydroindenyl, 1,2,3,4-tetrahydro-naphthyl, 5,6,7,8-tetrahydro-naphthyl, 8,9-dihydro-7H-benzocyclohepten-6-yl, 6,7,8,9-tetrahydro-5H-benzocycloheptenyl, 5,6,7,8,9,10-hexahydro-benzocyclooctenyl, fluorenyl, dicyclo[2.2.1]heptyl, 7,7-dimethyl-dicyclo[2.2.1]heptyl, dicyclo[2.2.1]heptenyl, dicyclo[2.2.2]octyl, dicyclo[3.1.1]heptyl, dicyclo[3.2.1]octyl, dicyclo[2.2.2]octenyl, dicyclo[3.2.1]octenyl, adamantyl, octahydro-4,7-methylene-1H-indenyl and octahydro-2,5-methylene-pentaleno, etc. Cyclic hydrocarbon groups also include divalent groups, i.e. connected to the rest of the molecule by two bonds. The connection can occur on the same ring carbon atom or on different ring carbon atoms.

In the present disclosure, as a group or part of other groups, the term "heterocyclyl" refers to a stable 3-20 membered non-aromatic cyclic group composed of 2 to 14 carbon atoms (e.g., 2, 3, 4, 5, 6, 7, 8, 9, 10, 11, 12, 13 or 14 carbon atoms) and 1 to 6 heteroatoms selected from nitrogen, phosphorus, oxygen and sulfur. Unless otherwise specified in the specification, heterocyclyls may be ring systems of single-ring, bicyclic ring, tricyclic ring or more rings, which may include a fused ring system, a bridge ring system, or a spiro ring system. The nitrogen, carbon or sulfur atoms in the heterocyclyls can be optionally oxidized. Nitrogen atoms can optionally be quaternized and the heterocyclyls can be partially or fully saturated. Heterocyclyls can be connected to the rest of the molecule via carbon atoms or heteroatoms and through single bonds. In a heterocyclyl containing a fused ring, one or more rings may be aryl or heteroaryl as defined below, provided that the connecting point to the rest of the molecule is a non-aromatic ring atom. For the object of the present disclosure, the heterocyclyl is preferably a stable 4-11 membered non-aromatic monocyclic, bicyclic, bridged ring or spiro ring group comprising 1 to 3 heteroatoms selected from nitrogen, oxygen and sulfur, more preferably a stable 4-10 membered non-aromatic monocyclic, bicyclic, bridged ring or spiro ring group comprising 1 to 3 heteroatoms selected from nitrogen, oxygen and sulfur. In some embodiments, the number of ring atoms of the heterocyclyl is 4-9,

4-8, or 4-7. In some embodiments, the number of ring atoms of the heterocyclyl is 3-8, preferably 3-7, more preferably 3-6. Examples of heterocyclyl include but are not limited to azetidinyl, pyrrolidinyl, morpholinyl, piperazinyl, homopiperazinyl, piperidinyl, thiomorpholinyl, 2,7-diaza-spiro[3.5]nonan-7-yl, 2-oxa-6-aza-spiro [3.3]heptan-6-yl, 2,5-diaza-bicyclo[2.2.1]heptan-2-yl, azetidinyl, pyranyl, tetrahydropyranyl, thiopyranyl, dihydrofuropyridinyl, pyrido 1,3-dioxolyl, tetrahydrofuranyl, oxazinyl, dioxolanyl, tetrahydroisoquinolyl, decahydroisoquinolyl, imidazolinyl, imidazolidinyl, quinazinyl, thiazolidinyl, isothiazolidinyl, isoxazolidinyl, dihydroindolyl, octahydroindolyl, octahydroisoindolyl, pyrazolindinyl, 6,7-dihydro-5H-cyclopenta[c]pyridine, phthalimidinyl, etc.

In the present disclosure, as a group or part of other groups, the term "aryl" means a conjugated hydrocarbon cyclic system group having 6 to 18 carbon atoms, such as 6 to 14 carbon atoms (preferably 6 to 10 carbon atoms, such as 6, 7, 8, 9 or 10 carbon atoms). For the purposes of the present disclosure, the aryl group may be a ring system of single ring, bicyclic ring, tricyclic ring or more rings, and may also be fused with cycloalkyl or heterocyclyl as defined above, provided that the aryl group is connected to the rest of the molecule by a single bond through an atom on the aromatic ring. Examples of the aryl group include, but are not limited to, phenyl, naphthyl, anthracenyl, phenanthrenyl, fluorenyl, 2,3-dihydro-1H-isoindolyl, 2-benzoxazolinonyl, 2H-1,4-benzoxazin-3(4H)-one-7-yl, etc.

In the present disclosure, the term "aralkyl" refers to the alkyl group defined above that is substituted by the aryl group defined above.

In the present disclosure, as a group or part of other groups, the term "heteroaryl" refers to a 5-16 membered, preferably 5-10 membered conjugated ring group, wherein the ring has 1 to 15 carbon atoms (preferably 1 to 10 carbon atoms, such as 1, 2, 3, 4, 5, 6, 7, 8, 9 or 10 carbon atoms) and 1 to 6 heteroatoms selected from nitrogen, oxygen and sulfur. Unless otherwise specified in this specification, the heteroaryl group may be a ring system of single-ring, bicyclic ring, tricyclic ring or more rings, and may also be fused to cycloalkyl or heterocyclyls as defined above, provided that the heteroaryl group is connected to the rest of the molecule by single bonds through atoms on the aromatic ring. The nitrogen, carbon or sulfur atoms in the heteroaryl group can be optionally oxidized. Nitrogen atoms can optionally be quaternized. For the purposes of the present disclosure, the heteroaryl group is preferably a stable 5-12 membered aromatic group comprising comprising 1 to 5 heteroatoms selected from nitrogen, oxygen and sulfur, more preferably a stable 5-10 membered aromatic group comprising 1 to 4 heteroatoms selected from nitrogen, oxygen and sulfur or 5-6 membered aromatic groups comprising 1 to 3 heteroatoms selected from nitrogen, oxygen and sulfur. Examples of the heteroaryl group include, but are not limited to, thienyl, imidazolyl, pyrazolyl, thiazolyl, oxazolyl, oxadiazolyl, isoxazolyl, pyridyl, pyrimidinyl, pyrazinyl, pyridazinyl, benzimidazolyl, benzopyrazolyl, indolyl, furanyl, pyrrolyl, triazolyl, tetrazoly, triazinyl, indolizinyl, isoindolyl, indazolyl, isoindazolyl, purinyl, quinolyl, isoquinolyl, phthalazinyl, naphthyridinyl, quinoxalinyl, peteridinyl, carbazolyl, carbolinyl, phenanthridinyl, phenanthrolinyl, acridine, phenazinyl, isothiazolyl, benzothiazole, pyrrolopyridyl, benzopyrrolyl, benzothienyl, oxatriazolyl, cinnolinyl, quinazolinyl, phenylthio, indolizinyl, o-phenanthrolinyl, isoxazolyl, phenoxazinyl, phenothiazinyl, 4,5,6,7-tetrahydrobenzo[b]thienyl, naphthopyridyl, [1,2,4]triazolo[4,3-b]pyridazine, [1,2,4]triazolo[4,3-a]pyrazine, [1,2,4]triazolo[4,3-c]pyrimidine, [1,2,4]triazolo[4,3 -a]pyridine, imidazolo[1,2-a]pyridine, imidazolo[1,2-b]pyridazine, imidazolo[1,2-a]pyrazine, etc.

In the present disclosure, the term "heteroaralkyl" refers to the alkyl group defined above that is substituted by the heteroaryl group defined above.

In the present disclosure, "optionally" means that the event or situation subsequently described may or may not occur, and the expression includes both the occurrence and non-occurrence of the event or situation. For example, "optionally substituted aryl" means that the aryl group is substituted or unsubstituted, and the expression includes both substituted aryl and unsubstituted aryl. The "optional" substituents described in the claims and specification of the present disclosure include, but are not limited to, one or more of alkyl, alkenyl, alkynyl, halogen, halogenated alkyl, halogenated epoxy, halogenated alkenyl, halogenated alkynyl, cyano, cycloalkyl-O-, nitro, amino, optionally substituted amino, alkoxy, cycloalkyl, optionally substituted aryl, optionally substituted heteroaryl, optionally substituted cyclic hydrocarbon group, and optionally substituted heterocyclyls. In a preferred embodiment, the substituents include, but are not limited to, one or more of C 1-C6 alkyl, halogenated alkyl, halogenated epoxy, cycloalkyl, amino, C1-C6 alkyl-substituted amino, halogen, cyano, C3-C8 cycloalkyl-O-, aryl and heteroaryl. In the present disclosure, when it is substituted, the number of substituents may be 1-5, such as 1-3, depending on the structure of the substituted group. For example, phenyl may be substituted by 1-3 substituents selected from C1-C6 alkoxy, C3-C8 cycloalkyl-O-, halogen and amino groups.

As used herein, "WDR5" refers to the WD40 domain repeat family protein 5, and the WD40 domain refers to the smallest conserved unit containing approximately 40 amino acids (typically supported by glycine-histidine and tryptophan-aspartic acid).

As used herein, the terms "part", "structural part", "chemical part", "group", "chemical group" refer to a specific fragment or functional group in a molecule. Chemical parts are often considered as chemical entities embedded or attached to molecules.

Those skilled in the art should also understand that in the method described below, the functional group of intermediate compounds may need protecting by an appropriate protective group. Such functional groups include hydroxyl, amino, mercapto and carboxyl. Suitable protective groups for hydroxyl include trialkyl silyl or diaryl alkylsilyl (e.g.,

tert-butyldimethylsilyl, tert-butyldiphenylsilyl or trimethylsilyl), tetrahydropyranyl, benzyl and the like. Suitable protective groups for amino, amidinyl and guanidinyl include tert-butoxycarbonyl, benzyloxycarbonyl, etc. Suitable protective groups for mercapto include -C(O)-R" (where R" is alkyl, aryl or aralkyl), p-methoxybenzyl, triphenylmethyl, etc. Suitable protective groups for carboxyl include esters of alkyl, aryl, or aralkyl.

The protective group may be introduced and removed according to standard techniques known to those skilled in the art and described herein. The use of protective groups is described in Greene, T. W. and P. G. M. Wuts, Protective Groups in Organi Synthesis, (1999), 4th Ed., Wiley. The protective group may also be a polymer resin.

**Compound of Formula I**

The compound of Formula I according to the present disclosure has a structure represented by the following formula:

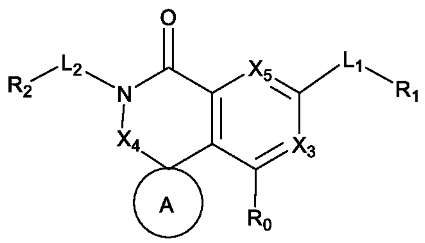

wherein,

ring A is a substituted or unsubstituted 3-8 membered saturated or unsaturated carboncycle, a substituted or unsubstituted 3-8 membered saturated or unsaturated heterocycle, wherein said heterocycle comprises 1-2 hetero atoms independently selected from N, O and S;
$X_3$ is selected from $CR_5$ and N;
$X_4$ is selected from optionally substituted $-(CH_2)_n-$, -C(=O)- and $NR_6$, wherein n is 1, 2 or 3;
$X_5$ is selected from N and $CR_5$;
$L_1$ is selected from a bond, optionally substituted C1-C6 alkylene, optionally substituted C1-C6 alkylene-O-, optionally substituted C1-C6 alkylene-NH-, optionally substituted C1-C6 alkylene-S- and optionally substituted C2-C4 alkenylene;
$L_2$ is selected from a bond, optionally substituted C1-C6 alkylene, optionally substituted C1-C6 alkylene-O-, optionally substituted C1-C6 alkylene-NH-, optionally substituted C1-C6 alkylene-S- and optionally substituted C2-C4 alkenylene;
$R_0$ is selected from H, C1-C6 alkyl, optionally substituted C1-C6 alkoxy, optionally substituted C1-C6 haloalkyl and C1-C6 alkyl substituted with $-NR_3R_4$;
$R_1$ is selected from optionally substituted cycloalkyl, optionally substituted aryl, optionally substituted heteroaryl, optionally substituted heterocyclyl, optionally substituted amino and guanidyl;
$R_2$ is selected from optionally substituted cycloalkyl, optionally substituted aryl, optionally substituted heteroaryl and optionally substituted heterocyclyl;
$R_3$ and $R_4$ are each independently selected from H, optionally substituted C1-C6 alkyl, optionally substituted C1-C6 alkoxy, optionally substituted C1-C6 haloalkyl, optionally substituted 4-10 membered heterocyclyl and optionally substituted 5-10 membered heteroaryl; or $R_3$ and $R_4$ form an optionally substituted 4-10 membered heterocyclyl or 5-10 membered heteroaryl with the nitrogen atom to which they are attached;
$R_5$ is selected from H, halogen, C1-C6 alkyl, halogenated C1-C6 alkyl and C1-C6 alkoxy;
$R_6$ is selected from H, C1-C6 alkyl, halogenated C1-C6 alkyl and C1-C6 alkoxy.
preferably, ring A is C3-C8 cycloalkyl, such as cyclopropyl, cyclobutyl, cyclopentyl or cyclohexyl; or ring A is a 3-7 membered heterocyclyl, such as aziridinyl, azetidinyl, pyrrolidinyl, piperidinyl, piperazinyl, oxiranyl, oxetanyl, tetrahydrofuranyl, tetrahydropyranyl and morpholinyl, etc. It should be noted that the group of ring A as described herein comprises C atoms shared by ring A and ring containing $X_4$.

Preferably, ring A may be optionally substituted with 1, 2 or 3 substituents selected from halogen, cyano, hydroxyl, C1-C6 alkyl, halogenated C1-C6 alkyl, hydroxyl-substituted C1-C6 alkyl, C1-C6 alkoxy, halogenated C1-C6 alkoxy, hydroxyl-substituted C1-C6 alkyl, $-NR_aR_b$, carbonyl substituted with $-NR_aR_b$, C1-C6 alkyl substituted with $-NR_aR_b$, C1-C6 alkylcarbonyl, C1-C6 alkoxycarbonyl, optionally substituted 6-14 membered aryl, optionally substituted 5-10 membered heteroaryl, optionally substituted C3-C8 cycloalkyl, optionally substituted 4-10 membered heteroaryl, C1-C6

alkyl substituted with optionally substituted 6-14 membered aryl, C1-C6 alkyl substituted with optionally substituted 5-10 membered heteroaryl, C1-C6 alkyl substituted with optionally substituted 4-10 membered heteroaryl and C1-C6 alkyl substituted with optionally substituted C3-C8 cycloalkyl; preferred substituents are one or more of halogen, C1-C6 alkyl, halogenated C1-C6 alkyl, hydroxyl-substituted C1-C6 alkyl, C1-C6 alkoxy, halogenated C1-C6 alkoxy, hydroxyl-substituted C1-C6 alkyl, $-NR_aR_b$, carbonyl substituted with $-NR_aR_b$, C1-C6 alkyl substituted with $-NR_aR_b$, C1-C6 alkylcarbonyl and C1-C6 alkoxycarbonyl. When said 6-14 membered aryl, 5-10 membered heteroaryl, 4-10 membered heteroaryl and C3-C8 cycloalkyl are substituted, the substituents may be 1, 2 or 3 substituents selected from halogen, C1-C6 alkyl, halogenated C1-C6 alkyl, C1-C6 alkoxy, halogenated C1-C6 alkoxy, $-NR_aR_b$, carbonyl substituted with $-NR_aR_b$, C1-C6 alkyl substituted with $-NR_aR_b$, C1-C6 alkylcarbonyl, C1-C6 alkoxycarbonyl, wherein said $R_a$ and $R_b$ are each independently selected from H and C1-C6 alkyl. When ring A is substituted, the substituents may be on C or N.

In a preferred embodiment, ring A is C3-C6 cycloalkyl or a 3-6 membered heterocyclyl, which is optionally substituted with 1, 2 or 3 substituents selected from C1-C4 alkyl, halogenated C1-C4 alkyl, C1-C4 alkyl substituted with hydroxyl, carbonyl substituted with $-NR_aR_b$ and C1-C4 alkyl substituted with $-NR_aR_b$, wherein said $R_a$ and $R_b$ are each independently selected from H and C1-C4 alkyl.

Preferably, $X_3$ is $CR_5$.

Preferably, $R_5$ is selected from H and C1-C4 alkyl.

Preferably, $X_4$ is $-(CH_2)_n-$, n is 1 or 2.

Preferably, when $X_4$ is $-(CH_2)_n-$ and substituted, the number of substituents may be 1 or 2, and the substituents may be selected from halogen, hydroxyl, amino, C1-C6 alkyl and halogenated C1-C6 alkyl, preferably halogen and C1-C6 alkyl.

Preferably, $L_1$ is selected from a bond, C1-C6 alkylene, C1-C6 alkylene-O-, C1-C6 alkylene-NH-, C1-C6 alkylene-S- and C2-C4 alkenylene, more preferably C1-C6 alkyl or C1-C6 alkylene-NH-. $L_1$ is optionally substituted, and the substituents may be selected from halogen, C1-C4 alkyl and hydroxyl. The number of substituents may be 1, 2 or 3. It should be noted that when said C1-C6 alkyl and C1-C6 alkylene- is substituted with C1-C4 alkyl, branched alkyl or branched alkylene may be formed.

Preferably, $R_1$ is selected from optionally substituted C3-C8 cycloalkyl, optionally substituted 6-14 membered aryl, optionally substituted 5-10 membered heteroaryl and optionally substituted 4-10 membered heterocyclyl. In $R_1$, preferred 6-14 membered aryl includes phenyl and naphthyl. In $R_1$, preferred 5-10 membered heteroaryl is heteroaryl having one or more (for example, no more than 4 or no more than 3) nitrogen ring atoms, especially 5 membered or 6 membered nitrogen-containing heteroaryl, including but not limited to, pyridyl, pyrrolyl, pyrazolyl, imidazolyl, triazolyl (such as 1H-1,2,3-triazolyl, 1,2,4-triazolyl, 1,2,5-triazolyl and 1,3,4-triazolyl), pyrimidinyl, pyrazinyl, triazinyl. The heteroaryl group may also be a fused ring, including but not limited to, indolyl, isoindolyl, indazolyl, benzimidazolyl, benzotriazolyl, benzoxazolyl, pyrrolopyridyl, benzisoxazolyl, quinolyl, etc. Preferred heteroaryl is pyridyl, pyrrolyl, imidazolyl and triazolyl, more preferably imidazolyl and pyridyl. Preferably, when $R_1$ is heteroaryl, it is connected to $L_1$ via nitrogen atoms on the heteroaromatic ring. In $R_1$, preferred 5-10 membered heterocyclyl includes, but is not limited to, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, decahydroisoquinolyl, imidazolinyl, imidazolidinyl, quinazinyl, thiazolidinyl, isothiazolidinyl, isoxazolidinyl, dihydroindolyl, octahydroindolyl, octahydroisoindolyl, pyrrolidinyl, pyrazolindinyl, phthalimidinyl, etc. In $R_1$, said cycloalkyl, aryl, heteroaryl and heterocyclyl are each optionally substituted with 1, 2 or 3 substituents. Preferred substituents may be selected from optionally substituted amino, alkyl (such as C1-C6 alkyl), halogen, hydroxyl, alkoxy (such as C1-C6 alkoxy), haloalkyl (such as C1-C6 haloalkyl) and cyano, etc. The optionally substituted amino may be $-NR_aR_b$, wherein $R_a$ and $R_b$ are each independently H or C1-C6 alkyl; more preferably, the substituents on the cycloalkyl, aryl, heteroaryl and heterocyclyl may be independently the optionally substituted amino, halogen, alkyl and cyano. When $R_1$ is optionally substituted amino or guanidyl, the substituents on amino may be aminocarbonyl optionally substituted with alkyl, i.e., $NR_aR_b$-CO-, wherein $R_a$ and $R_b$ are each independently H or C1-C6 alkyl. More preferred $R_1$ is 5 or 6 membered nitrogen-containing heteroaryl substituted with substituted amino, halogen, alkyl and/or cyano, especially imidazolyl, pyrimidinyl, triazolyl, pyridyl, pyrazinyl, pyrrolyl, pyrazolyl and the like substituted with substituted amino, halogen, alkyl and/or cyano, more preferably imidazolyl and pyridyl substituted with substituted amino, halogen, alkyl and/or cyano, wherein the substituted amino is preferably $-NR_aR_b$, wherein $R_a$ and $R_b$ are each independently H or C1-C6 alkyl.

In a particularly preferred embodiment, ring A is cyclopropyl, cyclobutyl, cyclopentyl, azetidinyl, oxetanyl, pyrrolidinyl or tetrahydrofuranyl, which is optionally substituted with 1, 2 or 3 substituents selected from F, Cl, Br, $CF_3$, $CH_3$, -OH, $-OCH_3$, $-NH_2$, $-NHCH_3$, and $-NH_2(CH_3)_2$.

In a particularly preferred embodiment, said $L_1$ is C1-C4 alkylene or C1-C4 alkylene-NH-, $R_1$ is 5 or 6 membered nitrogen-containing heteroaryl optionally substituted with 1 or 2 substituents selected from halogen, cyano, C1-C4 alkyl and $-NR_aR_b$, preferably pyridyl, pyrrolyl, pyrazolyl, imidazolyl, imidazolonyl, pyridinopyrrolyl, oxazolone, oxazolidinone, triazolyl, pyrimidinyl, pyrazinyl, triazinyl, wherein $R_a$ and $R_b$ are each independently H or C1-C4 alkyl. The alkylene may be linear or branched alkylene.

Preferably, $L_2$ may be selected from a bond, C3-C5 cycloalkyl and C1-C6 alkylene. $L_2$ may be optionally substituted with substituents selected from halogen, C1-C3 alkyl, C3-C5 cycloalkyl and hydroxyl. The number of substituents may be 1, 2 or 3. Preferred $L_2$ is optionally substituted C1-C4 alkylene. The alkylene may be linear or branched alkylene.

Preferably, $R_2$ is selected from optionally substituted 6-14 membered aryl, optionally substituted 5-10 membered heteroaryl and optionally substituted 4-10 membered heterocyclyl, more preferably optionally substituted monocyclic or bicyclic aryl, monocyclic or bicyclic heteroaromatic ring or monocyclic or bicyclic heterocyclyl. In $R_2$, the aryl is preferably phenyl or naphthyl. The heteroaryl is preferably heteroaryl having one or more (such as no more than 4 or no more than 3) nitrogen ring atoms, especially 5 or 6 membered nitrogen-containing heteroaryl, including but not limited to pyridyl, pyrrolyl, pyrazolyl, imidazolyl, triazolyl (such as 1H-1,2,3-triazolyl, 1,2,4-triazolyl, 1,2,5-triazolyl and 1,3,4-triazolyl), pyrimidinyl, pyrazinyl, triazinyl. The heteroaryl may also be a fused ring, including but not limited to indolyl, isoindolyl, indazolyl, benzopyrrolyl, benzimidazolyl, benzotriazolyl, benzoxazolyl, pyrrolopyridyl, benzisoxazolyl, quinolyl, etc. Preferred heteroaryl is pyridyl, pyrazolyl, pyrrolopyridyl, or indolyl. In $R_2$, the heterocyclyl is preferably azetidinyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, benzodihydrofuranyl, decahydroisoquinolyl, imidazolinyl, imidazolidinyl, 6,7-dihydro-5H-cyclopenta[c]pyridine, quinazinyl, thiazolidinyl, isothiazolidinyl, isoxazolidinyl, dihydroindolyl, octahydroindolyl, octahydroisoindolyl, pyrrolidinyl, pyrazolindinyl, etc.

Preferably, the number of substituents on $R_2$ may be 1, 2 or 3. The substituent is preferably selected from optionally substituted amino, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl (such as phenyl), 5-10 membered heteroaryl (preferably heteroaryl containing 1 or 2 nitrogen ring atoms, such as pyrazolyl, imidazolyl, pyridyl, pyrazinyl, piperazinyl, etc.), 4-10 membered heterocyclyl (such as oxetanyl, azetidinyl, pyrrolidinyl, tetrahydrofuranyl, piperidinyl, piperazinyl, tetrahydropyranyl, morpholinyl, etc.) and cyano, etc.; wherein optionally substituted amino may be $-NR_aR_b$, wherein $R_a$ and $R_b$ are each independently H or C1-C6 alkyl. Particularly preferred substituents on $R_2$ are C1-C6 alkoxy, halogen, C1-C6 alkyl, cyano, C3-C8 cycloalkyl-O- and halogenated C1-C6 alkoxy. Preferably, the substituents on $R_2$ at least comprise C1-C6 alkoxy, halogenated C1-C6 alkoxy or C3-C8 cycloalkyl oxy. Preferably, the number of substituents on $R_2$ is 2. Preferably, at least one of the substituents is C1-C6 alkoxy, halogenated C1-C6 alkoxy or C3-C8 cycloalkyl oxy. In some embodiments, preferably, said C1-C6 alkoxy, halogenated C1-C6 alkoxy or C3-C8 cycloalkyl oxy is at the meta position of C where $R_2$ is linked to $L_2$.

Preferably, $R_2$ is phenyl optionally substituted with 1 or 2 substituents selected from C1-C6 alkoxy, halogen, C1-C6 alkyl, cyano, C3-C8 cycloalkyl-O- and halogenated C1-C6 alkoxy.

Preferably, $R_2$ is 5-10 membered nitrogen-containing heteroaryl optionally substituted with 1 or 2 substituents selected from C1-C6 alkoxy, halogen, C1-C6 alkyl, cyano, C3-C8 cycloalkyl-O- and halogenated C1-C6 alkoxy (such as pyridyl, pyrimidinyl, pyrazolyl, pyrrolyl, imidazolyl, pyrazinyl, pyrrolopyridyl or indolyl etc.) or a 4-10 membered heterocyclyl ptionally substituted with 1 or 2 substituents selected from C1-C6 alkoxy, halogen, C1-C6 alkyl, cyano, C3-C8 cycloalkyl-O- and halogenated C1-C6 alkoxy, such as dihydrofuropyridinyl and pyrido 1,3-dioxolyl, etc.

Preferably, in Formula I, $L_2$ is C1-C4 alkylene, $R_2$ is 6-14 membered aryl (such as phenyl) optionally substituted with 1 or 2 substituents selected from C1-C6 alkoxy, halogen, C1-C6 alkyl, cyano, C3-C8 cycloalkyl-O- and halogenated C1-C6 alkoxyor 5-10 membered nitrogen-containing heteroaryl (such as pyridyl, pyrazolyl, pyrrolopyridyl, or indolyl) optionally substituted with 1 or 2 substituents selected from C1-C6 alkoxy, halogen, C1-C6 alkyl, cyano, C3-C8 cycloalkyl-O- and halogenated C1-C6 alkoxy or 4-10 membered heterocyclyl (such as dihydrofuropyridinyl and pyrido 1,3-dioxolyl) optionally substituted with 1 or 2 substituents selected from C1-C6 alkoxy, halogen, C1-C6 alkyl, cyano, C3-C8 cycloalkyl-O- and halogenated C1-C6 alkoxy.

Preferably, in some embodiments, $R_0$ is H. In some embodiments, $R_0$ is C1-C6 alkyl substituted with $-NR_3R_4$, wherein $R_3$ and $R_4$ are each independently selected from H, optionally substituted C1-C6 alkyl, optionally substituted 4-10 membered heterocyclyl and optionally substituted 5-10 membered heteroaryl. The C1-C6 alkyl may be optionally substituted with 1, 2, 3, 4, 5 or 6 substituents selected from halogen, cyano, C1-C6 alkoxy, halogenated C1-C6 alkoxy, $-NR_aR_b$ and hydroxyl, wherein $R_a$ and $R_b$ are each independently H or C1-C4 alkyl. The 4-10 membered heterocyclyls include but are not limited to nitrogen and/or oxygen containing heterocyclyl, such as oxetanyl, azetidinyl, pyrrolidinyl, tetrahydrofuranyl, piperidinyl, piperazinyl, tetrahydropyranyl, morpholinyl etc. The 5-10 membered heteroaryl includes but is not limited to 5-10 membered nitrogen and/or oxygen containing heteroaryl, such as pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl and quinolyl, etc. The 4-10 membered heterocyclyl and 5-10 membered heteroaryl may be each optionally substituted with 1, 2 or 3 substituents selected from halogen, cyano, C1-C6 alkyl, halogenated C1-C6 alkyl, C1-C6 alkoxy, halogenated C1-C6 alkoxy, $-NR_aR_b$ and hydroxyl, wherein $R_a$ and $R_b$ are each independently H or C1-C4 alkyl. Preferably, $R_3$ and $R_4$ are not said optionally substituted 4-10 membered heterocyclyl or said optionally substituted 5-10 membered heteroaryl or both at the same time.

Preferably, in some embodiments, $R_3$ and $R_4$ form optionally substituted 4-10 membered heterocyclyl or 5-10

membered heteroaryl with the nitrogen atom to which they are attached. The 4-10 membered heterocyclyl includes but is not limited to nitrogen and/or oxygen containing heterocyclyl, such as azetidinyl, pyrrolidinyl, piperidinyl, piperazinyl, morpholinyl, etc. The 5-10 membered heteroaryl includes but is not limited to 5-10 membered nitrogen and/or oxygen containing heteroaryl, such as pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl and quinolyl, etc. The 4-10 membered heterocyclyl and 5-10 membered heteroaryl may be each optionally substituted with 1, 2 or 3 substituents selected from halogen, cyano, sulfo, C1-C6 alkylsulfonyl, C1-C6 alkoxysulfonyl, C1-C6 alkylcarbonyl, halogenated C1-C6 alkylcarbonyl, C1-C6 alkoxycarbonyl, halogenated C1-C6 alkoxycarbonyl, C1-C6 alkyl, halogenated C1-C6 alkyl, C1-C6 alkoxy, halogenated C1-C6 alkoxy, $-NR_aR_b$ and hydroxyl, wherein $R_a$ and $R_b$ are each independently H or C1-C4 alkyl.

Preferably, in Formula I, $R_0$ is C1-C4 alkyl substituted with $-NR_3R_4$, wherein $R_3$ and $R_4$ are each independently selected from H, optionally substituted C1-C6 alkyl and optionally substituted 4-8 membered heterocyclyl, or $R_3$ and $R_4$ form optionally substituted 4-8 membered heterocyclyl with the nitrogen atom to which they are attached. Further preferably, said C1-C6 alkyl is optionally substituted with 1-6 substituents selected from hydroxyl, cyano and halogen, and said 4-8 membered heterocyclyls are each optionally substituted with 1, 2 or 3 substituents selected from halogen, C1-C6 alkyl, halogenated C1-C6 alkyl, C1-C6 alkoxy, halogenated C1-C6 alkoxy, sulfo, C1-C6 alkylsulfonyl, C1-C6 alkoxysulfonyl, C1-C6 alkylcarbonyl, halogenated C1-C6 alkylcarbonyl, C1-C6 alkoxycarbonyl, halogenated C1-C6 alkoxycarbonyl, $-NR_aR_b$ and hydroxyl, wherein $R_a$ and $R_b$ are each independently H or C1-C4 alkyl. Further preferably, said 4-8 membered heterocyclyls are each independently selected from oxetanyl, azetidinyl, pyrrolidinyl, tetrahydrofuranyl, piperidinyl, piperazinyl, tetrahydropyranyl and morpholinyl.

In some preferred embodiments of Formula I, ring A is C3-C6 cycloalkyl or 4-8 membered heterocyclyl, wherein said cycloalkyl or heterocyclyl may be optionally substituted with 1, 2 or 3 substituents selected from halogen, C1-C4 alkyl, halogenated C1-C4 alkyl, C1-C4 alkoxy, halogenated C1-C4 alkoxy, carbonyl substituted with $-NR_aR_b$ and C1-C4 alkyl substituted with - $NR_aR_b$; $X_3$ and $X_5$ are both CH; $X_4$ is $CH_2$; $L_1$ is C1-C4 alkylene; $R_1$ is 5 or 6 membered nitrogen-containing heteroaryl optionally substituted with 1 or 2 substituents selected from halogen, C1-C4 alkyl and $-NR_aR_b$, preferably pyridyl, pyrrolyl, pyrazolyl, imidazolyl, triazolyl, pyrimidinyl, pyrazinyl, triazinyl; $L_2$ is C1-C4 alkylene; $R_2$ is phenyl, 5-10 membered nitrogen-containing heteroaryl or 4-9 membered heterocyclyl, such as pyridyl, pyrimidinyl, pyrazolyl, pyrrolyl, imidazolyl, pyrazinyl, dihydrofuropyridinyl, pyrido 1,3-dioxolyl or indolyl, wherein said phenyl, 5-10 membered nitrogen-containing heteroaryl or 4-9 membered heterocyclyl may be optionally substituted with 1 or 2 substituents selected from C1-C6 alkoxy, halogen, C1-C6 alkyl, cyano, C3-C8 cycloalkyl-O- and halogenated C1-C6 alkoxy; $R_0$ is C1-C4 alkyl substituted with $-NR_3R_4$, wherein $R_3$ and $R_4$ are each independently selected from H, optionally substituted C1-C6 alkyl and optionally substituted 4-8 membered heterocyclyl, or $R_3$ and $R_4$ form optionally substituted 4-8 membered heterocyclyl with the nitrogen atom to which they are attached. Preferably, said C1-C6 alkyl is optionally substituted with 1-6 substituents selected from hydroxyl, cyano and halogen and said 4-8 membered heterocyclyl is each optionally substituted with 1, 2 or 3 substituents selected from halogen, C1-C6 alkyl, halogenated C1-C6 alkyl, C1-C6 alkoxy, halogenated C1-C6 alkoxy, $-NR_aR_b$ and hydroxy, wherein $R_a$ and $R_b$ are each independently H or C1-C4 alkyl, said 4-8 membered heterocyclyl is each independently selected from oxetanyl, azetidinyl, pyrrolidinyl, tetrahydrofuranyl, piperidinyl, piperazinyl, tetrahydropyranyl and morpholinyl.

In some preferred embodiments of Formula I, ring A is C3-C6 cycloalkyl, wherein said cycloalkyl may be optionally substituted with 1, 2 or 3 substituents selected from halogen, C1-C4 alkyl and halogenated C1-C4 alkyl; $X_3$ and $X_5$ are both CH; $X_4$ is $CH_2$; $L_1$ is C1-C4 alkylene; $R_1$ is 5 or 6 membered nitrogen-containing heteroaryl substituted with 1 or 2 substituents selected from C1-C4 alkyl and $-NR_aR_b$, preferably pyridyl, pyrrolyl, pyrazolyl, imidazolyl, triazolyl, pyrimidinyl, pyrazinyl, triazinyl, $R_a$ and $R_b$ are each independently H or C1-C4 alkyl; $L_2$ is C1-C4 alkylene; $R_2$ is 5-10 membered nitrogen-containing heteroaryl, such as pyridyl, pyrimidinyl, pyrazolyl, pyrrolyl, imidazolyl, pyrazinyl or indolyl, wherein said 5-10 membered nitrogen-containing heteroaryl may be optionally substituted with 1 or 2 substituents selected from C1-C6 alkoxy, C1-C6 alkyl, C3-C8 cycloalkyl-oxy, halogenated C1-C6 alkoxy, halogen and cyano; $R_0$ is C1-C4 alkyl substituted with $-NR_3R_4$, wherein $R_3$ and $R_4$ are each independently selected from H and C1-C6 alkyl optionally substituted with 1, 2, 3, 4, 5 or 6 substituents selected from hydroxyl, cyano and halogen, or $R_3$ and $R_4$ form 4-8 membered heterocyclyl optionally substituted with 1, 2 or 3 substituents selected from halogen, hydroxyl and C1-C4 alkyl with the nitrogen atom to which they are attached. Preferably, said 4-8 membered heterocyclyl is selected from oxetanyl, azetidinyl, pyrrolidinyl, tetrahydrofuranyl, piperidinyl, piperazinyl, tetrahydropyranyl and morpholinyl.

In some preferred embodiments of Formula I, the compound of Formula I has a structure represented by the following Formula II:

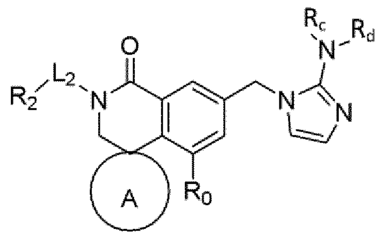

II

wherein, ring A, $R_0$, $L_2$ and $R_2$ are each as described in any embodiment herein, $R_c$ and $R_d$ are each independently H or C1-C6 alkyl. Preferably, in Formula II, ring A is C3-C6 cycloalkyl or 4-10 membered heterocyclyl, wherein said C3-C6 cycloalkyl or 4-10 membered heterocyclyl may be optionally substituted with 1, 2 or 3 substituents selected from halogen, C1-C4 alkyl, halogenated C1-C4 alkyl, C1-C4 alkoxy, halogenated C1-C4 alkoxy, carbonyl substituted with -$NR_aR_b$ and C1-C4 alkyl substituted with -$NR_aR_b$, wherein $R_a$ and $R_b$ are each independently H or C1-C4 alkyl; $R_0$ is C1-C4 alkyl substituted with -$NR_3R_4$, wherein $R_3$ and $R_4$ are each independently selected from H, optionally substituted C1-C6 alkyl and optionally substituted 4-8 membered heterocyclyl, or $R_3$ and $R_4$ form an optionally substituted 4-8 membered heterocyclyl with the nitrogen atom to which they are attached. Preferably, said C1-C6 alkyl is optionally substituted with 1-6 substituents selected from hydroxyl, cyano and halogen, and said 4-8 membered heterocyclyl is each optionally substituted with 1, 2 or 3 substituents selected from halogen, C1-C6 alkyl, halogenated C1-C6 alkyl, C1-C6 alkoxy, halogenated C1-C6 alkoxy, -$NR_aR_b$ and hydroxyl, wherein $R_a$ and $R_b$ are each independently H or C1-C4 alkyl, said 4-8 membered heterocyclyl is each independently selected from oxetanyl, azetidinyl, pyrrolidinyl, tetrahydrofuranyl, piperidinyl, piperazinyl, tetrahydropyranyl and morpholinyl; $L_2$ is C1-C4 alkylene; $R_2$ is phenyl, 5-10 membered nitrogen-containing heteroaryl or 4-10 membered heterocyclyl, such as pyridyl, pyrimidinyl, pyrazolyl, pyrrolyl, imidazolyl, pyrazinyl, pyrrolopyridyl, dihydrofuropyridinyl, pyrido 1,3-dioxolyl or indolyl, wherein said phenyl or 5-10 membered nitrogen-containing heteroaryl may be optionally substituted with 1 or 2 substituents selected from C1-C6 alkoxy, halogen, C1-C6 alkyl, cyano, C3-C8 cycloalkyl-O- and halogenated C1-C6 alkoxy; and said $R_c$ and $R_d$ are each independently H or C1-C4 alkyl.

Further preferably, in Formula II, ring A is C3-C6 cycloalkyl, wherein said cycloalkyl may be optionally substituted with 1, 2 or 3 substituents selected from halogen, C1-C4 alkyl and halogenated C1-C4 alkyl; $L_2$ is C1-C4 alkylene; $R_2$ is 5-10 membered nitrogen-containing heteroaryl, such as pyridyl, pyrimidinyl, pyrazolyl, pyrrolyl, imidazolyl, pyrazinyl or indolyl, wherein said 5-10 membered nitrogen-containing heteroaryl may be optionally substituted with 1 or 2 substituents selected from C1-C6 alkoxy, halogen and cyano; $R_0$ is C1-C4 alkyl substituted with -$NR_3R_4$, wherein $R_3$ and $R_4$ are each independently selected from H and C1-C6 alkyl, or $R_3$ and $R_4$ form a 4-8 membered heterocyclyl with the nitrogen atom to which they are attached. Preferably, said 4-8 membered heterocyclyl is selected from azetidinyl, pyrrolidinyl, piperidinyl, piperazinyl and morpholinyl; $R_c$ and $R_d$ are each independently H or C1-C4 alkyl.

Further preferably, in Formula II, ring A is C3-C6 cycloalkyl or C4-C6 heterocyclyl containing one nitrogen or one O atom that is optionally substituted with substituents selected from C1-C4 alkyl, carbonyl substituted with -$NR_aR_b$ and C1-C4 alkyl substituted with -$NR_aR_b$, preferably cyclopropyl, cyclobutyl, oxetanyl or azetidinyl, wherein said cyclopropyl, cyclobutyl, oxetanyl or azetidinyl is optionally substituted with C1-C4 alkyl, carbonyl substituted with - $NR_aR_b$ and C1-C4 alkyl substituted with -$NR_aR_b$. $R_0$ is H or C1-C4 alkyl substituted with -$NR_3R_4$, wherein $R_3$ and $R_4$ are each independently selected from H, 4-6 membered heterocyclyl and C1-C6 alkyl optionally substituted with 1, 2, 3, 4, 5 or 6 substituents selected from hydroxyl, cyano and halogen, or $R_3$ and $R_4$ form a 4-6 membered heterocyclyl optionally substituted with 1, 2 or 3 substituents selected from halogen, hydroxyl and C1-C4 alkyl with the nitrogen atom to which they are attached. Preferably, said 4-6 membered heterocyclyl is each independently oxygen and/or nitrogen containing 4-6 membered heterocyclyl, preferably each independently selected from oxetanyl, azetidinyl, pyrrolidinyl, tetrahydrofuranyl, piperidinyl, piperazinyl, tetrahydropyranyl and morpholinyl. $L_2$ is C1-C4 alkylene. $R_2$ is 6-14 membered aryl, 5-10 membered nitrogen-containing heteroaryl or 4-10 membered heterocyclyl, such as phenyl, pyridyl, pyrimidinyl, pyrazolyl, pyrrolyl, imidazolyl, pyrazinyl, indolyl, pyrrolopyridyl, dihydrofuropyridinyl or pyrido 1,3-dioxolyl, wherein said 6-14 membered aryl, 5-10 membered nitrogen-containing heteroaryl or 4-10 membered heterocyclyl is optionally substituted with 1 or 2 substituents selected from C1-C6 alkoxy, C1-C4 alkyl, halogen, C3-C6 cycloalkyl-oxy, halogenated C1-C4 alkoxy and cyano. $R_c$ and $R_d$ are each independently H or C1-C4 alkyl.

In some preferred embodiments of Formula I, the compound of Formula I has a structure represented by the following Formula III:

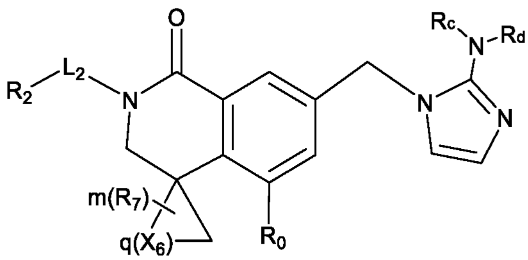

III

wherein ring A, $R_0$, $L_2$ and $R_2$ are each as described in any embodiment herein, $R_c$ and $R_d$ are each independently H or C1-C4 alkyl. $X_6$ is each independently selected from the group consisting of C, O, N, S, q is an integer of 1, 2 or 3. Preferably, $X_6$ is C. Preferably, q is 1 or 2. Preferably, $R_7$ is selected from F, Cl, cyano, hydroxyl, C1-C6 alkyl, fluorinated or chlorinated C1-C6 alkyl, hydroxyl-substituted C1-C6 alkyl, C1-C6 alkoxy, fluorinated or chlorinated C1-C6 alkoxy, hydroxyl-substituted C1-C6 alkyl, $-NR_aR_b$, carbonyl substituted with $-NR_aR_b$, C1-C6 alkyl substituted with $-NR_aR_b$, C1-C6 alkylcarbonyl, and C1-C6 alkoxycarbonyl; wherein said $R_a$ and $R_b$ are each independently selected from H and C1-C6 alkyl, m is an integer of 0, 1, 2 or 3; preferably, m is 0 or 2.

In another aspect according to the present disclosure, the compound of Formula I, or a pharmaceutically acceptable salt thereof, or an enantiomer, a diastereoisomer, a tautomer, an isotope substitute, a solvate, a polymorph, a prodrug or metabolite thereof is provided:

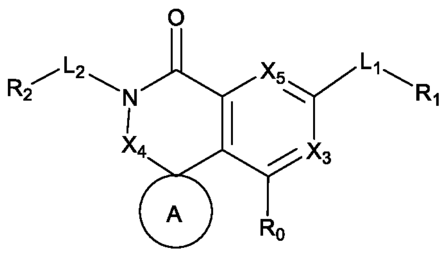

Formula I

wherein,

ring A is a substituted or unsubstituted 3-8 membered saturated carbocycle or a substituted or unsubstituted 3-8 membered saturated heterocycle, wherein said heterocycle contains 1-2 hetero atoms selected from N, O and S;
$X_3$ is selected from $CR_5$ and N;
$X_4$ is selected from optionally substituted $-(CH_2)_n-$, $-C(=O)-$ and $NR_6$, wherein n is 1, 2 or 3;
$X_5$ is selected from N and $CR_5$;
$L_1$ is selected from a bond, optionally substituted C1-C6 alkylene, optionally substituted C1-C6 alkylene-O-, optionally substituted C1-C6 alkylene-NH-, optionally substituted C1-C6 alkylene-S- and optionally substituted C2-C4 alkenylene;
$L_2$ is selected from a bond, optionally substituted C1-C6 alkylene, optionally substituted C1-C6 alkylene-O-, optionally substituted C1-C6 alkylene-NH-, optionally substituted C1-C6 alkylene-S- and optionally substituted C2-C4 alkenylene;
$R_0$ is selected from optionally substituted C1-C6 haloalkyl, $-C(O)NR_3R_4$, $-CH_2OR_3$ and C1-C6 alkyl substituted with $-NR_3R_4$ ;
$R_1$ is selected from optionally substituted cycloalkyl, optionally substituted aryl, optionally substituted heteroaryl, optionally substituted heterocyclyl, optionally substituted amino and guanidyl;
$R_2$ is selected from optionally substituted cycloalkyl, optionally substituted aryl, optionally substituted heteroaryl and optionally substituted heterocyclyl;
$R_3$ and $R_4$ are each independently selected from H, optionally substituted C1-C6 alkyl, optionally substituted C1-C6 alkoxy, optionally substituted C1-C6 haloalkyl, optionally substituted 4-10 membered heterocyclyl and optionally substituted 5-10 membered heteroaryl; or $R_3$ and $R_4$ form an optionally substituted 4-10 membered heterocyclyl or 5-10 membered heteroaryl with the nitrogen atom to which they are attached;
$R_5$ is selected from H, halogen, C1-C6 alkyl, halogenated C1-C6 alkyl and C1-C6 alkoxy; and

$R_6$ is selected from H, C1-C6 alkyl, halogenated C1-C6 alkyl and C1-C6 alkoxy.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is C3-C8 cycloalkyl or 3-7 membered heterocyclyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is selected from cyclopropyl, cyclobutyl, cyclopentyl, cyclohexyl, aziridinyl, azetidinyl, pyrrolidinyl, piperidinyl, piperazinyl, oxiranyl, oxetanyl, tetrahydrofuranyl, tetrahydropyranyl and morpholinyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is substituted with 1, 2 or 3 substituents selected from halogen, hydroxyl, cyano, C1-C6 alkyl, halogenated C1-C6 alkyl, hydroxyl-substituted C1-C6 alkyl, C1-C6 alkoxy, halogenated C1-C6 alkoxy and $-NR_aR_b$; wherein said $R_a$ and $R_b$ are each independently selected from Hand C1-C6 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is cyclopropyl, cyclobutyl, cyclopentyl, azetidinyl, oxetanyl, pyrrolidinyl or tetrahydrofuranyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is optionally substituted with 1, 2 or 3 substituents selected from the group consisting of F, Cl, Br, $CF_3$, $CH_3$, -OH, $-OCH_3$, $-NH_2$, $-NHCH_3$, $-NH_2(CH_3)_2$.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_0$ is C1-C6 alkyl substituted with $-NR_3R_4$; wherein $R_3$ and $R_4$ are each independently selected from H, C1-C6 alkyl optionally substituted with 1, 2, 3, 4, 5 or 6 substituents selected from halogen, cyano, C1-C6 alkoxy, halogenated C1-C6 alkoxy, $-NR_aR_b$ and hydroxyl, or $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

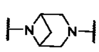 or 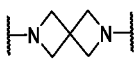

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

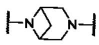 or 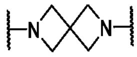

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, sulfo, C1-C6 alkylsulfonyl, C1-C6 alkoxysulfonyl, C1-C6 alkylcarbonyl, halogenated C1-C6 alkylcarbonyl, C1-C6 alkoxycarbonyl, halogenated C1-C6 alkoxycarbonyl, $-NR_aR_b$ and hydroxyl, wherein $R_a$ and $R_b$ are each independently H or C1-C4 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_0$ is C1-C6 alkyl substituted with $-NR_3R_4$, wherein $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

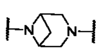 or 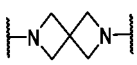

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

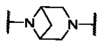 or 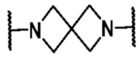

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, sulfo, C1-C6 alkylsulfonyl, C1-C6 alkoxysulfonyl, C1-C6 alkylcarbonyl, halogenated C1-C6 alkylcarbonyl, C1-C6 alkoxycarbonyl, halogenated C1-C6 alkoxycarbonyl, $-NR_aR_b$ and hydroxyl; wherein $R_a$ and $R_b$ are each independently H or C1-C4 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_0$ is C1-C6 alkyl substituted with $-NR_3R_4$, wherein $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

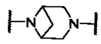 or 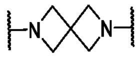

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

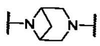 or 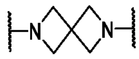

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, $-NR_aR_b$ and hydroxyl; wherein $R_a$ and $R_b$ are each independently H or C1-C4 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $X_3$ and/or $X_5$ is $CR_5$.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_5$ is selected from H and C1-C4 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $X_4$ is $-(CH_2)_n-$, n is 1 or 2; wherein said $-(CH_2)_n-$ is optionally substituted with 1 or 2 substituents selected from halogen, hydroxyl, amino, C1-C6 alkyl and halogenated C1-C6 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $X_3$ and $X_5$ are both $CR_5$; and $X_4$ is optionally substituted $-(CH_2)-$.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $L_1$ is selected from C1-C6 alkylene, C1-C6 alkylene-O-, C1-C6 alkylene-NH-, C1-C6 alkylene-S- and C2-C4 alkenylene, wherein said C1-C6 alkylene, C1-C6 alkylene-O-, C1-C6 alkylene-NH-, C1-C6 alkylene-S- and C2-C4 alkenylene may be optionally substituted with 1, 2 or 3 substituents selected from halogen, C1-C4 alkyl and hydroxyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $L_1$ is selected from C1-C6 alkylene and C1-C6 alkylene-NH-, wherein said C1-C6 alkylene and C1-C6 alkylene-NH- are optionally substituted with 1, 2 or 3 substituents selected from halogen, C1-C4 alkyl and hydroxyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_1$ is selected from optionally substituted C3-C8 cycloalkyl, optionally substituted 6-14 membered aryl, optionally substituted 5-10 membered heteroaryl and optionally substituted 4-10 membered heterocyclyl, wherein said cycloalkyl, aryl, heteroaryl and heterocyclyl are each optionally substituted with 1, 2 or 3 substituents selected from optionally substituted amino, C1-C6 alkyl, halogen, hydroxyl, C1-C6 alkoxy, C1-C6 haloalkyl and cyano; wherein said optionally substituted amino is $-NR_aR_b$, wherein $R_a$ and $R_b$ are each independently H or C1-C6 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_1$ is selected from optionally substituted phenyl, naphthyl pyridyl, pyrrolyl, pyrazolyl, imidazolyl, triazolyl, pyrimidinyl, pyrazinyl, triazinyl, imidazopyridinyl, indolyl, isoindolyl, indazolyl, benzimidazolyl, benzotriazolyl, benzoxazolyl, benzisoxazolyl, quinolyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, decahydroisoquinolyl, imidazolinyl, imidazolidinyl, quinazinyl, thiazolidinyl, isothiazolidinyl, isoxazolidinyl, dihydroindolyl, octahydroindolyl, octahydroisoindolyl, pyrrolidinyl, pyrazolindinyl, phthalimidinyl,

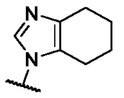
and
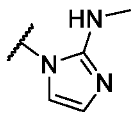
.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $L_1$ is C1-C4 alkylene or C1-C4 alkylene-NH-, $R_1$ is pyridyl, pyrrolyl, pyrazolyl, imidazolyl, triazolyl, pyrimidinyl, pyrazinyl, triazinyl or

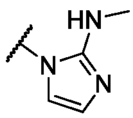
,

wherein said pyridyl, pyrrolyl, pyrazolyl, imidazolyl, triazolyl, pyrimidinyl, pyrazinyl, triazinyl or

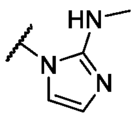

is optionally substituted with 1 or 2 substituents selected from halogen, C1-C4 alkyl and $-NR_aR_b$, wherein $R_a$ and $R_b$ are each independently H or C1-C4 alkyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $L_2$ is selected from a bond and C1-C6 alkylene, wherein said C1-C6 alkylene is optionally substituted with 1, 2 or 3 substituents selected from halogen, C1-C3 alkyl, C3-C5 cycloalkyl and hydroxyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_2$ is optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano; wherein $R_a$ and $R_b$ are each independently H or C1-C6 alkyl; wherein said heteroaryl is preferably a heteroaryl containing 1 or 2 nitrogen ring atoms, preferably selected from pyrazolyl, imidazolyl, pyridyl, pyrazinyl and piperazinyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_2$ is selected from 6-14 membered aryl, 5-10 membered heteroaryl and 4-10 membered heterocyclyl, wherein said 6-14 membered aryl, 5-10 membered heteroaryl and 4-10 membered heterocyclyl may be optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $R_2$ is selected from phenyl, naphthyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl, azetidinyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, benzodihydrofuranyl, decahydroisoquinolyl, imidazolinyl, imidazolidinyl, 6,7-dihydro-5H-cyclopenta[c]pyridine, quinazinyl, thiazolidinyl, isothiazolidinyl, isoxazolidinyl, dihydroindolyl, octahydroindolyl, octahydroisoindolyl, pyrrolidinyl, pyrazolindinyl,

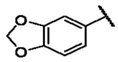 and 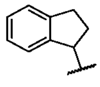,

wherein said phenyl, naphthyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl, azetidinyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, benzodihydrofuranyl, decahydroisoquinolyl, imidazolinyl, imidazolidinyl, 6,7-dihydro-5H-cyclopenta[c]pyridine, quinazinyl, thiazolidinyl, isothiazolidinyl, isoxazolidinyl, dihydroindolyl, octahydroindolyl, octahydroisoindolyl, pyrrolidinyl, pyrazolindinyl,

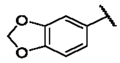

and

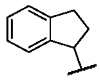

are optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, $L_2$ is C1-C4 alkylene, $R_2$ is 6-14 membered aryl, 5-10 membered nitrogen-containing heteroaryl or 4-10 membered heterocyclyl, wherein said 6-14 membered aryl, 5-10 membered nitrogen-containing heteroaryl or 4-10 membered heterocyclyl is optionally substituted with 1 or 2 substituents selected from C1-C6 alkoxy, halogen, C1-C6 alkyl, cyano, C3-C8 cycloalkyl-O- and halogenated C1-C6 alkoxy, preferably phenyl, pyridyl, pyrazolyl, pyrrolopyridyl, dihydrofuropyridinyl, pyrido 1,3-dioxolyl and indolyl.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is C3-C6 cycloalkyl or 4-8 membered heterocyclyl, wherein said cycloalkyl or heterocyclyl is optionally substituted with 1, 2 or 3 substituents selected from halogen, C1-C4 alkyl, halogenated C1-C4 alkyl, C1-C4 alkoxy and halogenated C1-C4 alkoxy;

$X_3$ and $X_5$ are both CH;
$X_4$ is $CH_2$;
$L_1$ is C1-C4 alkylene;
$R_1$ is

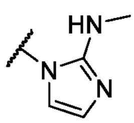;

$L_2$ is C1-C4 alkylene;
$R_2$ is phenyl, 5-10 membered nitrogen-containing heteroaryl or 4-10 membered heterocyclyl, preferably pyridyl, pyrimidinyl, pyrazolyl, pyrrolyl, imidazolyl, pyrazinyl, pyrrolopyridyl, dihydrofuropyridinyl or pyrido 1,3-dioxolyl or indolyl, wherein said phenyl, 5-10 membered nitrogen-containing heteroaryl and 4-10 membered heterocyclyl are each optionally substituted with 1 or 2 substituents selected from C1-C6 alkoxy, halogen, C1-C6 alkyl, cyano, C3-C8 cycloalkyl-O- and halogenated C1-C6 alkoxy;
$R_0$ is C1-C4 alkyl substituted with $-NR_3R_4$; wherein $R_3$ and $R_4$ are each independently selected from H, C1-C6 alkyl

optionally substituted with 1-6 substituents selected from hydroxyl, cyano and halogen, and a 4-8 membered heterocyclyl optionally substituted with 1, 2 or 3 substituents selected from halogen, C1-C6 alkyl, C3-C6 cycloalkyl, halogenated C1-C6 alkyl, C1-C6 alkoxy, halogenated C1-C6 alkoxy, $-NR_aR_b$ and hydroxyl, wherein $R_a$ and $R_b$ are each independently H or C1-C4 alkyl; or $R_3$ and $R_4$ form a 4-8 membered heterocyclyl optionally substituted with 1, 2 or 3 substituents selected from halogen, C1-C6 alkyl, halogenated C1-C6 alkyl, C1-C6 alkoxy, halogenated C1-C6 alkoxy, $-NR_aR_b$ and hydroxyl with the nitrogen atom to which they are attached, wherein $R_a$ and $R_b$ are each independently H or C1-C4 alkyl; preferably, said 4-8 membered heterocyclyl is each independently selected from the group consisting of oxetanyl, azetidinyl, pyrrolidinyl, tetrahydrofuranyl, piperidinyl, piperazinyl, tetrahydropyranyl, morpholinyl,

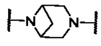 or 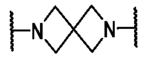.

In particular, in the compound of Formula I according to the present disclosure, or a pharmaceutically acceptable salt thereof, or an enantiomer, a diastereoisomer, a tautomer, isotope substitute, solvate, a polymorph, a prodrug or metabolite thereof, ring A is C3-C6 cycloalkyl, wherein said cycloalkyl is optionally substituted with 1, 2 or 3 substituents selected from halogen, C1-C4 alkyl and halogenated C1-C4 alkyl;

$X_3$ and $X_5$ are both CH;
$X_4$ is $CH_2$;
$L_1$ is C1-C4 alkylene;
$R_1$ is

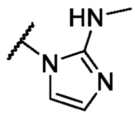;

$L_2$ is C1-C4 alkylene;
$R_2$ is 5-10 membered nitrogen-containing heteroaryl or 4-10 membered heterocyclyl, preferably pyridyl, pyrimidinyl, pyrazolyl, pyrrolyl, imidazolyl, pyrazinyl, pyrrolopyridyl, dihydrofuropyridinyl or pyrido 1,3-dioxolyl or indolyl, wherein said 5-10 membered nitrogen-containing heteroaryl or 4-10 membered heterocyclyl is each optionally substituted with 1 or 2 substituents selected from C1-C6 alkoxy, C1-C6 alkyl, C3-C8 cycloalkyl-O-, halogenated C1-C6 alkoxy, halogen and cyano;
$R_0$ is C1-C4 alkyl substituted with $-NR_3R_4$, wherein $R_3$ and $R_4$ are each independently selected from Hand C1-C6 alkyl optionally substituted with 1, 2, 3, 4, 5 or 6 substituents selected from hydroxyl, cyano and halogen, or $R_3$ $R_4$ form a 4-8 membered heterocyclyl optionally substituted with 1, 2 or 3 substituents selected from halogen, hydroxyl, C1-C4 alkoxy and C1-C4 alky with the nitrogen atom to which they are attached, preferably, said 4-8 membered heterocyclyl is selected from azetidinyl, pyrrolidinyl, piperidinyl, piperazinyl, morpholinyl,

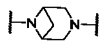 and 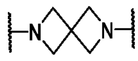.

Exemplary compounds of Formula I according to the present disclosure include but are not limited to the following compounds and a pharmaceutically acceptable salt thereof, or an enantiomer, a diastereoisomer, a tautomer, isotope substitute, solvate, a polymorph, a prodrug or metabolite thereof:

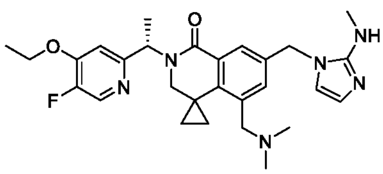, 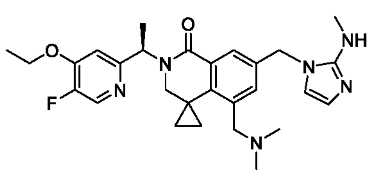, 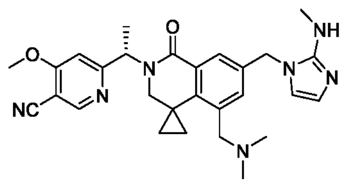,

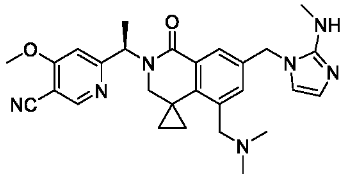,
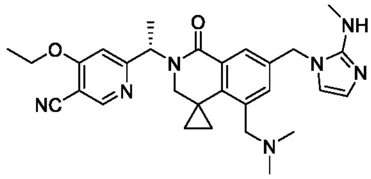,
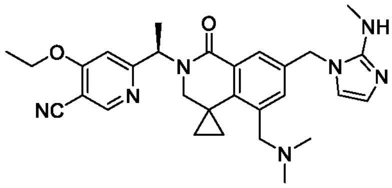,

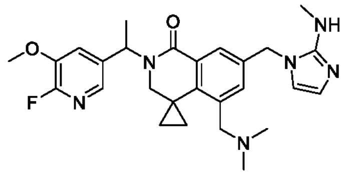,
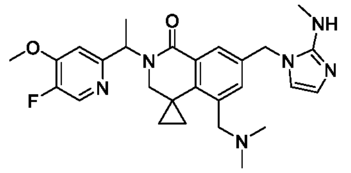,
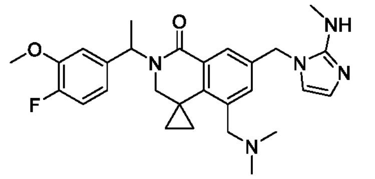,

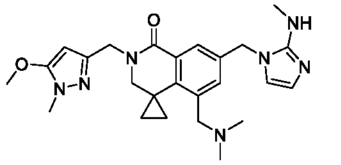,
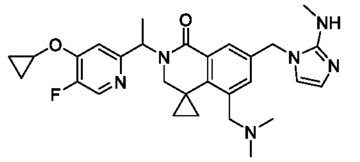,
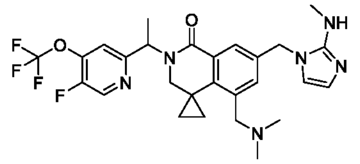,

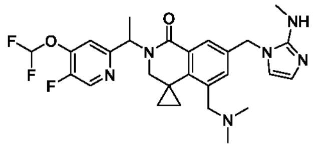,
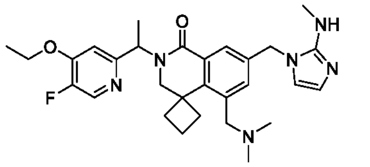,
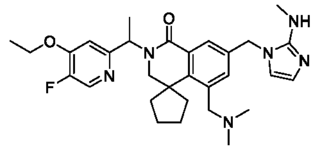,

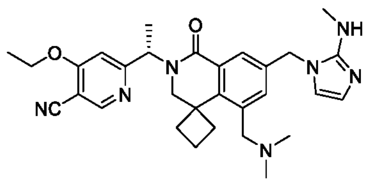,
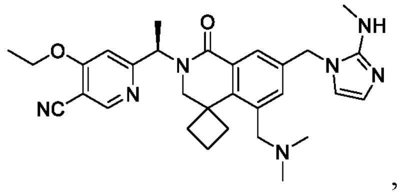,
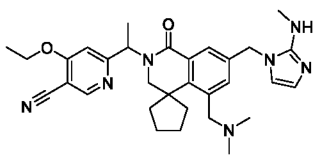,

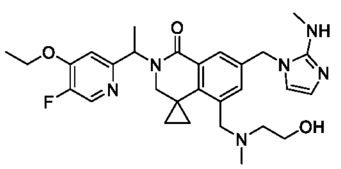,
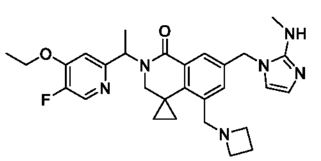,
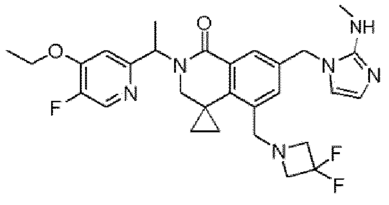,

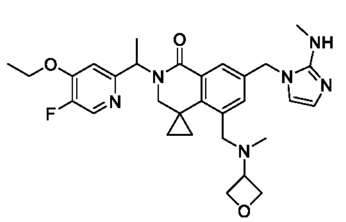,
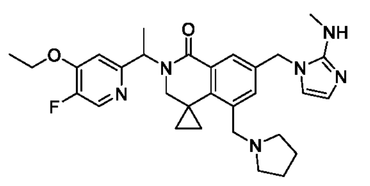,
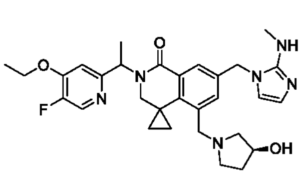,

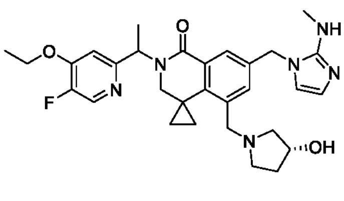,
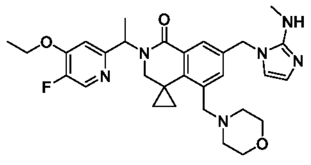,
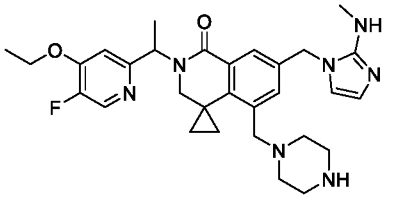,

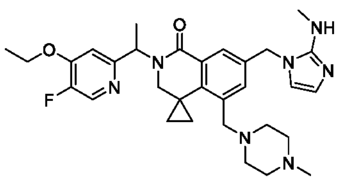,
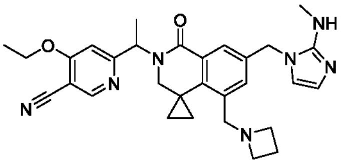,
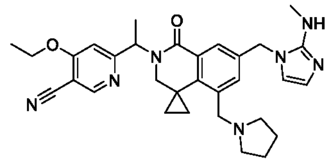,

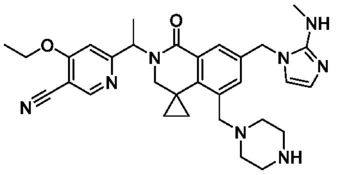,
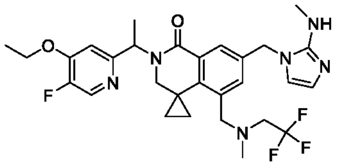,
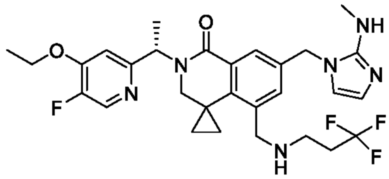,

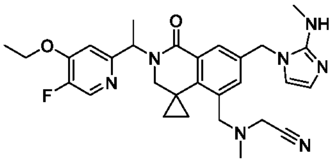,
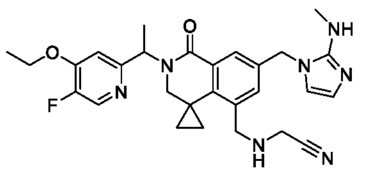,
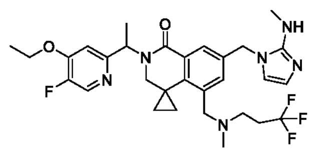,

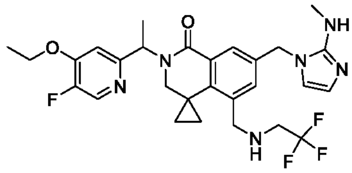,
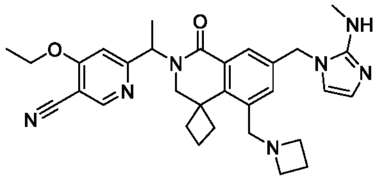,
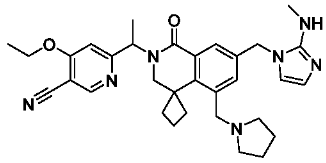,

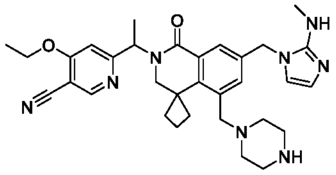,
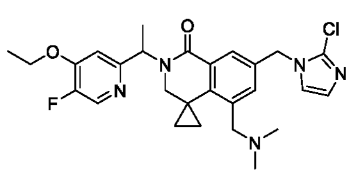,
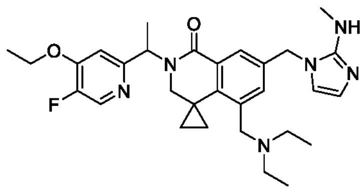,

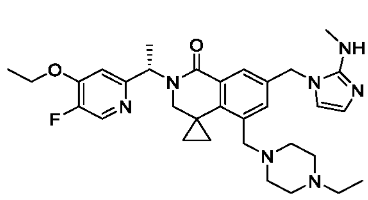,
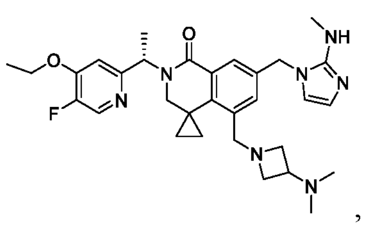,
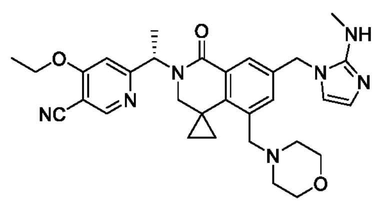,

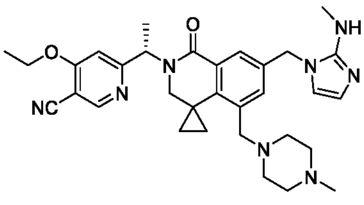, 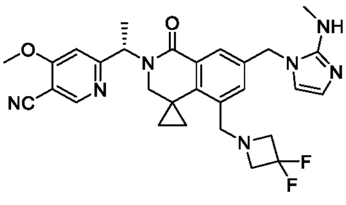, 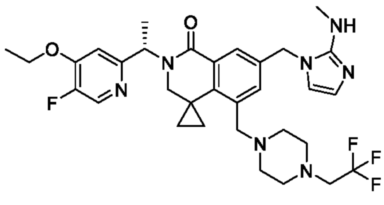,

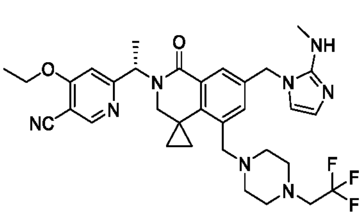, 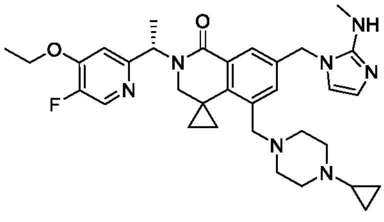, 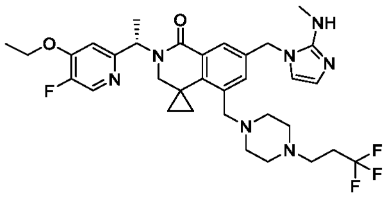,

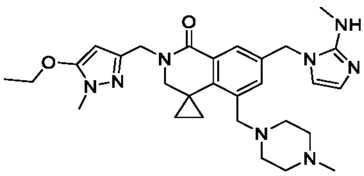, 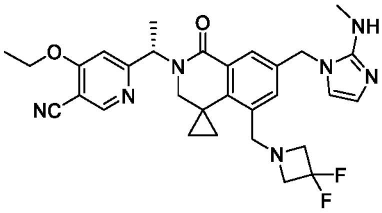, 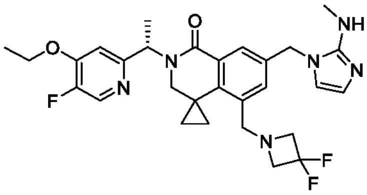,

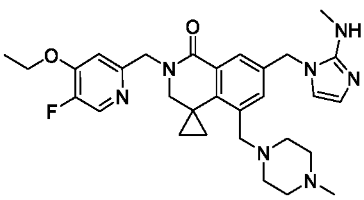, 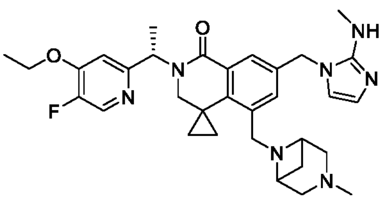, 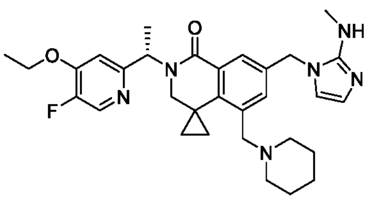,

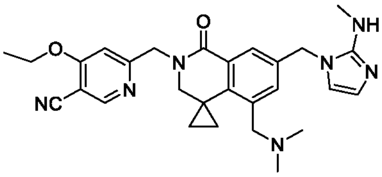, 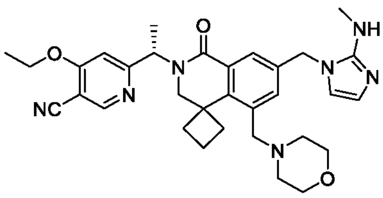, 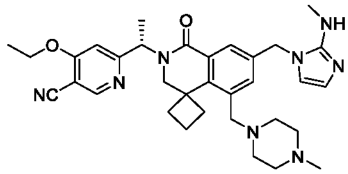,

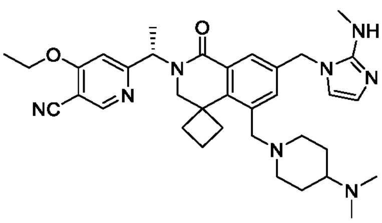, 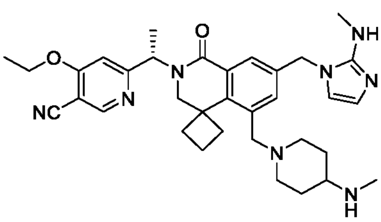, 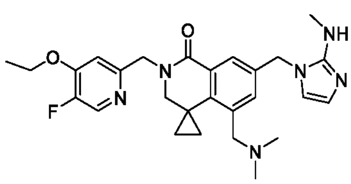,

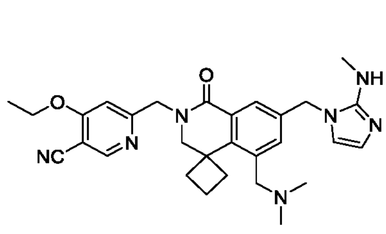 , 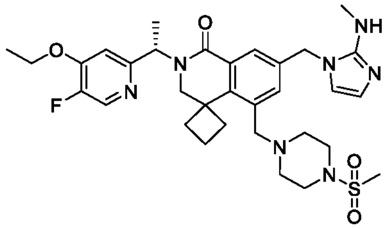 , 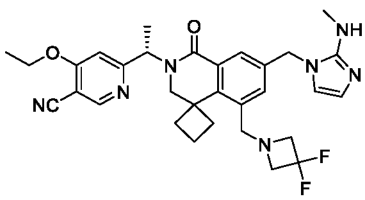 ,

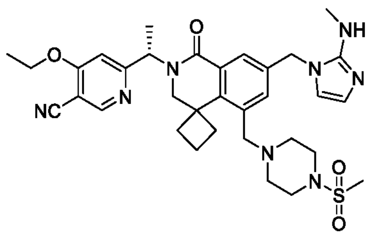 , 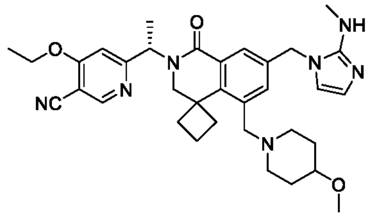 , 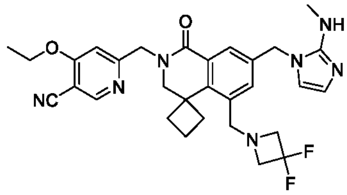 ,

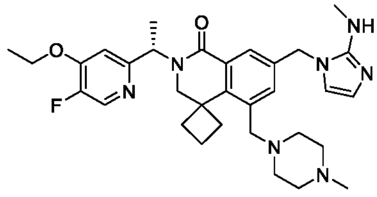 , 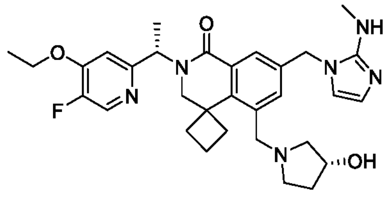 , 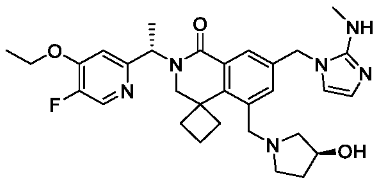 ,

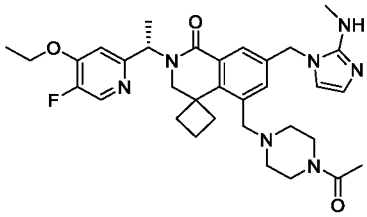 , 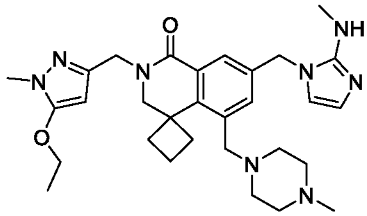 , 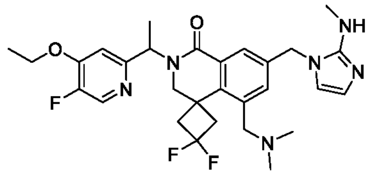 ,

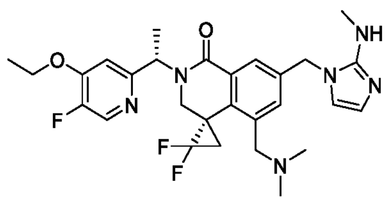 , 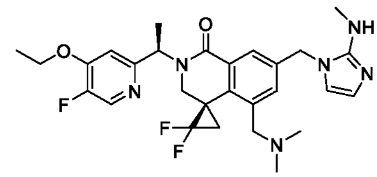 , 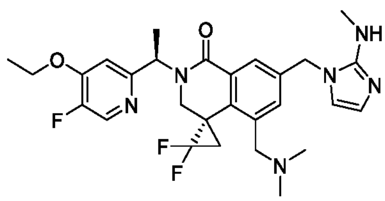 ,

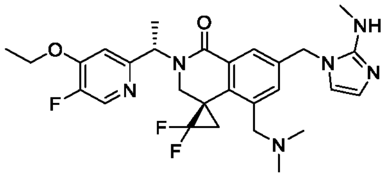 , 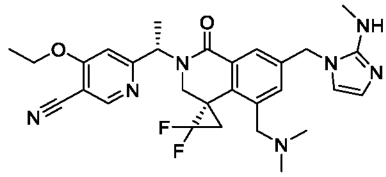 , 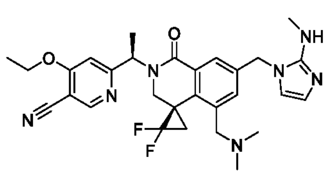 ,

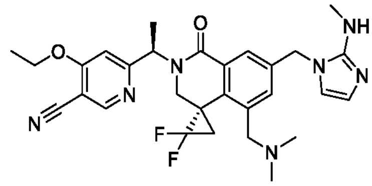 , 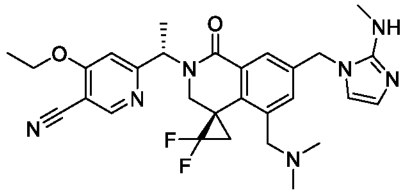 , 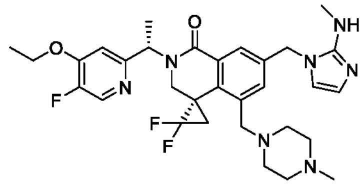 ,

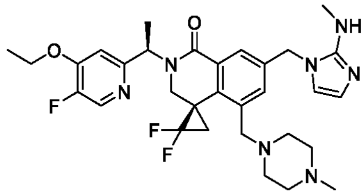, 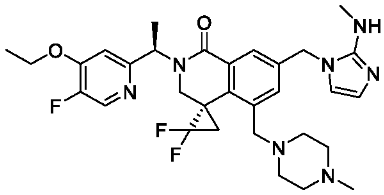, 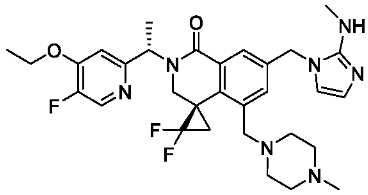,

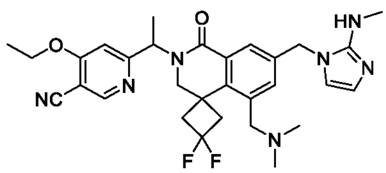, 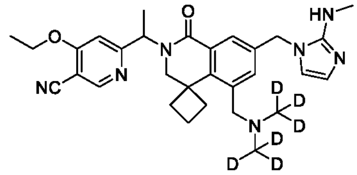, 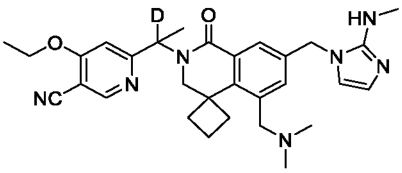,

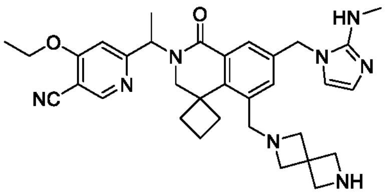, 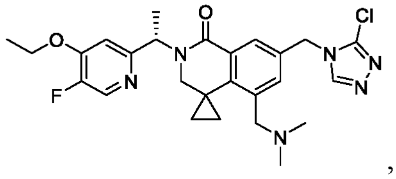, 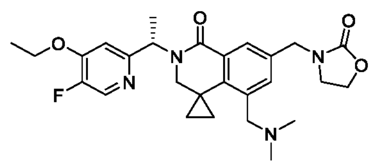,

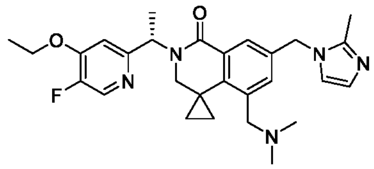, 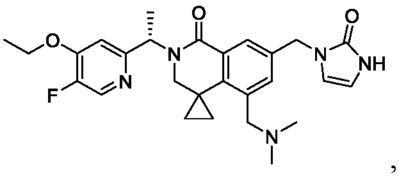, 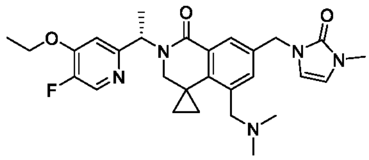,

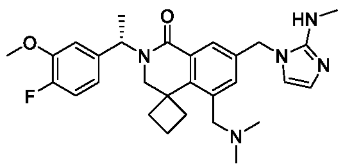, 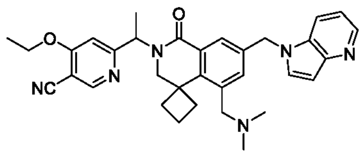, 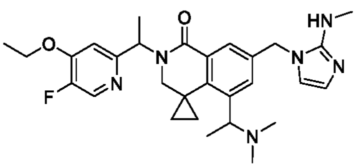,

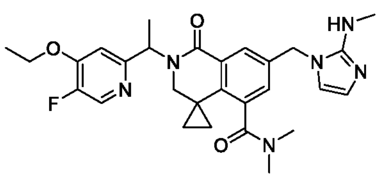, 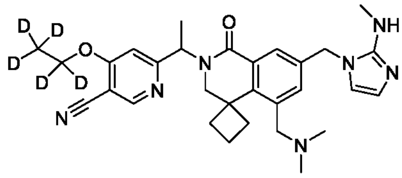, 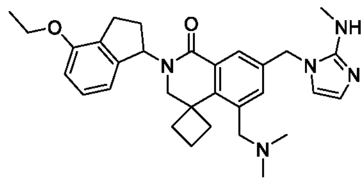,

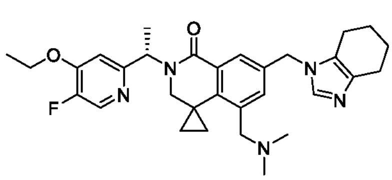, 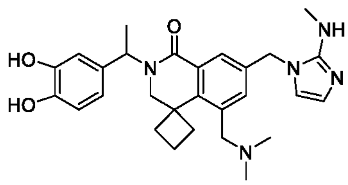, 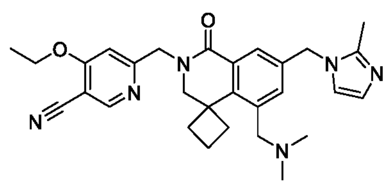,

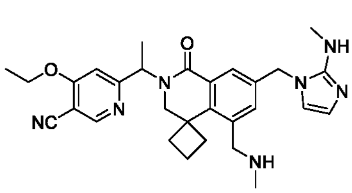,
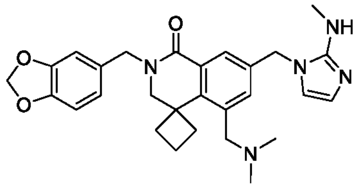,
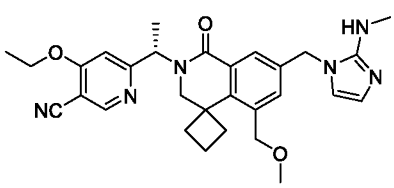,

and

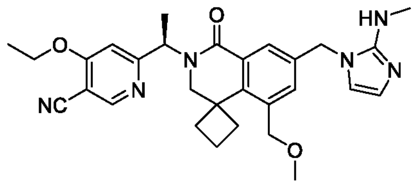.

It should be understood by the person skilled in the art that the present disclosure provides the embodiments including but not limited to those listed below:

Embodiment 1. A compound of Formula I, or a pharmaceutically acceptable salt thereof, or an enantiomer, a diastereoisomer, a tautomer, an isotope substitute, a solvate, a polymorph, a prodrug or metabolite thereof:

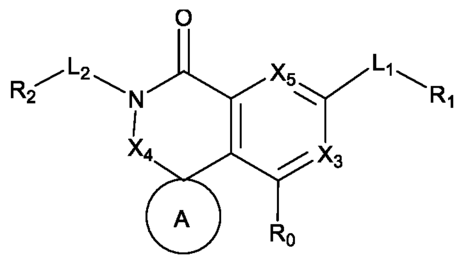

Formula I

wherein,

ring A is a substituted or unsubstituted 3-8 membered saturated or unsaturated carboncycle or a substituted or unsubstituted 3-8 membered saturated or unsaturated heterocycle, wherein said heterocycle contains 1-2 hetero atoms selected from N, O and S;
$X_3$ is selected from $CR_5$ and N;
$X_4$ is selected from optionally substituted $-(CH_2)_n-$, -C(=O)- and $NR_6$, wherein n is 1, 2 or 3;
$X_5$ is selected from N and $CR_5$;
$L_1$ is selected from a bond, optionally substituted C1-C6 alkylene, optionally substituted C1-C6 alkylene-O-, optionally substituted C1-C6 alkylene-NH-, optionally substituted C1-C6 alkylene-S- and optionally substituted C2-C4 alkenylene;
$L_2$ is selected from a bond, optionally substituted C1-C6 alkylene, optionally substituted C1-C6 alkylene-O-, optionally substituted C1-C6 alkylene-NH-, optionally substituted C1-C6 alkylene-S- and optionally substituted C2-C4 alkenylene;
$R_0$ is selected from H, C1-C6 alkyl, optionally substituted C1-C6 alkoxy, optionally substituted C1-C6 haloalkyl and C1-C6 alkyl substituted with $-NR_3R_4$;
$R_1$ is selected from optionally substituted cycloalkyl, optionally substituted aryl, optionally substituted heteroaryl, optionally substituted heterocyclyl, optionally substituted amino and guanidyl;
$R_2$ is selected from optionally substituted cycloalkyl, optionally substituted aryl, optionally substituted heteroaryl and optionally substituted heterocyclyl;
$R_3$ and $R_4$ are each independently selected from H, optionally substituted C1-C6 alkyl, optionally substituted C1-C6 alkoxy, optionally substituted C1-C6 haloalkyl, optionally substituted 4-10 membered heterocyclyl and optionally substituted 5-10 membered heteroaryl; or $R_3$ and $R_4$ form optionally substituted 4-10 membered heterocyclyl or 5-10 membered heteroaryl with the nitrogen atom to which they are attached;

$R_5$ is selected from H, halogen, C1-C6 alkyl, halogenated C1-C6 alkyl and C1-C6 alkoxy;
$R_6$ is selected from H, C1-C6 alkyl, halogenated C1-C6 alkyl and C1-C6 alkoxy.

Embodiment 2. The compound of Formula I according to Embodiment 1, or a pharmaceutically acceptable salt thereof, or an enantiomer, a diastereoisomer, a tautomer, an isotope substitute, a solvate, a polymorph, a prodrug or metabolite thereof, wherein ring A is C3-C8 cycloalkyl or 3-7 membered heterocyclyl, preferably selected from cyclopropyl, cyclobutyl, cyclopentyl, cyclohexyl, aziridinyl, azetidinyl, pyrrolidinyl, piperidinyl, piperazinyl, oxiranyl, oxetanyl, tetrahydrofuranyl, tetrahydropyranyl and morpholinyl;
preferably, ring A is optionally substituted with 1, 2 or 3 substituents selected from halogen, hydroxyl, cyano, C1-C6 alkyl, halogenated C1-C6 alkyl, hydroxyl-substituted C1-C6 alkyl, C1-C6 alkoxy, halogenated C1-C6 alkoxy, $-NR_aR_b$, carbonyl substituted with $-NR_aR_b$, C1-C6 alkyl substituted with $-NR_aR_b$, C1-C6 alkylcarbonyl, C1-C6 alkoxycarbonyl, optionally substituted 6-14 membered aryl, optionally substituted 5-10 membered heteroaryl, optionally substituted 4-10 membered heterocyclyl, optionally substituted C3-C8 cycloalkyl, C1-C6 alkyl optionally substituted with 6-14 membered aryl, C1-C6 alkyl optionally substituted with 5-10 membered heteroaryl, C1-C6 alkyl optionally substituted with 4-10 membered heterocyclyl, and C1-C6 alkyl optionally substituted with C3-C8 cycloalkyl; wherein said 6-14 membered aryl, 5-10 membered heteroaryl, 4-10 membered heterocyclyl and C3-C8 cycloalkyl are substituted, the substituent is 1, 2 or 3 substituents selected from halogen, C1-C6 alkyl, halogenated C1-C6 alkyl, C1-C6 alkoxy, halogenated C1-C6 alkoxy, $-NR_aR_b$, carbonyl substituted with $-NR_aR_b$, C1-C6 alkyl substituted with $-NR_aR_b$, C1-C6 alkylcarbonyl, and C1-C6 alkoxycarbonyl; wherein said $R_a$ and $R_b$ are each independently selected from H and C1-C6 alkyl.
Embodiment 3. The compound of Formula I according to Embodiment 1 or 2, or a pharmaceutically acceptable salt thereof, or an enantiomer, a diastereoisomer, a tautomer, an isotope substitute, a solvate, a polymorph, a prodrug or metabolite thereof, wherein ring A is cyclopropyl, cyclobutyl, cyclopentyl, azetidinyl, oxetanyl, pyrrolidinyl or tetrahydrofuranyl,
preferably, ring A is optionally substituted with 1, 2 or 3 substituents selected from F, Cl, Br, $CF_3$, $CH_3$, -OH, $-OCH_3$, $-NH_2$, $-NHCH_3$, and $-NH_2(CH_3)_2$.
Embodiment 4. The compound of Formula I according to Embodiment 1, or a pharmaceutically acceptable salt thereof, or an enantiomer, a diastereoisomer, a tautomer, an isotope substitute, a solvate, a polymorph, a prodrug or metabolite thereof, wherein

$X_3$ is $CR_5$, $R_5$ is selected from H and C1-C4 alkyl; and/or
$X_4$ is $-(CH_2)_n-$, n is 1 or 2; wherein said $-(CH_2)_n-$ is optionally substituted with 1 or 2 substituents selected from halogen, hydroxyl, amino, C1-C6 alkyl and halogenated C1-C6 alkyl.

Embodiment 5. The compound of Formula I according to Embodiment 1, or a pharmaceutically acceptable salt thereof, or an enantiomer, a diastereoisomer, a tautomer, an isotope substitute, a solvate, a polymorph, a prodrug or metabolite thereof, wherein $L_1$ is selected from C1-C6 alkylene-O-, C1-C6 alkylene-NH-, C1-C6 alkylene-S- and C2-C4 alkenylene and C1-C6 alkylene optionally substituted with 1, 2 or 3 substituents selected from halogen, C1-C4 alkyl and hydroxyl, , preferably selected from C1-C6 alkylene-NH- or C1-C6 alkylene optionally substituted with 1, 2 or 3 substituents selected from halogen, C1-C4 alkyl and hydroxyl.
Embodiment 6. The compound of Formula I according to Embodiment 1, or a pharmaceutically acceptable salt thereof, or an enantiomer, a diastereoisomer, a tautomer, an isotope substitute, a solvate, a polymorph, a prodrug or metabolite thereof, wherein $R_1$ is selected from optionally substituted C3-C8 cycloalkyl, optionally substituted 6-14 membered aryl, optionally substituted 5-10 membered heteroaryl and optionally substituted 4-10 membered heterocyclyl;

preferably, said 6-14 membered aryl is phenyl or naphthyl; said 5-10 membered heteroaryl is selected from pyridyl, pyrrolyl, pyrazolyl, imidazolyl, triazolyl, pyrimidinyl, pyrazinyl, triazinyl, indolyl, isoindolyl, indazolyl, benzimidazolyl, benzotriazolyl, benzoxazolyl, benzisoxazolyl and quinolyl, preferably pyridyl, pyrrolyl, imidazolyl and triazolyl; said 4-10 membered heterocyclyl is selected from pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, decahydroisoquinolyl, imidazolinyl, imidazolidinyl, quinazinyl, thiazolidinyl, isothiazolidinyl, isoxazolidinyl, dihydroindolyl, octahydroindolyl, octahydroisoindolyl, pyrrolidinyl, pyrazolindinyl and phthalimidinyl;
preferably, said alkoxy, aryl, heteroaryl and heterocyclyl are each optionally substituted with 1, 2 or 3 substituents selected from optionally substituted amino, C1-C6 alkyl, halogen, hydroxyl, C1-C6 alkoxy, C1-C6 haloalkyl and cyano; wherein said optionally substituted amino is $-NR_aR_b$, wherein $R_a$ and $R_b$ are each independently H or C1-C6 alkyl.

Embodiment 7. The compound of Formula I according to Embodiment 1, or a pharmaceutically acceptable salt thereof, or an enantiomer, a diastereoisomer, a tautomer, an isotope substitute, a solvate, a polymorph, a prodrug or

metabolite thereof, wherein said $L_1$ is C1-C4 alkylene or C1-C4 alkylene-NH-, $R_1$ is 5 or 6 membered nitrogen-containing heteroaryl optionally substituted with 1 or 2 substituents selected from halogen, C1-C4 alkyl and $-NR_aR_b$, wherein said 5 or 6 membered nitrogen-containing heteroaryl is preferably pyridyl, pyrrolyl, pyrazolyl, imidazolyl, triazolyl, pyrimidinyl, pyrazinyl or triazinyl, wherein $R_a$ and $R_b$ are each independently H or C1-C4 alkyl.

Embodiment 8. The compound of Formula I according to Embodiment 1, or a pharmaceutically acceptable salt thereof, or an enantiomer, a diastereoisomer, a tautomer, an isotope substitute, a solvate, a polymorph, a prodrug or metabolite thereof, wherein $L_2$ is selected from a bond and C1-C6 alkylene, wherein said C1-C6 alkylene is optionally substituted with 1, 2 or 3 substituents selected from halogen, C1-C3 alkyl, C3-C5 cycloalkyl and hydroxyl.

Embodiment 9. The compound of Formula I according to Embodiment 1, or a pharmaceutically acceptable salt thereof, or an enantiomer, a diastereoisomer, a tautomer, an isotope substitute, a solvate, a polymorph, a prodrug or metabolite thereof, wherein $R_2$ is selected from optionally substituted 6-14 membered aryl, optionally substituted 5-10 membered heteroaryl and optionally substituted 4-10 membered heterocyclyl;

preferably, said 6-14 membered aryl is phenyl or naphthyl; said 5-10 membered heteroaryl is selected from pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl and quinolyl, preferably pyridyl, pyrrolopyridyl, pyrazolyl or indolyl; said 4-10 membered heterocyclyl is selected from azetidinyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, benzodihydrofuranyl, decahydroisoquinolyl, imidazolinyl, imidazolidinyl, 6,7-dihydro-5H-cyclopenta[c]pyridine, quinazinyl, thiazolidinyl, isothiazolidinyl, isoxazolidinyl, dihydroindolyl, octahydroindolyl, octahydroisoindolyl, pyrrolidinyl and pyrazolindinyl;

preferably, $R_2$ is optionally substituted with 1, 2 or 3 substituents selected from optionally substituted amino, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano; wherein said optionally substituted amino is $-NR_aR_b$, wherein $R_a$ and $R_b$ are each independently H or C1-C6 alkyl; wherein said heteroaryl is preferably a heteroaryl containing 1 or 2 nitrogen ring atoms, preferably selected from pyrazolyl, imidazolyl, pyridyl, pyrazinyl and piperazinyl.

Embodiment 10. The compound of Formula I according to Embodiment 1, or a pharmaceutically acceptable salt thereof, or an enantiomer, a diastereoisomer, a tautomer, an isotope substitute, a solvate, a polymorph, a prodrug or metabolite thereof, wherein $L_2$ is C1-C4 alkylene, $R_2$ is 6-14 membered aryl, 5-10 membered nitrogen-containing heteroaryl or 4-10 membered heterocyclyl substituted with 1 or 2 substituents selected from C1-C6 alkoxy, halogen, C1-C6 alkyl, cyano, C3-C8 cycloalkyl-O- and halogenated C1-C6 alkoxy, preferably phenyl, pyridyl, pyrazolyl, pyrrolopyridyl, dihydrofuropyridinyl, pyrido 1,3-dioxolyl or indolyl.

Embodiment 11. The compound of Formula I according to Embodiment 1, or a pharmaceutically acceptable salt thereof, or an enantiomer, a diastereoisomer, a tautomer, an isotope substitute, a solvate, a polymorph, a prodrug or metabolite thereof, wherein $R_0$ is H or C1-C6 alkyl substituted with $-NR_3R_4$; wherein $R_3$ and $R_4$ are each independently selected from H, C1-C6 alkyl optionally substituted with 1, 2, 3, 4, 5 or 6 substituents selected from halogen, cyano, C1-C6 alkoxy, halogenated C1-C6 alkoxy, $-NR_aR_b$ and hydroxyl, or $R_3$ and $R_4$ form optionally substituted 4-10 membered heterocyclyl and optionally substituted 5-10 membered heteroaryl with the nitrogen atom to which they are attached, wherein $R_a$ and $R_b$ are each independently H or C1-C4 alkyl;

preferably, said 4-10 membered heterocyclyl is preferably nitrogen and/or oxygen containing heterocyclyl, preferably selected from oxetanyl, azetidinyl, pyrrolidinyl, tetrahydrofuranyl, piperidinyl, piperazinyl, tetrahydropyranyl and morpholinyl;

preferably, said 5-10 membered heteroaryl is 5-10 membered nitrogen and/or oxygen containing heteroaryl, preferably selected from pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl and quinolyl;

preferably, said 4-10 membered heterocyclyl and 5-10 membered heteroaryl are each optionally substituted with 1, 2 or 3 substituents selected from halogen, cyano, C1-C6 alkyl, halogenated C1-C6 alkyl, C1-C6 alkoxy, halogenated C1-C6 alkoxy, $-NR_aR_b$ and hydroxyl, wherein $R_a$ and $R_b$ are each independently H or C1-C4 alkyl.

Embodiment 12. The compound of Formula I according to Embodiment 1, or a pharmaceutically acceptable salt thereof, or an enantiomer, a diastereoisomer, a tautomer, an isotope substitute, a solvate, a polymorph, a prodrug or metabolite thereof, wherein $R_0$ is C1-C6 alkyl substituted with $-NR_3R_4$, wherein $R_3$ and $R_4$ form optionally substituted 4-10 membered heterocyclyl or 5-10 membered heteroaryl with the nitrogen atom to which they are attached;

preferably, said 4-10 membered heterocyclyl is nitrogen and/or oxygen containing heterocyclyl, preferably selected

from oxetanyl, azetidinyl, pyrrolidinyl, tetrahydrofuranyl, piperidinyl, piperazinyl, tetrahydropyranyl and morpholinyl; preferably, said 5-10 membered heteroaryl is 5-10 membered nitrogen and/or oxygen containing heteroaryl, preferably selected from pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl and quinolyl;
preferably, said 4-10 membered heterocyclyl and 5-10 membered heteroaryl are each optionally substituted with 1, 2 or 3 substituents selected from halogen, cyano, C1-C6 alkyl, halogenated C1-C6 alkyl, C1-C6 alkoxy, halogenated C1-C6 alkoxy, $-NR_aR_b$ and hydroxyl, wherein $R_a$ and $R_b$ are each independently H or C1-C4 alkyl.

Embodiment 13. The compound of Formula I according to Embodiment 1, or a pharmaceutically acceptable salt thereof, or an enantiomer, a diastereoisomer, a tautomer, an isotope substitute, a solvate, a polymorph, a prodrug or metabolite thereof, wherein $R_0$ is C1-C6 alkyl substituted with $-NR_3R_4$, wherein $R_3$ and $R_4$ form optionally substituted 4-10 membered heterocyclyl or 5-10 membered heteroaryl with the nitrogen atom to which they are attached;

preferably, said 4-10 membered heterocyclyl is nitrogen and/or oxygen containing heterocyclyl, preferably selected from oxetanyl, azetidinyl, pyrrolidinyl, tetrahydrofuranyl, piperidinyl, piperazinyl, tetrahydropyranyl and morpholinyl; preferably, said 5-10 membered heteroaryl is 5-10 membered nitrogen and/or oxygen containing heteroaryl, preferably selected from pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl and quinolyl;
preferably, said 4-10 membered heterocyclyl and 5-10 membered heteroaryl are each optionally substituted with 1, 2 or 3 substituents selected from halogen, cyano, C1-C6 alkyl, halogenated C1-C6 alkyl, C1-C6 alkoxy, halogenated C1-C6 alkoxy, sulfo, C1-C6 alkylsulfonyl, C1-C6 alkoxysulfonyl, C1-C6 alkylcarbonyl, halogenated C1-C6 alkylcarbonyl, C1-C6 alkoxycarbonyl, halogenated C1-C6 alkoxycarbonyl, $-NR_aR_b$ and hydroxyl, wherein $R_a$ and $R_b$ are each independently H or C1-C4 alkyl.

Embodiment 14. The compound of Formula I according to Embodiment 1, or a pharmaceutically acceptable salt thereof, or an enantiomer, a diastereoisomer, a tautomer, an isotope substitute, a solvate, a polymorph, a prodrug or metabolite thereof, wherein

ring A is C3-C6 cycloalkyl or 4-8 membered heterocyclyl, wherein said cycloalkyl or heterocyclyl are optionally substituted with 1, 2 or 3 substituents selected from halogen, C1-C4 alkyl, halogenated C1-C4 alkyl, C1-C4 alkoxy, halogenated C1-C4 alkoxy, carbonyl substituted with $-NR_aR_b$ and C1-C4 alkyl substituted with $-NR_aR_b$; wherein $R_a$ and $R_b$ are each independently H or C1-C4 alkyl;
$X_3$ and $X_5$ are both CH;
$X_4$ is $CH_2$;
$L_1$ is C1-C4 alkylene;
$R_1$ is 5 or 6 membered nitrogen-containing heteroaryl optionally substituted with 1 or 2 substituents selected from halogen, C1-C4 alkyl and $-NR_aR_b$, preferably pyridyl, pyrrolyl, pyrazolyl, imidazolyl, triazolyl, pyrimidinyl, pyrazinyl, triazinyl; wherein $R_a$ and $R_b$ are each independently H or C1-C4 alkyl;
$L_2$ is C1-C4 alkylene;
$R_2$ is phenyl, 5-10 membered nitrogen-containing heteroaryl or 4-10 membered heterocyclyl, preferably pyridyl, pyrimidinyl, pyrazolyl, pyrrolyl, imidazolyl, pyrazinyl, pyrrolopyridyl, dihydrofuropyridinyl or pyrido 1,3-dioxolyl or indolyl, wherein said phenyl, 5-10 membered nitrogen-containing heteroaryl and 4-10 membered heterocyclyl are each optionally substituted with 1 or 2 substituents selected from C1-C6 alkoxy, halogen, C1-C6 alkyl, cyano, C3-C8 cycloalkyl-O- and halogenated C1-C6 alkoxy;
$R_0$ is C1-C4 alkyl substituted with $-NR_3R_4$; wherein $R_3$ and $R_4$ are each independently selected from H, C1-C6 alkyl optionally substituted with 1-6 substituents selected from hydroxyl, cyano and halogen, and 4-8 membered heterocyclyl optionally substituted with 1, 2 or 3 substituents selected from halogen, C1-C6 alkyl, halogenated C1-C6 alkyl, C1-C6 alkoxy, halogenated C1-C6 alkoxy, $-NR_aR_b$ and hydroxyl, wherein $R_a$ and $R_b$ are each independently H or C1-C4 alkyl; or $R_3$ and $R_4$ form 4-8 membered heterocyclyl optionally substituted with 1, 2 or 3 substituents selected from halogen, C1-C6 alkyl, halogenated C1-C6 alkyl, C1-C6 alkoxy, halogenated C1-C6 alkoxy, $-NR_aR_b$ and hydroxyl with the nitrogen atom to which they are attached, wherein $R_a$ and $R_b$ are each independently H or C1-C4 alkyl; preferably, said 4-8 membered heterocyclyl is each independently selected from oxetanyl, azetidinyl, pyrrolidinyl, tetrahydrofuranyl, piperidinyl, piperazinyl, tetrahydropyranyl and morpholinyl.

Embodiment 15. The compound of Formula I according to Embodiment 1, or a pharmaceutically acceptable salt thereof, or an enantiomer, a diastereoisomer, a tautomer, an isotope substitute, a solvate, a polymorph, a prodrug or metabolite thereof, wherein

ring A is C3-C6 cycloalkyl, wherein said cycloalkyl is optionally substituted with 1, 2 or 3 substituents selected from halogen, C1-C4 alkyl and halogenated C1-C4 alkyl;
$X_3$ and $X_5$ are both CH;
$X_4$ is $CH_2$;
$L_1$ is C1-C4 alkylene;
$R_1$ is 5 or 6 membered nitrogen-containing heteroaryl substituted with 1 or 2 substituents selected from the group consisting of C1-C4 alkyl and $-NR_aR_b$, preferably pyridyl, pyrrolyl, pyrazolyl, imidazolyl, triazolyl, pyrimidinyl, pyrazinyl, triazinyl; wherein $R_a$ and $R_b$ are each independently H or C1-C4 alkyl;
$L_2$ is C1-C4 alkylene;
$R_2$ is 5-10 membered nitrogen-containing heteroaryl or 4-10 membered heterocyclyl, preferably pyridyl, pyrimidinyl, pyrazolyl, pyrrolyl, imidazolyl, pyrazinyl, pyrrolopyridyl, dihydrofuropyridinyl or pyrido 1,3-dioxolyl or indolyl, wherein said 5-10 membered nitrogen-containing heteroaryl or 4-10 membered heterocyclyl is each optionally substituted with 1 or 2 substituents selected from C1-C6 alkoxy, C1-C6 alkyl, C3-C8 cycloalkyl-O-, halogenated C1-C6 alkoxy, halogen and cyano;
$R_0$ is C1-C4 alkyl substituted with $-NR_3R_4$, wherein $R_3$ and $R_4$ are each independently selected from Hand C1-C6 alkyl optionally substituted with 1, 2, 3, 4, 5 or 6 substituents selected from hydroxyl, cyano and halogen, or $R_3$ and $R_4$ form a 4-8 membered heterocyclyl optionally substituted with 1, 2 or 3 substituents selected from halogen, hydroxyl and C1-C4 alkyl with the nitrogen atom to which they are attached, preferably, said 4-8 membered heterocyclyl is selected from oxetanyl, azetidinyl, pyrrolidinyl, tetrahydrofuranyl, piperidinyl, piperazinyl, tetrahydropyranyl and morpholinyl.

Embodiment 16. The compound of Formula I according to Embodiment 1, or a pharmaceutically acceptable salt thereof, or an enantiomer, a diastereoisomer, a tautomer, an isotope substitute, a solvate, a polymorph, a prodrug or metabolite thereof, wherein the compound of Formula I has a structure represented by the following Formula II:

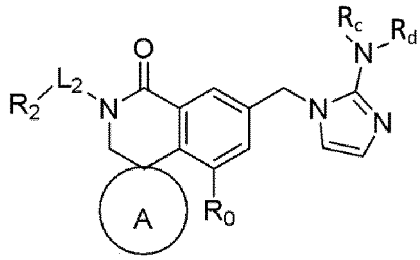

**II**

wherein, ring A is as defined in Embodiment 1, 2, 3, 14 or 15; $R_0$ is as defined in Embodiment 11, 12, 13, 14 or 15; $L_2$ is as defined in Embodiment 8, 10, 14 or 15; $R_2$ is as defined in Embodiment 9, 10, 14 or 15;
$R_c$ and $R_d$ are each independently H or C1-C4 alkyl.

Embodiment 17. The compound of Formula I according to Embodiment 16, or a pharmaceutically acceptable salt thereof, or an enantiomer, a diastereoisomer, a tautomer, an isotope substitute, a solvate, a polymorph, a prodrug or metabolite thereof, wherein in Formula II:

ring A is C3-C6 cycloalkyl optionally substituted with substituents selected from F, Cl, hydroxyl, C1-C4 alkyl, carbonyl substituted with $-NR_aR_b$ and C1-C4 alkyl substituted with - $NR_aR_b$ or C4-C6 heterocyclyl containing one nitrogen or one O atom, preferably cyclopropyl, cyclobutyl, oxetanyl or azetidinyl optionally substituted with C1-C4 alkyl, carbonyl substituted with $-NR_aR_b$ and C1-C4 alkyl substituted with $-NR_aR_b$; wherein $R_a$ and $R_b$ are each independently H or C1-C4 alkyl;
$R_0$ is H or C1-C4 alkyl substituted with $-NR_3R_4$, wherein $R_3$ and $R_4$ are each independently selected from H, 4-6 membered heterocyclyl and C1-C6 alkyl optionally substituted with 1, 2, 3, 4, 5 or 6 substituents selected from hydroxyl, cyano and halogen, or $R_3$ and $R_4$ form a 4-6 membered heterocyclyl optionally substituted with 1, 2 or 3 substituents selected from halogen, hydroxyl and C1-C4 alkyl with the nitrogen atom to which they are attached; wherein said 4-6 membered heterocyclyl is preferably an oxygen and/or nitrogen-containing heterocyclyl, preferably selected from oxetanyl, azetidinyl, pyrrolidinyl, tetrahydrofuranyl, piperidinyl, piperazinyl, tetrahydropyranyl and morpholinyl;
$L_2$ is C1-C4 alkylene;
$R_2$ is 6-14 membered aryl, 5-10 membered nitrogen-containing heteroaryl or 4-10 membered heterocyclyl, preferably

phenyl, pyridyl, pyrimidinyl, pyrazolyl, pyrrolyl, imidazolyl, pyrazinyl, indolyl, pyrrolopyridyl, dihydrofuropyridinyl or pyrido 1,3-dioxolyl, wherein said 6-14 membered aryl, 5-10 membered nitrogen-containing heteroaryl or 4-10 membered heterocyclyl is each optionally substituted with 1 or 2 substituents selected from C1-C6 alkoxy, C1-C4 alkyl, halogen, C3-C6 cycloalkyl-oxy, halogenated C1-C4 alkoxy and cyano;

$R_c$ and $R_d$ are each independently H or C1-C4 alkyl.

Embodiment 18. The compound of Formula I according to Embodiment 1, or a pharmaceutically acceptable salt thereof, or an enantiomer, a diastereoisomer, a tautomer, an isotope substitute, a solvate, a polymorph, a prodrug or metabolite thereof, wherein the compound of Formula I has a structure represented by the following Formula III:

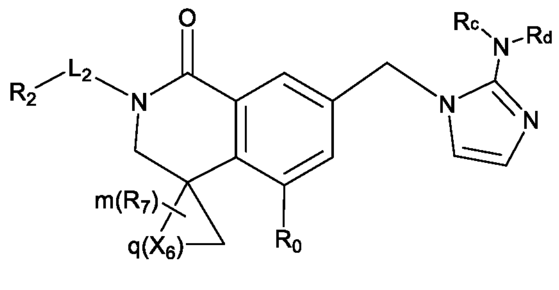

**III**

wherein, $R_0$ is as defined in Embodiment 11, 12, 13, 14, 15 or 17; $L_2$ is as defined in Embodiment 8, 10, 14, 15 or 17; $R_2$ is as defined in Embodiment 9, 10, 14, 15 or 17; $R_c$ and $R_d$ are as defined in Embodiment 16 or 17,

$X_6$ is each independently selected from the group consisting of C, O, N, S,

q is an integer of 1, 2 or 3,

$R_7$ is selected from the group consisting of F, Cl, cyano, hydroxyl, C1-C6 alkyl, fluorinated or chlorinated C1-C6 alkyl, hydroxyl-substituted C1-C6 alkyl, C1-C6 alkoxy, fluorinated or chlorinated C1-C6 alkoxy, hydroxyl-substituted C1-C6 alkyl, $-NR_aR_b$, carbonyl substituted with $-NR_aR_b$, C1-C6 alkyl substituted with $-NR_aR_b$, C1-C6 alkylcarbonyl, C1-C6 alkoxycarbonyl; wherein said $R_a$ and $R_b$ are each independently selected from H and C1-C6 alkyl,

m is an integer of 0, 1, 2 or 3.

Embodiment 19. The compound of Formula I according to Embodiment 1, or a pharmaceutically acceptable salt thereof, or an enantiomer, a diastereoisomer, a tautomer, an isotope substitute, a solvate, a polymorph, a prodrug or metabolite thereof, wherein the compound is selected from a group consisting of:

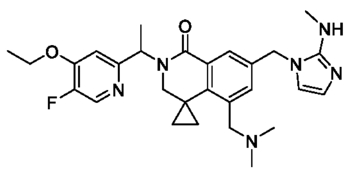 ,

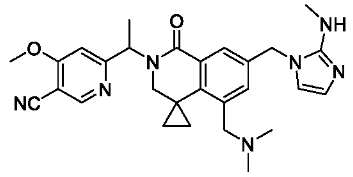 ,

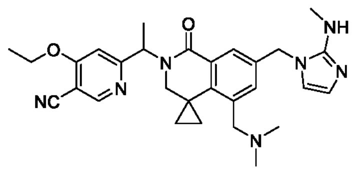 ,

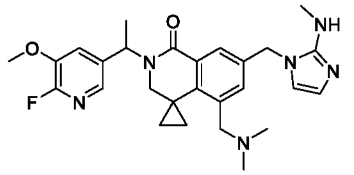 ,

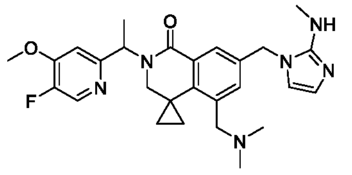 ,

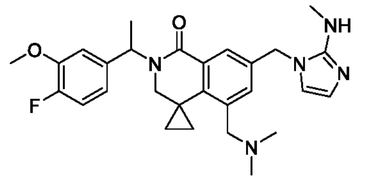 ,

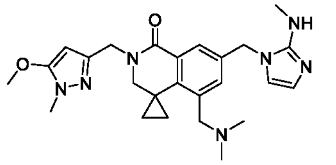 ,

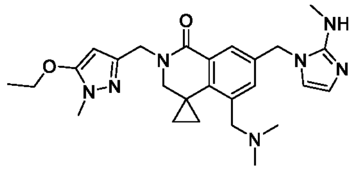 ,

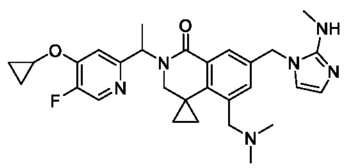 ,

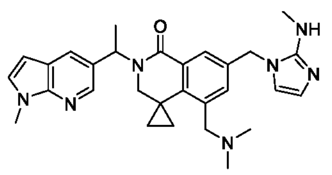,

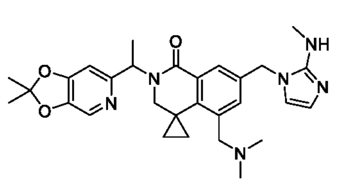,

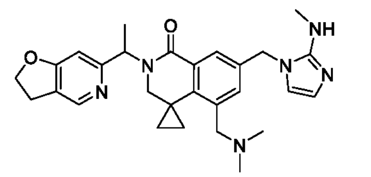,

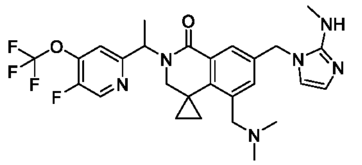,

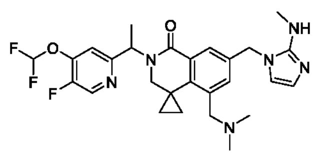,

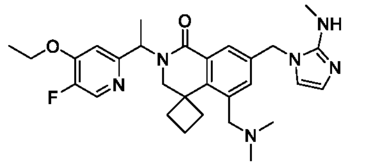,

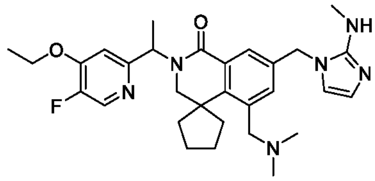,

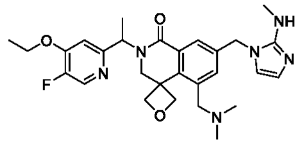,

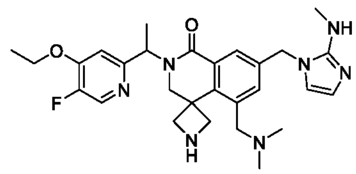,

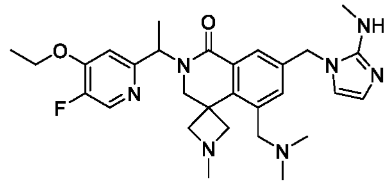,

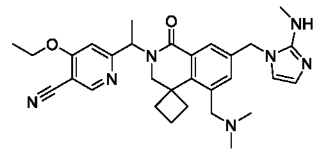,

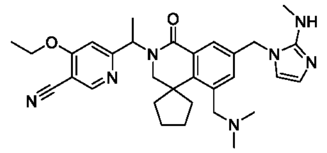,

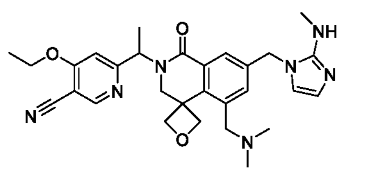,

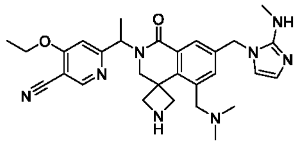,

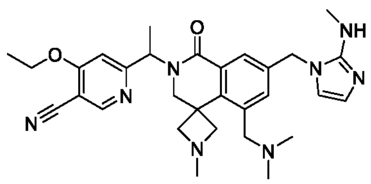,

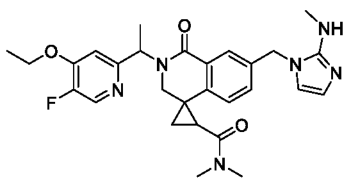,

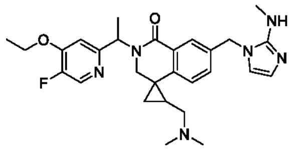,

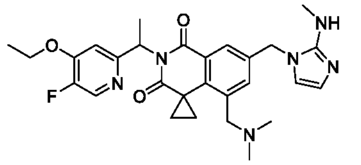,

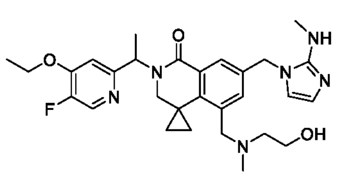,

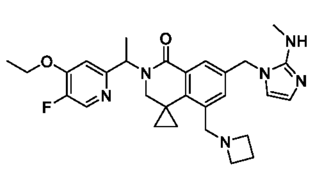,

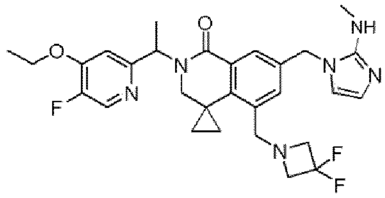,

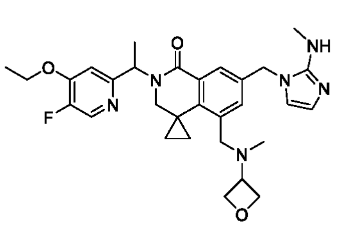 , 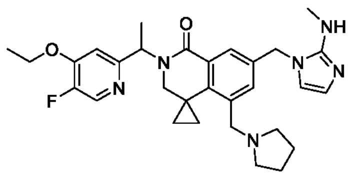 , 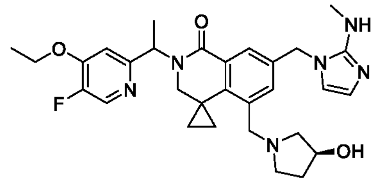 ,

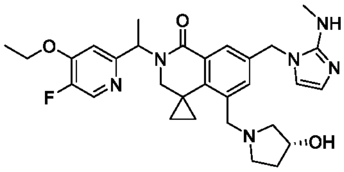 , 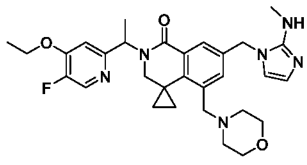 , 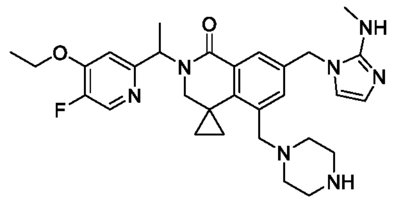 ,

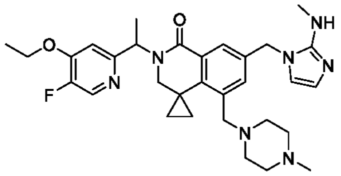 , 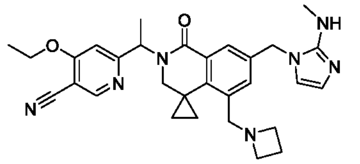 , 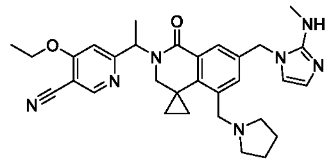 ,

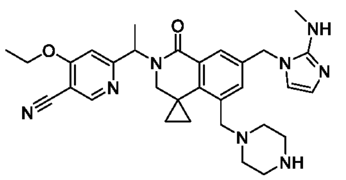 , 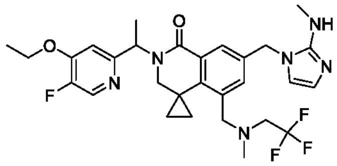 , 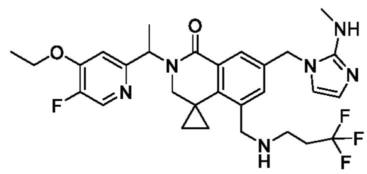 ,

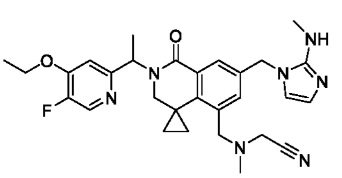 , 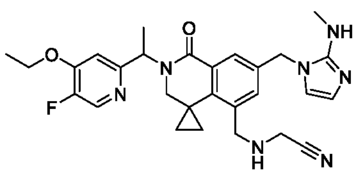 , 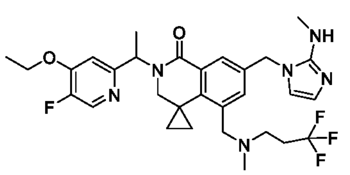 ,

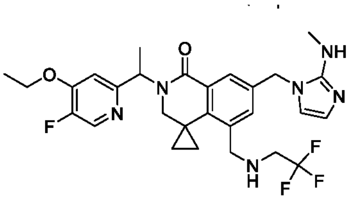 , 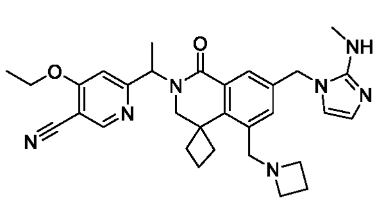 , 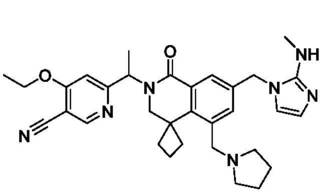 ,

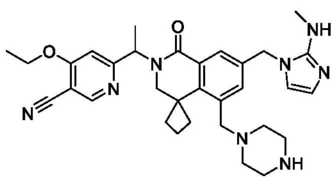 , 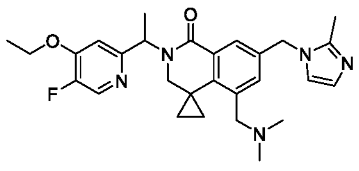 , 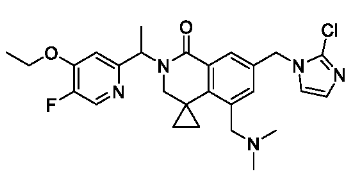 ,

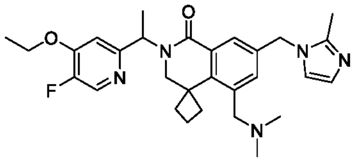 , 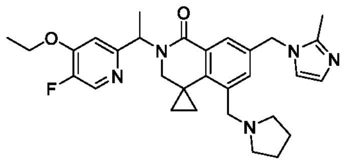 , 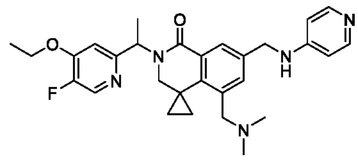 ,

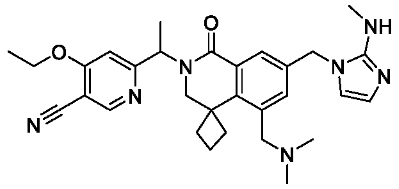 , 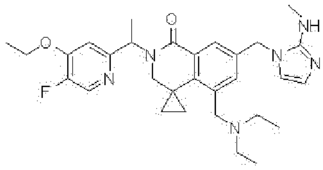 , 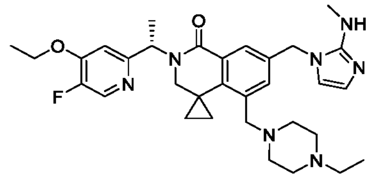 ,

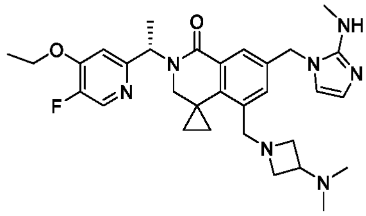 , 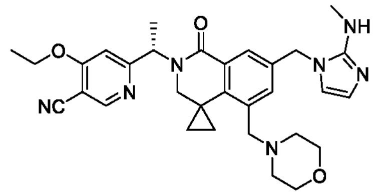 , 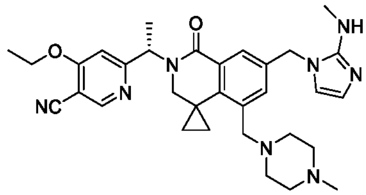 ,

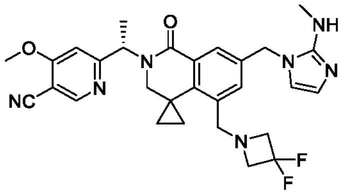 , 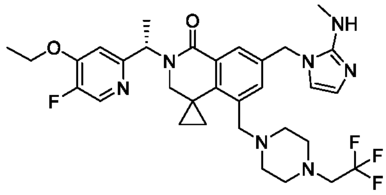 , 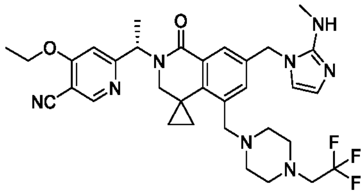 ,

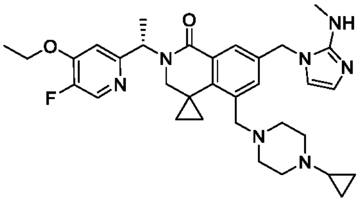 , 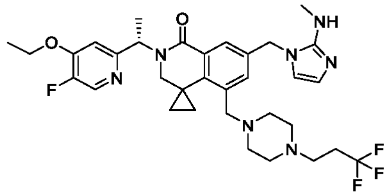 , 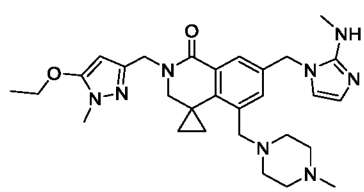 ,

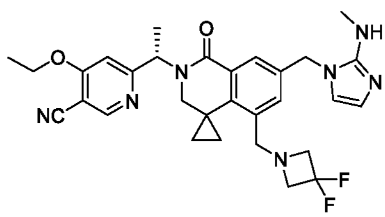 , 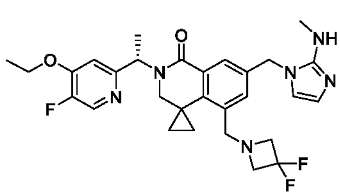 , 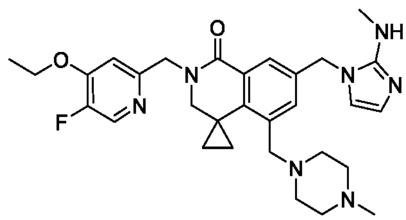

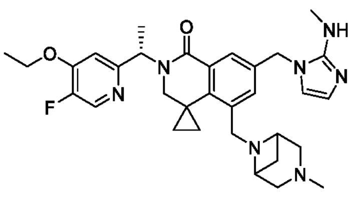,
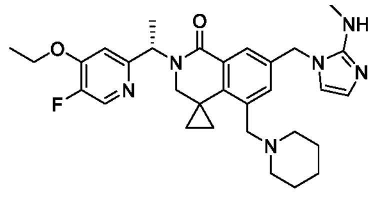,
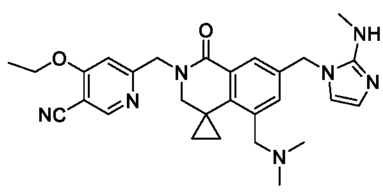,

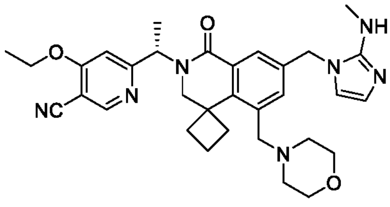,
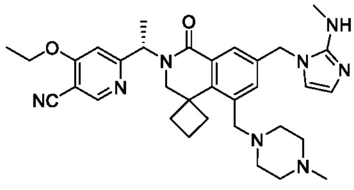,
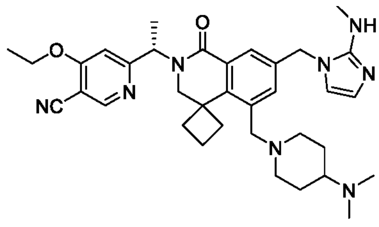,

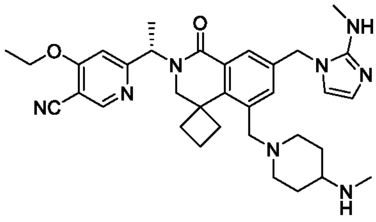,
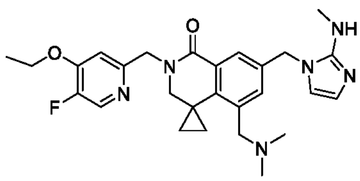,
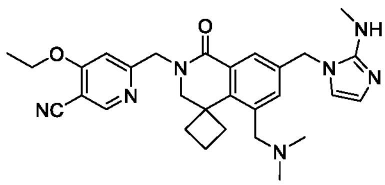,

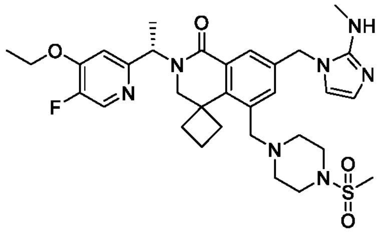,
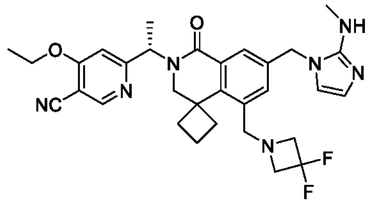,
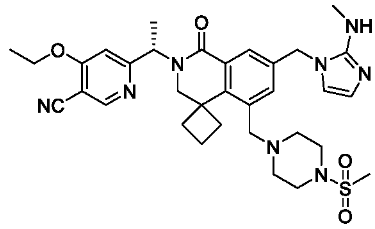,

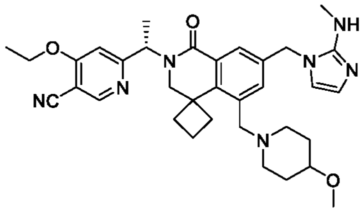,
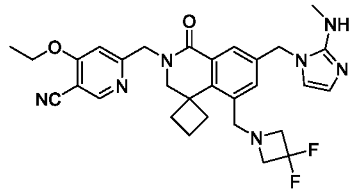,
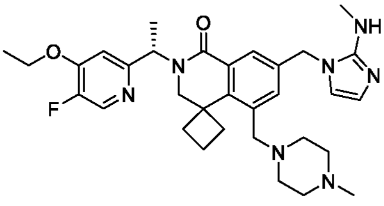,

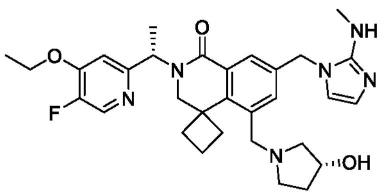,
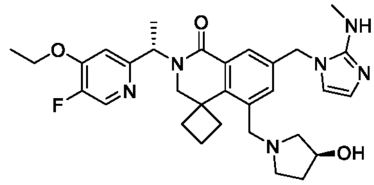,
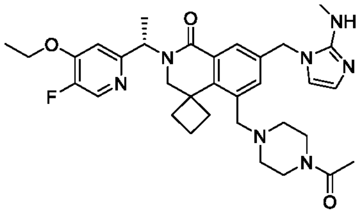,

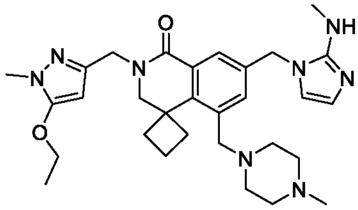 , 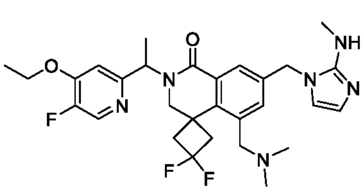 , 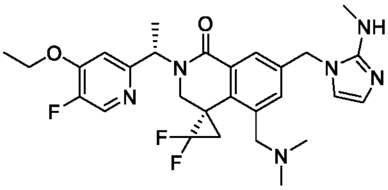 ,

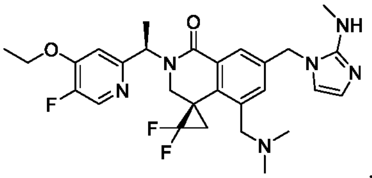 , 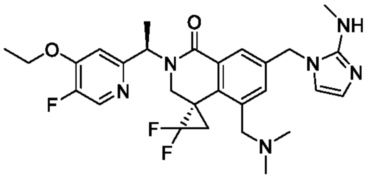 , 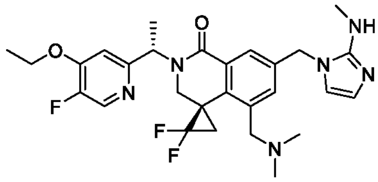 ,

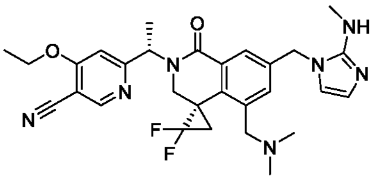 , 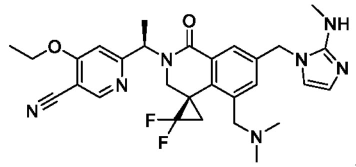 , 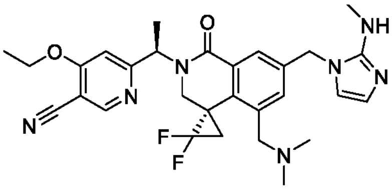 ,

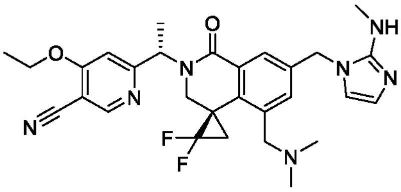 , 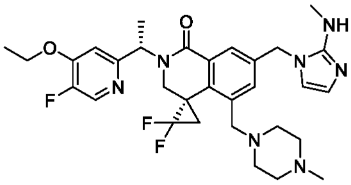 , 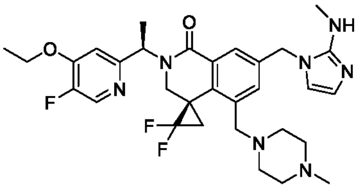 ,

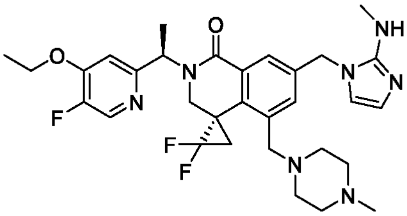 , 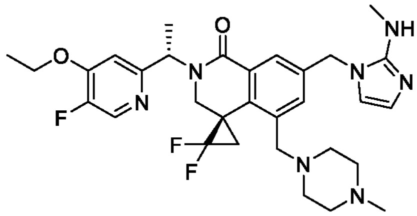 .

Embodiment 20. A pharmaceutical composition comprising the compound according to any one of Embodiments 1-16, or a pharmaceutically acceptable salt thereof, or an enantiomer, a diastereoisomer, a tautomer, an isotope substitute, a solvate, a polymorph, a prodrug or metabolite thereof, and a pharmaceutically acceptable carriers or excipients.

Embodiment 21. Use of the compound according to any one of Embodiments 1-19, or a pharmaceutically acceptable salt thereof, or an enantiomer, a diastereoisomer, a tautomer, an isotope substitute, a solvate, a polymorph, a prodrug or metabolite thereof in the manufacture of drugs for the treatment or prevention of WDR5-mediated diseases.

Embodiment 22. Use according to Embodiment 21, wherein said WDR5-mediated disease comprises Kabuki syndrome as well as various solid tumors and hematological tumors; preferably, solid tumors and hematological tumors are selected from the group consisting of Noonan syndrome, leopard syndrome, neuroblastoma, sarcoma, melanoma, articular chondroma, cholangioma, leukemia, breast cancer, gastrointestinal stromal tumor, histiocytic lymphoma, lung cancer, esophageal cancer, pancreatic cancer, breast cancer, prostate cancer, liver cancer, skin cancer, epithelial cell carcinoma, cervical cancer, ovarian cancer, bowel cancer, nasopharyngeal cancer, brain cancer, bone cancer, kidney cancer, oral cancer, head cancer, neuroblastoma, squamous cell carcinoma of the head and neck, anaplastic large cell lymphoma or glioblastoma, wherein leukemia is preferably juvenile myeloid monocytic leukemia, human myeloid monocytic leukemia, and lung cancer is preferably non-small cell lung cancer, small cell lung cancer, lung squamous cell carcinoma, lung adenocarcinoma.

The present disclosure includes a pharmaceutically acceptable salt of Compound of Formula I. In the present

disclosure, the term "pharmaceutically acceptable salt" includes pharmaceutically acceptable acid addition salt and pharmaceutically acceptable base addition salt.

"Pharmaceutically acceptable acid-addition salt" means salts formed with inorganic or organic acids that retain the bioavailability of free bases without other side effects. Inorganic salts include but are not limited to hydrochloride, hydrobromate, sulfate, nitrate, phosphate, etc. Organic salts include, but are not limited to, formate, acetate, 2,2-dichloroacetate, trifluoroacetate, propionate, caproate, octanoate, caprate, undecylenate, glycolate, gluconate, lactate, sebacinate, adicate, glutarate, malonate, oxalate, maleate, succinate, fumarate, tartrate, citrate, palmitate, stearate, oleate, cinnamate, laurate, malate, glutamate, pyroglutamate, aspartate, benzoate, mesylate, benzenesulfonate, p-toluenesulfonate, alginate, ascorbate, salicylate, 4-aminosalicylate, naphthalene disulfonate, etc. These salts can be prepared by methods known in the art.

"Pharmaceutically acceptable base addition salt" means salts formed with inorganic or organic bases that retain the bioavailability of free acids without other side effects. Salts derived from inorganic bases include but are not limited to sodium salts, potassium salts, lithium salts, ammonium salts, calcium salts, magnesium salts, iron salts, zinc salts, copper salts, manganese salts, aluminum salts, etc. Preferred inorganic salts are ammonium salt, sodium salt, potassium salt, calcium salt and magnesium salt. Salts derived from organic bases include, but are not limited to, those derived from primary amines, secondary amines and tertiary amines, substituted amines, including natural substituted amines, cyclic amines and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methyl glucosamine, theobromine, purine, piperazine, piperidine, N-ethylpiperidine, polyamine resin, etc. Preferred organic bases include isopropylamine, diethylamine, ethanolamine, trimethylamine, dicyclohexylamine, choline and caffeine. These salts can be prepared by methods known in the art.

The present disclosure further comprises isomers of Compound of Formula I, including stereoisomers, enantiomers or tautomers. In the present disclosure, a "stereoisomer" refers to compounds composed of the same atoms, bonded by the same bonds, but with different three-dimensional structures. The present disclosure covers various stereoisomers and mixtures thereof. A "tautomer" is an isomer formed by the transfer of protons from one atom of a molecule to another atom of the same molecule. All tautomeric forms of the compound according to the present disclosure will also be included within the scope of the present disclosure.

The compounds of the present disclosure or pharmaceutically acceptable salts thereof may comprise one or more chiral carbon atoms, and may thus produce enantiomers, diastereomers and other stereoisomeric forms. Each chiral carbon atom can be defined as (R)- or (S)- stereochemistry. The present disclosure is intended to include all possible isomers, and their racemates and optically pure forms. The preparation of compounds of the present disclosure may select racemates, diastereomers or enantiomers as raw materials or intermediates. Optically active isomers can be prepared using chiral synthons or chiral reagents, or resolved using conventional techniques such as crystallization and chiral chromatography, etc.

Conventional techniques for the preparation/separation of individual isomers include chiral synthesis from suitable optically pure precursors, or resolving racemates (or racemates of salts or derivatives) using, for example, chiral HPLC. Reference is made to, for example, Gerald Gübitz and Martin G. Schmid (Eds.), Chiral Separations, Methods and Protocols, Methods in Molecular Biology, Vol. 243, 2004; A.M. Stalcup, Chiral Separations, Annu. Rev. Anal. Chem. 3:341-63, 2010; Fumiss et al. (eds.), VOGEL'S ENCYCLOPEDIA OF PRACTICAL ORGANIC CHEMISTRY 5.sup.TH ED., Longman Scientific and Technical Ltd., Essex, 1991, 809-816; Heller, Acc. Chem. Res. 1990, 23, 128.

When the compounds of the present disclosure contain alkene double bonds, unless otherwise stated, the compounds of the present disclosure are intended to comprise E- and Z- geometric isomers.

The present disclosure further comprises all suitable isotopic variants of compounds of the present disclosure, pharmaceutically acceptable salts or isomers thereof. Isotopic variants of compounds of the present disclosure, pharmaceutically acceptable salts or isomers thereof are defined as those in which at least one atom is replaced by atoms having the same atomic number, but atomic mass is different from atomic mass often found in nature. Isotopes that may be incorporated into compounds of the present disclosure, pharmaceutically acceptable salts or isomers thereof, include but are not limited to, isotopes of H, C, N and O, such as $^{2}H$, $^{3}H$, $^{11}C$, $^{13}C$, $^{14}C$, $^{15}N$, $^{17}O$, $^{18}O$, $^{35}S$, $^{18}F$, $^{36}Cl$ and $^{125}I$. The isotopic variant of the compound of the present disclosure, a pharmaceutically acceptable salt or isomer thereof may be prepared by adopting appropriate isotopic variants of suitable reagents through conventional techniques.

Preparation of Compound of Formula I

In some embodiments, Compound of Formula IIa according to the present disclosure may be prepared by the method shown in the following scheme:

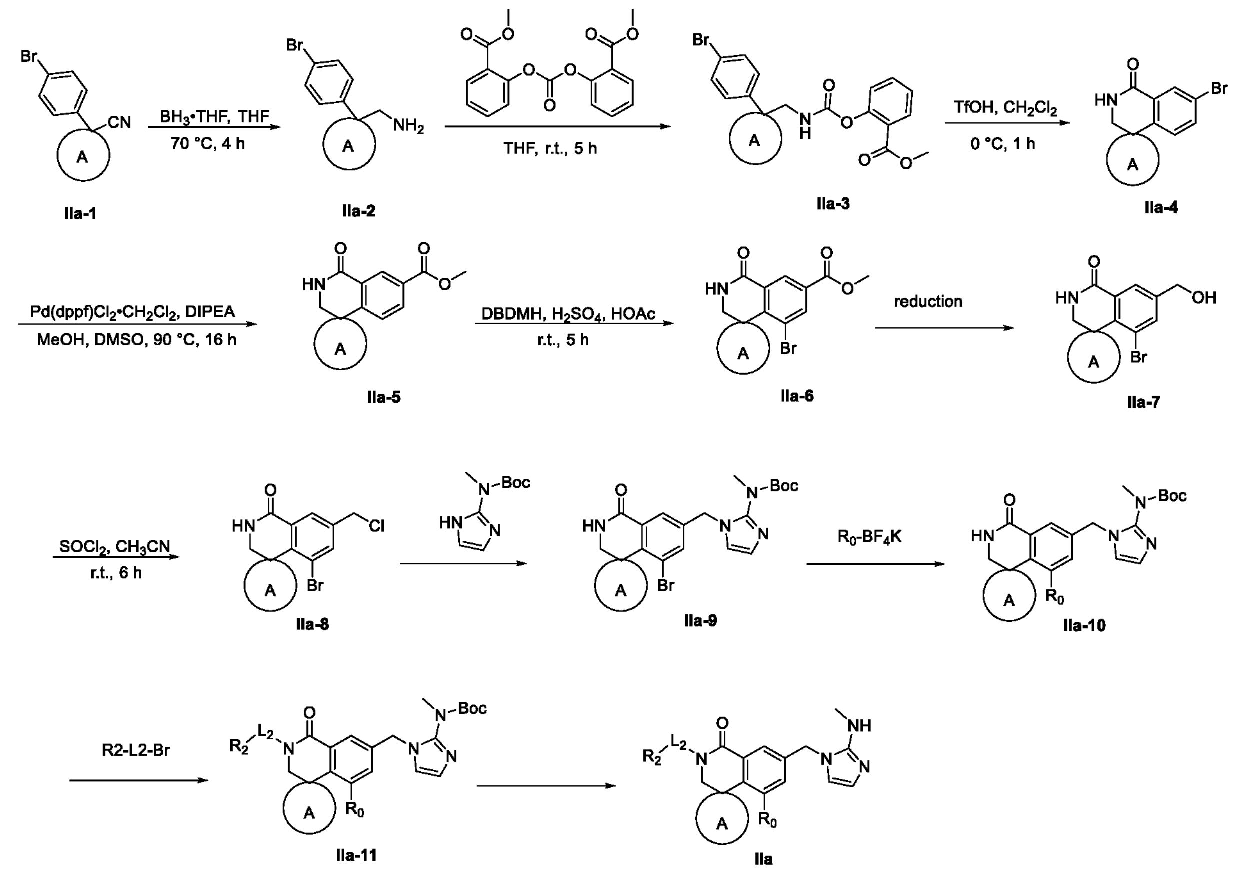


IIa-1 IIa-2 IIa-3 IIa-4 IIa-5 IIa-6 IIa-7 IIa-8 IIa-9 IIa-10 IIa-11 IIa

In the scheme, Intermediate IIa-1 is subjected to reduction to obtain Intermediate IIa-2; IIa-2 is subjected to amine transesterification to obtain IIa-3; IIa-3 is subjected to acid catalyzed cyclization to obtain IIa-4; IIa-4 is subjected to catalyzed carbonyl insertion to form ester compound of IIa-5. IIa-5 is subjected to aromatic cyclohalogenation to obtain IIa-6; IIa-6 is subjected to ester reduction to form IIa-7; The hydroxyl group of IIa-7 is further halogenated to form IIa-8; IIa-8 and imidazolamide undergo alkylation reaction to obtain IIa-9; IIa-9 is catalytically coupled with boric acid derivatives to obtain IIa-10. IIa-10 and $R_2$-$L_2$-Br undergo alkylation to form IIa-11; IIa-11 is deprotected to obtain Compound of Formula IIa. The reaction conditions for each step are shown in the reaction scheme.

In each formula of the above reaction scheme, each group is defined as described above.

In the preparation method of the present disclosure, the catalytic cyclization reaction of IIa-3 to IIa-4 is used to construct an aromatic spirocyclic intermediate of Formula IIa-4. The reaction is catalyzed by an acid (preferably trifluoromethanesulfonic acid) in a halogenated alkane (preferably dichloromethane) at low or room temperature.

In the presence of a base and a phase transfer catalyst (preferably cesium carbonate, tetrabutylammonium iodide), IIa-8 can react with imidazoles to introduce imidazole group on the right to obtain IIa-9.

IIa-9 and boric acid compounds undergo a catalytic coupling reaction to introduce $R_0$ substituents, which can be carried out in the presence of water and organic solvents (such as alcohols or ether solvents, preferably dioxane). IIa-10 is obtained in the presence of a base (e.g., sodium carbonate) and a catalyst (e.g., Pd(dppf)$Cl_2$) under heating.

In the presence of a base (preferably sodium hydride), IIa-10 reacts with halide to introduce $R_2$-$L_2$- group. The solvent of the alkylation reaction may be an organic solvent, preferably tetrahydrofuran, DMF or NMP.

The above scheme also includes a Boc-deprotection step. The deprotection reaction can be performed in a conventional manner.

In the above scheme, if the final product has a chiral center and needs chiral resolution, conventional methods in the art, such as SFC or chiral HPLC, can be used for chiral resolution.

In the reaction, when purification is required, purification can be performed using conventional methods in the art, such as extraction first, followed by column chromatography.

The synthesis of Compounds of Formula I and Formula II of the present disclosure may be performed according to the general method of Compound of Formula IIa by changing different raw materials to obtain different compounds. The preparation method usually includes the steps described above such as catalytic ring-closing, catalytic coupling, reduction halogenation, and alkylation substitution.

## Pharmacology, use, method and pharmaceutical composition

WDR5 belongs to the WD40-repeat protein family. WD40 is an important domain composed of about 40 amino acid residues. WDR domain contains a ring-shaped seven-bladed β-propeller domain, wherein each blade contains a conserved serine-histidine (SH) and tryptophan-aspartic acid (WD) sequence. WDR5 protein contains a central cavity formed from 7-bladed β-propeller that runs up and down. WDR5 protein mainly plays a role in regulation through a deep arginine-binding pocket, that is, a core catalytic complex composed of WIN site and MLL1, and forms MWRAD (MLL1, WDR5, RbBP5, ASH2L and DYP-30) core catalytic complex with other proteins. It catalyzes methylation of histone H3, activates gene transcription and participates in epiregulation.

The MLL family protein complex, which is widely present in eukaryotes, is an important class of histone methyltransferases, which catalyzes the methylation of histone H3K4, activates transcription by catalyzing H3K4 methylation and/or by recruiting other transcription-related proteins and mediators, and plays a crucial role in development. Mutations in these proteins, including genomic translocations that fuse them with other transcription factors, can cause a variety of diseases, such as mixed-lineage leukemia (MLL), lymphoma, Kabuki syndrome, etc. Compounds designed for WIN pockets where WDR5 binds to histones and MLL1 by using WDR5 as a drug target can reduce gene expression downstream of WDR5 and treat MLL rearrangement-dependent leukemia.

TP53 gene is the most common variant in tumors, and TP53 mutations occur in 50% of human cancers. The high frequency of missense mutations and the high expression level of mutant p53 (mp53) protein in cancer cells promote the new function of mp53 protein ("gain-of" (GOF)), and also actively promote the development and progression of cancer. The growth of tumor cells driven by p53 gene mutation depends on the binding of WDR5 to MLL. MLL, MOZ and histone modifications are all regulated by the expression of GOF p53. WDR5 WIN site inhibitors effectively block the growth of breast cancer cell lines driven by p53 gene mutations as well as the growth of leukemia cells.

WIN site inhibitors of WDR5 can also inhibit the co-binding locus of MYC and WDR5. MYC is a class of oncoproteins with transcription factor function, which is overexpressed in most malignant tumors. MYC can bind to the regulatory sequence of thousands of target genes, regulate target gene expression, and affect the processes such as cell growth, proliferation, metabolism, genome stability and apoptosis. Early studies have found that the extensive binding of MYC to chromatin also relies on WDR5 interactions, and MYC plays an important role in the process of tumorigenesis in vivo through its evolutionarily conserved "MYC box IIIb" sequence binding to hydrophobic fissures on the surface of WDR5. Blocking the interaction of MYC with WDR5 inhibits the driving cancer ability of MYC. Inhibitors targeting the WDR5 WIN site can be used as targeted therapy drugs for the treatment of MYC-driven tumors.

It has been found in clinical studies that WDR5 is highly expressed in a variety of solid tumors, and there is a certain correlation with poor tumor prognosis. On the one hand, it is due to the regulatory effect of WDR5 on EMT. Epithelial cell-mesenchymal transformation (EMT) refers to the biological process by which epithelial cells are transformed into cells with an interstitial phenotype through specific procedures. EMT is an important biological process for epithelial cell-derived malignant tumor cells to acquire migration and invasion capacity. WDR5 can directly regulate the TGFβ1 signaling pathway, thereby participating in the ETM and migration process of tumor cells. WDR5 inhibitors do not significantly kill breast cancer cells having drug resistance, but can enhance the sensitivity of cells to PTX by reducing the level of TGFβ1, thereby inhibiting tumor metastasis. This also suggests the potential of WDR5 inhibitors in combination therapy. WDR5 also mediates the expression of multiple EMT characteristic molecules such as N-cadherin, ZNF407, HOXA9, SNAIL1, and VIMENTIN. On the other hand, the researchers also found that WDR5 regulates the expression of tumor-related signaling molecules, but is also regulated by multiple upstream signaling pathways or molecules. PI3K/AKT-mediated upregulation of WDR5 expression promoted the metastasis of colorectal cancer, while in gastric cancer, the induced expression of cyclin D1 could upregulate WDR5 and promote tumor formation.

In summary, WDR5 protein is widely involved in the activation of signaling pathways such as MI,L1, TP53, MYC, TGFβ1 and PI3K/AKT in cancer cells, which promotes the growth, proliferation, differentiation and migration of cancer cells. Therefore, WDR5 protein-protein interaction inhibitors can be used to treat a variety of diseases, including Kabuki syndrome as well as various solid tumors and hematological tumors, including but not limited to Noonan syndrome, leopard syndrome, neuroblastoma, sarcoma, melanoma, articular chondroma, cholangioma, leukemia (such as juvenile myeloid monocytic leukemia, human myeloid monocytic leukemia, etc.), breast cancer, gastrointestinal stromal tumor, histiocytic lymphoma, lung cancer (such as non-small cell lung cancer, small cell lung cancer, lung squamous cell carcinoma, lung adenocarcinoma, etc.), esophageal cancer, pancreatic cancer, breast cancer, prostate cancer, liver cancer, skin cancer, epithelial cell carcinoma, cervical cancer, ovarian cancer, bowel cancer, nasopharyngeal cancer, brain cancer, bone cancer, kidney cancer, oral cancer, head cancer, neuroblastoma, squamous cell carcinoma of the head and neck, anaplastic large cell lymphoma or glioblastoma, etc.

The compound of Formula I of the present disclosure, a pharmaceutically acceptable salt and an isomer thereof are inhibitors of WDR5. They can inhibit the binding of WDR5 to MLL1 or MYC, and can also inhibit the activity of WDR5 itself. Thus, the compound of Formula I of the present disclosure, a pharmaceutically acceptable salt and an isomer thereof may be used to treat or prevent WDR5-mediated diseases. In the present disclosure, "WDR5-mediated disease"

refers to a disease in which WDR5 is involved in the onset and/or progression of the disease and in which remission, treatment and/or prophylaxis can be achieved by inhibiting WDR5 expression and/or activity. In the present disclosure, WDR5-mediated diseases include, but are not limited to, Kabuki syndrome, various solid tumors and hematologic tumors as described above, in particular including MLL rearrangement-dependent leukemia (such as human myeloid monocytic leukemia), MYC-driven tumors and breast cancer, etc. Herein, MYC-driven tumors refer to tumors caused by MYC activity. The compound of Formula I of the present disclosure, a pharmaceutically acceptable salt and an isomer thereof may also be used to inhibit epithelial cell-mesenchymal transformation (EMT), thereby inhibiting the metastasis of epithelial cell-derived malignant tumor cells. Thus, the "WDR5-mediated disease" described in the present disclosure also includes metastasis of cancer, especially metastasis of epithelial cell-derived malignant tumors.

Accordingly, the present disclosure provides a method for treating or preventing WDR5-mediated diseases described herein, which comprises administering a therapeutically effective amount of the compound of Formula I of the present disclosure, a pharmaceutically acceptable salt and an isomer thereof or a pharmaceutical composition containing the compound of Formula I of the present disclosure, a pharmaceutically acceptable salt and an isomer thereof.

The term "subject" or "individual" herein refers to mammals, especially primates and, more specifically, humans.

As used herein, the terms "prevention" and "preventing" include reducing the likelihood of occurrence or deterioration of the disease or condition. The term also includes preventing the occurrence of a disease or condition in mammals, especially when such mammals are susceptible to the disease or condition but have not yet been diagnosed with the disease or condition. "Treatment" and other similar synonyms include the following meanings: (i) inhibiting a disease or condition, i.e., inhibiting its progression; (ii) alleviating the disease or condition, i.e., making the state of the disease or condition subside; or (iii) alleviating symptoms caused by the disease or condition.

As used herein, the terms "effective amount", "therapeutic effective amount", "amount of administration" and "pharmaceutically effective amount" refer to the amount of at least one agent or compound that is taken to relieve one or more symptoms of the disease or condition being treated to some extent. The result can be a reduction and/or remission of signs, symptoms, or causes, or any other desired change in biological systems. For example, the "effective amount" for treatment is the amount of a composition comprising a compound of Formula I disclosed herein, a pharmaceutically acceptable salt or an isomer that is required to provide a clinically significant remission effect. The amount of administration can be determined according to factors such as the age, gender, disease and severity of the subject. Techniques such as dose escalation tests can be used to determine the effective amount appropriate for any individual case.

As used herein, the terms "take", "apply", "administer" and the like refer to a method capable of delivering a compound or composition to the desired site for biological action. All well-known administration methods in the art may be used in the present disclosure. These methods include, but are not limited to, oral administration, transduodenum administration, parenteral injection (including intrapulmonary, intranasal, intrathecal, intravenous, subcutaneous, intraperitoneal, intramuscular, intraarterial injection or infusion), topical administration, and rectal administration. Those skilled in the art are familiar with the application techniques that may be used for the compounds and methods described herein, for example, those discussed in Goodman and Gilman, The Pharmacological Basis of Therapeutics, current ed.; Pergamon; and Remington's, Pharmaceutical Sciences (current edition), Mack Publishing Co., Easton, Pa. In a preferred embodiment, a compound of Formula I according to the present disclosure, a pharmaceutically acceptable salt or an isomer thereof or a pharmaceutical composition comprising the compound of Formula I according to the present disclosure, a pharmaceutically acceptable salt or isomer is orally administered.

The inventive compound of Formula I according to the present disclosure, a pharmaceutically acceptable salt and an isomer thereof or the pharmaceutical composition comprising the compound of Formula I according to the present disclosure, a pharmaceutically acceptable salt and an isomer thereof may be used in combination with other compounds having pharmacological activity, in particular may be used in combination in the treatment of cancer. For example, the inventive compound of Formula I according to the present disclosure, a pharmaceutically acceptable salt and an isomer thereof or the pharmaceutical composition comprising the compound of Formula I according to the present disclosure, a pharmaceutically acceptable salt and an isomer thereof may be administered concurrently, sequentially or separately with one or more drugs selected from the group consisting of chemotherapy agents, such as mitosis inhibitors, such as taxane, vinca alkaloids, paclitaxel, docetaxel, vincristine, vinblastine, vinorelbine or vinflunine, other anticancer agents such as cisplatin, 5-Fluorouracil or 5-fluoro-2-4 (1H,3H)-pyrimidinedione (5FU), flutamide, or gemcitabine, etc. In some embodiments, the inventive compound of Formula I according to the present disclosure, a pharmaceutically acceptable salt and an isomer thereof or the pharmaceutical composition comprising the compound of Formula I of the present disclosure, a pharmaceutically acceptable salt and an isomer thereof may also be used in the treatment of cancer together with well-known tumor immunotherapy drugs in the art, such as anti-PD1 antibodies. Alternatively, the compound of Formula I according to the present disclosure, a pharmaceutically acceptable salt and an isomer thereof or the pharmaceutical composition comprising the compound of Formula I according to the present disclosure, a pharmaceutically acceptable salt and an isomer may also be used in combination with conventional radiotherapy.

As used herein, "in combination", "in combination with drug ", "together with" or "in combination with therapy"

refers to drug treatment obtained by mixing or combining more than one active ingredients, which includes fixed and unfixed combinations of active ingredients, or a combination of two or more different therapeutic methods. The term "fixed combination" refers to the simultaneous administration of at least one compound as described herein and at least one synergistic agent to a patient in the form of a single entity or a single dosage form. The term "unfixed combination" refers to the administration of at least one compound and at least one synergistic formulation described herein to patients in the form of separate entities concurrently, in combination, or sequentially at variable intervals. These are also applied to cocktail therapies, such as the administration of three or more active ingredients.

The present disclosure also provides a pharmaceutical composition comprising a compound of Formula I of the present disclosure, a pharmaceutically acceptable salt or isomer, and a pharmaceutically acceptable carrier or excipient.

In the present disclosure, "pharmaceutical composition" means a preparation containing a compound of Formula I of the present disclosure, a pharmaceutically acceptable salt or an isomer thereof and a medium generally accepted in the art for delivering a bioactive compound to a mammal (e.g., human). The medium includes pharmaceutically acceptable carriers. The purpose of the pharmaceutical composition is to promote the administration of the organism, facilitate the absorption of the active ingredient and then exert biological activity. As used herein, the term "pharmaceutically acceptable" refers to a substance (such as a carrier or diluent) that does not affect the biological activity or properties of the compound of Formula I of the present disclosure, a pharmaceutically acceptable salt or an isomer thereof and is relatively non-toxic, i.e., the substance may be administered to an individual without causing adverse biological reactions or interacting in an undesirable manner with any component contained in the composition. "Pharmaceutically acceptable carrier or excipient" includes, but is not limited to, any adjuvant, carrier, excipient, flow aid, sweetener, diluent, preservative, dyes/colorant, corrigent, surfactant, wetting agent, dispersant, suspension, stabilizer, isotonic agent, solvent or emulsifier that is licensed by the relevant government authority for use in humans or livestock.

In some embodiments, the active ingredient of the pharmaceutical composition of the present disclosure, in addition to the compound of Formula I of the present disclosure, a pharmaceutically acceptable salt or an isomer thereof, may also contain other known anticancer agents, including but not limited to taxane, vinca alkaloids, paclitaxel, docetaxel, vincristine, vinblastine, vinorelbine, vinofonin, cisplatin, 5-fluorouracil, 5-fluoro-2-4 (1H,3H)-pyrimidinedione (5FU), flutamide and gemcitabine, etc.

The present disclosure relates to use of the compound of Formula I of the present disclosure, a pharmaceutically acceptable salt and an isomer thereof or the pharmaceutical composition comprising the compound of Formula I of the present disclosure, a pharmaceutically acceptable salt and an isomer thereof in the treatment or prevention of WDR5-mediated diseases described herein, or in the preparation of drugs for the treatment or prevention of WDR5-mediated diseases described herein. The present disclosure also provides a compound of Formula I of the present disclosure, a pharmaceutically acceptable salt and an isomer thereof, or a pharmaceutical composition containing the compound of Formula I of the present disclosure, a pharmaceutically acceptable salt and an isomer thereof for treating or preventing WDR5-mediated diseases described herein.

The present disclosure is further explained below in conjunction with specific Examples. It should be understood that these Examples are intended only to illustrate the present disclosure and are not intended to limit the scope of the present disclosure. In the following Examples, unless otherwise stated, experimental conditions are usually according to the conventional conditions, or according to the conditions recommended by the manufacturer. Unless otherwise stated, percentages and parts are calculated by weight.

The starting materials used in the following Examples may be purchased from chemical sellers such as Aldrich, TCI, Alfa Aesar, Bide, Energy, etc., or may be synthesized by known methods.

The meanings of the English abbreviations involved in the following Examples are described in the following table.

| | | | |
|---|---|---|---|
| $Ti(OEt)_4$ | tetraethyl titanate | DMF | N,N-dimethylformamide |
| $LiBH_4$ | lithium borohydride | $Na_2CO_3$ | sodium carbonate |
| TFA | trifluoroacetic acid | EtOH | ethanol |
| LDA | lithium N, N-diisopropylamide | $CBr_4$ | tetrabromomethane |
| $Pd(AmPhos)_2Cl_2$ | Dichlorobis[di-tert-butyl(p-dimethylaminophenyl) phosphino]palladium (II) | $Ph_3P$ | triphenylphosphine |
| $Cs_2CO_3$ | cesium carbonate | NBS | N-bromosuccinimide |
| DMAc or DMA | N,N-dimethylacetamide | BPO | benzoyl Peroxide |

(continued)

| | | | |
|---|---|---|---|
| THF | tetrahydrofuran | TMP | 2,2,6,6-Tetramethylpiperidine |
| DCM | dichloromethane | $PBr_3$ | Phosphorus tribromide |
| MszO | methanesulfonic anhydride | $CCl_4$ | tetrachloromethane |
| n-BuLi | n-butyl lithium | $N_2H_4$ | hydrazine |
| Dibal-H | diisobutylaluminium hydride | $H_2SO_4$ | Sulfuric acid |
| Dioxane | 1,4-dioxane | $POCl_3$ | Phosporus Oxychoride |
| PPA | Polyphosphoric acid | $Pd(OAc)_2$ | Palladiumacetate |
| $NaBH_4$ | Sodium borohydride | EtONa | sodium ethoxide |
| MeOH | methanol | $Pd_2(dba)_3$ | Tris(dibenzylideneacetone) dipalladi um |
| $Et_3N$ or TEA | triethylamine | XantPhos | 4,5-Bis(diphenylphosphino)-9,9-dimethylxanthene |
| DIPEA | N,N-diisopropylethanamine | $CH_3CN$ | acetonitrile |
| HCl | hydrochloride | $BOC_2O$ | di-tertbutyl dicarbonate |
| PMB-Br | 4-methoxybenzyl bromide | $K_2CO_3$ | Potassium carbonate |
| DMAP | 4-dimethylaminopyridine | DMF-DMA | N,N-dimethylformamidedimethyl acetal |

In the following Examples, ice bath refers to -5 °C to 0 °C; room temperature refers to 10 °C to 30 °C, and reflux temperature generally refers to the reflux temperature of the solvent under normal pressure. "reacted overnight" generally refers to reacting for 8-15 hours. In the following Examples, unless the operating temperature is otherwise specified, it is carried out at room temperature.

In the following Examples, separation and purification of the intermediate and the final product is carried out through normal-phase or reversed-phase flash column or other suitable methods. Normal-phase flash column uses ethyl acetate and n-hexane or methanol and methylene chloride as mobile phases. Reversed-phase preparative high-pressure liquid chromatography (HPLC) uses a C18 column and is detected at UV 214 nm and 254 nm in mobile phase of A (water and 0.1% formic acid), B (acetonitrile), or mobile phase of A (water and 0.1% ammonium bicarbonate), B (acetonitrile).

In each Example: LCMS instrument: pump: Agilent 1260;

UV detector: Agilent 1260 DAD Mass Spectrometer API 3000;
chromatography column: Waters sunfire C18, 4.6×50mm, 5um;
Mobile phase: A-$H_2O$ (0.1% HCOOH); B-acetonitrile NMR;
instrument: Bruker Ascend 400M (1H NMR:400MHz; 13C NMR:100 MHz).
Synthesis of Intermediate A1: synthesis of 2-(1-bromoethyl)-5-fluoro-4-ethoxypyridine

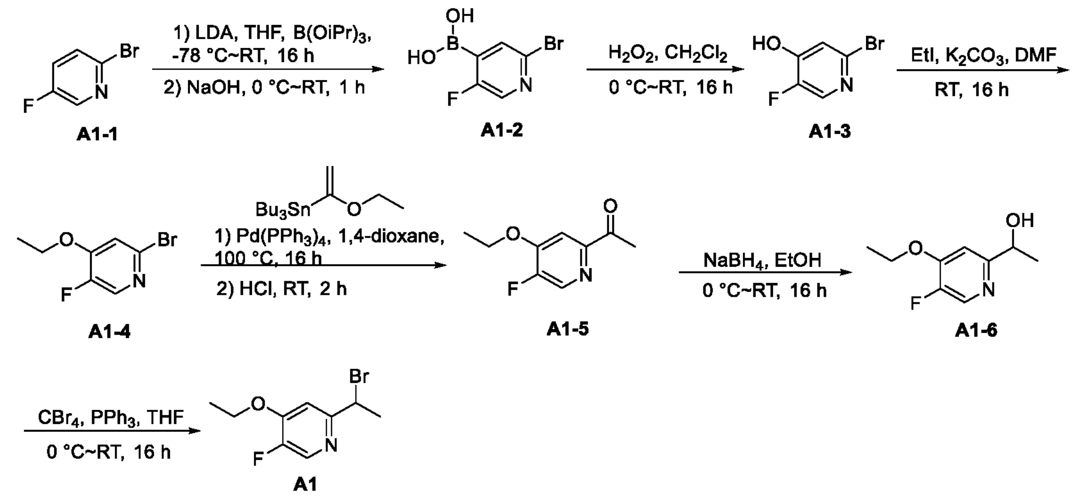


Step 1: at -78°C, under argon protection, LDA (2M, in tetrahydrofuran, 434 mL, 868.0 mmol) was add dropwise into a solution of 2-bromo-5-fluoropyridine (**A1-1**, 102 g, 580.0 mmol) in tetrahydrofuran (1.0 L). After the reaction was performed at this temperature for 1 hour, triisopropyl borate (163 g, 870.0 mmol) was added. After 30 mins at this temperature, the reaction mixture was brought to room temperature and left to react for 16 hours. After the reaction liquid was cooled to 0°C and an aqueous solution of sodium hydroxide (4M, 2.5 L) was added, the reaction mixture was brought to room temperature and stirred for 1 hour. The water phase was separated, adjusted to pH 3 with 6M aqueous HCl and extracted with ethyl acetate (3 * 1.5 L). The organic phase was washed with saturated saline, dried, filtered and concentrated to obtain a crude product of (2-bromo-5-fluoropyridine-4-yl) boric acid (**A1-2**, 114.5 g, yield: 89.9%).

LCMS: m/z 222.0 $[M+H]^+$.

Step 2: at 0°C, hydrogen peroxide (30% aq., 177.1 g, 1562.7 mmol) was added to a suspension of (2-bromo-5-fluoropyridine-4-yl) boric acid (**A1-2**, 114.5 g, 520.9 mmol) in dichloromethane (1.5 L). After the completion of addition, the reaction mixture was brought to room temperature and left to react for 16 hours. The reaction liquid was concentrated to remove solvent. Then water (500 mL) and ethyl acetate (1.0 L) were added. The water phase and the organic phase were separated. The water phase was extracted with ethyl acetate (2 * 1.0 L). The organic phase was combined and concentrated to dryness to obtain a crude product of 2-bromo-5-fluoropyridine-4-ol (**A1-3**, 96 g, yield: 96.0%).

LCMS: m/z 192.1 $[M+H]^+$.

Step 3: potassium carbonate (138.1 g, 1000 mmol) and ethyl iodide (234 g, 1500 mmol) were added into a solution of 2-bromo-5-fluoropyridine-4-ol (**A1-3**, 96 g, 500 mmol) in DMF (600 mL) and reacted at room temperature for 16 hours. The reaction liquid was poured into saturated saline, extracted with ethyl acetate (2 * 1000 mL). The organic phase was washed with water (3 * 1500 mL) and saturated saline (2 * 1500 mL) in sequence, dried, filtered, and concentrated under reduced pressure to dryness. After column chromatography purification, a colorless liquid of 2-bromo-4-ethoxy-5-fluoropyridine (**A1-4**, 85 g, yield:77.3%) was obtained.

LCMS: m/z 222.0 $[M+H]^+$.

Step 4: tetrakis (triphenylphosphine)palladium (7.3 g, 0.03 mmol) was added into a solution of 2-bromo-4-ethoxy-5-fluoropyridine (**A1-4**, 42 g, 190.9 mmol) and tributyl(1-ethoxyvinyl) stannane (103.4 g, 286.4 mmol) in 1,4-dioxane under argon protection. The reaction mixture was brought to 100°C and left to react for 16 hours. The reaction liquid was concentrated under reduced pressure to remove solvent, diluted with ethyl acetate (800 mL), added with a saturated aqueous solution of potassium fluoride, and stirred at room temperature for 1 hour. The water phase and the organic phase were separated. The water phase was extracted with ethyl acetate (500 mL). The organic phase was combined, washed with saturated saline, dried, filtered, and concentrated under reduced pressure to dryness. The residue was dissolved in tetrahydrofuran (500 mL), added with 2M aqueous HCl (382 mL, 764.0 mmol) and reacted at room temperature for 2 hours. The reaction liquid was separated. The water phase was extracted with dichloromethane (2 * 1000 mL). The organic phase was combined, washed with saturated saline, dried, filtered, and concentrated under reduced pressure to dryness. After column chromatography purification, **A1-5**(32.5 g, yield:93.0%) was obtained.

LCMS: m/z 184.1 $[M+H]^+$.

Step 5: 1-(5-fluoro-4-ethoxypyridine-2-yl)ethan-1-one (**A1-5**, 28.4 g, 155.0 mmol) was dissolved in ethanol (560 mL), cooled to 0 °C, and added with sodium borohydride (5.85 g, 155.0 mmol) in three portions. After the end of addition, the reaction mixture was kept reacting at 0°C for 10 mins, then brought to room temperature and left to react overnight. The reaction liquid was poured into ice water and extracted with dichloromethane (4 * 1000 mL). The organic phase was combined, washed with saturated saline, dried, filtered, and concentrated under reduced pressure to dryness. After column chromatography purification, a light yellow oil of 1-(5-fluoro-4-ethoxypyridine2-yl)ethan-1-ol (**A1-6**, 25 g,

yield:87.1%) was obtained.

LCMS: m/z 186.2 $[M+H]^+$.

Step 6: a solution of carbon tetrabromide (65.0 g, 196.0 mmol) in tetrahydrofuran (100 mL) was added into a solution of 1-(5-fluoro-4-ethoxypyridine2-yl)ethan-1-ol (**A1-6**, 24.2 g, 130.7 mmol) and triphenylphosphine (61.7 g, 235.2 mmol) in tetrahydrofuran (500 mL) at 0°C under argon protection. After the end of addition, the reaction mixture was kept reacting at 0°C for 10 mins, then brought to room temperature and left to react overnight. The reaction liquid was filtered. The filter cake was washed with tetrahydrofuran. The filtrate was concentrated under reduced pressure to dryness. After column chromatography purification, a light yellow oil **A1** (24 g, yield:74.1%) was obtained.

LCMS: m/z 247.9 $[M+H]^+$.

$^1H$ NMR (400 MHz, chloroform-*d*), δ ppm 8.26 (d, *J* = 2.8 Hz, 1H), 7.06 (d, *J* = 6.4 Hz, 1H), 5.19~5.14 (m, 1H), 4.23~4.18 (m, 2H), 2.05 (d, *J* = 7.2 Hz, 3H), 1.50 (t, *J* = 6.8 Hz, 3H).

Synthesis of Intermediate A2: synthesis of 6-(1-bromoethyl)-4-methoxynicotinonitrile

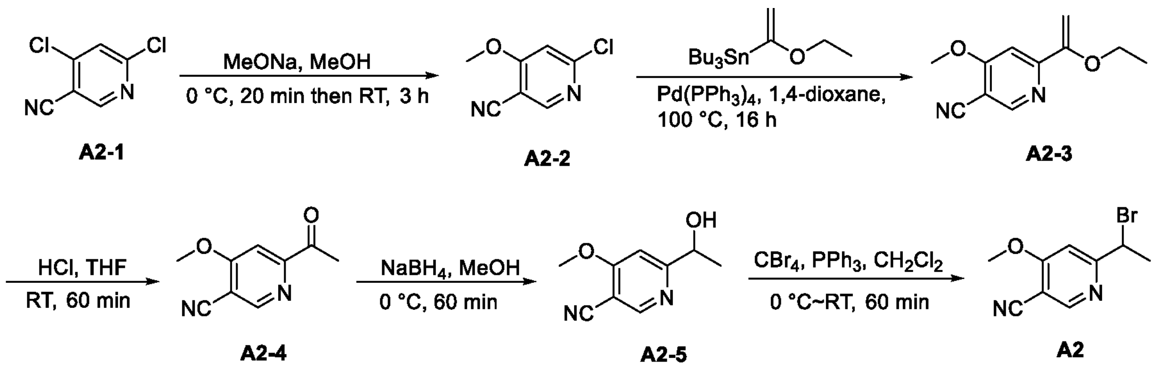


Step 1: sodium methoxide (5.5 g, 101.7 mmol) was added into a solution of 4,6-dichloronicotinonitrile (**A2-1**, 16.0 g, 92.5 mmol) in methanol (200 mL) at 0°C under argon protection. After the end of addition, the reaction mixture was reacted at 0°C for 20 mins, then brought to room temperature and left to react for 3 hours. The reaction liquid was poured into a saturated aqueous ammonium chloride solution, extracted with ethyl acetate (3 * 200 mL). The organic phase was washed with saturated saline, dried, filtered and concentrated to dryness. After column chromatography purification (petroleum ether: ethyl acetate = 10:1), a white solid of 6-chloro-4-methoxynicotinonitrile (**A2-2**, 9.8 g, yield: 62.8%) was obtained.

$^1H$ NMR (400 MHz, chloroform-*d*): δ ppm 8.47 (s, 1H), 6.96 (s, 1H), 4.02(s, 3 H).

Step 2: tetrakis (triphenylphosphine) palladium (3.78 g, 3.26 mmol) was added into a solution of 6-chloro-4-methoxynicotinonitrile (**A2-2**, 11 g, 65.2 mmol) and tributyl (1-ethoxyvinyl) stannane (35.3 g, 97.8 mmol) in 1,4-dioxane under argon protection. The reaction mixture was brought to 100°C and left to react for 16 hours. The reaction liquid was concentrated under reduced pressure to remove solvents, diluted with ethyl acetate (300 mL), added with a saturated aqueous solution of potassium fluoride, stirred at room temperature for 1 hour. The water phase and the organic phase were separated. The water phase was extracted with ethyl acetate (300 mL). The organic phase was combined, washed with saturated saline, dried, filtered, and concentrated under reduced pressure to dryness to obtain a crude product of 6-(1-ethoxyvinyl)-4-methoxynicotinonitrile (**A2-3**, 14 g).

LCMS: m/z 205.1 $[M+H]^+$.

Step 3: 6-(1-ethoxyvinyl)-4-methoxynicotinonitrile (**A2-3**, 14 g, 65.2 mmol) was dissolved in tetrahydrofuran (200 mL), added with aqueous HCl (2M, 130 mL, 260 mmol) and reacted for 60 mins at room temperature. After diluted with ethyl acetate (300 mL), the reaction liquid was washed with saturated sodium bicarbonate and saturated saline in sequence, dried, filtered, and concentrated under reduced pressure to dryness. After column chromatography purification (petroleum ether: ethyl acetate =8:1), a white solid of 6-acetyl-4-methoxynicotinonitrile (**A2-4**, 8.5 g, two step yield: 73.9%) was obtained.

LCMS: m/z 177.0 $[M+H]^+$.

Step 4: sodium borohydride (2.7 g, 71.63 mmol) was added into a solution of 6-acetyl-4-methoxynicotinonitrile (**A2-4**, 8.5 g, 47.75 mmol) in methanol (50 mL) in ice-water bath and reacted for 60 mins at 0°C. The reaction was quenched with ice water. The reaction liquid was concentrated under reduced pressure to remove most of methanol solvent, diluted with ethyl acetate (80 mL), washed with saturated saline (3 * 80 mL), dried with anhydrous sodium sulfate and filtered. The filtrate was concentrated under reduced pressure to dryness. After column chromatography purification

(petroleum ether: ethyl acetate =1:1), a light yellow solid of 6-(1-hydroxylethyl)-4-methoxynicotinonitrile (**A2-5**, 6.8 g, yield:79.1%) was obtained.

LCMS: m/z 179.1 $[M+H]^+$.

Step 5: a solution of triphenylphosphine (12.36 g, 47.19 mmol) in dichloromethane (30 mL) was added into a solution of 6-(1-hydroxylethyl)-4-methoxynicotinonitrile (**A2-5**, 5.6 g, 31.46 mmol) and carbon tetrabromide (15.63 g, 47.19 mmol) in dichloromethane (120 mL) at 0°C under argon protection and reacted for 60 mins at 0°C under stirring. The reaction liquid was concentrated to dryness. After column chromatography purification (petroleum ether: ethyl acetate =5:1), a white solid of 6-(1-bromoethyl)-4-methoxynicotinonitrile (**A2**, 3.5 g, yield:46.2%) was obtained.

$^1$H NMR (400 MHz, chloroform-*d*): δ ppm 8.62 (s, 1H), 7.06 (s, 1H), 5.17-5.15 (m, 1H), 4.04 (s, 3H), 2.07 (d, *J* = 7.2 Hz, 3H).

Synthesis of Intermediate **A3**: Synthesis of 6-(1-bromoethyl)-4-ethoxynicotinonitrile

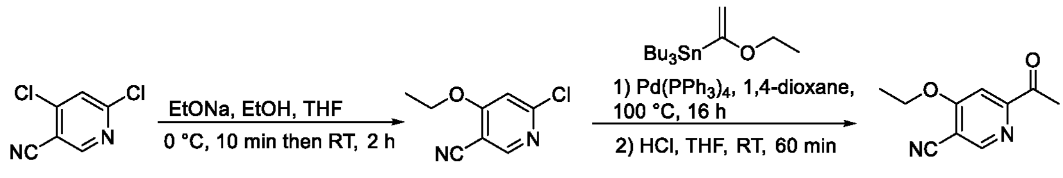


**A2-1** **A3-2** **A3-3**

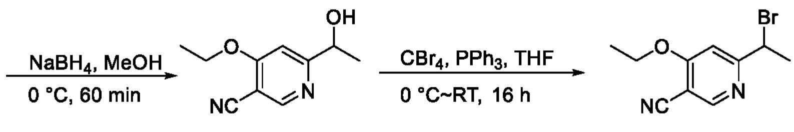


**A3-4** **A3**

Step 1: a solution of sodium ethoxide in ethanol (20%, 101.3 g, 121.4 mmol) was added into a solution of 4,6-dichloronicotinonitrile (**A2-1**, 20.0 g, 115.6 mmol) in tetrahydrofuran (400 mL) at 0°C, under argon protection. After the end of addition, the reaction mixture was reacted for 10 mins, then brought to room temperature and left to react for 2 hours. The reaction liquid was poured into a saturated aqueous solution of ammonium chloride, extracted with ethyl acetate (3 * 200 mL). The organic phase was combined, washed with saturated saline, dried and filtered. The filtrate was concentrated under reduced pressure to dryness. After column chromatography purification (ethyl acetate/petroleum ether=1/20), a white solid of 6-chloro-4-ethylnicotinonitrile (**A3-2**, 16.5 g, yield:78.2 %) was obtained.

LCMS: m/z 183.0 $[M+H]^+$.

$^1$H NMR (400 MHz, chloroform-*d*): δ ppm 8.47 (s, 1H), 6.93 (s, 1H), 4.27 (q, *J* = 6.8 Hz, 2H), 1.54 (t, *J* = 7.2 Hz, 3H).

Step 2: tetrakis (triphenylphosphine)palladium (5.2 g, 4.52 mmol) was added into a solution of 6-chloro-4-ethylnicotinonitrile (**A3-2**, 16.5 g, 90.36 mmol) and tributyl (1-ethoxyvinyl) stannane (49.0 g, 135.54 mmol) in 1,4-dioxane (300 mL) under argon protection. The reaction mixture was brought to 100°C and left to react for 16 hours. The reaction liquid was concentrated under reduced pressure to remove solvents, diluted with ethyl acetate (150 mL), added with a saturated aqueous solution of potassium fluoride, and stirred at room temperature for 1 hour. Then the water phase and the organic phase were separated. The water phase was extracted with ethyl acetate (200 mL). The organic phase was combined, washed with saturated saline, dried and filtered. The filtrate was concentrated under reduced pressure to dryness. The residue was dissolved in tetrahydrofuran (100 mL), added with aqueous HCl (2M, 120 mL, 240 mmol) and reacted at room temperature for 1 hour. The reaction liquid was diluted with ethyl acetate (300 mL), washed with saturated sodium bicarbonate and saturated saline in sequence, dried, filtered, and concentrated under reduced pressure to dryness. After column chromatography purification (ethyl acetate/petroleum ether=1/30), a white solid of 6-acetyl-4-ethoxynicotinonitrile (**A3-3**, 16.6 g, yield:96.6 %) was obtained.

LCMS: m/z 191.1 $[M+H]^+$

$^1$H NMR (400 MHz, chloroform-*d*): δ ppm 8.72 (s, 1H), 7.61 (s, 1H), 4.35~4.30 (q, *J* = 7.2 Hz, 2H), 2.72 (s, 3H), 1.54 (t, *J* = 7.2 Hz, 3H).

Step 3: sodium borohydride (3.3 g, 87.28 mmol) was added into a solution of 6-acetyl-4-ethoxynicotinonitrile (**A3-3**, 16.6 g, 87.28 mmol) in methanol (400 mL) at 0°C and reacted at 0°C for 1 hour. The reaction was quenched with ice water. The reaction liquid was concentrated under reduced pressure to remove most of methanol solvent, diluted with ethyl acetate (150 mL), washed with saturated saline (3 * 50 mL), dried with anhydrous sodium sulfate and filtered. The filtrate was concentrated under reduced pressure to dryness. After silica gel chromatography (ethyl acetate /petroleum ether=1/10) purification, a light yellow solid of 4-ethoxy-6-(1-hydroxylethyl) nicotinonitrile (**A3-4**, 12.3 g, yield: 73.3%) was obtained.

LCMS: m/z 193.0 $[M+H]^+$.

$^1H$ NMR (400 MHz, DMSO-d6): δ ppm 8.71 (s, 1H), 7.31 (s, 1H), 5.63 (d, *J* = 4.4 Hz, 1H), 4.76~4.69 (m, 1H), 4.36~4.28 (m, 2H), 1.41~1.36 (m, 6H).

Step 4: at 0 °C under argon protection, carbon tetrabromide (31.8 g, 95.98 mmol) was added into a solution of 4-ethoxy-6-(1-hydroxylethyl) nicotinonitrile (**A3-4**, 12.3 g, 63.99 mmol) and triphenylphosphine (33.6 g, 127.98 mmol) in tetrahydrofuran (400 mL) and reacted for 5 mins at 0°C. Then the reaction mixture was brought to room temperature and left to react for 16 hours under stirring. The reaction liquid was filtered. The filtrate was concentrated under reduced pressure to dryness. After silica gel chromatography (ethyl acetate: petroleum ether=1/20) purification, a light yellow solid of 6-(1-bromoethyl)-4-ethoxynicotinonitrile (**A3**, 14.2 g, yield:86.9 %) was obtained.

LCMS: m/z 255.0 $[M+H]^+$.

$^1H$ NMR (400 MHz, DMSO-d6): δ ppm 8.81 (s, 1H), 7.47 (s, 1H), 5.46 (q, *J* = 6.8 Hz, 1H), 4.39~4.28 (m, 2H), 1.99 (d, *J* = 6.8 Hz, 3H), 1.39 (t, *J* = 7.2 Hz, 3H).

Synthesis of Intermediate **A4**: Synthesis of 5-(1-bromoethyl)-2-fluoro-3-methoxypyridine

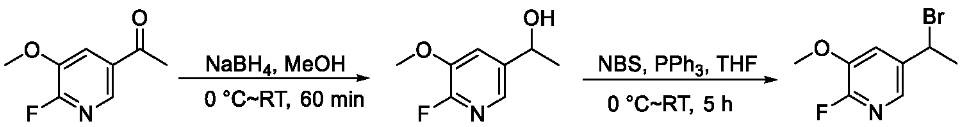


**A4-1** **A4-2** **A4**

Step 1: sodium borohydride (671 mg, 17.73 mmol) was added into a solution of 1-(6-fluoro-5-methoxypyridin-3-yl)ethan-1-one (**A4-1**, 3.0 g, 17.73 mmol) in methanol (30 mL) in an ice-water bath and reacted at room temperature for 60 mins under stirring. The reaction liquid was quenched with ice water, diluted with ethyl acetate (100 mL), washed with saturated saline (3 * 100 mL), dried with anhydrous sodium sulfate and filtered. The filtrate was concentrated under reduced pressure. After silica gel chromatography (0 to 50% gradient, ethyl acetate: petroleum ether) purification, a colorless oil of **A4-2** (2.9 g, yield: 96%) was obtained.

Step 2: triphenylphosphine (6.0 g, 22.78 mmol) and NBS (4.1 g, 22.78 mmol) were added into a solution of 1-(6-fluoro-5-methoxypyridine-3-yl)ethan-1-ol (**A4-2**, 2.6 g, 15.19 mmol) in dichloromethane(40 mL) at 0°C, under argon protection and reacted at room temperature under stirring for 5 hours. The reaction liquid was diluted with dichloromethane (100 mL), washed with saturated saline (3 * 100 mL), dried with anhydrous sodium sulfate and filtered. The filtrate was concentrated under reduced pressure. The residue was purified by silica gel chromatography (0 to 20% gradient, ethyl acetate: petroleum ether) to obtained a solid **A4** (2.5 g, yield: 70%).

$^1H$ NMR (400 MHz, chloroform-*d*): δ ppm 7.74 (s, 1H), 7.39 (dd, *J* =9.6, 2.0 Hz, 1H), 5.18 (q, *J* = 7.2 Hz, 1H), 3.94 (s, 3H), 2.06 (d, *J* = 7.2 Hz, 3H).

$^{19}F$ NMR (376 MHz, DMSO-d6): δ ppm -84.25.

Synthesis of Intermediate **A5**: Synthesis of 2-(1-bromoethyl)-5-fluoro-4-methoxypyridine

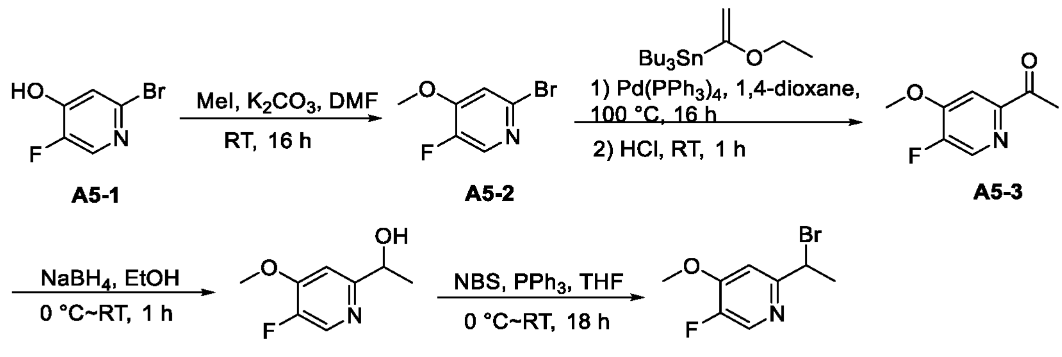


A5-1 A5-2 A5-3

A5-4 A5

Step 1: potassium carbonate (10.8 g, 78.2 mmol) and methyl iodide (11.0 g, 77.5 mmol) were added into a solution of 2-bromo-5-fluoropyridine-4-ol (**A5-1**, 6.0 g, 31.3 mmol) in DMF (50 mL) and reacted at room temperature for 16 hours. The reaction liquid was diluted with water (50 mL), extracted with ethyl acetate (2 * 150 mL). The organic phase was washed with water (3 * 200 mL) and saturated saline (2 * 200 mL), dried and filtered. The filtrate was concentrated under reduced pressure to dryness. After column chromatography purification, a white solid of **A5-2** (2.6 g, yield: 40.4%) was obtained.

LCMS: m/z 206.1 $[M+H]^+$.

Step 2: tetrakis (triphenylphosphine)palladium (1.0 g, 0.87 mmol) was added into a solution of 2-bromo-5-fluoro-4-methoxypyridine (**A5-2**, 2.6 g, 12.6 mmol) and tributyl(1-ethoxyvinyl) stannane (6.8 g, 18.9 mmol) in dioxane under argon protection. The reaction mixture was brought to 100°C and left to react for 16 hours. The reaction liquid was concentrated under reduced pressure to remove solvents, diluted with ethyl acetate (100 mL), added with a saturated aqueous solution of potassium fluoride and stirred at room temperature for 1 hour. The water phase and the organic phase were separated. The water phase was extracted with ethyl acetate (100 mL). The organic phase was combined, washed with saline, dried, filtered, and concentrated under reduced pressure to dryness. The residue was dissolved in tetrahydrofuran (50 mL), added with aqueous HCl (2M, 25.5 mL, 51.0 mmol), and reacted at room temperature for 1 hour. The reaction liquid was diluted with ethyl acetate (100 mL), then washed with saturated sodium bicarbonate and saturated saline in sequence, dried, filtered, and concentrated under reduced pressure to dryness. After column chromatography purification, a white solid of **A5-3** (1.8 g, yield:84.3%) was obtained.

LCMS: m/z 170.1 $[M+H]^+$.

$^1$H NMR (400 MHz, chloroform-*d*), δ ppm 8.37 (d, *J* = 2.8 Hz, 1H), 7.72 (d, *J* = 6.8 Hz, 1H), 4.00 (s, 3H), 2.69 (s, 3H).

Step 3: sodium borohydride (5.75 g, 152.5 mmol) was added into a solution of 1-(5-fluoro-4-methoxypyridine-2-yl) acetone (**A5-3**, 12.9 g, 76.3 mmol) in methanol (100 mL) in batches at 0°C under argon protection and reacted at room temperature under stirring for 1 hour. The reaction was quenched with ice water, extracted with ethyl acetate (3 * 100 mL), washed with saturated saline (100 mL), dried with anhydrous sodium sulfate, filtered and concentrated. After silica gel column chromatography (dichloromethane: methanol=40 :1), a yellow solid of 1-(5-fluoro-4-methoxypyridine-2-yl)ethan-1-ol (**A5-4**, 6.1 g, yield: 46.7%) was obtained.

LCMS: m/z 172.1 $[M+H]^+$.

Step 4: triphenylphosphine (2.14 g, 8.18 mmol), NBS (1.25 g, 7.01 mmol) were added into a solution of **A5-4** (1.0 g, 5.84 mmol) in dichloromethane (10 mL) in sequence at 0°C under argon protection and reacted at room temperature under stirring for 18 hours. The reaction was quenched with ice water, extracted with ethyl acetate (3 * 50 mL), washed with saturated saline (50 mL), dried with anhydrous sodium sulfate, filtered and concentrated. After silica gel column chromatography (petroleum ether: ethyl acetate = 30:1), a white solid of **A5** (770 mg, yield:56.3%) was obtained.

LCMS: m/z 234.0 $[M+H]^+$.

Synthesis of Intermediate **A6**: Synthesis of 4-(1-bromomethyl)-1-fluoro-2-methoxybenzene

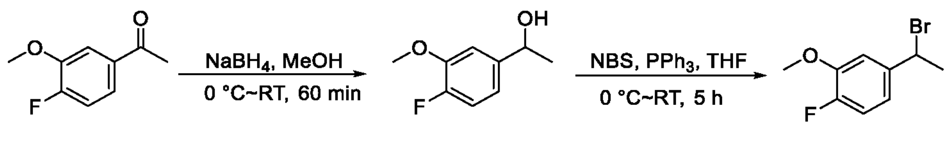


A6-1 A6-2 A6

Referring to the synthesis of **A4**, **A6** was synthesized by two steps with **A6**-1 instead of **A4**-1: LCMS: m/z 232.99 $[M+H]^+$.

Synthesis of Intermediate A7: 3-(chloromethyl)-5-methoxy-1-methyl-1H-pyrazole

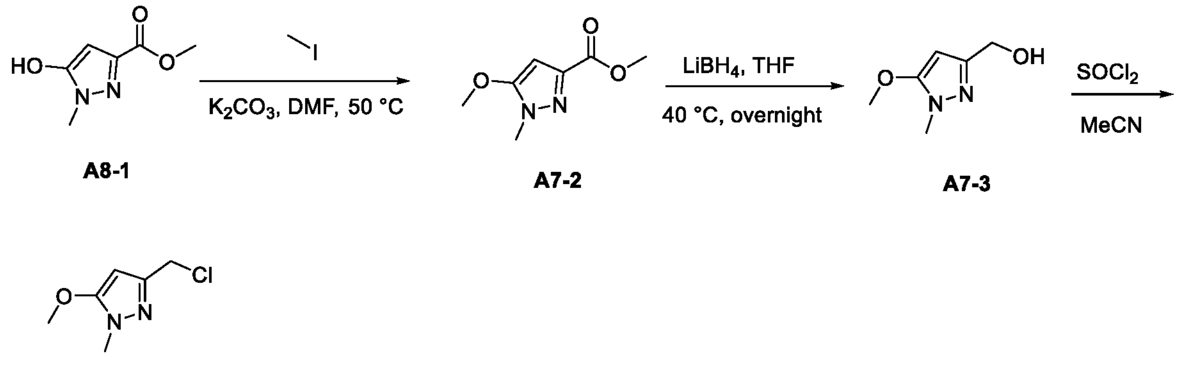


A8-1 A7-2 A7-3

A7

Referring to Synthesis of Intermediate A8, A7 was synthesized by four steps.
LCMS: m/z 161.04 $[M+H]^+$ .

Synthesis of Intermediate **A8**: 3-(chloromethyl)-5-ethoxy-1-methyl-1H-pyrazole

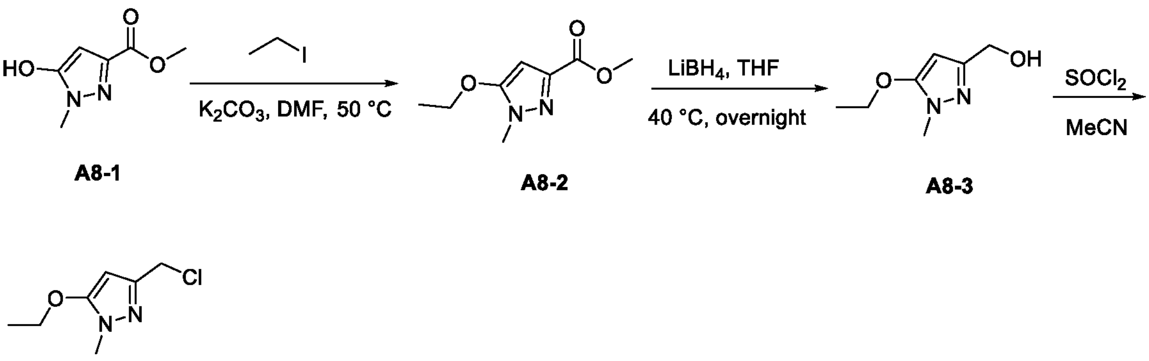


A8-1 A8-2 A8-3

A8

Step 1: ethyl iodide (5.0 g, 32.02 mmol) was added dropwise into **A8-1** (1.0 g, 6.40 mmol) and potassium carbonate (2.66 g, 19.21 mmol) in DMF (10 mL), heated to 50 °C and reacted under stirring for 5 hours. The reaction liquid was diluted with ethyl acetate (30 mL), washed with saturated saline (3 * 50 mL), dried with anhydrous sodium sulfate and filtered. The filtrate was concentrated under reduced pressure. After silica gel chromatography (0 to 30% gradient, ethyl acetate: petroleum ether) purification, a white solid of **A8-2** (1.1 g, yield:93.2%) was obtained.
$^1$H NMR (400 MHz, chloroform-d): $\delta$ ppm 6.05 (s, 1H), 4.16 (q, *J* = 7.2 Hz, 2H), 3.90 (s, 3H), 3.72 (s, 3H), 1.44 (t, *J* = 7.2 Hz, 3H).
Step 2: lithium borohydride (2.0 M in THF, 5.4 mL, 10.86 mmol) was added dropwise into **A8-2** (1.0 g, 5.43 mmol) in tetrahydrofuran (15 mL), heated to 40 °C, and reacted under stirring overnight. The reaction was quenched with sodium sulfate decahydrate (3.0 g) and filtered. The filtrate was concentrated under reduced pressure. After silica gel chromatography (0 to 50% gradient, ethyl acetate: petroleum ether) purification, a colorless oil of **A8-3** (580 mg, yield: 68.4%) was obtained.
$^1$H NMR (400 MHz, chloroform-d): $\delta$ ppm 5.51 (s, 1H), 4.56 (s, 2H), 4.12 (q, *J* = 7.2 Hz, 2H), 3.61 (s, 3H), 1.42 (t, *J* = 7.2 Hz, 3H).
Step 3: thionyl chloride (714 mg, 6 mmol) was added into a solution of **A8-3** (312 mg, 2 mmol) in acetonitrile (10 mL) and reacted at room temperature for 3 h after the atmosphere was replaced with nitrogen. The reaction liquid

was dried by a rotary evaporator to obtain a crude product, neutralized with a saturated aqueous solution of sodium bicarbonate to pH=7 and extracted with dichloromethane (2×100 mL). The organic phase was combined, washed with saturated saline (100 mL), dried with anhydrous sodium sulfate and filtered. The filtrate was concentrated under reduced pressure to dryness. After silica gel column chromatography purification, a white solid of 3-(chloromethyl)-5-ethoxy-1-methyl-1H-pyrazole (260 mg, yield: 74.1 %) was obtained.

LCMS: m/z: 175.5 $[M+H]^+$.

Synthesis of Intermediate **A9:** Synthesis of 2-(1-bromoethyl)-4-cyclopropoxy-5-fluoropyridine

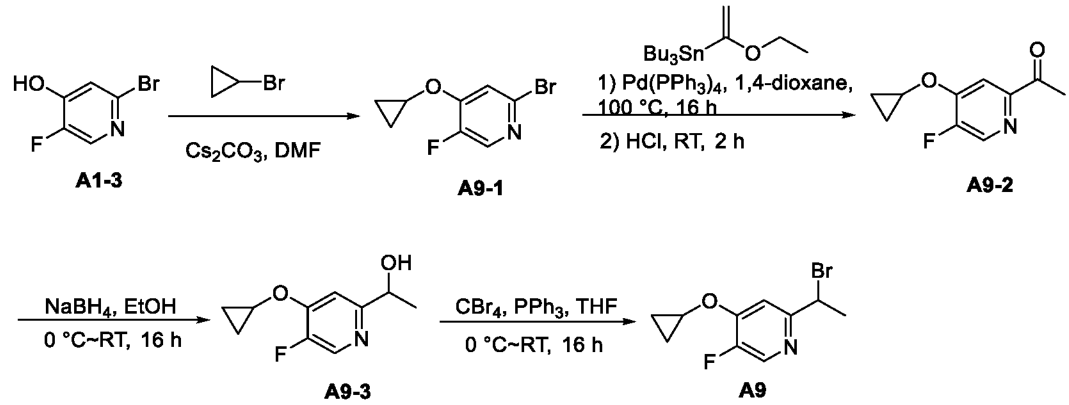


A1-3, A9-1, A9-2, A9-3, A9

Step 1: cesium carbonate (848 mg, 2.60 mmol), potassium iodide (190 mg, 1.15 mmol) and bromocyclopropane (1.76 g, 14.58 mmol) were added into a solution of **A1-3** (200 mg, 1.04 mmol) in DMA (10 mL) and reacted for 4 hours at 180°C under microwave. 16 parallel reaction batches were carried out. The reaction liquid was combined and diluted with ethyl acetate (200 mL) and water. The water phase and the organic phase were separated. The organic phase was washed with water (4 * 200 mL), washed with saturated saline (2 * 200 mL), dried with anhydrous sodium sulfate and then concentrated. After column chromatography purification (petroleum ether: ethyl acetate =50:1), a yellow liquid of **A9-1** (1.6 g, yield:41.3%) was obtained.

LCMS: m/z: 232.3 $[M+H]^+$ .

$^1H$ NMR (400 Hz, chloroform-d) δ ppm 8.09 (d, *J* = 2.4 Hz, 1 H), 7.38 (d, *J* = 6.0 Hz, 1 H), 3.89~3.85 (m, 1 H), 0.92~0.90 (m, 4 H).

$^{19}F$ NMR (376 Hz, chloroform-d) δ ppm -151.47.

Referring to the synthesis of Intermediate A1, **A9** was obtained from Intermediate A9-1 by three steps.

LCMS: m/z 260.0 $[M+H]^+$ .

Synthesis of Intermediate A13: Synthesis of 6-(chloromethyl)-4-ethoxynicotinonitrile

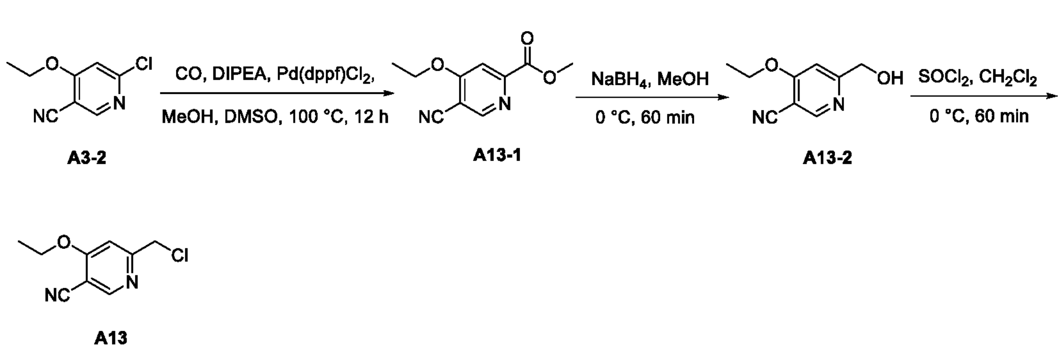


A3-2, A13-1, A13-2, A13

Step 1: [1,1'-bis(diphenylphosphino)ferrocene] palladium (II) chloride (321 mg, 0.44 mmol) and DIPEA (1.69 g, 13.17 mmol) were added into a mixed solution of **A3-2** (800 mg, 4.38 mmol) in methanol (15 mL) and dimethyl sulfoxide (50 mL). After the air was replaced with carbon monoxide, the reaction mixture was heated to 100°C and reacted for 12

hours. The reaction liquid was diluted with ethyl acetate (400 mL), added with saturated saline, stirred at room temperature for 1 hour. The water phase and the organic phase were separated. The water phase was extracted with ethyl acetate (300 mL). The organic phase was combined, washed with saturated saline, dried and filtered. The filtrate was concentrated under reduced pressure to dryness. After column chromatography purification, **A13-1** (180 mg, yield:19.9 %) was obtained.

LCMS: m/z: 207.1 $[M+H]^+$.

Step 2: sodium borohydride (75.6 mg, 2 mmol) was added into a solution of **A13-1** (206 mg, 1 mmol) in methanol (10 mL) in an ice-water bath and reacted for 60 mins at 0°C. The reaction was quenched with ice water. The reaction liquid was concentrated under reduced pressure to remove most of methanol solvent, then diluted with ethyl acetate (80 mL), washed with saturated saline (3 * 80 mL), dried with anhydrous sodium sulfate and filtered. The filtrate was concentrated under reduced pressure to dryness. silica gel chromatography (0 to 50% gradient, ethyl acetate: petroleum ether) purification, a light yellow oily product of **A13-2** (78 mg, yield: 43.8%) was obtained.

LCMS: m/z: 179.1 $[M+H]^+$.

Step 3: thionyl chloride (107 mg, 0.9 mmol) was added into a solution of **A13-2** (80 mg, 0.45 mmol) in dichloromethane (10 mL) in an ice-water bath and reacted at 0°C for 60 mins. The reaction liquid was concentrated under reduced pressure to remove most of solvents, and neutralized to pH=7 with a saturated aqueous solution of sodium bicarbonate, diluted with ethyl acetate (80 mL), washed with saturated saline (3 * 80 mL), dried with anhydrous sodium sulfate and filtered. The filtrate was concentrated under reduced pressure to dryness. After silica gel chromatography (0 to 50% gradient, ethyl acetate:petroleum ether) purification, a light yellow solid of **A13** (60 mg, yield: 68.0 %) was obtained.

LCMS: m/z: 197.1 $[M+H]^+$.

Synthesis of Intermediate A14: 2-(chloromethyl)-4-ethoxy-5-fluoropyridine

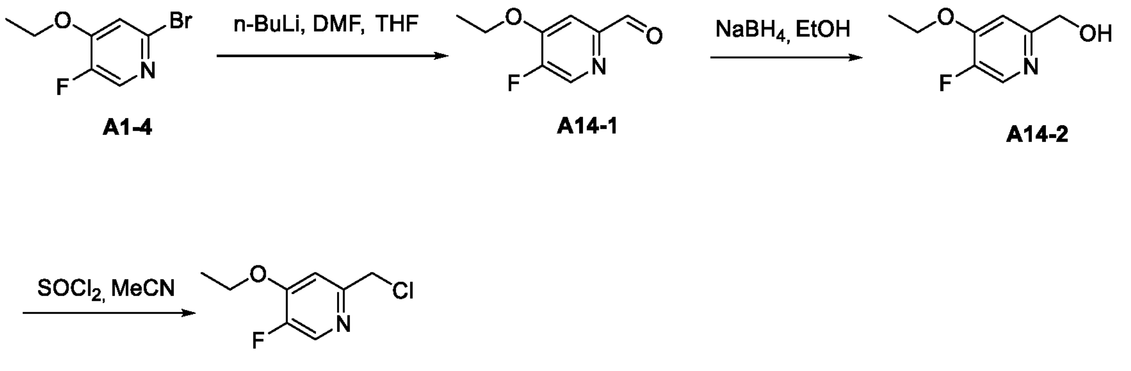


**A1-4** **A14-1** **A14-2**

**A14**

Step 1: n-BuLi (1.6 M in hexane, 11.5 ml, 18.41 mmol) was added dropwise into **A1-4** (2.70 g, 12.27 mmol) in anhydrous tetrahydrofuran (40 mL) at -75 °C under argon protection and reacted at -75 °C for 15 mins. Anhydrous DMF (1.42 ml, 2.05 mmol) was added and reacted at -75 °C for 15 mins. The reaction was quenched with water, extracted with ethyl acetate (3 * 60 mL). The organic phase was combined, washed with water, dried, filtered and concentrated. After column chromatography purification, **A14-1** (915 mg, yield:44.1%) was obtained.

LCMS: m/z: 170.0 $[M+H]^+$

Step 2: sodium borohydride (671 mg, 17.74 mmol) was added into a solution of **A14-1** (1.0 g, 5.91 mmol) in ethanol (20 mL) at 0 °C under argon protection and reacted at room temperature for 3 hours. The reaction was quenched with saturated aqueous solution ammonium chloride, added with water, extracted with ethyl acetate, dried and concentrated. After column chromatography purification, **A14-2** (690 mg, yield: 68.2%) was obtained.

LCMS: m/z:172.0 $[M+H]^+$ .

Step 3: thionyl chloride (0.32 ml, 4.39 mmol) was added dropwise into a solution of **A14-2** (250 mg, 1.46 mmol) in acetonitrile (10 mL) and reacted at room temperature for 1 hour. The reaction liquid was distilled under reduced pressure, added with dichloromethane, washed with sodium bicarbonate, dried and concentrated. After column chromatography purification, Intermediate **A14** (240 mg, yield:86.6%) was obtained.

LCMS: m/z: 190.0 $[M+H]^+$ .

Synthesis of Intermediate A15: 1-chloro-4-ethoxy-2,3-dihydro-1H-indene

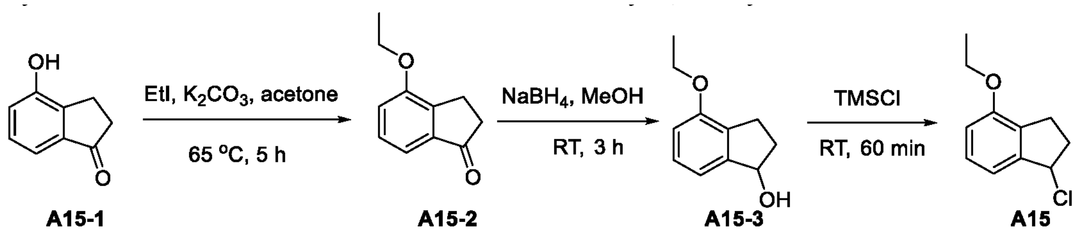


**A15-1** **A15-2** **A15-3** **A15**

Step 1: potassium carbonate (12.1 g, 87.74 mmol) and ethyl iodide (27.4 g, 175.49 mmol) were added into a solution of **A15-1**(2.6 g, 17.55 mmol) in acetone (100 mL) in sequence and reacted at 60°C for 5 hours. The reaction liquid was cooled to room temperature and then filtered. The filtrate was concentrated under reduced pressure. After column chromatography purification (petroleum ether: ethyl acetate = 20:1), **A15-2** (2.9 g, yield: 93.8 %) was obtained.

$^1H$ NMR (400 Hz, chloroform-d) δ ppm: 7.34~7.27 (m, 2H), 7.02~7.00 (m, 1H), 4.15 (q, *J* = 6.8 Hz, 2H), 3.05~3.02 (m, 2H), 2.68~2.66 (m, 2H), 1.46 (t, *J* = 7.2 Hz, 3H).

Step 2: sodium borohydride (690 mg, 18.16 mmol) was added into a solution of **A15-2** (3.2 g, 18.16 mmol) in methanol (50 mL) in batches in an ice-water bath and reacted at room temperature for 3 hours. After the reaction liquid was concentrated and purified through column chromatography (petroleum ether: ethyl acetate =10:1), **A15-3** (3.0 g, yield:92.7 %) was obtained.

$^1H$ NMR (400 Hz, chloroform-d) δ ppm: 7.21~7.17 (m, 1H), 7.01~6.99 (m, 1H), 6.75~6.73 (m, 1H), 5.23~5.20 (m, 1H), 4.08~4.03 (m, 2H), 3.04~2.97 (m, 1H), 2.77~2.69 (m, 1H), 2.50~2.44 (m, 1H), 1.95~1.87(m, 1H), 1.42 (t, J=7.2 Hz, 3 H).

Step 3: **A15-3** (1.0 g, 5.61 mmol) and trimethylchlorosilane (10 mL) were mixed and stirred at room temperature for 60 mins. The reaction liquid was poured into ice water and extracted with ethyl acetate (2 * 50 mL). The organic phase was combined, washed with saturated aqueous solution of NaCl (100 mL), dried with anhydrous sodium sulfate and concentrated to obtain Intermediate **A15** (1.1 g, yield: record as 100%), which was directly used in the next step.

Synthesis of Intermediate **A16:** 5-(chloromethyl)benzo[d][1,3]dioxane

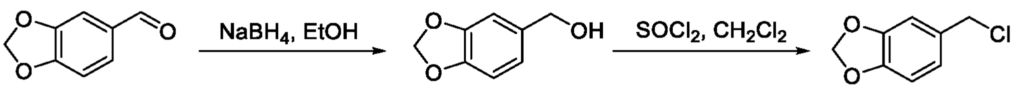


**A16-1** **A16-2** **A16**

Step 1: sodium borohydride (453 mg, 12.0 mmol) was added into a solution of **A16-1** (600 mg, 4.0 mmol) in ethanol (60 mL) at 0 °C and reacted at room temperature under stirring for 12 hours. The reaction liquid was poured into ice water (50 mL), extracted with dichloromethane (2 * 50 mL). The organic phase was combined, washed with saturated saline (100 mL), dried with anhydrous sodium sulfate and filtered. The filtrate was concentrated under reduced pressure to dryness. After column chromatography purification, a colorless oil of **A16-2** (450 mg, yield: 74.0 %) was obtained.

Step 2: thionyl chloride (714 mg, 3.0 mmol) was added dropwise into a solution of **A16-2** (304 mg, 2.0 mmol) in dichloromethane (50 mL) and reacted at room temperature for 4 hours. The reaction liquid was concentrated under reduced pressure to dryness. The resultant crude product was diluted with dichloromethane (100 mL), neutralized with a saturated aqueous solution of sodium bicarbonate to pH=7 and extracted with dichloromethane (2 * 100 mL). The organic phase was combined, washed with saturated saline (100 mL), dried with anhydrous sodium sulfate and filtered. The filtrate was concentrated under reduced pressure to dryness. After column chromatography purification, a colorless oil of **A16** (280 mg, yield: 82.3 %) was obtained.

Synthesis of Intermediate **A17:** 2-(1-bromoethyl)-4-(difluoromethoxy)-5-fluoropyridine

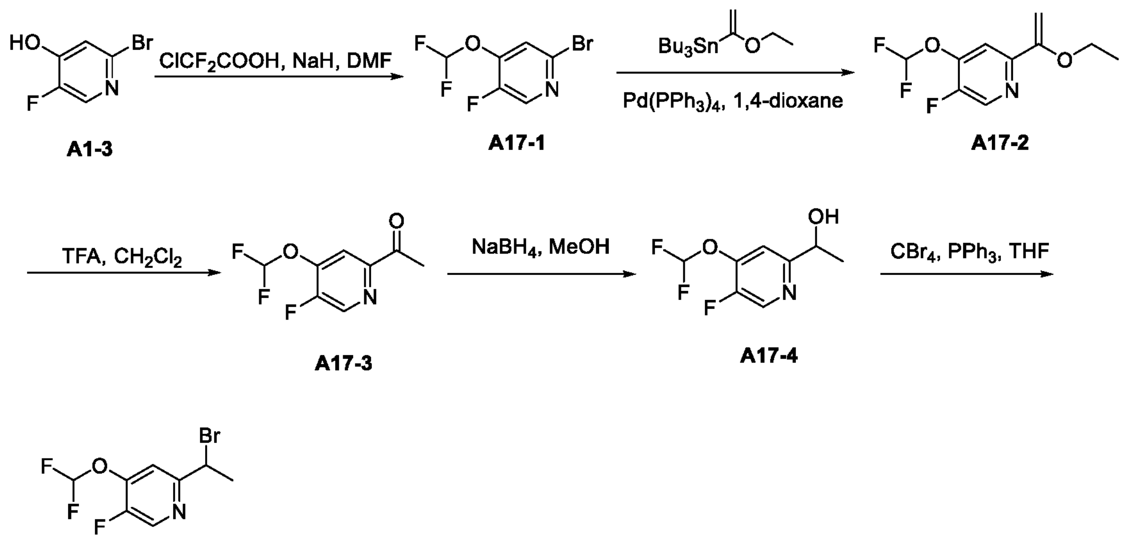


A1-3 A17-1 A17-2 A17-3 A17-4 A17

Step 1: sodium hydride (60% dispersed in oil, 2.87 g, 71.75 mmol) was added slowly into a solution of **A1-3** (4.59 g, 23.91 mmol) in N,N-dimethylformamide (135 mL) in an ice-water bath under argon protection. After the addition, the reaction mixture was kept stirring for 10 min, then added dropwise with chlorodifluoroacetic acid (4.66 g, 35.85 mmol) and reacted at 70°C overnight. The reaction was cooled to room temperature, quenched with water (80 mL), extracted with ethyl acetate (3 * 30 mL). The organic phase was combined, washed with saturated saline (2 * 20 mL), dried with anhydrous sodium sulfate, filtered and then concentrated under reduced pressure. After silica gel chromatography purification, a light yellow liquid of **A17-1** (622 mg, yield: 10.8%) was obtained.

LCMS: m/z: 242.0 $[M+H]^+$ .

Step 2: Compound **A17-1** (622 mg, 2.57 mmol), tributyl (1-ethoxyvinyl) stannane (1.39 g, 3.85 mmol), tetrakis(triphenylphosphine)palladium (149 mg, 0.13 mmol), 1,4-dioxane (40 mL) were added into a dry flask in sequence. After the addition, the reaction mixture was reacted at 100°C overnight under nitrogen protection. The reaction liquid was concentrated, dissolved with ethyl acetate (20 mL), added with a saturated aqueous solution of potassium fluoride (15 mL) and stirred at room temperature for 1 hour. The reaction liquid was filtered, added with water (30 mL) for separation and extracted. The water phase was extracted with ethyl acetate (2 * 15 mL). The organic phase was combined, washed with saturated saline (15 mL), dried with anhydrous sodium sulfate, filtered and then concentrated under reduced pressure. After silica gel chromatography purification, a yellow liquid of **A17-2** (590 mg, yield: 98.6%) was obtained.

LCMS: m/z: 234.0 $[M+H]^+$ .

Step 3: trifluoroacetic acid (6 mL) was added dropwise into a solution of **A17-2** (590 mg, 2.53 mmol) in dichloromethane (20 mL) in an ice-water bath and reacted at room temperature overnight. The reaction liquid was stirred in an ice bath, adjusted to weak alkaline with a sodium bicarbonate solution, added with water (15 mL) for separation and extracted. The water phase was washed with dichloromethane (2 * 15 mL). The organic phase was combined, washed with saturated saline (2 * 15 mL), dried with anhydrous sodium sulfate, filtered and then concentrated under reduced pressure. After silica gel chromatography purification, a colorless liquid **A17-3** (308 mg, yield: 59.3%) was obtained.

LCMS: m/z: 206.0 $[M+H]^+$ .

Step 4: sodium borohydride (114 mg, 3.0 mmol) was added slowly into a solution of **A17-3** (308 mg, 1.50 mmol) in methanol (10 mL) in an ice-water bath and reacted at room temperature overnight after the addition. The reaction liquid was quenched with water (20 mL) and extracted with ethyl acetate (3 * 20 mL). The organic phase was combined, washed with saturated saline (2 * 15 mL), dried with anhydrous sodium sulfate, filtered and then concentrated under reduced pressure. After Prep-TLC purification, a colorless oil of **A17-4** (295 mg, yield: 94.8%) was obtained.

LCMS: m/z: 208.0 $[M+H]^+$ .

Step 5: a solution of carbon tetrabromide (180 mg, 0.54 mmol) in tetrahydrofuran (1 mL) was added dropwise into a solution of **A17-4** (75 mg, 0.36 mmol) and triphenylphosphine (171 mg, 0.65 mmol) in tetrahydrofuran (4 mL) in an ice-water bath under nitrogen protection and reacted at room temperature overnight. The reaction liquid was added with water (15 mL) and extracted with ethyl acetate (20 mL * 3). The organic phase was combined, washed with saturated saline (2 * 15 mL), dried with anhydrous sodium sulfate, filtered and then concentrated under reduced pressure. After Prep-TLC purification, a light yellow solid of **A17** (72 mg, yield: 73.6%) was obtained.

LCMS: m/z: 269.9 $[M+H]^+$ .

Synthesis of Intermediate **A18:** 2-(1-bromoethyl)-4-(trifluoromethoxy)-5-fluoropyridine

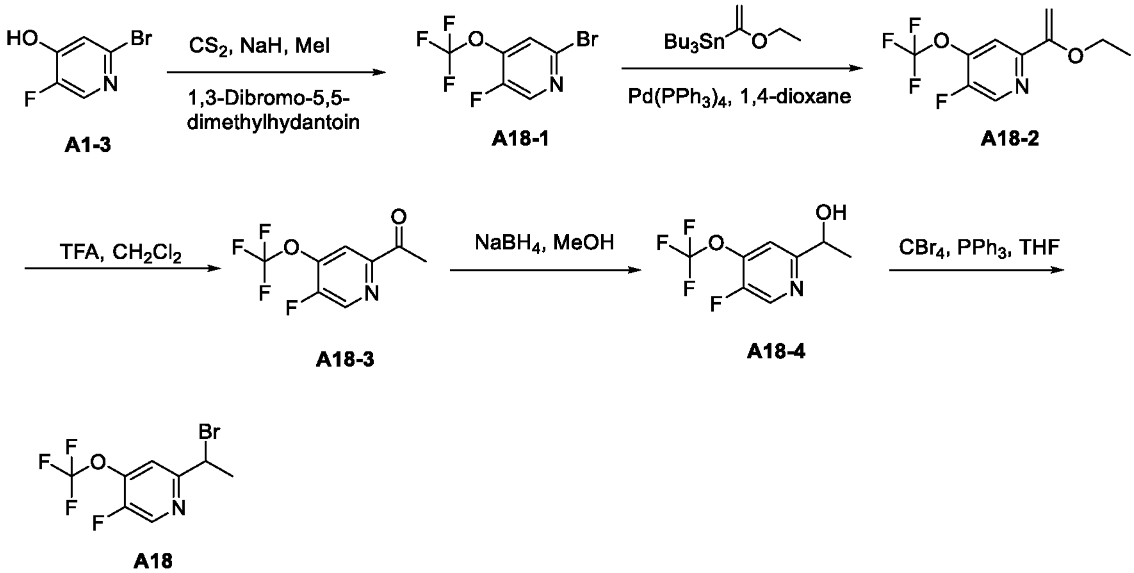


**A1-3** **A18-1** **A18-2** **A18-3** **A18-4** **A18**

Step 1: A18-1 was prepared from A1-3 in one step according to the synthesis method described in reference Tetrahedron Letters (2008), 49(44), 6368-6370.

Step 2: Intermediate A18 was prepared from Intermediate A18-1 through four-step reaction according to the synthesis route of Intermediate A17.

LCMS: m/z: 287.9 $[M+H]^+$ .

Synthesis of Intermediate **A19:** 5-(1-chloroethyl)-2,2-dimethylbenzo[d][1,3]dioxole

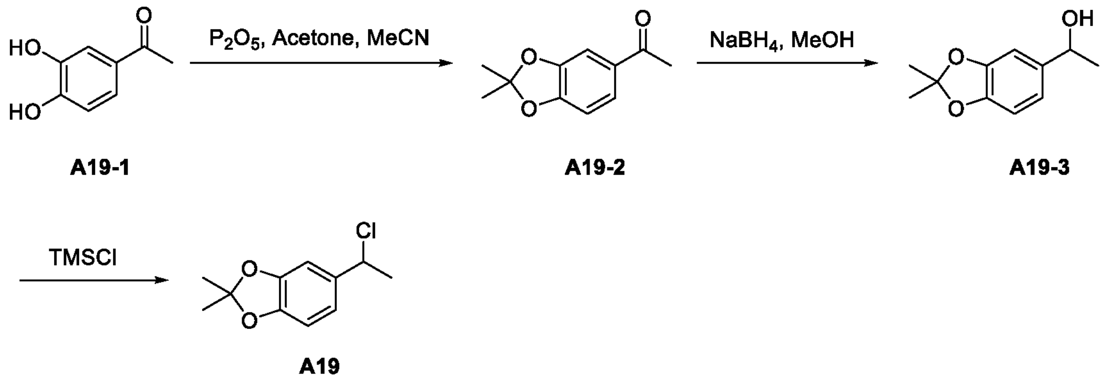


**A19-1** **A19-2** **A19-3** **A19**

Step 1: phosphorus pentoxide (9.3 g, 65.72 mmol) was added into a solution of **A19-1**(2.0 g, 13.14 mmol) in acetonitrile/acetone (40 mL/10 mL), heated to 75 °C and left to react for 3 hours. Phosphorus pentoxide (4.67g * 4, 131.50 mmol) was added in batches every 4 hours and kept reacting for 48 hours. After cooled to room temperature, the reaction mixture was poured into ice water (100 mL) and extracted with ethyl acetate (3 * 30 mL). The organic phase was combined, washed with a saturated aqueous solution of NaCl (100 mL), dried with anhydrous sodium sulfate and then concentrated. After column chromatography (petroleum ether: ethyl acetate =10:1) purification, **A19-2** (900 mg, yield:35.6 %) was obtained.

$^1H$ NMR (400 Hz, chloroform-d): $\delta$ ppm 7.53 (dd, *J* =1.6 Hz, 8.0 Hz, 1H), 7.36 (d, *J* =1.6 Hz, 1H), 6.77 (d, *J* =4.4 Hz, 1H), 2.53 (s, 3 H), 1.70 (s, 6H).

Step 2: sodium borohydride (355 mg, 9.36 mmol) was added into a solution of **A19-2** (900 mg, 4.68 mmol) in

methanol (10 mL) in an ice-water bath in batches and reacted at room temperature for 3 hours. The reaction liquid was concentrated and then purified through column chromatography (petroleum ether: ethyl acetate =10:1-5:1) to obtain **A19-3** (580 mg, yield: 63.8 %).

$^1$H NMR (400 Hz, chloroform-d): δ ppm 6.80~6.79 (m, 1 H), 6.77~6.75 (m, 1 H), 6.68~6.66 (m, 1 H), 4.80~4.78 (m, 1H), 1.82 (s, 1H), 1.67 (d, *J*= 1.2Hz, 6H), 1.46 (d, *J*= 2.4Hz, 3H).

Step 3: **A19-3** (490 mg, 2.52 mmol) and trimethylchlorosilane (5 mL) were mixed and stirred at room temperature for 1 hour. The reaction liquid was poured into ice water, extracted with ethyl acetate (2 * 20 mL). The organic phase was combined, washed with a saturated aqueous solution of NaCl (30 mL), dried with anhydrous sodium sulfate and concentrated to obtain **A19** (500 mg, yield: 93.2%).

Synthesis of Intermediate **B1:** 5'-bromo-7'-(chloromethyl)-2',3'-dihydro-1'H - spiro[cyclopropan-1,4'-isoquinoline]-1'-one

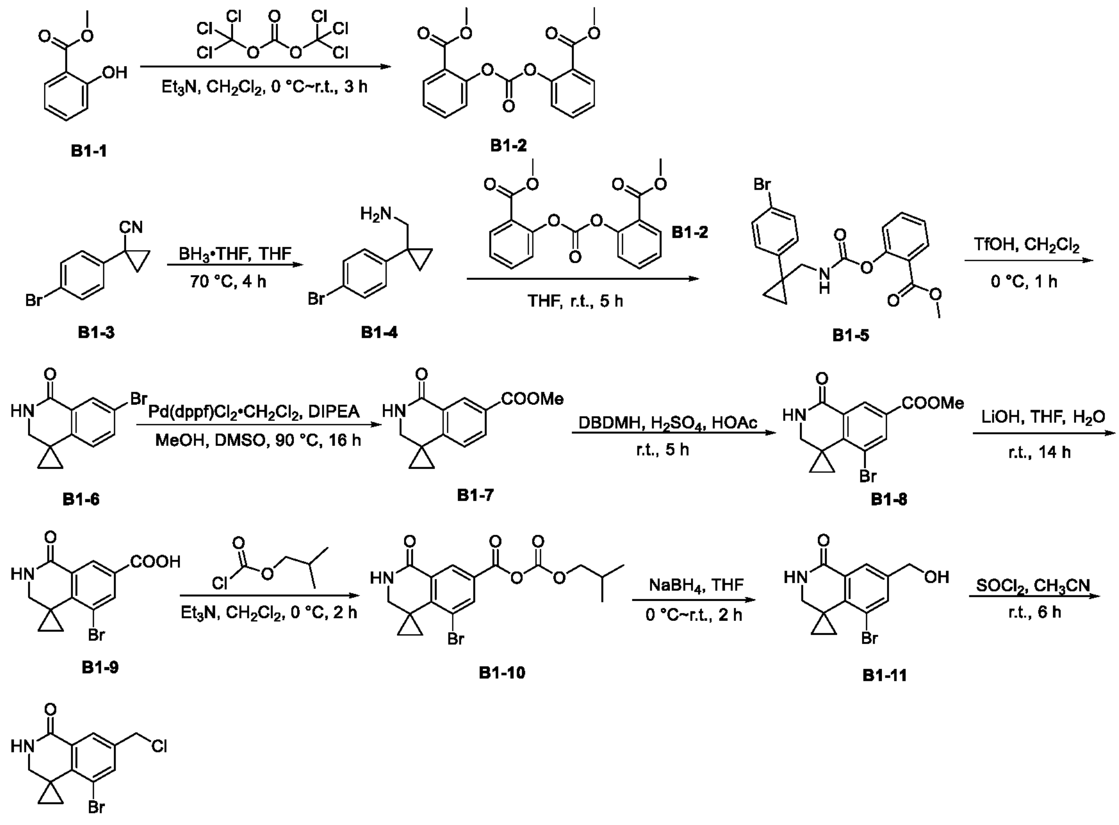

B1-1, B1-2, B1-3, B1-4, B1-2, B1-5, B1-6, B1-7, B1-8, B1-9, B1-10, B1-11, B1

Step 1: triethylamine (34 mL, 246.47 mmol) was added into a solution of methyl 2-hydroxybenzoate (**B1-1**, 25 g, 164.31 mmol) in dichloromethane (300 mL). A solution of triphosgene (36.6 g, 123.23 mmol) in dichloromethane (100 mL) was added slowly under argon protection at 0°C and reacted at room temperature for 3 hours. The reaction liquid was concentrated and purified through column chromatography (ethyl acetate /petroleum ether=1/10) to obtain **B1-2** (26 g, yield: 47.9 %).

$^1$H NMR (400 MHz, DMSO-d6): δ 8.02-7.99 (m, 2H), 7.80-7.66 (m, 2H), 7.53-7.44 (m, 4H), 3.90 (s, 6H).

Step 2: $BH_3$·THF (1M, in tetrahydrofuran, 566 mL, 566 mmol) was added dropwise into a solution of 1-(4-bromophenyl)cyclopropanenitrile (**B1-3**, 50 g, 228.13 mmol) in tetrahydrofuran (500 mL) under argon protection at 0°C. The reaction mixture was brought to 70°C and left to react for 4 hours until the completion of reaction monitored by LCMS. The reaction was quenched with methanol and concentrated. After column chromatography purification (dichloromethane/methanol=35/1), (1-(4-bromophenyl)cyclopropyl)methylamine (**B1-4**, 48 g, yield:94.3 %) was obtained.

LCMS: m/z 226.1 [M+H]$^+$.

Step 3: dimethyl 2,2'-(carbonylbis(oxy)dibenzoate (**B1-2**, 26 g, 78.79 mmol) was added into a solution of (1-(4-bromophenyl)cyclopropyl)methylamine (**B1-4,** 17.73 g, 78.41 mmol) in tetrahydrofuran(400 mL) in batches under argon protection and reacted at room temperature for 5 hours. The reaction liquid was concentrated. After column chromatography purification (ethyl acetate /petroleum ether=1/20), **B1-5** (27.8 g, yield: 87.2 %) was obtained.

LCMS: m/z 404.0 $[M+H]^+$.

Step 4: trifluoromethanesulfonic acid (30.4 mL, 344.91 mmol) was added dropwise slowly into a solution of **B1-5** (27.8 g, 68.77 mmol) in dichloromethane (500 mL) at 0°C under argon protection and reacted at 0°C for 1 hour under stirring. The reaction was quenched with a saturated aqueous solution of sodium bicarbonate and extracted with dichloromethane. The extract was combined, dried with anhydrous sodium sulfate, filtered and concentrated. After column chromatography purification, 7'-bromo-2', 3'-dihydro-1'H-spiro[cyclopropan-1,4'-isoquinoline] - 1'-one (**B1-6**, 10.72 g, yield:61.9 %) was obtained.

LCMS: m/z: 252.0$[M+H]^+$.

Step 5: $Pd(dppf)Cl_2{\cdot}CH_2Cl_2$ (3.32 g, 4.07 mmol) and N,N-diisopropylethanamine (21.1 mL, 128.13 mmol) were added into a solution of 7'-bromo-2', 3'-dihydro-1'H-spiro[cyclopropan-1,4'-isoquinoline]-1'-one (**B1-6**, 10.72 g, 42.52 mmol) in methanol (20 mL) and DMSO(20 mL). After the air was replaced with CO three times, the reaction mixture was heated to 90 °C and reacted for 16 hours. The reaction liquid was poured into water, extracted with dichloromethane. The organic phase was combined, dried with anhydrous sodium sulfate, filtered and dried by a rotary evaporator. After column chromatography purification (ethyl acetate /petroleum ether=1/10), methyl 1'-one-2',3'-dihydro-1'H-spiro[cyclopropan-1,4'-isoquinoline]-7'-carboxylate (**B1-7**, 6.13 g, yield:62.3 %) was obtained.

LCMS: m/z: 232.1$[M+H]^+$.

Step 6: methyl 1'-one -2',3'-dihydro-1'H-spiro[cyclopropan-1,4'-isoquinoline]-1'-carboxylate (**B1-7,** 4.0 g, 17.3 mmol) was added into a mixed solution of concentrated sulfuric acid (20 mL) and glacial acetic acid (20 mL) at 0°C. After stirred for 20 mins, the reaction liquid was added dropwise with a solution of 1,3-dibromo-5,5-dimethylhydantoin (4.9 g, 17.3 mmol) in glacial acetic acid (20 mL), reacted at room temperature for 5 hours and diluted with ethyl acetate (200 mL). The reaction liquid was poured into ice water, stirred at room temperature for 1 hour. The water phase and the organic phase were separated. The water phase was extracted with ethyl acetate (300 mL). The organic phase was combined, washed with saturated saline, dried with anhydrous sodium sulfate, filtered, and concentrated under reduced pressure to dryness. After column chromatography purification (ethyl acetate/petroleum ether=1/10), a white solid of methyl 5'-bromo-1'-one-2',3'-dihydro-1'H-spiro[cyclopropan-1,4'-isoquinoline]-7'-carboxylate (**B1-8**, 2.0 g, yield:37.3 %) was obtained.

LCMS: m/z 310.0 /312.0$[M+H]^+$.

$^1$H NMR (400 MHz, DMSO-*d6*): $\delta$ ppm 8.46 (d, $J$ = 2.0 Hz, 2H), 8.11 (d, $J$ = 2.0 Hz, 1 H), 3.88 (s, 3H), 3.16 (d, $J$ = 2.8 Hz, 2 H), 1.90~1.87(m, 2 H), 1.07~1.05(m, 2 H).

Step 7: lithium hydroxide monohydrate (800 mg, 20 mmol) was added into a solution of methyl 5'-bromo-1'-one-2',3'-dihydro-1'H-spiro[cyclopropan-1,4'-isoquinoline]-7'- carboxylate (**B1-8**, 3.1 g, 10 mmol) in tetrahydrofuran (50 mL) and water (50 mL) and reacted at room temperature for 14 hours. It was shown by LCMS that 80% of product was present. The solvent tetrahydrofuran was removed under reduced pressure and the reaction liquid was adjusted to pH 7 with 1 M aq. HCl solution. A large amount of white solid was precipitated and filtered to obtain 5'-bromo-1'-one-2',3'-dihydro-1'H-spiro[cyclopropan-1,4'-isoquinoline] -7'-carboxylic acid (**B1-9**, 2.2 g, yield:74.3%).

LCMS: m/z 295.7/297.7 $[M+H]^+$ .

Step 8: isobutyl chloroformate (2.02 g, 14.8 mmol) was added into a solution of 5'-bromo-1'-one-2', 3'-dihydro-1'H-spiro[cyclopropan-1,4'-isoquinoline] -7'-carboxylic acid (**B1-9**, 2.2 g, 7.4 mmol) and triethylamine (2.24 g, 22.2 mmol) in dichloromethane (30 mL) at 0 °C and reacted at room temperature under stirring for 2 hours. The reaction liquid was poured into ice water and stirred at room temperature for 1 hour. The water phase and the organic phase were separated. The water phase was extracted with dichloromethane (300 mL). The organic phase was combined, washed with saturated saline, dried with anhydrous sodium sulfate, filtered, and concentrated under reduced pressure to dryness to obtain a crude product **B1-10**(1.68 g, yield:57.3 %).

LCMS: m/z 396.1/398.1 $[M+H]^+$.

Step 9: sodium borohydride (472 mg, 12.5 mmol) was added into a solution of **B1-10** (1.65 g, 4.2 mmol) in tetrahydrofuran (30 mL) at 0°C and reacted at room temperature for 2 hours. The reaction was quenched with methanol (10 mL). The reaction liquid was poured into ice water and stirred at room temperature for 0.5 hour. The water phase and the organic phase were separated. The water phase was extracted with dichloromethane (200 mL). The organic phase was combined, washed with saturated saline, dried with anhydrous sodium sulfate, filtered, and concentrated under reduced pressure to dryness. After column chromatography purification (ethyl acetate /petroleum ether=1/3), an oil of **B1-11** (460 mg, yield: 39.2 %) was obtained.

LCMS: m/z: 282.2/284.2$[M+H]^+$.

Step 10: thionyl chloride (127 mg, 1.068 mmol) was added into a solution of **B1-11**(100 mg, 0.356 mmol) in acetonitrile (20 mL) at 0°C and reacted at room temperature for 6 hours. The reaction liquid was dried by a rotary evaporator to obtain a crude product 5'-bromo-7'-(chloromethyl)-2',3'-dihydro-1'H-spiro[cyclopropan-1,4'-isoquinoline]-1'-one (**B1**, 110 mg, yield: 100 %).

LCMS: m/z: 299.97 $[M+H]^+$.

Synthesis of Intermediate **B2:** 5'-bromo-7'-(chloromethyl)-2',3'-dihydro-1'H - spiro[cyclobutan-1,4'-isoquinoline]-1'-one

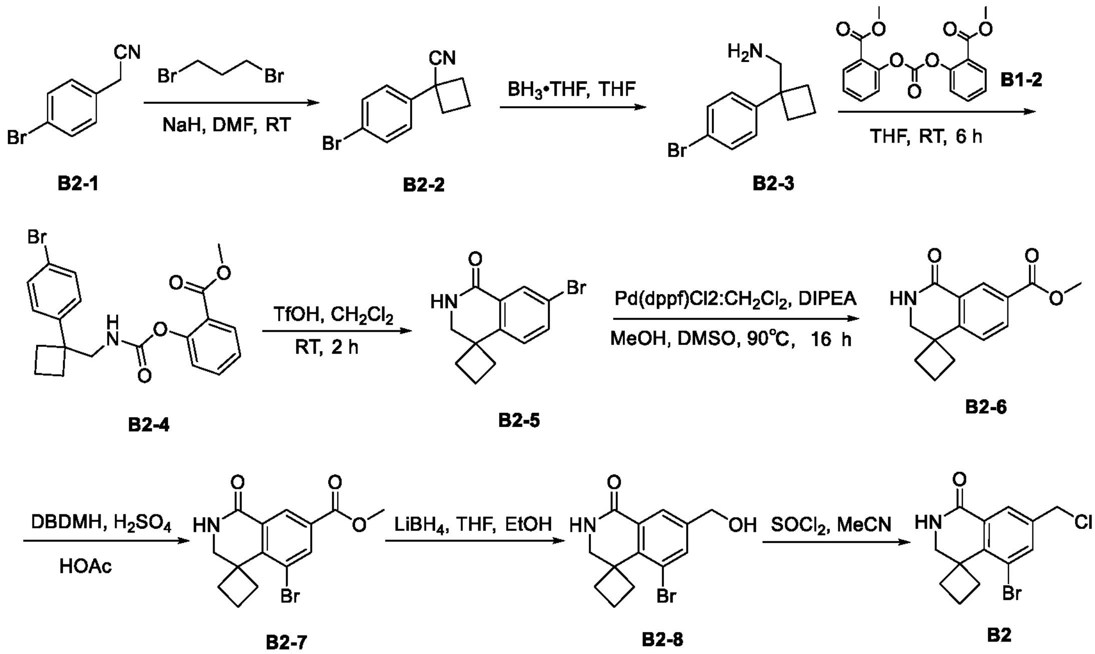


Step 1: sodium hydride (60% dispersed in mineral oil, 20.4 g, 510.07 mmol) was added into a solution of **B2-1** (25 g, 127.52 mmol) in DMF (500 mL) in batches at 0 °C under argon protection and reacted at 0 °C for 30 mins, added dropwise with a solution of 1,3-dibromopropane (19.42 mL, 191.28 mmol) in DMF (50 mL) and reacted at room temperature for 1 hour. The reaction was quenched with ice water and extracted with ethyl acetate (3 * 300 mL). The organic phase was combined, washed with saturated saline, dried, filtered and concentrated. After column chromatography purification (petroleum ether: ethyl acetate =20:1), **B2-2** (20 g, yield:66.4 %) was obtained.

$^1$H NMR (400 MHz, DMSO-d6): δ ppm 7.64 (d, *J* = 8.8 Hz, 2H), 7.43 (d, *J* = 8.4 Hz, 2H), 2.77-2.71 (m, 2H), 2.64 -2.56 (m, 2H), 2.33-2.22 (m, 1H), 2.05 - 1.96 (m, 1H).

Step 2: $BH_3$·THF (1M in THF, 212 mL, 212 mmol) was added dropwise into a solution of **B2-2** (20 g, 84.71 mmol) in THF (200 mL) at 0 °C under argon protection and reacted at 70 °C for 4 hours until the completion of reaction monitored by LCMS. The reaction was quenched with methanol and concentrated under reduced pressure. After column chromatography purification (dichloromethane: methanol=20:1), **B2-3** (5.5 g, yield: 27.0 %) was obtained.

LCMS: m/z: 242.1 $[M+H]^+$.

Step 3: **B1-2** (6.2 g, 18.75 mmol) was added into a solution of **B2-3**(4.5 g, 18.75 mmol) in THF (40 mL) in batches under argon protection and reacted at room temperature for 6 hours. The reaction liquid was concentrated under reduced pressure. After column chromatography purification (petroleum ether: ethyl acetate =1:1), **B2-4** (7.2 g, yield:91.9 %) was obtained.

$^1$H NMR (400 MHz, DMSO-d6): δ ppm 7.86 (t, *J* = 6.4 Hz, 1H), 7.82-7.80 (m, 1H), 7.61-7.57 (m, 1H), 7.51-7.49 (m, 2H), 7.32 (t, *J* = 7.2 Hz, 1H), 7.15-7.13 (m, 2H), 7.86 (d, *J* = 8.0 Hz, 1H), 3.73 (s, 3H), 3.36 (s, 1H), 3.34 (s, 1H), 2.36-2.29 (m, 2H), 2.23 -2.16 (m, 2H), 2.10-2.03 (m, 1H), 1.81 - 1.72 (m, 1H).

Step 4: trifluoromethanesulfonic acid (7.6 mL, 86.06 mmol) was added dropwise a solution of **B2-4** (7.2 g, 17.21 mmol) in dichloromethane (100 mL) at 0 °C under argon protection and reacted at room temperature for 2 hours. The reaction was quenched with a saturated aqueous solution of sodium bicarbonate and extracted with dichloromethane (3 * 100 mL). The organic phase was combined, dried and filtered, concentrated. After column chromatography purification (petroleum ether: ethyl acetate =1:1), **B2-5** (1.8 g, yield: 39.3 %) was obtained.

LCMS: m/z: 266.0 $[M+H]^+$.

Step 5: N,N-diisopropylethanamine (3.3 mL, 20.30 mmol) and [1,1'-bis(diphenylphosphino)ferrocene]palladium (II) chloride dichloromethane complex (552 mg, 0.677 mmol) was added into a solution of **B2-5** (1.8 g, 6.77 mmol) in methanol(10 mL) and DMSO(30 mL). After the air was replaced with CO three times, the reaction mixture was heated to 90 °C and left to react for 16 hours. The reaction liquid was diluted with water (50 mL) and extracted with ethyl acetate (3 * 50 mL). The organic phase was combined, dried and filtered and concentrated. After column chromatography purification (petroleum ether: ethyl acetate =1:3), **B2-6**(1.3 g, yield:78.4 %) was obtained.

LCMS: m/z: 246.1 $[M+H]^+$.

Step 6: a solution of 1,3-dibromo-5,5-dimethylhydantoin (3.0 g, 10.6 mmol) in glacial acetic acid (10 mL) was added slowly dropwise into a mixed solution of **B2-6** (1.3 g, 5.3 mmol) in concentrated sulfuric acid (10 mL) and glacial acetic acid (10 mL) at 0 °C and reacted at room temperature for 16 hours. The reaction was quenched with ice water, extracted with ethyl acetate (3 * 50 mL). The organic phase was combined, dried and filtered and concentrated. After column chromatography purification, **B2-7**(1.2 g, yield:69.8 %) was obtained.

LCMS: m/z: 326.0 $[M+H]^+$.

Step 7:To lithium borohydride (2M in THF, 9.3 mL, 18.51 mmol) was added dropwise into a mixed solution of **B2-7** (1.2 g, 3.70 mmol) in THF (10 mL) and ethanol (2 mL) at 0 °C and reacted at room temperature for 10 mins. Then the reaction mixture was heated to 45°C and left to react for 2 hours. The reaction was quenched with ice water and extracted with ethyl acetate (3 * 50 mL). The organic phase was combined, dried, filtered and concentrated. After column chromatography purification, **B2-8** (580 mg, yield: 52.7 %) was obtained.

LCMS: m/z: 296.0 $[M+H]^+$.

Step 8: thionyl chloride (0.15 mL, 2.03 mmol) was added dropwise into a solution of **B2-8** (200 mg, 0.675 mmol) in acetonitrile (10 mL) at 0 °C and reacted at room temperature under stirring for 1 hour. The reaction liquid was concentrated under reduced pressure to obtain **B2** (215 mg, 0.675 mmol). The crude product was directly used in the next step.

LCMS: m/z: 316.0 $[M+H]^+$.

Synthesis of Intermediate **B3:** 5'-bromo-7'-(chloromethyl)-2',3'-dihydro-1'H-spiro[cyclopentan-1,4'-isoquinoline]-1'-one

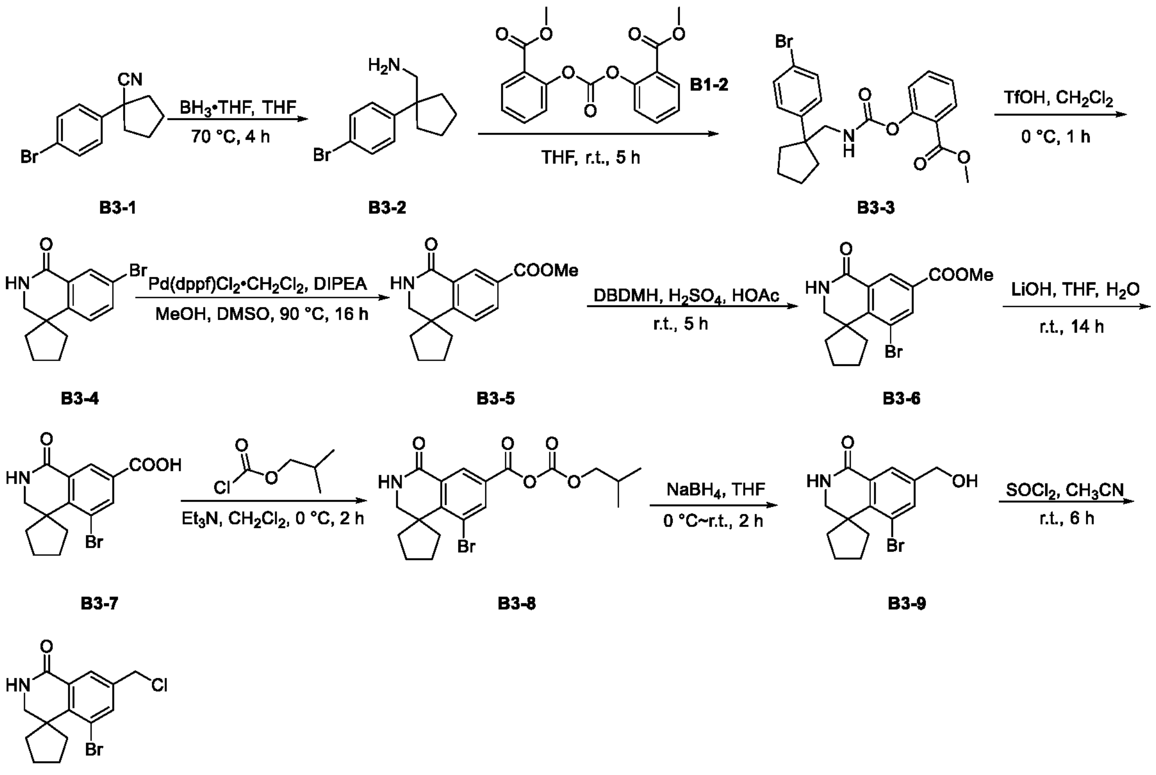


B3

Intermediate B3 was obtained by a nine-step reaction with B3-1 instead of B1-3 according to the synthesis route of B1.

LCMS: m/z: 328.0 $[M+H]^+$.

Synthesis of Intermediate **B10:** 5'-bromo-7'-(chloromethyl)-2,2-difluoro-2',3'-dihydro-1'H-spiro[cyclopropan-1,4'-isoquinoline]-1'-one

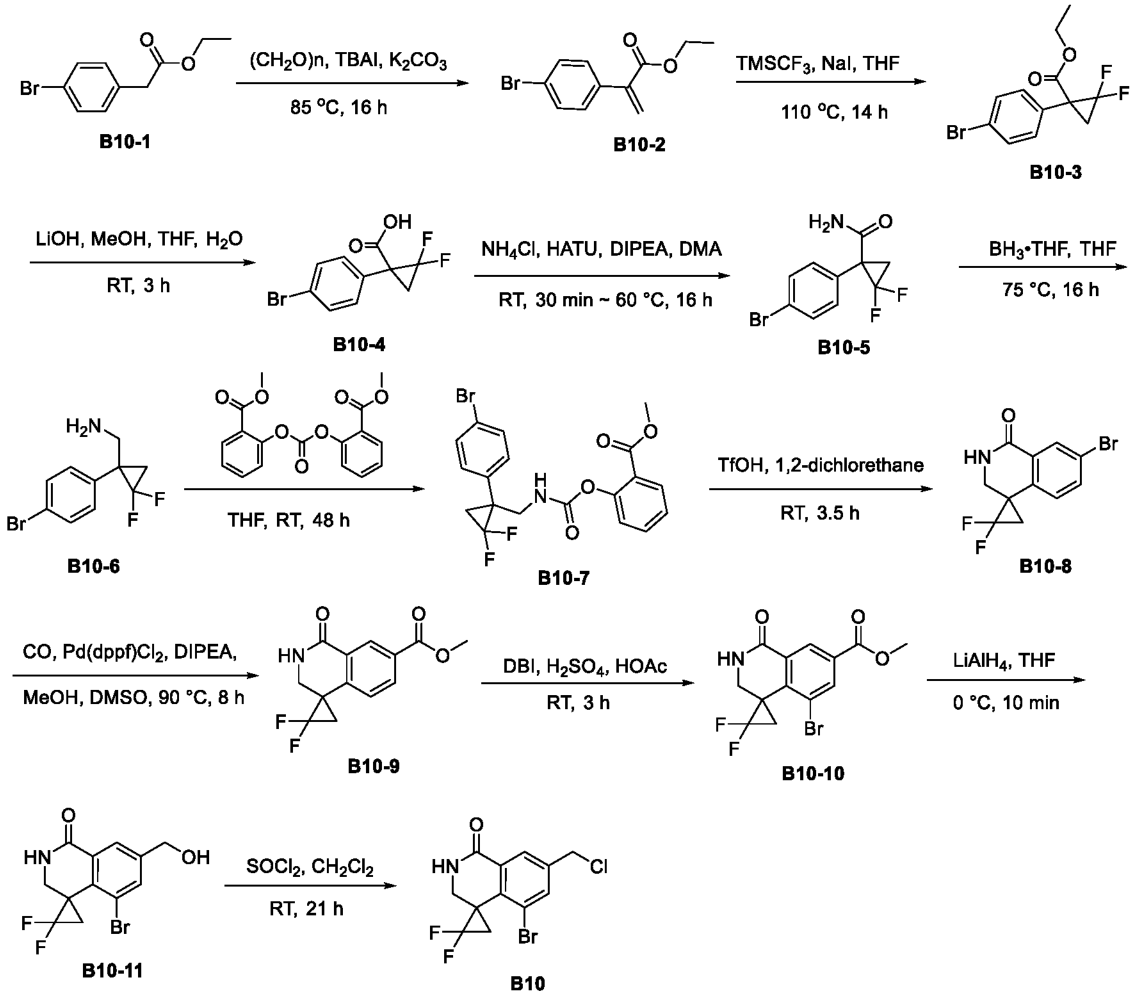


B10-1 B10-2 B10-3 B10-4 B10-5 B10-6 B10-7 B10-8 B10-9 B10-10 B10-11 B10

Step 1: paraformaldehyde (18.51 g, 617.03 mmol), tetrabutylammonium iodide (7.60 g, 20.57 mmol) and potassium carbonate (85.28 g, 617.03 mmol) were added into a solution of **B10-1**(100 g, 411.35 mmol) in toluene (1.5 L), heated to 85°C and left to react for16 hours. The reaction mixture was cooled to room temperature and filtered. The filtrate was concentrated under reduced pressure. After column chromatography purification (petroleum ether: ethyl acetate =50:1), **B10-2** (61 g, yield: 58.1 %) was obtained.

$^1$H NMR (400 Hz, chloroform-d) δ ppm: 7.50~7.47 (m, 2 H), 7.31~7.28 (m, 2 H), 6.38 (d, *J* = 1.2 Hz, 1 H), 5.89 (d, *J* = 0.8 Hz, 1 H), 4.31~4.26 (m, 2 H), 1.33 (t, *J* = 7.2 Hz, 3 H).

Step 2: (trifluoromethyl)trimethylsilane (93.6 g, 658.54 mmol) and sodium iodide (7.4 g, 49.39 mmol) was added into a solution of **B10-2** (21 g, 82.32 mmol) in tetrahydrofuran (300 mL) and reacted at 110°C in a closed tank for 14 hours. The reaction liquid was diluted with ethyl acetate (700 mL), washed with water (2 * 700 mL), washed with a saturated aqueous solution of NaCl (700 mL), dried with anhydrous sodium sulfate and then concentrated. After column chromatography separation and purification (petroleum ether: ethyl acetate =50:1), **B10-3** (21 g, yield: 83.7%) was obtained.

$^1$H NMR (400 Hz, chloroform-d) δ ppm: 7.44~7.40 (m, 2 H), 7.24~7.18 (m, 2 H), 4.15~4.05 (m, 2H), 2.59~2.53 (m, 1 H), 1.84-1.78 (m, 1 H), 1.13 (t, *J* = 7.2 Hz, 3 H).

Step 3: **B10-3** (73.5 g, 240.89 mmol) was dissolved in methanol (600 mL), added with water (300 mL) and tetrahydrofuran (150 mL), followed by lithium hydroxide (28.91 g, 1.20 mol) and reacted at room temperature under stirring for 3 hours. The reaction liquid was concentrated under reduced pressure to remove solvents methanol and tetrahydrofuran. The residue was diluted with water (700 mL), adjusted to pH=3~4 with dilute hydrochloric acid (3 M) and extracted with ethyl acetate (3 * 600 mL). The organic phase was combined, dried with anhydrous sodium sulfate and filtered. The filtrate was concentrated under reduced pressure. After column chromatography purification (petroleum ether: ethyl acetate: acetic acid =10:1:0.001), **B10-4** (31.8 g, yield:47.6 %) was obtained.

$^1$H NMR (400 Hz, DMSO-$d_6$) δ ppm: 13.45 (s, 1 H), 7.59~7.57 (m, 2 H), 7.37~7.35 (m, 2 H), 2.58~2.53 (m, 1 H), 2.30~2.24 (m, 1 H).

Step 4: DIPEA (89.0 g, 688.66 mmol) and HATU (52.37 g, 137.73 mmol) were added into a solution of **B10-4**

(31.8 g, 114.78 mmol) in DMA (600 mL) with stirring at room temperature for 30 mins. Ammonium chloride (18.42 g, 344.33 mmol) was added. After stirred at room temperature for 30 mins, the reaction mixture was heated to 60°C, left to react for 16 hours, cooled to room temperature, then diluted with ethyl acetate (1.5 L), washed with water (3 * 1 L) and a saturated aqueous solution of NaCl (1 L), dried with anhydrous sodium sulfate and filtered., The filtrate was concentrated under reduced pressure. After column chromatography purification (petroleum ether: ethyl acetate =2:1), **B10-5** (17 g, yield: 53.6%) was obtained.

LCMS: m/z: 275.9 $[M+H]^+$.

Step 5: $BH_3$·THF (1 M in tetrahydrofuran, 143 mL, 143.0 mmol) was added dropwise into a solution of **B10-5** (15.8 g, 57.23 mmol) in tetrahydrofuran (300 mL) in an ice-water bath. After the end of addition, the reaction mixture was heated gradually to 75°C and reacted for 16 hours. The reaction liquid was cooled to 0°C and added with methanol (100 mL) dropwise to quench the reaction. The reaction liquid was concentrated under reduced pressure to remove the solvent. The residue was dissolved in and diluted with methanol, stirred at 75°C for 10 hours and concentrated. After column chromatography separation and purification (dichloromethane: methanol=50:1~20:1), **B10-6** (11 g, yield:73.3 %) was obtained.

LCMS: m/z: 263.9 $[M+H]^+$.

Step 6: dimethyl 2,2'-(carbonylbis(oxy))dibenzoate (15.1 g, 45.78 mmol) was added into a solution of **B10-6** (12 g, 45.78 mmol) in tetrahydrofuran (150 mL) and stirred at room temperature for 48 hours. The reaction liquid was directly concentrated. After column chromatography purification (petroleum ether: ethyl acetate =10:1~5:1), **B10-7** (17 g, yield:84.6 %) was obtained.

LCMS: m/z 464.0 $[M+Na]^+$

Step 7: trifluoromethanesulfonic acid (200 mL) was added dropwise into a solution of **B10**-7 (17 g, 38.61 mmol) in 1,2-dichloroethane (400 mL) in an ice-water bath and stirred at room temperature for 3.5 hours. The reaction liquid was poured into ice water and extracted with dichloromethane (2 * 300 mL). The organic phase was combined, washed with a saturated aqueous solution of NaCl (300 mL), dried with anhydrous sodium sulfate and filtered. The filtrate was concentrated under reduced pressure and purified through column chromatography (dichloromethane: methanol=100:1) to obtain **B10-8** (6.5 g, yield: 58.5 %).

LCMS: m/z 289.9 $[M+H]^+$ .

Step 8: DIPEA(10.5 g, 81.22 mmol) and [1,1'-bis(diphenylphosphino)ferrocene] palladium (II) chloride (1.98 g, 2.71 mmol) were added into a solution of **B10-8**(7.8 g, 27.07 mmol) in methanol(80 mL) and dimethyl sulfoxide (160 mL). After the air was replaced with CO, the reaction mixture was reacted at 90°C for 8 hours, cooled to room temperature, then diluted with dichloromethane (400 mL), washed with water (3 * 400 mL), washed with a saturated aqueous solution of NaCl (400 mL), dried with anhydrous sodium sulfate and filtered. The filtrate was concentrated under reduced pressure. After column chromatography purification (dichloromethane: methanol=50:1), **B10-9**(6.4 g, yield: 88.9%) was obtained.

LCMS: m/z 268.0 $[M+H]^+$.

Step 9: 1,3-dibromo-1,3,5-triazine-2,4,6-trione (8 g, 28.07 mmol) was added into a solution of **B10-9** (3 g, 11.23 mmol) in acetic acid (30 mL) and concentrated sulfuric acid (60 mL) and reacted at room temperature for 3 hours. The reaction liquid was poured into ice water, extracted with ethyl acetate (2 * 150 mL). The organic phase was combined, washed with a saturated aqueous solution of sodium sulfite (100 mL), washed with a saturated aqueous solution of NaCl (150 mL), dried with anhydrous sodium sulfate and filtered. The filtrate was concentrated under reduced pressure. After column chromatography purification (dichloromethane: methanol=200:1), **B10-10** (900 mg, yield:23.2%) was obtained.

LCMS: m/z 348.0$[M+H]^+$.

Step 10: $LiAlH_4$ (1 M in tetrahydrofuran, 2.9 mL, 2.89 mmol) was added dropwise into **B10-10** (500 mg, 1.44 mmol) in tetrahydrofuran (20 mL) at 0°C under argon protection and kept reacting at this temperature for 10 mins. The reaction liquid was poured into ice water and extracted with dichloromethane (2 * 50 mL). The organic phase was combined, dried with anhydrous sodium sulfate and filtered. The filtrate was concentrated under reduced pressure. After column chromatography purification (dichloromethane: methanol=20:1), **B10-11**(350 mg, yield:76.2 %) was obtained.

LCMS: m/z 317.9$[M+H]^+$.

Step 11: thionyl chloride (375 mg, 3.14 mmol) was added dropwise into a solution of **B10-11** in dichloromethane (6 mL) and reacted at room temperature for 5 hours. The reaction was incomplete under monitoring, added with additional thionyl chloride (750 mg, 6.28 mmol) and continued to react for 16 hours. The reaction liquid was poured into ice water (20 mL) and extracted with dichloromethane (50 mL) twice. The organic phase was combined, washed with saturated saline (200 mL), dried with anhydrous sodium sulfate and concentrated. After column chromatography purification (dichloromethane: methanol=50:1), **B10** (200 mg, yield:94.8 %) was obtained.

LCMS: m/z: 337.9$[M+H]^+$.

Synthesis of Intermediate **B11:** 5'-bromo-7'-(chloromethyl)-3,3-difluoro-2',3'-dihydro-1'H-spiro[cyclobutan-1,4'-isoquinoline]-1'-one

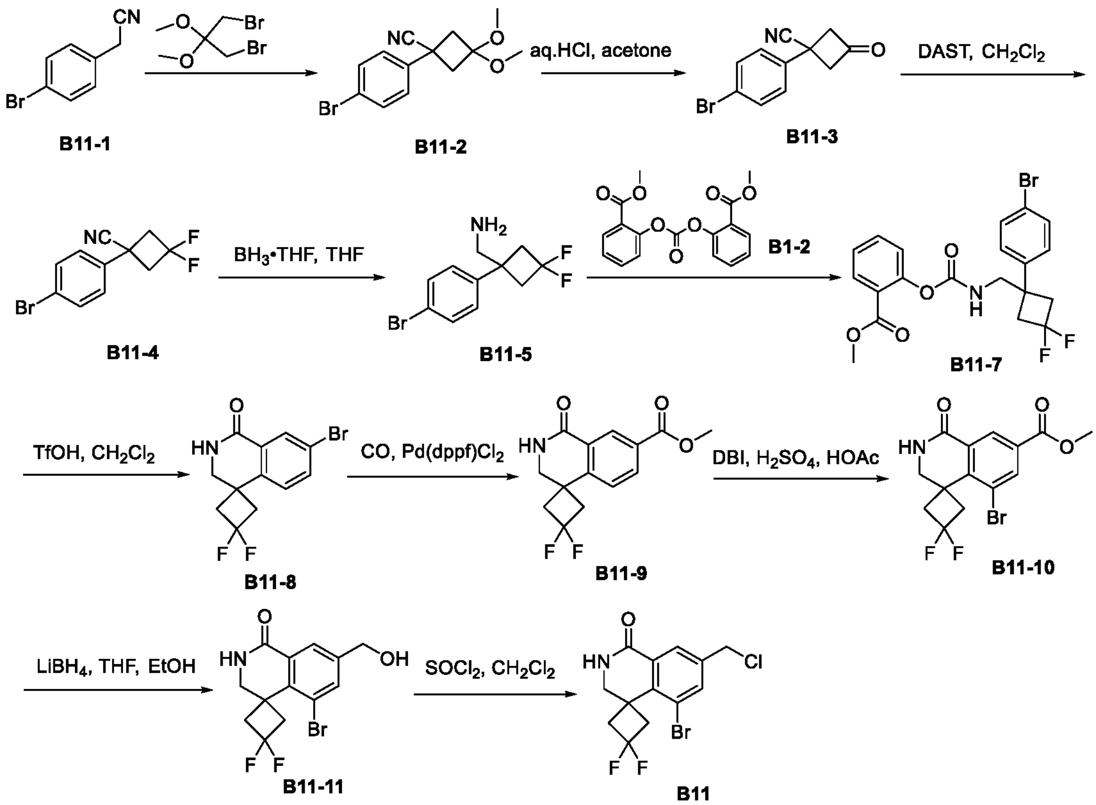


Step 1: sodium hydride (60% dispersed in oil, 22.4 g, 561.08 mmol) was added into a solution of **B11-1** (50 g, 255.03 mmol) in DMF (1 L) in an ice-water bath in batches and stirred at room temperature for 1 hour. 1,3-dibromo-2,2-dimethoxypropane (73.5 g, 280.54 mmol) was added dropwise, heated to 60°C and left to react for 16 hours. The reaction liquid was cooled to room temperature, then poured into ice water and extracted with ethyl acetate (2 * 700 mL). The organic phase was combined, washed with water (3 * 500 mL), a saturated aqueous solution of NaCl (2 * 700 mL), dried with anhydrous sodium sulfate and then concentrated. The residue was dissolved in petroleum ether and filtered to obtain **B11-2** (55 g, yield:72.8 %).

$^1$H NMR (400 Hz, chloroform-d) δ ppm: 7.54~7.51 (m, 2H), 7.38~7.34 (m, 2H), 3.27 (s, 3H), 3.17 (s, 3H), 3.12~3.11 (m, 1H), 3.09~3.08 (m, 1H), 2.71~2.69 (m, 1H), 2.67~2.66 (m, 1H).

Step 2: HCl (6 M, 180 mL) was added dropwise into a solution of **B11-2**(59 g, 199.2 mmol) in acetone (700 mL). After the end of addition, the reaction mixture was heated to 60°C and left to react for 3 hours. The reaction liquid was cooled to room temperature, then diluted with ethyl acetate (1 L), washed with water (700 mL), then a saturated aqueous solution of NaCl (700 mL), dried with anhydrous sodium sulfate and then concentrated. After column chromatography separation and purification (petroleum ether: ethyl acetate =15:1), **B11-3** (47 g, yield:94.4%) was obtained.

$^1$H NMR (400 Hz, chloroform-d) δ ppm: 7.61~7.58 (m, 2H), 7.39~7.36 (m, 2H), 4.09~4.03 (m, 2H), 3.72~3.65 (m, 2H).

Step 3: **B11-3** (30 g, 120.0 mmol) was dissolved in dichloromethane (300 mL) and methanol (0.3 mL). After the reaction mixture was stirred for 10 mins, DAST (92 mL, 319.8 mmol) was added dropwise in an ice-water bath and reacted at room temperature for 3 hours. The reaction liquid was poured into ice water, extracted with dichloromethane (2 * 400 mL). The organic phase was combined, washed with a saturated aqueous solution of NaCl (500 mL), dried with anhydrous sodium sulfate, filtered and concentrated. After column chromatography purification (petroleum ether: ethyl acetate =100:1~60:1), **B11-4** (18 g, yield:55.2 %) was obtained.

$^1$H NMR (400 Hz, chloroform-d) δ ppm: 7.60~7.56 (m, 2H), 7.36~7.32 (m, 2H), 3.56~3.47 (m, 2H), 3.23~3.12 (m, 2H).

$^{19}$F NMR (376 Hz, chloroform-d) δ ppm: -85.72, -86.25, -92.02, -92.55

Step 4: a $BH_3$·THF solution (1M, 213 mL, 213.00 mmoL) was added dropwise into a solution of **B11-4** (19.3 g,

70.93 mmol) in tetrahydrofuran (150 mL) in an ice-water bath. The reaction mixture was heated and reacted for 16 hours under refluxing. Methanol (30 mL) was added dropwise into the reaction liquid in an ice-water bath to quench the reaction. After concentration, the residue was diluted with methanol (300 mL), heated under refluxing for 5 hours and concentrated. After column chromatography purification (dichloromethane/methanol=50:1), **B11-5** (13.0 g, yield: 66.4%) was obtained.

LCMS: m/z: 278.0 $[M+H]^+$.

Step 5: **B11-5** (13.6 g, 49.25 mmol) and **B1-2** (16.3 g, 49.25 mmol) were dissolved in tetrahydrofuran (150 mL) and stirred at room temperature for 16 hours. The reaction liquid was directly concentrated. After column chromatography purification (petroleum ether: ethyl acetate =10:1~5:1), **B11-7** (22.0 g, yield: 98.3%) was obtained.

LCMS: m/z: 477.9 $[M+H]^+$.

Step 6: trifluoromethanesulfonic acid (32.7 mL, 369.82 mmoL) was added dropwise into a solution of **B11-7** (24.0 g, 52.83 mmol) in dichloromethane (300 mL) in an ice-water bath and reacted at room temperature for 3 hours. The reaction liquid was poured into ice water and added with dichloromethane (500 mL). The organic phase was washed with water (2 * 500 mL) and saturated aqueous solution of NaCl (2 * 500 mL), dried with anhydrous sodium sulfate and concentrated. The crude product was dissolved in petroleum ether/dichloromethane (10/1) and filtered to obtain **B11-8** (8.3 g, yield: 51.9 %).

LCMS: m/z 301.7 $(M+H)^+$

$^1$H NMR (400 Hz, DMSO-d6) δ ppm: 8.28 (s, 1H), 7.97 (d, *J* = 2.0 Hz, 1H), 7.81~7.79 (m, 1H), 7.60~7.58 (m, 1H), 3.46 (d, *J* = 2.0 Hz, 2H), 2.99~2.89 (m, 2H), 2.84~2.75 (m, 2H).

$^{19}$F NMR (400 Hz, DMSO-d6) δ ppm: -84.92, -84.44, -87.73, -88.25.

Step 7: DIPEA (9.6 g, 74.47 mmol) and [1,1'-bis(diphenylphosphino)ferrocene]palladium (II) chloride (1.27 g, 1.74 mmol) were added into **B11-8** (7.5 g, 24.82 mmol) in methanol (25 mL) and dimethyl sulfoxide (100 mL) and reacted for 4 hours in CO atmosphere at 90°C. The reaction liquid was cooled to room temperature, diluted with dichloromethane (40 mL), washed with water (3 * 500 mL) and saturated aqueous solution of NaCl (300 mL), and concentrated. After column chromatography purification (dichloromethane: methanol=100:1~50:1), **B11-9** (6.1 g, yield: 87.4%) was obtained.

LCMS: m/z 282.1 $[M+H]^+$ .

Step 8: 1,3-dibromo-1,3,5-triazine-2,4,6-trione (785 mg, 2.74 mmol) was added into a solution of **B11-9** (700 mg, 2.49 mmol) in acetic acid (6 mL) and concentrated sulfuric acid (3 mL). After the end of addition, the reaction mixture was reacted at room temperature for 1 hour. The reaction liquid was poured into ice water and extracted with ethyl acetate (2 * 500 mL). The organic phase was combined, washed with a saturated aqueous solution of sodium sulfite (50 mL) and a saturated aqueous solution of NaCl (50 mL) and concentrated. After column chromatography purification (dichloromethane: ethyl acetate =20:1~5:1), **B11-10** (600 mg, yield:66.9%) was obtained.

LCMS: m/z 360.0 $[M+H]^+$.

$^1$H NMR (400 Hz, DMSO-d6) δ ppm: 8.51~8.48 (m, 2H), 8.27 (d, *J* = 2.0 Hz, 1H), 3.90 (s, 3H), 3.87~3.74 (m, 2H), 3.49 (s, 2H), 2.72~2.63 (m, 2H).

$^{19}$F NMR (400 Hz, DMSO-d6) δ ppm: -81.54, -82.06, -87.19, -87.71.

Step 9: a solution of lithium borohydride in tetrahydrofuran (2 M, 2.8 mL, 5.60 mmol) was added dropwise into a solution of **B11-10** (800 mg, 2.22 mmol) in tetrahydrofuran (20 mL) at 0°C, under argon protection and kept reacting for 2 hours at this temperature. The reaction liquid was poured into ice water, extracted with dichloromethane (3 * 100 mL). The organic phase was concentrated and purified through column chromatography (dichloromethane: methanol=25: 1) to obtain **B11-11** (500 mg, yield: 67.8 %).

LCMS: m/z 332.0$[M+H]^+$.

Step 10: thionyl chloride (970 mg, 6.47 mmol) was added into a solution of **B11-11** (610 mg, 1.85 mmol) in acetonitrile at 0°C and reacted for 1 hour at ambient reaction. The reaction liquid was diluted with dichloromethane (50 mL), added with saturated sodium bicarbonate and extracted with dichloromethane. The organic phase was washed with saline, dried, filtered and concentrated to dryness. After column chromatography (petroleum ether/ethyl acetate =2/1) purification, a yellow solid of **B11**(600 mg, yield: 93.2%) was obtained.

LCMS: m/z: 350 $[M+H]^+$.

Synthesis of Intermediate **C1**: tert-butyl (1H-imidazol-2-yl)(methyl)carbamate

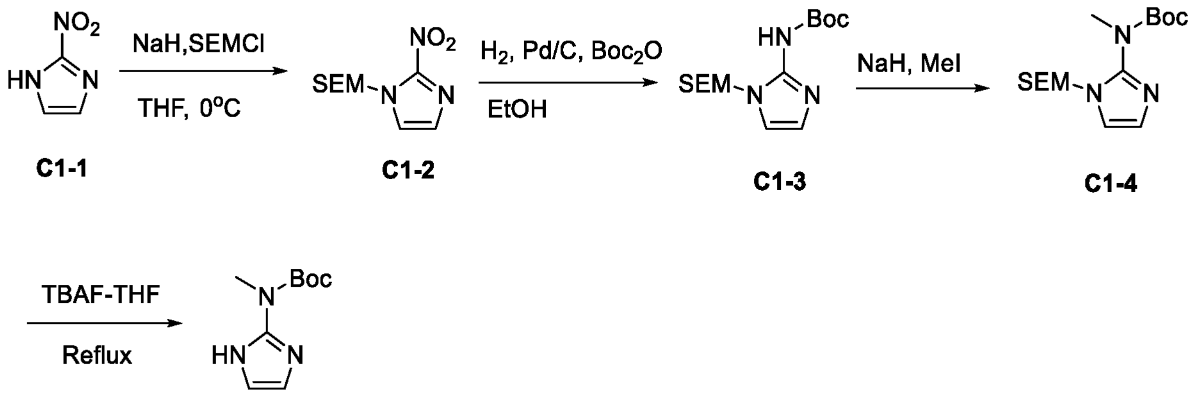


C1-1 C1-2 C1-3 C1-4

C1

Step 1: 2-nitro-1H-imidazole (**C1-1**, 9000 mg, 79.8 mmol) was added into anhydrous tetrahydrofuran (270 mL). Sodium hydride (60%, 3.5 g, 87.5 mmol) was added in an ice bath. The reaction mixture was reacted for 0.5 hours under argon protection at 0°C. (2-(chloromethoxy)ethyl)trimethylsilane (14.6 g, 87.6 mmol) was added dropwise, then reacted at 0°C for 2 hours. The reaction liquid was poured into ice-water (200 mL) slowly, extracted with ethyl acetate (3 * 200 mL). The organic phase was combined, washed with saturated saline (200 mL), dried with anhydrous sodium sulfate, filtered and concentrated. The crude product was purified through column chromatography (ethyl acetate/ petroleum ether=1/5) to obtain a yellow solid of 2-nitro-1-((2-(trimethylsilyl)ethoxy)methyl)-1H-imidazole (**C1-2,** 14.3 g, yield: 73.8 %).

Step 2: 2-nitro-1-((2-(trimethylsilyl)ethoxy)methyl)-1H-imidazole 14-2 (**C1-2,** 14.3 g, 58.8 mmol) was added into anhydrous ethanol (420 mL).The atmosphere was replaced with hydrogen three times. The reaction mixture was reacted at room temperature for 16 hours under hydrogen protection. The reaction liquid was filtered. The filtrate was concentrated to obtain a crude product of 1-((2-(trimethylsilyl)ethoxy)methyl)-1H-imidazol-2-amine. The crude product of 1-((2-(trimethylsilyl)ethoxy)methyl)-1H-imidazol-2-amine was dissolved with dichloromethane (300 mL), added with potassium carbonate (35.0 g, 253.3 mmol), added dropwise with di-tert-butyl dicarbonate (44.0 g, 201.6 mmol), and reacted at room temperature for 16 hours. The reaction liquid was filtered. The filtrate was concentrated to dryness, dissolved with methanol (600 mL), added with potassium carbonate (35.0 g, 253.3 mmol) and stirred at room temperature for 16 hours. The reaction liquid was filtered. The filtrate was concentrated to dryness. After column chromatography (ethyl acetate /petroleum ether=1/20), tert-butyl (1-(((2-(trimethylsilyl)ethoxy)methyl)-1H-imidazol-2-yl)carbamate (**C1-3,** 9.6 g, yield: 58.1%) was obtained.

LCMS:m/z 314.5 $[M+H]^+$.

Step 3: tert-butyl (1-(((2-(trimethylsilyl)ethoxy)methyl)-1H-imidazol-2-yl)carbamate (**C1-3,** 9.6 g, 30.7 mmol) was added into anhydrous tetrahydrofuran (300 mL), cooled to 0°C and added with sodium hydride (60%, 1.5 g, 37.5 mmol). The reaction mixture was reacted for 15 mins under argon protection at 0°C, added dropwise with methyl iodide (6.5 g, 45.8 mmol), then reacted at 0°C for 3 hours. The reaction liquid was poured slowly into ice water, extracted with ethyl acetate (3 * 200 mL). The organic phase was combined, washed with saturated saline (200 mL) and concentrated. The crude product was purified through column chromatography (ethyl acetate/petroleum ether=1/20) to obtain a yellow solid of tert-butyl methyl(1-(((2-(trimethylsilyl)ethoxy)methyl)-1H-imidazol-2-yl)carbamate (**C1-4,** 8.2 g, yield:81.4 %).

LCMS:m/z 328.5 $[M+H]^+$.

Step 4: tert-butyl methyl(1-(((2-(trimethylsilyl)ethoxy)methyl)-1H-imidazol-2-yl)carbamate (C1-4, 8.2 g, 25.0 mmol) was added into anhydrous tetrahydrofuran(200 mL) and added with a solution of tetrabutylammonium fluoride in tetrahydrofuran (1M, 125 mL, 125 mmol). The reaction mixture was reacted for 1 hour under argon protection at 80°C. The reaction liquid was concentrated. The crude product was purified through column chromatography (ethyl acetate: petroleum ether=1:10) to obtain a yellow solid of tert-butyl (1H-imidazol-2-yl)(methyl)carbamate(**C1**, 2.3 g, yield:48.0 %).

LCMS:m/z 198.5 $[M+H]^+$.

The following intermediates are commercially available:

| No. | Structural Formula | name |
|---|---|---|
| C2 | 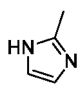 | 2-methyl-1H-imidazole |

(continued)

| No. | Structural Formula | name |
|---|---|---|
| **C3** | 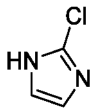 | 2-chloro-1H-imidazole |
| **C4** | 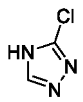 | 3-chloro-4H-1,2,4-triazole |
| **C5** | 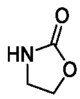 | oxazolin-2-one |
| **C6** | 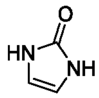 | 1,3-dihydro-2H-imidazol-2-one |
| **C7** | 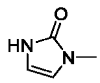 | 1-methyl-1,3-dihydro-2H-imidazol-2-one |
| **C8** | 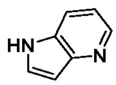 | 1H-pyrrolo[3,2-b]pyridine |
| **C9** | 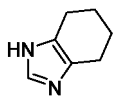 | 4,5,6,7-tetrahydro-1H-benzo[d]imidazole |

Synthesis of Intermediate **D57:** potassium ((4-ethylpiperazin-1-yl)methyl) trifluoroborate

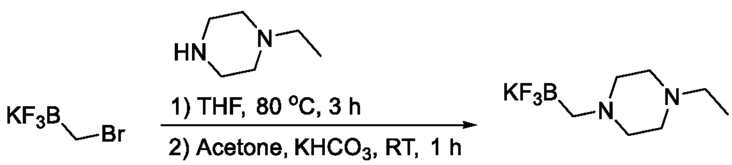


**D57**

Step 1: potassium bromomethyl trifluoroborate (200 mg, 1 mmol) was added into a solution (4 mL) of 1-ethylpiperazine (460 mg, 4 mmol) in tetrahydrofuran (20 mL). After the air was replaced with nitrogen, the reaction mixture was reacted for 3 hours at 80°C. The reaction liquid was dried by a rotary evaporator to obtain a crude product. The resultant crude product was dissolved in acetone (20 mL) and added with potassium bicarbonate (100 mg, 1 mmol). The resultant suspension was stirred at room temperature for 1 hour and filtered. The filtrate was concentrated under reduced pressure to dryness to obtain a white solid of potassium ((4-ethylpiperazin-1-yl)methyl)trifluoroborate (**D57,** 250 mg, yield: 100 %).

The following intermediates were synthesized according to the above method:

| No. | Structural Formula | name |
|---|---|---|
| **D58** | 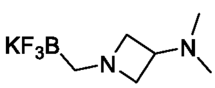 | potassium ((N, N-dimethylazetidinyl-3-yl)methyl)trifluoro borate |

(continued)

| No. | Structural Formula | name |
|---|---|---|
| D59 | 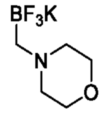 | potassium (morpholinylmethyl)trifluoroborate |
| D61 | 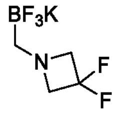 | potassium salt of 3,3-difluoro-1-((trifluoro-14-boranyl)methyl)azetidine |
| D62 | 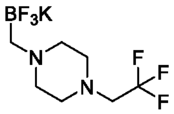 | potassium ((4-trifluoroethylpiperazin-1-yl)methyl) trifluoroborate |
| D64 | 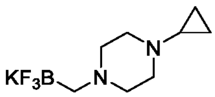 | potassium ((4-cyclopropylpiperazin-1-yl)methyl) trifluoroborate |
| D65 | 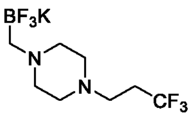 | potassium 1-(3,3,3-trifluoropropyl)piperazine trifluoroborate |
| D66 | 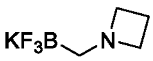 | potassium azetidinyl-1-methyltrifluoroborate |
| D67 | 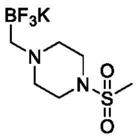 | potassium 4-(methylsulfonyl)piperazin-1-methyltrifluoroborate |
| D68 | 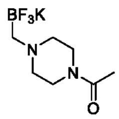 | potassium 4-(acetyl)piperazin-1-methyltrifluoroborate |
| D69 | 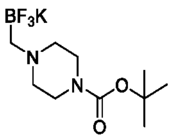 | potassium 4-(tertbutyloxycarbonyl)piperazin-1- methyltrifluoroborate |
| D70 | 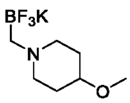 | potassium 4-(methoxy)pyridin-1-methyltrifluoroborate |
| D71 | 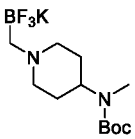 | potassium 4-(N-methyl-N-tertbutyloxycarbonylamino)-pyridin-1-methyltrifluoroborate |
| D72 | 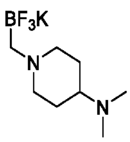 | potassium 4-(N, N-dimethylamino)-pyridin-1-methyltrifluoroborate |

(continued)

| No. | Structural Formula | name |
|---|---|---|
| **D73** | 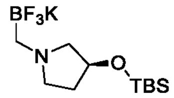 | potassium S-3-tert-butyl dimethylsilylpyrrolidin-1-methyltrifluoroborate |
| **D74** | 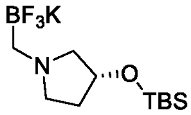 | potassium R-3-tert-butyl dimethylsilylpyrrolidin-1-methyltrifluoroborate |
| **D75** | 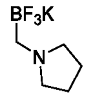 | Potassium pyrrolidin-1-methyltrifluoroborate |
| **D76** | 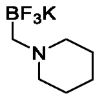 | Potassium pyridin-1-methyltrifluoroborate |
| **D77** | 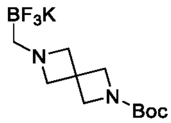 | potassium salt of tert-butyl 6-((trifluoro-λ4-boryl)methyl)-2,6-diazaspiro [3.3] heptan-2-carboxylate |
| **D78** | 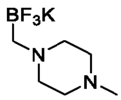 | potassium ((4-methylpiperazin-1-yl)methyl)trifluoroborate |
| **D79** | 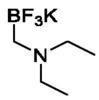 | potassium N,N-diethyl-amino-methyltrifluoroborate |
| **D80** | 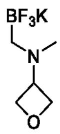 | potassium N-methyl-N-(epoxybutan-3)-amino-methyltrifluoroborate |

**Example 1: Synthesis of Compound 1 and 1'**

(S)-5'-((dimethylamino)methyl)-2'-(1-(4-ethoxy-5-fluoropyridine-2-yl)ethyl)-7'-((2-(methylamino)-1H-imidazol-1-yl)methyl)-2',3'-dihydro-1'H-spiro[cyclopropan-1,4'-isoquinoline]-1'-one

(R)-5'-((dimethylamino)methyl)-2'-(1-(4-ethoxy-5-fluoropyridine-2-yl)ethyl)-7'-((2-(methylamino)-1H-imidazol-1-yl)methyl)-2',3'-dihydro-1'H-spiro[cyclopropan-1,4'-isoquinoline]-1' -one

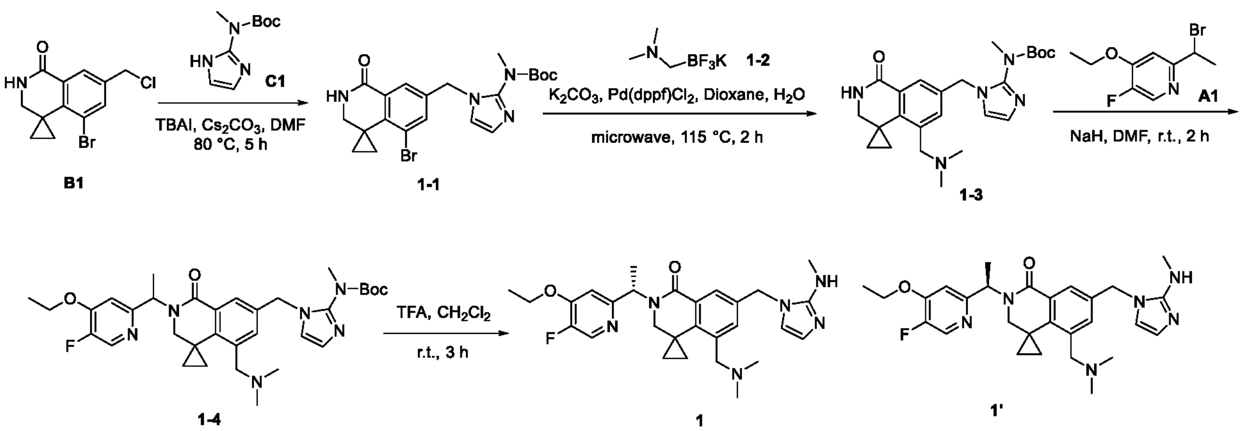


B1 1-1 1-3 1-4 1 1'

Step 1: 5'-bromo-7'-(chloromethyl)-2',3'-dihydro-1'H-spiro[cyclopropan-1,4'-isoquinoline]-1'-one (**B1**, 302 mg, 1 mmol) was added into a solution of tert-butyl (1H-imidazol-2-yl)(methyl)carbamate (**C1**, 197 mg, 1 mmol), tetrabutylammonium iodide (369 mg, 1 mmol) and cesium carbonate (650 mg, 2 mmol) in DMF (45 mL) at 0 °C and reacted at 80°C for 5 hours. The reaction liquid was poured into ice water (20 mL), extracted with dichloromethane (2 * 150 mL). The organic phase was combined, washed with saturated saline (200 mL), dried with anhydrous sodium sulfate and filtered. The filtrate was concentrated under reduced pressure to dryness. After column chromatography purification (ethyl acetate: petroleum ether=1:3), a yellow solid of tert-butyl (1-(((5'-bromo-1'-one-2', 3'-dihydro-1'H-spiro[cyclopropan-1,4'-isoquinoline] -7'-yl)methyl)- 1H-imidazol-2-yl)(methyl)carbamate (**1-1,** 350 mg, yield:75.9 %) was obtained.

LCMS: m/z: 461.1/463.1$[M+H]^+$.

Step 2: Pd(dppf)$Cl_2$ (37 mg, 0.05 mmol), $K_2CO_3$ (138 mg, 1 mmol) were added into a mixed solution of tert-butyl (1-(((5'-bromo-1'-one-2', 3'-dihydro-1'H-spiro[cyclopropan-1,4'-isoquinoline] -7'-yl)methyl)-1H-imidazol-2-yl)(methyl)carbamate (**1-1,** 230 mg, 0.5 mmol) and potassium N,N-dimethylaminomethyl trifluoroborate (**1-2,** 165 mg, 1 mmol) in dioxane (6 mL) and water (2 mL). After the atmosphere was replaced with nitrogen, the reaction mixture was reacted under microwave at 115 °C for 2 hours until 50% product was observed by LCMS. The reaction liquid was diluted with ethyl acetate (200 mL) followed by adding a saturated saline solution. The water phase and the organic phase were separated. The water phase was extracted with ethyl acetate (100 mL). The organic phase was combined, washed with saturated saline, dried with anhydrous sodium sulfate, filtered, and concentrated under reduced pressure to dryness. After column chromatography purification (ethyl acetate: petroleum ether=1:2), a yellow solid of tert-butyl (1-((5'-(((dimethylamino)methyl)-1'-one-2', 3 '-dihydro-1'H-spiro[cyclopropan-1,4'-isoquinoline]-7'-(methyl)-1H-imidazol-2-yl)(methyl)carbamate (**1-3,** 105 mg, yield:47.8 %) was obtained.

LCMS: m/z: 440.5$[M+H]^+$.

Step 3: NaH (60% dispersed in mineral oil, 30 mg, 0.72 mmol) was added into a solution of tert-butyl (1-((5'-(((dimethylamino)methyl)-1'-one-2',3'-dihydro-1'H-spiro[cyclopropan-1,4'-isoquinoline]-7'-(methyl)-1H-imidazol-2-yl)(methyl)carbamate (**1-3,** 100 mg, 0.228 mmol) in DMF (15 mL) at 0 °C and stirred at 0 °C for 20 mins. 2-(1-bromoethyl)-4-ethoxy-5-fluoropyridine (**A1**, 90 mg, 0.363 mmol) was added. The reaction mixture was reacted at room temperature under stirring for 2 hours. The reaction liquid was poured into ice water (20 mL), extracted with dichloromethane (2 * 50 mL). The organic phase was combined, washed with saturated saline (100 mL), dried with anhydrous sodium sulfate and filtered. The filtrate was concentrated under reduced pressure to dryness. After column chromatography purification (ethyl acetate: petroleum ether=2:1), a yellow solid of tert-butyl (1-((5'-((dimethylamino)methyl)-2'-(1-(4-ethoxy-5-fluoropyridine-2-yl)ethyl)-1'-one-2', 3' -dihydro-1'H-spiro[cyclopropan-1,4'-isoquinoline] -7'-yl)methyl)-1H-imidazol-2-yl)(methyl)carbamate (**1-4,** 90 mg, yield: 65.2%) was obtained.

LCMS: m/z: 607.8 $[M+H]^+$ .

Step 4: trifluoroacetic acid (4 mL) was added into a solution of tert-butyl (1-((5'-((dimethylamino)methyl)-2'-(1-(4-ethoxy-5-fluoropyridine-2-yl)ethyl)-1'-one-2',3'-dihydro-1'H-spiro[cyclopropan-1,4'-isoquinoline]-7'-yl)methyl)-1H-imidazol-2-yl)(methyl) carbamate (**1-4,** 90 mg, 0.148 mmol) in dichloromethane (10 mL) at 0 °C and reacted at room temperature under stirring for 2 hours. The reaction liquid was poured into ice water (30 mL), neutralized to pH 7 with a saturated aqueous solution of sodium bicarbonate and extracted with dichloromethane (2 * 100 mL). The organic phase was combined, washed with saturated saline (100 mL), dried with anhydrous sodium sulfate and filtered. The filtrate was concentrated under reduced pressure to dryness. After high performance liquid chromatography separation and purification (SunFire Prep C18 OBD 10um, acetonitrile/water system), a white solid was obtained. The white solid was separated through SFC (column model: DAICELCHIRALCEL®AD; Mobile phase: redistilled grade EtOH and Supercritical $CO_2$; wavelength: 214 nm) to obtained Compound **1** (formate salt, 16 mg, yield: 19.5%) and Compound **1'** (formate salt, 18 mg, yield: 22%).

Compound **1:** (S)-5'-((dimethylamino)methyl)-2'-(1-(4-ethoxy-5-fluoropyridine-2-yl)ethyl)-7'-((2-(methylamino)-1H-imidazol-1-yl)methyl)-2',3'-dihydro-1'H-spiro[cyclopropan-1,4'-isoquinoline]-1'-one

LCMS: m/z 507.4 $[M+H]^+$.

$^1$H NMR (400 MHz, methanol-$d_4$): δ ppm 8.48 (s, 1H), 8.23 (d, *J* = 3.2 Hz, 1H), 7.84 (s, 1H), 7.29 (s, 1H), 7.17 (d, *J* = 6.8 Hz, 1H), 6.95(d, *J* = 2.0 Hz, 1H), 6.91(d, *J* = 2.4 Hz, 1H), 5.96~5.90 (m, 1H), 5.08 (s, 2H), 4.24~4.16 (m, 2H), 3.60~3.47 (m, 2H), 3.37-3.34 (m, 1H), 3.01-2.98 (m, 1H), 2.98 (s, 3H), 2.24 (s, 6H), 1.56~1.51(m, 4 H), 1.43 (t, *J* = 7.2 Hz, 3H), 1.08~1.05 (m, 1 H), 0.95~0.90 (m, 1 H), 0.53~0.48 (m, 1 H).

$^{19}$F NMR (376 MHz, methanol-$d_4$): δ ppm -153.96.

Compound **1':** (R)-5'-((dimethylamino)methyl)-2'-(1-(4-ethoxy-5-fluoropyridine-2-yl)ethyl)-7'-((2-(methylamino)-1H-imidazol-1-yl)methyl)-2',3'-dihydro-1'H-spiro[cyclopropan-1,4'-isoquinoline]-1' -one

Data analysis:

LCMS: m/z 507.1 $[M+H]^+$.
$^1$H NMR (400 MHz, DMSO-$d_6$): δ ppm 8.37 (d, *J* = 2.8 Hz, 1H), 7.74 (d, *J* = 1.6 Hz, 1H), 7.18 (d, *J* = 1.6 Hz, 1H), 7.13 (d, *J* = 6.8 Hz, 1H), 6.74 (s, 1H), 6.59 (s, 1H), 6.23-6.21 (m, 1H), 5.89-5.83 (m, 1H), 4.96 (s, 2H), 4.25-4.11 (m, 2H), 3.34-3.17 (m, 3H), 2.91 (d, *J* = 12.8 Hz, 1H), 2.76 (d, *J* = 4.8 Hz, 3H), 2.06 (s, 6H), 1.47 (d, *J* = 7.2 Hz, 3H), 1.43-1.39 (m, 1H), 1.35 (t, *J* = 6.8 Hz, 3H), 1.16-1.12 (m, 1H), 0.83-0.78 (m, 1H), 0.52-0.47 (m, 1H).
$^1$F NMR (376 MHz, DMSO-$d_6$): δ ppm-153.19.

The following compounds were synthesized by the same method or modified method using the corresponding intermediates with reference to the synthesis method for Compound 1 of Example 1.

The following Examples refer to free compounds or pharmaceutically acceptable salts thereof.

| No | Starting intermediate and material, reference step | Compound structure | Compound name | Data analysis |
|---|---|---|---|---|
| Compound **4** | **1-3, A4,** With reference to Step 3 and Step 4 Of Example 1 | 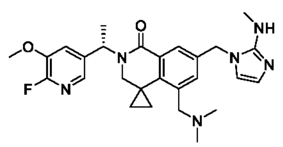 | (S)-5'-(((dimethylamino) methyl) -2'-(1-(6-fluoro-5-methoxypyridine-3 - yl) ethyl)-7'-((2-(methylamino)-1H-imidazol-l-yl)methyl)-2', 3'-dihydro-1'H-spiro [cyclopropan-1,4'-isoquinoline] -1'-one | LCMS: m/z: 493.7 $[M+H]^+$ . $^1$H NMR (400 MHz, DMSO-$d_6$): δ ppm 7.78 (s, 1H), 7.69 (s, 1H), 7.50 (d, *J* = 10.4 Hz, 1H), 7.32 (s, 1H), 7.26 (s, 1H), 6.95 (s, 1H), 6.85 (s, 1H), 5.92-5.89 (m, 1H), 5.05 (s, 2H), 3.86 (s, 3H), 3.27-3.21 (m, 3H), 2.83 (d, *J* = 4.8 Hz, 3H), 2.76 (d, *J* = 12.8 Hz, 1H), 2.07 (s, 6H), 1.50 (d, *J* = 7.2 Hz, 3H), 1.42-1.39(m, 1H), 1.18-1.16 (m, 1H), 0.87-0.82 (m, 1H), 0.44-0.40 (m, 1H). |

(continued)

| No | Starting intermediate and material, reference step | Compound structure | Compound name | Data analysis |
|---|---|---|---|---|
| Co mp ou nd 5 | **1-3, A5,** with reference to Step 3 and Step 4 of Example 1 | 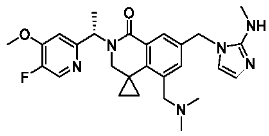 | (S)-5'-(((dimethylamino) methyl) -2'-(1-(5-fluoro-4-methoxypyridine-2-yl) ethyl)-7'-((2-(methylamino)-1H-imidazol-l-yl)methyl)-2', 3'-dihydro-1'H-spiro [cyclopropan-1,4'-isoquinoline] -1'-one | LCMS: m/z: 493.7 $[M+H]^+$ . $^1H$ NMR (400 MHz, DMSO-$d_6$): δ ppm 8.36 (d, *J* = 2.8 Hz, 1H), 7.75 (s, 1H), 7.19(s, 1H), 7.14 (d, *J* = 7.2 Hz, 1H), 6.8 (s, 1H), 6.68 (s, 1H), 6.61 (brs, 1H), 5.89-5.84 (m, 1H), 4.98 (s, 2H), 3.91 (s, 3H), 3.27-3.21 (m, 3H), 2.92 (d, *J* = 12.8 Hz, 1H), 2.78 (d, *J* = 3.2 Hz, 3H), 2.08 (s, 6H), 1.47 (d, *J* = 7.2 Hz, 3H), 1.41-1.38 (m, 1 H), 1.17-1.13 (m, 1 H), 0.84-0.79 (m, 1 H), 0.55-0.49 (m, 1 H). |
| Co mp ou nd 7 | **1-1, A1,** D76 with reference to Step 2 to Step 4 of Example 1 | 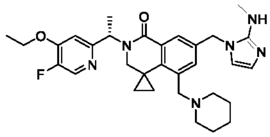 | (S)-2'-(l-(4-ethoxy-5-fluoropyridine-2-yl)ethyl)-7'-((2-(methylamino)-1H-imidazol-1-yl)methyl)-5'-(pyridin-l-ylmethyl)-2',3'-dihydro-1'H-spiro [cyclopropan-1,4'-isoquinoline]-1'-one | LCMS: m/z: 547.3 $[M+H]^+$ . $^1H$ NMR (400 MHz, DMSO-$d_6$): δ ppm 8.36 (d, *J* = 3.2 Hz, 1H), 7.75-7.70 (m, 2H), 7.23 (s, 1H), 7.12 (d, *J* = 7.2 Hz, 1H), 7.05 (s, 1H), 6.98 (s, 1H), 5.88-5.83 (m, 1H), 5.08 (s, 2H), 4.24-4.16 (m, 2H), 3.42-3.38 (m, 2H), 3.20-3.17 (m, 1H), 2.92-2.89 (m, 1H), 2.85 (d, *J* = 4.8 Hz, 3H), 2.21 (s, 4H), 1.46 (d, *J* = 7.2 Hz, 3H), 1.43-1.33 (m, 10 H), 1.19-1.13 (m, 1 H), 0.83-0.78 (m, 1 H), 0.52-0.47 (m, 1 H). |

(continued)

| No | Starting intermediate and material, reference step | Compound structure | Compound name | Data analysis |
|---|---|---|---|---|
| Co mp ou nd **8** | **1-1, A1,** D59 with reference to Step 2 to Step 4 of Example 1 | 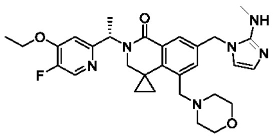 | (S)-2'-(1-(4-ethoxy-5 -fluoropyridine-2-yl)ethyl)-7'-((2-(methylamino)-1H-imidazol-1-yl)methyl)-5'-(morpholinylmethyl)-2',3'-dihydro-1'H-spiro [cyclopropan-1,4'-isoquinoline]-1'-one | LCMS: m/z: 549.3 $[M+H]^+$ . $^1H$ NMR (400 MHz, methanol-$d_4$): δ ppm 8.51 (s, 1H), 8.22 (d, *J* = 3.2 Hz, 1H), 7.81 (s, 1H), 7.22 (s, 1H), 7.15 (d, *J* = 6.8 Hz, 1H), 6.89 (dd, *J* = 14.4, 2.4 Hz, 2H), 5.95-5.90 (m, 1H), 5.04 (s, 2H), 4.24-4.16 (m, 2 H), 3.59-3.54 (m, 5H), 3.42-3.34 (m, 2H), 2.98-2.95 (m, 4 H), 2.31 (s, 4H), 1.63-1.59 (m, 1H), 1.55 (d, *J* = 7.2 Hz, 3H), 1.43 (t, *J* = 7.2 Hz, 3H), 1.16-1.11 (m, 1 H), 0.89-0.84 (m, 1 H), 0.48-0.43 (m, 1 H). |
| Co mp ou nd **9** | **1-1,** D69, **A1,** with reference to Step 2 to Step 4 of Example 1 | 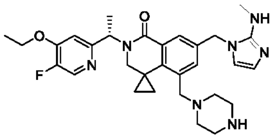 | (S)-2'-(1-(4-ethoxy-5 -fluoropyridine-2-yl)ethyl)-7'-((2-(methylamino)-1H-imidazol-1-yl)methy l)-5'-(piperazin-1-ylmethyl)-2',3'-dihydro-1'H-spiro [cyclopropan-1,4'-isoquinoline]-1'-one | LCMS: m/z: 548.3 $[M+H]^+$ . |
| Co mp ou nd **10** | with reference to Step 2 to Step 4 of Example 1 | 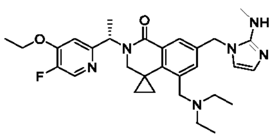 | (S)-5'-((diethylamino) methyl)-2'-(l-(4-ethoxy-5-fluoropyridine-2-yl)ethyl)-7'-((2-(methylamino)-1H-imidazol-1-yl)methyl)-2',3'-dihydro-1'H-spiro [cyclopropan-1,4'-isoquinoline]-1'-one | LCMS: m/z: 535.3 $[M+H]^+$ . $^1H$ NMR (400 MHz, methanol-$d_4$): δ ppm 8.23 (d, *J* = 3.2 Hz, 1H), 7.83 (s, 1H), 7.47 (s, 1H), 7.16 (d, *J* = 6.8 Hz, 1H), 6.97-6.93 (m, 2H), 5.96-5.90 (m, 1H), 5.10 (s, 2H), 4.25-4.17 (m, 2H), 3.78 (s, 2H), 3.38-3.35 (m, 1H), 3.03-3.00 (m, 1H), 3.00 (s, 3H), 2.63-2.31 (m, 4H), 1.57-1.51 (m, 4H), 1.43 (t, *J* = 7.2 Hz, 3H), 1.09-0.97 (m, 7H), 0.95-0.89 (m, 1 H), 0.56-0.52 (m, 1 H). |

(continued)

| No | Starting intermediate and material, reference step | Compound structure | Compound name | Data analysis |
|---|---|---|---|---|
| Co mp ou nd **11** | **1-3, A6,** with reference to Step 3 to Step 4 of Example 1 | 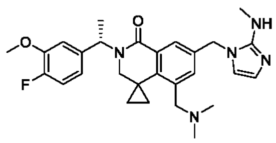 | (S)-5'-((methylamino) methyl)-2'-(1-(4-fluoro-3-methoxyphenyl)ethyl)-7'-((2-(methylamino)-1H-imidazol-1-yl)methyl)-2',3'-dihydro-1'H-spiro [cyclopropan-1,4'-isoquinoline]-1'-one | LCMS: m/z: 492.2 $[M+H]^+$ . |
| Co mp ou nd **12** | **1-3, A7,** with reference to Step 3 to Step 4 of Example 1 | 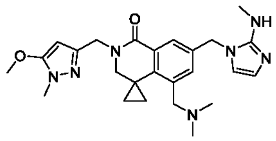 | 5'-((dimethylamino) methyl)-2'-((5-methoxy-1-methyl-1H-pyrazole-3-yl) methyl)-7'-((2-(methylamino)-1H-imidazol-1-yl)methyl)-2',3'-dihydro-1'H-spiro [cyclopropan-1,4'-isoquinoline]-1'- | LCMS: m/z: 463.3 $[M+H]^+$ . |
| Co mp ou nd **14** | **1-3, A9,** with reference to Step 3 to Step 4 of Example 1 | 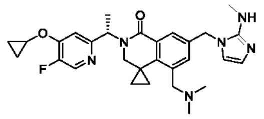 | (S)-2'-(1-(4-cyclopropyloxy-5-fluoropyridine-2-yl)ethyl)-5'-((dimethylamino) methyl)-7'-((2-(methylamino)-1H-imidazol-1-yl)methyl)-2',3'-dihydro-1'H-spiro [cyclopropyl- 1,4'-isoquinoline]-1'-one | LCMS: m/z: 519.2 $[M+H]^+$. $^1H$ NMR (400 Hz, methanol-d4) δ ppm 8.44 (brs, 2 H), 8.24 (d, *J* = 2.8 Hz, 1 H), 7.88 (s, 1 H), 7.48~7.46 (m, 1 H), 7.39~7.34 (m, 1 H), 6.98~6.93 (m, 2 H), 5.99~5.94 (m, 1 H), 5.11 (s, 2 H), 4.02~3.98 (m, 1 H), 3.86~3.76 (m, 2 H), 3.45~3.41 (m, 1 H), 3.03~2.99 (m, 4 H), 2.43 (s, 6 H), 1.59~1.50 (m, 4 H), 1.07~0.98 (m, 2 H), 0.91~0.71 (m, 4 H), 0.60~0.54 (m, 1 H). $^{19}F$ NMR (376 Hz, methanol-d4) δ ppm -153.87. |
| Compound **32** | **1-1, A1, D66** with reference to Step 2 to Step 4 of Example 1 | 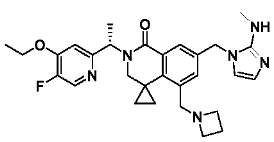 | (S)-5'-((azetidinyl-l-yl) methyl)-2'-(1-(4-ethoxy-5-fluoropyridine-2-yl)ethyl)-7'-((2-(methylamino)-1H-imidazol-l-yl)methyl)-2',3'-dihydro-l'H-spiro [cyclopropan-1,4'-isoquinoline]-1'-one | LCMS: m/z: 519.3 $[M+H]^+$. |

(continued)

| No | Starting intermediate and material, reference step | Compound structure | Compound name | Data analysis |
|---|---|---|---|---|
| Compound **33** | **1-1, A1, D61** with reference to Step 2 to Step 4 of Example 1 | 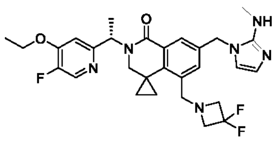 | (S)-5'-((3, 3-difluoroazetidinyl-1-yl) methyl)-2'-(1-(4-ethoxy-5-fluoropyridine-2-yl)ethyl)-7'-((2-(methylamino)-1H-imidazol-1-yl)methyl)-2',3'-dihydro-1'H-spiro [cyclopropan-1,4'-isoquinoline]-1'-one | LCMS: m/z: 555.2 $[M+H]^+$ . $^1H$ NMR (400 MHz, methanol-$d_4$): δ ppm 8.22 (d, *J* = 3.2 Hz, 1H), 7.81 (d, *J* = 2.0 Hz, 1H), 7.23 (d, *J* = 1.6 Hz, 1H), 7.15 (d, *J* = 1.6 Hz, 1H), 6.78 (d, *J* = 2.4 Hz, 1H), 6.77 (d, *J* = 2.4 Hz, 1H), 5.96~5.90 (m, 1H), 5.00 (s, 2H), 4.24~4.16 (m, 2H), 3.81(d, *J* = 12.4 Hz, 1H), 3.69 (d, *J* = 12.4 Hz, 1H), 3.55~3.47 (m, 4H), 3.35 (d, *J* = 12.4 Hz, 1H), 2.98 (d, *J* = 12.4 Hz, 1H), 2.92(s, 3H), 1.56~1.51 (m, 4H), 1.43 (t, *J* = 7.2 Hz, 3H), 1.11~1.06 (m, 1H), 0.92~0.87 (m, 1H), 0.51~0.46 (m, 1H). |
| Compound **34** | **1-1, A1, D80** with reference to Step 2 to Step 4 of Example 1 | 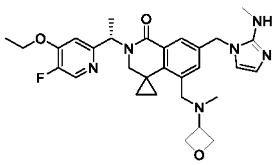 | (S)-2'-(l-(4-ethoxy-5-fluoropyridine-2-yl)ethyl)-5'-((methyl(oxetan-3-yl) amino)methyl)-7'-((2-(methylamino)-1H-imidazol-1-yl)methyl)-2',3'-dihydro-1'H-spiro [cyclopropan-1,4'-isoquinoline]-1'-one | LCMS: m/z: 549.3 $[M+H]^+$ . |
| Compound **35** | **1-1, A1, D73** with reference to Step 2 to Step 4 of Example 1 | 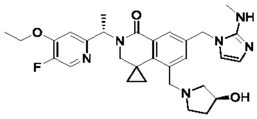 | 2'-((S)-l-(4-ethoxy-5-fluoropyridine-2-yl)ethyl)-5'-(((S)-3-hydroxylpyrrolidin-1-yl) methyl)-7'-((2-(methylamino)-1H-imidazol-1-yl)methyl)-2',3'-dihydro-1'H-spiro [cyclopropan-1,4'-isoquinoline]-1'-one | LCMS: m/z: 549.3 $[M+H]^+$ . |

(continued)

| No | Starting intermediate and material, reference step | Compound structure | Compound name | Data analysis |
|---|---|---|---|---|
| Co mp ou nd **36** | **1-1, A1, D74** with reference to Step 2 to Step 4 of Example 1 | 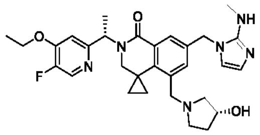 | 2'-((S)-l-(4-ethoxy-5-fluoropyridine-2-yl)ethyl)-5'-(((R)-3-hydroxylpyrrolidin-1-yl) methyl)-7'-((2-(methylamino)-1H-imidazol-1-yl)methyl)-2',3'-dihydro-1'H-spiro [cyclopropan-1,4'-isoquinoline]-1'-one | LCMS: m/z: 549.3 $[M+H]^+$. |
| Co mp ou nd **37** | **1-1, A1, D78** with reference to Step 2 to Step 4 of Example 1 | 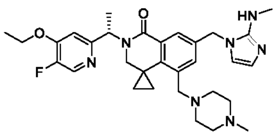 | (S)-2'-(1-(4-ethoxy-5 -fluoropyridine-2-yl)ethyl)-7'-((2-(methylamino)-1H-imidazol-1-yl)methy l)-5'-((4-methylpiperazin-1-yl) methyl)-2',3'-dihydro-1'H-spiro [cyclopropan-1,4'-isoquinoline]-1'-one | LCMS: m/z: 562.3 $[M+H]^+$. $^1$H NMR (400 MHz, methanol-d4): δ ppm 8.48 (br 2H), 8.23 (d, *J* = 3.2 Hz, 1H), 7.82 (d, *J* = 1.6 Hz, 1H), 7.26(d, *J* = 1.6 Hz, 1H), 7.17 (d, *J* = 6.8 Hz, 1H), 6.95 (d, *J* = 2.4 Hz, 1H), 6.91 (d, *J* = 2.4 Hz, 1H), 5.96~5.91 (m, 1H), 5.07 (s, 2H), 4.24~4.16 (m, 2H), 3.67 (d, *J* = 12.4 Hz, 1H), 3.51 (d, *J* = 12.4 Hz, 1H), 3.36~3.29 (m, 4H), 3.00~2.93 (m, 5H), 2.65~2.54 (m, 7H), 1.59~1.54 (m, 4H), 1.43 (t, *J* = 7.2 Hz, 3H), 1.12~1.07 (m, 1H), 0.92~0.87 (m, 1H), 0.50~0.44 (m, 1H). |
| Co mp ou nd **38** | **1-1, A3, D66** with reference to Step 2 to Step 4 of Example 1 | 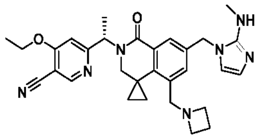 | (S)-6-(1-(5'-(azetidinyl-1-ylmethyl)-7'-((2-(methylamino)-1H-imidazol-1-yl)methyl)-1'-one-1'H-spiro [cyclopropan-1,4'-isoquinoline]-2'(3'H)-yl) ethyl)-4-ethoxy nicotinonitrile | LCMS: m/z: 526.3 $[M+H]^+$ . |

(continued)

| No | Starting intermediate and material, reference step | Compound structure | Compound name | Data analysis |
|---|---|---|---|---|
| Co mp ou nd **39** | **1-1, A3, D75** with reference to Step 2 to Step 4 of Example 1 | 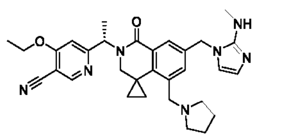 | (S)-4-ethoxy-6-(l-(7'-((2-(methylamino)-1H-imidazol-1-yl)methyl)-1'-one-5'-(pyrrolidin-1-ylmethyl)-l'H-spiro [cyclopropan-1,4'-isoquinoline]-2'(3'H)-yl) ethyl)-4-ethoxy nicotinonitrile | LCMS: m/z: 540.3 $[M+H]^+$ . |
| Co mp ou nd **40** | **1-1, A3, D69** with reference to Step 2 to Step 4 of Example 1 | 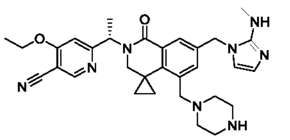 | (S)-4-ethoxy-6-(l-(7'-((2-(methylamino)-1H-imidazol-1-yl)methyl)-1'-one-5'-(piperazin-1-ylmethyl)-l'H-spiro [cyclopropan-1,4'-isoquinoline]-2'(3'H)-yl) ethyl)-4-ethoxy nicotinonitrile | LCMS: m/z: 555.3 $[M+H]^+$. |
| Co mp ou nd **55** | **1-3, A17,** with reference to Step 3 to Step 4 of Example 1 | 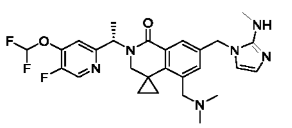 | (S)-2'-(1-(4-(difluoromethoxy)-5-fluoropyridine-2-yl) ethyl)-5'-((dimethylamino) methyl)-7'-((2-(methylamino)-1H-imidazol-1-yl)methyl)-2',3'-dihydro-1'H-spiro [cyclopropan-1,4'-isoquinoline]-1'-one | LCMS: m/z: 529.2 $[M+H]^+$ . $^1$H NMR(400 MHz methanol-d4):δ 8.49 (d, *J* = 2.4 Hz, 1H), 7.83 (d, *J* = 2.4 Hz, 1H), 7.36 (d, *J* = 6.4 Hz, 1H), 7.32-6.95 (m, 2H), 6.62 (d, *J* = 1.6 Hz, 1H), 6.59 (s, 1H), 5.97 (q, *J* = 7.2 Hz, 1H), 4.94 (s, 2H), 3.42-3.26 (m, 3H), 2.99 (d, *J* = 13.2 Hz, 1H), 2.84 (s, 3H), 2.11 (s, 6H), 1.58-1.53 (m, 4H), 1.13-1.08 (m, 1H), 0.90-084 (m, 1H), 0.53-0.48 (m, 1H). $^{19}$F NMR (376 MHz, methanol-d4): δ ppm -85.66, -85.78, -150.26. |
| Co mp ou nd **56** | **1-3, A18,** with reference to Step 3 to Step 4 of Example 1 | 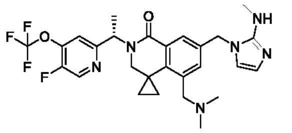 | (S)-5'-((dimethylamino) methyl)-2'-(1-(5-fluoro-4-(trifluoromethoxy)pyridin e-2-yl)ethyl)-7'-((2-(methylamino)-1H-imidazol-1-yl)methyl)-2',3'-dihydro-1'H-spiro [cyclopropan-1,4'-isoquinoline]-1'-one | LCMS: m/z: 547.3 $[M+H]^+$ |

(continued)

| No | Starting intermediate and material, reference step | Compound structure | Compound name | Data analysis |
|---|---|---|---|---|
| Co mp ou nd **57** | **1-1, A1**, **D57** with reference to Step 2 to Step 4 of Example 1 | 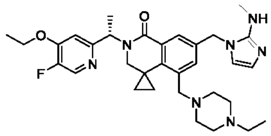 | (S)-2'-(1-(4-ethoxy-5 -fluoropyridine-2-yl)ethyl)-5'-(((4-ethylpiperazin-1-yl) methyl)-7'-((2-(methylamino)-1H -imidazol-l-yl)methyl)-2', 3'-dihydro-1'H-spiro [cyclopropan-1,4'-isoquinoline] -1'-one | LCMS: m/z: 576.3 $[M+H]^+$ . $^1$H NMR (400 MHz, methanol-d4): δ ppm 8.56 (br, 1H), 8.23 (d, *J* = 3.2 Hz, 1H), 7.82 (d, *J* = 1.6 Hz, 1H), 7.26 (d, *J* = 1.6 Hz, 1H), 7.17 (d, *J* = 6.8 Hz, 1H), 6.94 (d, *J* = 2.0 Hz, 1H), 6.89 (d, *J* = 2.0 Hz, 1H), 5.96~5.90 (m, 1H), 5.06 (s, 2H), 4.24~4.16 (m, 2H), 3.67 (d, *J* = 12.4 Hz, 1H), 3.51 (d, *J* = 12.8 Hz, 1H), 3.35~3.30 (m, 2H), 3.01~2.92 (m, 9H), 2.59~2.53 (m, 4H), 1.58~1.55 (m, 4H), 1.43 (t, *J* = 7.2 Hz, 3H), 1.24 (t, *J* = 7.2 Hz, 3H), 1.12~1.07 (m, 1H), 0.92~0.87 (m, 1H), 0.50~0.44 (m, 1H). $^{19}$F NMR (376 MHz, methanol-d4): -153.96. |
| Co mp ou nd **58** | **1-1, A1**, **D58** with reference to Step 2 to Step 4 of Example 1 | 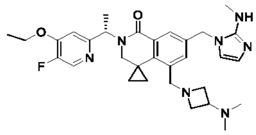 | (S)-5'-((3-(dimethylamino) azetidinyl -1-yl)methyl)-2'-(1-(4-ethoxy-5-fluoropyridine-2-yl)ethyl)-7'-((2-(methylamino)-1H-imidazol-l-yl)methyl)-2', 3'-dihydro-1'H-spiro [cyclopropan-1,4'-isoquinoline] -1'-one | LCMS: m/z: 562.2 $[M+H]^+$ . $^1$H NMR (400 MHz, methanol-d4): δ ppm 8.23 (d, *J* = 3.2 Hz, 1H), 7.81 (d, *J* = 2.4 Hz, 1H), 7.18~7.14 (m, 2H), 6.74~6.73 (m, 2H), 5.96~5.91 (m, 1H), 4.99 (s, 2H), 4.24~4.15 (m, 2H), 3.67~3.54 (m, 2H), 3.40~3.31 (m, 3H), 2.97~2.85 (m, 7H), 2.13 (s, 6H), 1.56~1.50 (m, 4H), 1.43 (t, *J* = 6.8 Hz, 3H), 1.10~1.05 (m, 1H), 0.91~0.86 (m, 1H), 0.49~0.44 (m, 1H). $^{19}$F NMR (376 MHz, methanol-d4): -154.04. |

(continued)

| No | Starting intermediate and material, reference step | Compound structure | Compound name | Data analysis |
|---|---|---|---|---|
| Co mp ou nd **59** | **1-1, A3, D59** with reference to Step 2 to Step 4 of Example 1 | 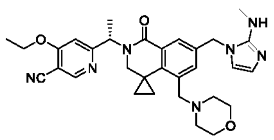 | (S)-4-ethoxy-6-(l-(7'-((2-(methylamino)-1H-imidazol-l-yl)methyl)-5'-(morpholinomethyl)-1'-one-1'H-spiro [cyclopropan-1,4'-isoquinoline] -2'(3'H)-yl) ethyl)nicotinonitrile | LCMS: m/z: 556.3 $[M+H]^+$ . $^1$H NMR (400 MHz, methanol-d4): δ ppm 8.62 (s, 1H), 8.51 (brs, 1H), 7.78 (d, *J* = 2.0 Hz, 1H), 7.24(d, *J* = 1.6 Hz, 1H), 7.18 (d, *J* = 6.8 Hz, 1H), 6.91 (d, *J* = 2.4 Hz, 1H), 6.87 (d, *J* = 2.4 Hz, 1H), 5.92~5.87 (m, 1H), 5.04 (s, 2H), 4.34~4.26 (m, 2H), 3.60~3.54(m, 5H), 3.44 (d, *J* = 12.4 Hz, 1H), 3.35 (d, *J* = 12.4 Hz, 1H), 3.14 (d, *J* = 12.4 Hz, 1H), 2.96 (s, 3H), 2.33 (s, 4H), 1.62~1.58 (m, 4H), 1.46 (t, *J* = 7.2 Hz, 3H), 1.25~1.20 (m, 1H), 0.95~0.90 (m, 1H), 0.67~0.62 (m, 1H). |
| Co mp ou nd **60** | **1-1, A3, D78** with reference to Step 2 to Step 4 of Example 1 | 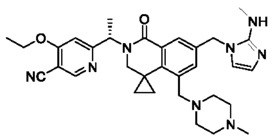 | (S)-4-ethoxy-6-(l-(7'-((2-(methylamino)-1H-imidazol-1-yl)methy l)-5'-(morpholinomethyl)- 1'-one-1'H-spiro [cyclopropan-1,4'-isoquinoline] -2'(3'H)-yl) ethyl)nicotinonitrile | LCMS: m/z: 556.3 $[M+H]^+$ . $^1$H NMR (400 MHz, methanol-d4): δ ppm 8.62 (s, 1H), 8.48 (s, 2H), 7.79 (d, *J* = 2.0 Hz, 1H), 7.25 (d, *J* = 1.6 Hz, 1H), 7.19 (s, 1H), 6.93 (d, *J* = 2.4 Hz, 1H), 6.88 (d, *J* = 2.4 Hz, 1H), 5.93~5.87 (m, 1H), 5.06 (s, 2H), 4.34~4.27 (m, 2H), 3.64 (d, *J* = 12.4 Hz, 1H), 3.50 (d, *J* = 12.4 Hz, 1H), 3.36 (d, *J* = 12.4 Hz, 1H), 3.15 (d, *J* = 12.4 Hz, 1H), 2.97 (s, 3H), 2.86 (s, 4H), 2.59 (s, 3H), 2.52 (s, 4H), 1.60~1.54 (m, 4H), 1.47 (t, *J* = 7.2 Hz, 3H), 1.18~1.15 (m, 1H), 0.96~0.92 (m, 1H), 0.67~0.62 (m, 1H). |

(continued)

| No | Starting intermediate and material, reference step | Compound structure | Compound name | Data analysis |
|---|---|---|---|---|
| Co mp ou nd **61** | **1-1, A2, D61** with reference to Step 2 to Step 4 of Example 1 | 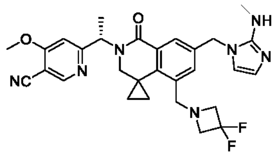 | (S)-6-(l-(5'-(((3,3-difluoroazetidinyl-1 - yl) methyl)-7)-((2-(methylamino)-1H-imidazol-1-yl)methyl)-1'-oxo-l'H-spiro [cyclopropan-1,4'-isoquinoline] -2'(3'H)-yl)ethyl)-4-methoxynicotinonitrile | LCMS: m/z: 548.1 $[M+H]^+$. $^1$H NMR (400 MHz, methanol-d4): δ ppm 8.63 (s, 1H), 8.52 (br 1H), 7.80 (s, 1H), 7.28 (s, 1H), 7.23 (s, 1H), 6.84 (s, 1H), 6.82 (s, 1H), 5.93~5.89 (m, 1H), 5.03 (s, 2H), 4.04 (s, 3H), 3.82~3.71 (m, 2H), 3.56~3.50 (m, 4H), 3.39 (d, *J* = 13.2 Hz, 1H), 3.17 (d, *J* = 13.2 Hz, 1H), 2.95 (s, 3H), 1.61~1.52 (m, 4H), 1.20~1.18 (m, 1H), 0.98~0.95 (m, 1H), 0.71~0.67 (m, 1H). |
| Co mp ou nd **62** | **1-1, A1, D62** with reference to Step 2 to Step 4 of Example 1 | 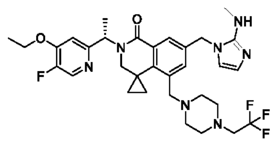 | (S)-2'-(1-(4-ethoxy-5 -fluoropyridine-2-yl)ethyl)-7'-(((2-(methylamino)-1H-imidazol-1-yl)methy l)-5'-((4 -(2,2,2-trifluoroethyl) piperazin-1 - yl)methyl)-2',3'-dihydro-1'H-spiro [cyclopropan-1,4'-isoquinoline]-1'-one | LCMS: m/z: 630.3 $[M+H]^+$ . Hnmr: |
| Co mp ou nd **63** | **1-1, A3, D62** with reference to Step 2 to Step 4 of Example 1 | 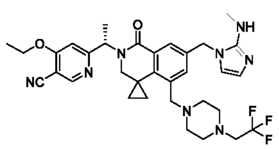 | (S)-4-ethoxy-6-(l-(7'-((2-(methylamino)-1H-imidazol-1-yl)methyl)-1'-one-5'-((4-(2,2,2 -trifluoroethyl)piperazin-1-yl)methyl)-l'H-spiro [cyclopropan-1,4'-isoquinoline] -2'(3'H)-yl) ethyl)nicotinonitrile | LCMS: m/z: 637.8 $[M+H]^+$ . $^1$H NMR (400 MHz, methanol-d4): δ ppm 8.61 (s, 1H), 8.51 (brs, 1H), 7.78 (d, *J* =2.0 Hz, 1H), 7.18(d, *J* = 3.6 Hz, 2H), 6.79 (d, *J* = 2.0 Hz, 1H), 6.77 (d, *J* = 2.0 Hz, 1H), 5.92~5.87 (m, 1H), 4.99 (s, 2H), 4.33~4.26 (m, 2H), 3.54 (d, *J* = 12.4 Hz, 1H), 3.42-3.33 (m, 2H), 3.13~2.97 (m, 3H), 2.92(s, 3H), 2.59 (s, 4H), 2.35 (s, 4H), 1.63~1.56 (m, 4H), 1.46 (t, *J* = 7.2 Hz, 3H), 1.21~1.17 (m, 1H), 0.92~0.87 (m, 1H), 0.62~0.57 (m, 1H). |

(continued)

| No | Starting intermediate and material, reference step | Compound structure | Compound name | Data analysis |
|---|---|---|---|---|
| Co mp ou nd **64** | **1-1, A1, D64** with reference to Step 2 to Step 4 of Example 1 | 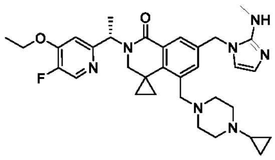 | (S)-5'-((4-cyclopropylpiperazin-1 - yl) methyl)-2'-(1-(4-ethoxy-5-fluoropyridine-2-yl)ethyl)-7'-((2-(methylamino)-1H -imidazol-l-yl)methyl)-2', 3'-dihydro-1'H-spiro [cyclopropan-1,4'-isoquinoline] -1'-one | LCMS: m/z: 588.2 $[M+H]^+$ $^1H$ NMR (400 MHz, DMSO -d6): $\delta$ ppm 8.37 (d, *J* = 2.8 Hz, 1H), 8.30 (s, 2H), 7.71 (d, *J* = 1.6 Hz, 1H), 7.13-7.11 (m, 2H), 6.65 (d, *J* = 1.2 Hz, 1H), 6.47 (d, *J* = 1.6 Hz, 1H), 5.86 (q, *J* = 7.2 Hz, 1H), 5.79-5.76 (m, 1H), 4.92 (s, 2H), 4.23-4.13 (m, 2H), 3.43 (d, *J* = 12.4 Hz, 1H), 3.31 (d, *J* = 12.4 Hz, 1H), 3.20 (d, *J* = 13.2 Hz, 1H), 2.90 *(d, J*= 13.2 Hz, 1H), 2.72 (d, *J* = 4.4 Hz, 3H), 2.51-2.38 (m, 4H), 2.21~2.16 (m, 4H), 1.55~1.53 (m, 1H), 1.47-1.45 (m, 4H), 1.35 (t, *J* = 6.8 Hz, 3H), 1.16-1.11 (m, 1H), 0.81-0.76 (m, 1H), 0.48-0.43 (m, 1H), 0.38-0.35 (m, 2H), 0.27-0.25 (m, 2H). $^{19}F$ NMR (376 MHz, DMSO-d6): $\delta$ ppm - 153.18. |
| Co mp ou nd **65** | **1-1, A1, D65** with reference to Step 2 to Step 4 of Example 1 | 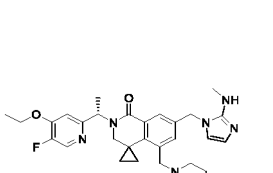 | (S)-2'-(1-(4-ethoxy-5 -fluoropyridine-2-yl)ethyl)-7'-(((2-(methylamino)-1H-imidazol-1-yl)methy l)-5'-((4 -(3,3,3-trifluoropropyl)piperazin-l-yl)methyl)-2', 3'-dihydro-1'H-spiro [cyclopropan-1,4'-isoquinoline] -1'-one | LCMS: m/z: 644.6 $[M+H]^+$ . $^1H$ NMR (400 MHz, methanol-d4): $\delta$ ppm 8.22 (s, 1H), 7.82 (s, 1H), 7.15-7.09 (m, 2H), 6.62 (s, 1H), 6.59 (s, 1H), 5.94-5.92 (m, 1H), 4.93 (s, 2H), 4.20-4.17 (m, 2H), 3.55(d, *J* = 12.8 Hz, 1H), 3.37-3.30 (m, 2H), 2.95(d, *J* = 12.8 Hz, 1H), 2.84 (s, 3H), 2.56-2.36 (m, 11H), 1.55-1.53 (m, 4H), 1.43-1.40 (m, 3H), 1.39-1.28 (m, 1H), 1.08-1.07 (m, 1H), 0.83-0.82 (m, 1H), 0.40-0.39 (m, 1H). |

(continued)

| No | Starting intermediate and material, reference step | Compound structure | Compound name | Data analysis |
|---|---|---|---|---|
| Co mp ou nd **66** | **1-1, A8, D78** with reference to Step 2 to Step 4 of Example 1 | 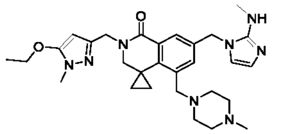 | 2'-((5-ethoxy-1-methyl-1H-pyrazole-3-yl)methyl)-7'-((2-(methylamino)-1H-imidazol-1-yl)methy l)-5'-((4-methylpiperazin-1-yl) methyl)-2',3'-dihydro-1'H-spiro[cyclopropan-1,4'-isoquinoline]-1'-one | LCMS: m/z 533.3 $[M+H]^+$ . $^1$H NMR (400 MHz, methanol-d4): δ ppm 8.48 (brs, 1H), 7.81 (s, 1H), 7.25 (s, 1H), 6.94 (s, 1H), 6.90 (s, 1H), 5.60 (s, 1H), 5.06 (s, 2H), 4.55 (s, 2H), 4.12-4.07 (m, 2H), 3.57-3.55 (m, 5H), 3.19 (s, 2H), 2.98 (s, 3H), 2.88 (s, 4H), 2.61 (s, 3H), 2.50 (s, 4H), 1.40-1.34 (m, 5H), 0.81-0.78 (m, 2 H). |
| Co mp ou nd **67** | **1-1, A3, D61** with reference to Step 2 to Step 4 of Example 1 | 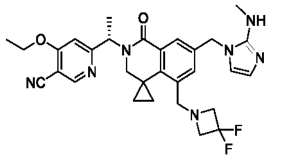 | (S)-6-(l-(5'-((3,3-difluoroazetidinyl-1 - yl) methyl)-7'-((2-(methylamino)-1H-imidazol-1-yl)methyl)-1'-one-1'H-spiro [cyclopropan-1,4'-isoquinoline]-2'(3'H)-yl) ethyl)-4-ethoxynicotinonitrile | LCMS: m/z: 562.3 $[M+H]^+$ . $^1$H NMR (400 MHz, methanol-d4): δ ppm 8.62 (s, 1H), 7.79 (d, *J* = 2.4 Hz, 1H), 7.24(d, *J* = 1.6 Hz, 1H), 7.17 (d, *J* = 1.6 Hz, 1H), 6.77 (d, *J* = 2.4 Hz, 1H), 6.76 (d, *J* = 2.4 Hz, 1H), 5.92~5.87 (m, 1H), 5.00 (s, 2H), 4.34~4.26 (m, 2H), 3.87 (d, *J* = 12.4 Hz, 1H), 3.71 (d, *J* = 12.4 Hz, 1H), 3.55~3.47 (m, 4H), 3.38 (d, *J* = 12.4 Hz, 1H), 3.12 (d, *J* = 12.4 Hz, 1H), 2.91 (s, 3H), 1.59~1.51 (m, 4H), 1.47 (t, *J* = 7.2 Hz, 3H), 1.19~1.14 (m, 1H), 0.98~0.93 (m, 1H), 0.69~0.64 (m, 1H). |

(continued)

| No | Starting intermediate and material, reference step | Compound structure | Compound name | Data analysis |
|---|---|---|---|---|
| Co mp ou nd **68** | **1-1, A1, D61** with reference to Step 1 to Step 4 of Example 1 | 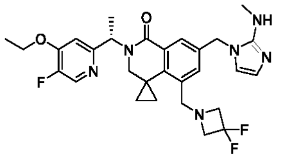 | (S)-5'-((3,3-difluoroazetidinyl-1 - yl) methyl)-2'-(1-(4-ethoxy-5-fluoropyridine-2-yl)ethyl)-7'-((2-(methylamino)-1H-imidazol-1-yl)methyl)-2',3'-dihydro-1'H-spiro [cyclopropan-1,4'-isoquinoline]-1'-one | LCMS: m/z: 555.3 $[M+H]^+$ . $^1$H NMR (400 MHz, methanol-d4): δ ppm 8.22 (d, *J* = 3.2 Hz, 1H), 7.81 (d, *J* = 2.0 Hz, 1H), 7.23 (d, *J* = 1.6 Hz, 1H), 7.15 (d, *J* = 1.6 Hz, 1H), 6.78 (d, *J* = 2.4 Hz, 1H), 6.77 (d, *J* = 2.4 Hz, 1H), 5.96~5.90 (m, 1H), 5.00 (s, 2H), 4.24~4.16 (m, 2H), 3.81(d, *J* = 12.4 Hz, 1H), 3.69 (d, *J* = 12.4 Hz, 1H), 3.55~3.47 (m, 4H), 3.35 (d, *J* = 12.4 Hz, 1H), 2.98 (d, *J* = 12.4 Hz, 1H), 2.92(s, 3H), 1.56~1.51 (m, 4H), 1.43 (t, *J* = 7.2 Hz, 3H), 1.11~1.06 (m, 1H), 0.92~0.87 (m, 1H), 0.51~0.46 (m, 1H). |
| Co mp ou nd **69** | **1-1, A14, D78** with reference to Step 2 to Step 4 of Example 1 | 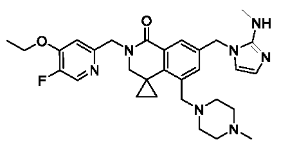 | 2'-((4-ethoxy-5-fluoropyridine-2-yl)methyl)-7'-((2-(methylamino)-1H-imidazol-1-yl)methy l)-5'-((4-methylpiperazin-1-yl) methyl)-2',3'-dihydro-1'H-spiro [cyclopropan-1,4'-isoquinoline] - 1'-one | LCMS: m/z: 547.7 $[M+H]^+$ . $^1$H NMR (400 MHz, methanol-d4) δ 8.15 (d, *J* = 5.6 Hz, 1H), 7.83 (d, *J* = 2.0 Hz, 1H), 7.11~7.08 (m, 2H), 6.64 (dd, *J* = 12.0, 1.6 Hz, 2H), 4.94 (s, 2H), 4.90 (d, J = 2.0 Hz, 2H), 4.21 (q, *J* = 7.2 Hz, 2H), 3.48 (s, 2H), 3.29 (s, 2H), 2.84 (s, 3H), 2.43~2.31 (m, 8H), 2.25 (s, 3H), 1.45 (t, *J* = 7.2 Hz, 3H), 1.40~1.37 (m, 2H), 0.86~0.83 (m, 2H). |

(continued)

| No | Starting intermediate and material, reference step | Compound structure | Compound name | Data analysis |
|---|---|---|---|---|
| Co mp ou nd **71** | **1-3, A13,** with reference to Step 3 to Step 4 of Example 1 | 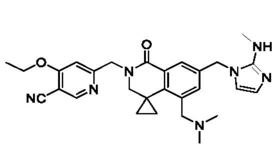 | 6-((5'-((dimethylamino) methyl)-7'-((2-(methylamino)-1H-imidazol-1-yl)methyl)-1'-one-1'H-spiro [cyclopropan-1,4'-isoquinoline]-2'(3'H)-yl) methyl)-4-ethoxynicotinonitrile | LCMS: m/z: 500.2 $[M+H]^+$ . $^1H$ NMR (400 MHz, methanol-d4): δ ppm 8.59 (s, 1H), 8.39 (s, 2H), 7.86 (s, 1H), 7.38 (s, 1H), 7.19 (s, 1H), 6.94 (d, *J* = 16 Hz, 2H), 5.10 (s, 2H), 4.82 (s, 2H), 4.32~4.27 (m, 2H), 3.74 (s, 2H), 3.44 (s, 2H), 2.98 (s, 3H), 2.38 (s, 6H), 1.47 (t, *J* = 7.2 Hz, 3H), 1.39~1.37 (m, 2H), 1.04~1.02 (m, 2H). |
| Co mp ou nd **76** | **1-3, A14,** with reference to Step 3 to Step 4 of Example 1 | 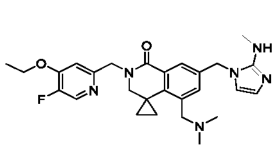 | 5'-((dimethylamino) methyl)-2'-((4-ethoxy-5-fluoropyridine-2-yl)methyl)-7'-((2-(methylamino)-1H-imidazol-1-yl)methyl)-2',3'-dihydro-1'H-spiro [cyclopropan-1,4'-isoquinoline]-1'-one | LCMS: m/z: 493.3 $[M+H]^+$ $^1H$ NMR (400 MHz, methanol-d4) δ ppm: 8.31 (s, 1H), 8.14 (d, *J* = 5.5 Hz, 1H), 7.86 (s, 1H), 7.35 (s, 1H), 7.10 (t, *J* = 6.0 Hz, 1H), 6.96~6.93 (m, 2H), 5.09 (s, 2H), 4.91 (s, 2H), 4.21 (q, *J* = 6.9 Hz, 2H), 3.77 (s, 2H), 3.36 (s, 2H), 2.99 (s, 3H), 2.41 (s, 6H), 1.45 (t, *J* = 6.9 Hz, 3H), 1.34 (s, 2H), 1.01 (s, 2H). |

**Example 2: Synthesis of Compound 2 and 2'**

(S)-6-(1-(5'-(((dimethylamino)methyl)-7'-((2-(methylamino)-1H-imidazol-1-yl)methyl)-1'-one-1'H-spiro[cyclopropan-1,4'-isoquinoline]-2'(3'H)-yl)ethyl)-4-methoxynicotinonitrile

(R)-6-(1-(5'-(((dimethylamino)methyl)-7'-((2-(methylamino)-1H-imidazol-1-yl)methyl)-1'-oxo-1'H-spiro[cyclopropan-1,4'-isoquinoline]-2'(3'H)-yl)ethyl)-4-methoxynicotinonitrile

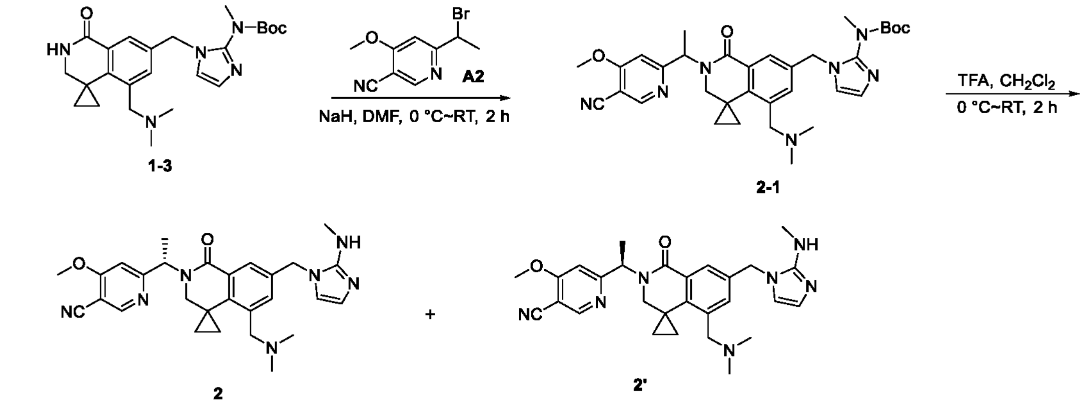


1-3

2-1

2 + 2'

Step 1: NaH (60% dispersed in mineral oil, 21 mg, 0.55 mmol) was added into a solution of tert-butyl (1-((5'-(((dimethylamino)methyl)-1'-one-2', 3'-dihydro-1'H-spiro[cyclopropan-1,4'-isoquinoline] -7'-(methyl)-1H-imidazol-2-yl)(methyl)carbamate (**1-3,** 80 mg, 0.18 mmol) in DMF(10 mL) at 0 °C under argon protection and stirred at 0 °C for 20 mins. 6-(1-bromoethyl)-4-methoxynicotinonitrile (**A2,** 65 mg, 0.27 mmol) was added and the reaction mixture was reacted at room temperature under stirring for 2 hours. The reaction liquid was poured into ice water (20 mL), extracted with dichloromethane (2 * 50 mL). The organic phase was combined, washed with saturated saline (100 mL), dried with anhydrous sodium sulfate and filtered. The filtrate was concentrated under reduced pressure to dryness. After column chromatography purification (dichloromethane: methanol=15:1), a yellow solid of tert-butyl (1-((2'-(1-(5-cyano-4-methoxypyridine-2-yl)ethyl)-5'-((dimethylamino)methyl)-1'-one2', 3' -dihydro-1'H-spiro[cyclopropan-1,4'-isoquinoline] -7'-yl)methyl)-1H-imidazol-2-yl)(methyl)carbamate(**2-1**, 30 mg, yield: 27.5 %) was obtained.

LCMS: m/z: 600.4 $[M+H]^+$.

Step 2: trifluoroacetic acid (2 mL) was added into a solution of tert-butyl (1-((2'-(1-(5-cyano-4-methoxypyridine-2-yl)ethyl)-5'-((dimethylamino)methyl)-1'-one 2', 3' -dihydro-1'H-spiro[cyclopropan-1,4'-isoquinoline]-7'-yl)methyl)-1H-imidazol-2-yl)(methyl)carbamate (**2-1,** 30 mg, 0.05 mmol) in dichloromethane (5 mL) at 0 °C and reacted at room temperature under stirring for 2 hours. The reaction liquid was poured into ice water (20 mL), neutralized to pH 7 with a saturated aqueous solution of sodium bicarbonate, extracted with dichloromethane (2 * 100 mL). The organic phase was combined, washed with saturated saline (100 mL), dried with anhydrous sodium sulfate and filtered. The filtrate was concentrated under reduced pressure to dryness. After column chromatography (dichloromethane: methanol=10:1), a white solid was obtained. The white solid was separated through SFC (column model: DAICELCHIRALCEL®AD; Mobile phase: redistilled grade EtOH and Supercritical $CO_2$; wavelength: 214 nm) to obtain (S)-6-(1-(5'-(((dimethylamino)methyl)-7'-((2-(methylamino)-1H-imidazol-1-yl)methyl)-1'-one-1'H-spiro[cyclopropan -1,4'-isoquinoline] -2'(3'H)-yl)ethyl)-4-methoxynicotinonitrile (Compound **2,** 6 mg, yield: 24.0 %) and (R)-6-(1-(5'-(((dimethylamino)methyl)-7'-((2-(methylamino)-1H-imidazol-1-yl)methyl)-1'-one-1'H-spiro[cyclopropan-1,4'-isoquinoline]-2'(3'H)-yl)ethyl)-4-methoxynicotinonitrile (Compound **2',** 5 mg, yield: 20.0 %).

Compound **2**:(S)-6-(1-(5'-(((dimethylamino)methyl)-7'-((2-(methylamino)-1H-imidazol-1-yl)methyl)-1'-one-1'H-spiro[cyclopropan-1,4'-isoquinoline]-2'(3'H)-yl)ethyl)-4-methoxynicotinonitrile

LCMS: m/z: 500.4 $[M+H]^+$ .

$^1$H NMR (400 MHz, DMSO-$d_6$): δ ppm 8.75 (s, 1H), 7.76(s, 1H), 7.65 (brs, 1H), 7.29 (s, 1H), 7.21 (s, 1H),7.02 (s, 1H), 6.94 (s, 1H), 5.88-5.83 (m, 1H), 5.08 (s, 2H), 3.99 (s, 3H), 3.33~3.21 (m. 2H), 3.20 (d, *J* = 13.6Hz, 1H), 3.06 (d, *J* = 14.4 Hz, 1H), 2.85 (s, 3H), 2.09 (s, 6H), 1.51 (d, *J* = 7.2 Hz, 3H), 1.41-1.35(m, 1 H), 1.29-1.23 (m, 1 H), 0.89-0.82 (m, 1 H), 0.72-0.69 (m, 1 H).

Compound **2':** (R)-6-(1-(5'-(((dimethylamino)methyl)-7'-((2-(methylamino)-1H-imidazol-1-yl)methyl)-1'-one-1'H-spiro[cyclopropan-1,4'-isoquinoline] -2'(3'H)-yl)ethyl)-4-methoxynicotinonitrile

LCMS: m/z: 500.4 $[M+H]^+$ .

**Example 3: Synthesis of Compound 3 and 3'**

(S)-6-(1-(5'-(((dimethylamino)methyl)-7'-((2-(methylamino)-1H-imidazol-1-yl)methyl)-1'-one-1'H-spiro[cyclopropan-1,4'-isoquinoline]-2'(3'H)-yl)ethyl)-4-ethoxynicotinonitrile

(R)-6-(1-(5'-(((dimethylamino)methyl)-7'-((2-(methylamino)-1H-imidazol-1-yl)methyl)-1'-one-1'H-spiro[cyclopropan-1,4'-isoquinoline]-2'(3'H)-yl)ethyl)-4-ethoxynicotinonitrile

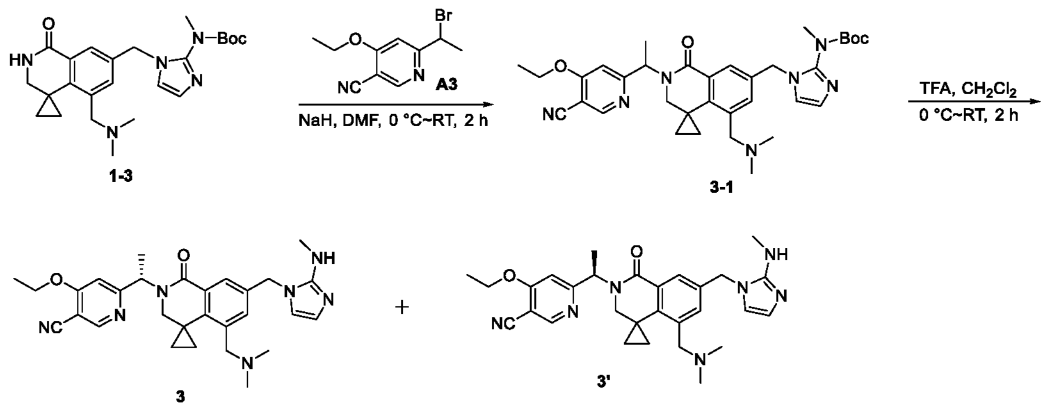


1-3 3-1 3 + 3'

Step 1: NaH (60% dispersed in mineral oil, 21 mg, 0.55 mmol) was added into a solution of tert-butyl (1-((5'-(((dimethylamino)methyl)-1'-one-2',3'-dihydro-1'H-spiro[cyclopropan-1,4'-isoquinoline]-7'-(methyl)-1H-imidazol-2-yl)(methyl)carbamate (**1-3,** 80 mg, 0.18 mmol) in DMF (10 mL) at 0 °C under argon protection and stirred at 0°C for 20 mins. 6-(1-bromoethyl)-4-ethoxynicotinonitrile (**A3,** 70 mg, 0.27 mmol) was added and the reaction mixture was reacted at room temperature under stirring for 2 hours. The reaction liquid was poured into ice water (20 mL), extracted with dichloromethane (2 * 50 mL). The organic phase was combined, washed with saturated saline (100 mL), dried with anhydrous sodium sulfate and filtered. The filtrate was concentrated under reduced pressure to dryness. After column chromatography purification (dichloromethane: methanol=15:1), a yellow solid of tert-butyl (1-((2'-(1-(5-cyano-4-ethoxypyridine-2-yl)ethyl)-5)-((dimethylamino)methyl)-1'-one2', 3' -dihydro-1'H-spiro[cyclopropan-1,4'-isoquinoline]-7'-yl)methyl)-1H-imidazol-2-yl)(methyl)carbamate (**3-1,** 50 mg, yield:44.8 %) was obtained.

LCMS: m/z: 614.9 $[M+H]^+$.

Step 2: trifluoroacetic acid (1 mL) was added into a solution of tert-butyl (1-((2'-(1-(5-cyano-4-ethoxypyridine2-yl)ethyl)-5)-((dimethylamino)methyl)-1'-one2', 3' -dihydro-1'H-spiro[cyclopropan-1,4'-isoquinoline] -7'-yl)methyl)-1H-imidazol-2-yl)(methyl)carbamate (**3-1,** 45 mg, 0.073 mmol) in dichloromethane (5 mL) at 0 °C and reacted at room temperature under stirring for 2 hours. The reaction liquid was poured into ice water (20 mL), neutralized to pH 7 with a saturated aqueous solution of sodium bicarbonate, extracted with dichloromethane (100 mL). The organic phase was combined, washed with saturated saline (100 mL), dried with anhydrous sodium sulfate and filtered. The filtrate was concentrated under reduced pressure to dryness. After silica gel column chromatography (dichloromethane: methanol=10:1), a white solid was obtained. Then the white solid was separated through chiral column to obtain (S)-6-(1-(5'-(((dimethylamino)methyl)-7'-((2-(methylamino)-1H-imidazol-1-yl)methyl)-1'-one-1'H-spiro[cyclopropan-1,4'-isoquinoline] -2'(3'H)-yl)ethyl)-4-ethoxynicotinonitrile (Compound **3,** 10 mg, yield: 26.5 %) and (R)-6-(1-(5'-(((dimethylamino)methyl)-7'-((2-(methylamino)-1H-imidazol-1-yl)methyl)-1'-one-1'H-spiro[cyclopropan-1,4'-isoquinoline] -2'(3'H)-yl)ethyl)-4-ethoxynicotinonitrile (Compound **3',** 12 mg, yield: 32 %).

Compound **3:** (S)-6-(1-(5'-(((dimethylamino)methyl)-7'-((2-(methylamino)-1H-imidazol-1-yl)methyl)-1'-one-1'H-spiro[cyclopropan-1,4'-isoquinoline] -2'(3'H)-yl)ethyl)-4-ethoxynicotinonitrile

LCMS: m/z: 514.9 $[M+H]^+$.

$^1H$ NMR (400 MHz, DMSO-$d_6$): δ ppm 8.75 (s, 1H), 7.75 (s, 1H), 7.25 (s, 2H), 7.18 (s, 1H), 6.94 (s, 1H), 6.85 (s, 1H), 5.86-5.81 (m, 1H), 5.04 (s, 2H), 4.33-4.25 (m, 2 H), 3.33-3.30 (m, 2 H), 3.23-3.17(m, 1 H), 3.05-3.03 (m, 1 H), 2.81(d, $J$ = 4.4 Hz, 3H), 2.08 (s, 6H), 1.50(d, $J$ = 7.2 Hz, 3H), 1.43(t, $J$ = 6.8 Hz, 4H), 1.28-1.25 (m, 1 H), 0.89-0.82 (m, 1 H), 0.69-0.63 (m, 1 H).

Compound **3'**: (R)-6-(1-(5'-(((dimethylamino)methyl)-7'-((2-(methylamino)-1H-imidazol-1-yl)methyl)-1'-one-1'H-spiro[cyclopropan-1,4'-isoquinoline] -2'(3'H)-yl)ethyl)-4-ethoxynicotinonitrile

LCMS: m/z: 514.9 $[M+H]^+$.

**Example 4: Synthesis of Compound 6**

(S)-2'-(1-(4-ethoxy-5-fluoropyridine-2-yl)ethyl)-7'-((2-(methylamino)-1H-imidazol-1-yl)methyl)-5'-(pyrrol-1-ylmethyl)-2',3'-dihydro-1'H-spiro[cyclopropan-1,4'-isoquinoline]-1'-one

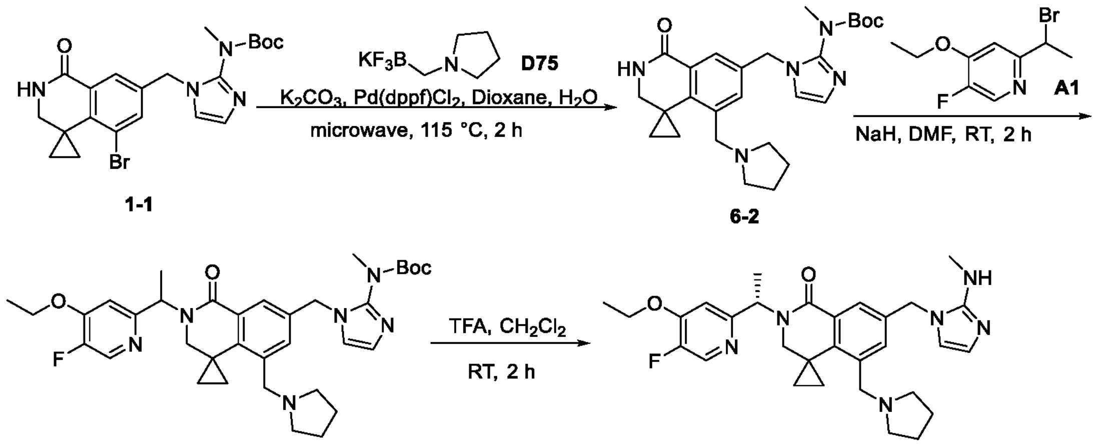


1-1

6-2

6-3

6

Step 1: 1,1-bis(diphenylphosphino) ferrocene palladium (II) chloride (7.3 mg, 0.01 mmol) and potassium carbonate (26.8 mg, 0.2 mmol) were added into a solution of tert-butyl (1-(((5'-bromo-1'-one-2',3'-dihydro-1'H-spiro[cyclopropan-1,4'-isoquinoline]-7'-yl)methyl]-1H-imidazol-2-yl)methyl)carbamate (**1-1,** 46 mg, 0.1 mmol) and potassium trifluoro[(pyrrolidine-1-yl)methyl]borate (**D75,** 38.4 mg, 0.2 mmol) in water (1 mL) and dioxane (4 mL). After the atmosphere was replaced with nitrogen, the reaction mixture was reacted in mocrowave at 115°C for 2 hours, diluted with ethyl acetate (100 mL) following by adding saturated saline. The water phase and the organic phase were separated. The water phase was extracted with ethyl acetate (150 mL). The organic phase was combined, washed with saturated saline, dried, filtered, and concentrated under reduced pressure to dryness. After column chromatography (dichloromethane: methanol=10:1) purification, a yellow solid of tert-butyl methyl(1-((1'-one-5'-(pyrrolidinyl-1-ylmethyl)-2', 3'-dihydro-1'H-spiro[cyclopropan-1,4'-isoquinoline]-7'-yl)methyl)-1H-imidazol-2-yl)carbamate (**6-2,** 12 mg, yield: 25.8 %) was obtained.

LCMS: m/z: 466.6 $[M+H]^+$.

Step 2: sodium hydride (60% dispersed in mineral oil, 10 mg, 0.204 mmol) was added into a solution of tert-butyl methyl(1-((1'-one-5'-(pyrrolidinyl-1-ylmethyl)-2', 3'-dihydro-1'H-spiro[cyclopropan-1,4'-isoquinoline]-7'-yl)methyl)-1H-imidazol-2-yl)carbamate (**6-2,** 30 mg, 0.064 mmol) in N,N-dimethylformamide (10 mL) at 0°C and stirred at 0 °C for 20 mins. 2-(1-bromoethyl)-4-ethoxy-5-fluoropyridine (**A1,** 25.3 mg, 0.103 mmol) was added and the reaction mixture was reacted at room temperature under stirring for 2 hours. The reaction liquid was poured into ice water (20 mL), extracted with dichloromethane (2 * 50 mL). The organic phase was combined, washed with saturated saline (100 mL), dried with anhydrous sodium sulfate and filtered. The filtrate was concentrated under reduced pressure to dryness. After column chromatography purification (dichloromethane: methanol=10:1), a yellow solid of tert-butyl (1-((2'-(1-(4-ethoxy-5-fluoropyridine-2-yl)ethyl)-1)-one5'-(pyrrolidine-1-ylmethyl)-2',3'-dihydro-1'H-spiro[cyclopropan-1,4'-isoquinoline]-7'-yl)methyl)-1H-imidazol-2-yl)(methyl)carbamate (**6-3**, 25 mg, yield:61.3%) was obtained.

LCMS: m/z: 633.9 $[M+H]^+$ .

Step 3: trifluoroacetic acid (1 mL) was added into a solution of tert-butyl (1-((2'-(1-(4-ethoxy-5-fluoropyridine-2-yl)ethyl)-1)-one5'-(pyrrolidine-1-ylmethyl)-2', 3'-dihydro-1'H-spiro[cyclopropan-1,4'-isoquinoline]-7'-yl)methyl)-1H-imidazol-2-yl)(methyl)carbamate (**6-3,** 20 mg, 0.0316 mmol) in dichloromethane (4 mL) at 0°C and reacted at room temperature under stirring for 2 hours. The reaction liquid was poured into ice water (20 mL), neutralized to pH 7 with a saturated aqueous solution of sodium bicarbonate and extracted with dichloromethane (2 * 50 mL). The organic phase was combined, washed with saturated saline (100 mL), dried with anhydrous sodium sulfate and filtered. The filtrate was concentrated under reduced pressure to dryness. After column chromatography (dichloromethane: methanol=10:1),

a white solid was obtained. The white solid was separated through chiral column to obtain Compound 6 (6 mg, yield:35 %).

LCMS: m/z: 533.3 $[M+H]^+$ .

$^1H$ NMR (400 MHz, methanol-$d_4$): δ ppm 8.23 (d, *J* = 3.6 Hz, 1H), 7.87 (s, 1H), 7.48 (s, 1H), 7.16 (d, *J* = 7.2 Hz, 1H), 6.95 (s, 2H), 5.95-5.90 (m, 1H), 5.12 (s, 2H), 4.60 (s, 1H), 4.25-4.16 (m, 2H), 4.03 (s, 2H), 3.42-3.34 (m, 1H), 3.06-3.03 (m, 1H), 2.99 (s, 3H), 2.86 (s, 3H), 1.91 (s, 4H), 1.56 (d, *J* = 7.2 Hz, 3H), 1.51-1.47 (m, 1H), 1.43 (t, *J* = 7.2 Hz, 3H), 1.09-0.99 (m, 2H), 0.61-0.56 (m, 1 H).

$^{19}F$ NMR (376 MHz, methanol-$d_4$): -153.93.

**Example 5: Synthesis of Compound 23 and 23'**

**Compound 23** :(S)-6-(1-(5'-((dimethylamino)methyl)-7'-((2-(methylamino)-1H-imidazol-1-yl)methyl)-1'-one-1'H-spiro[cyclobutan-1,4'-isoquinoline]-2'(3'H)-yl)ethyl)-4-ethoxynicotinonitrile

**Compound 23'** :(R)-6-(1-(5'-((dimethylamino)methyl)-7'-((2-(methylamino)-1H-imidazol-1-yl)methyl)-1'-one-1'H-spiro[cyclobutan-1,4'-isoquinoline]-2'(3'H)-yl)ethyl)-4-ethoxynicotinonitrile

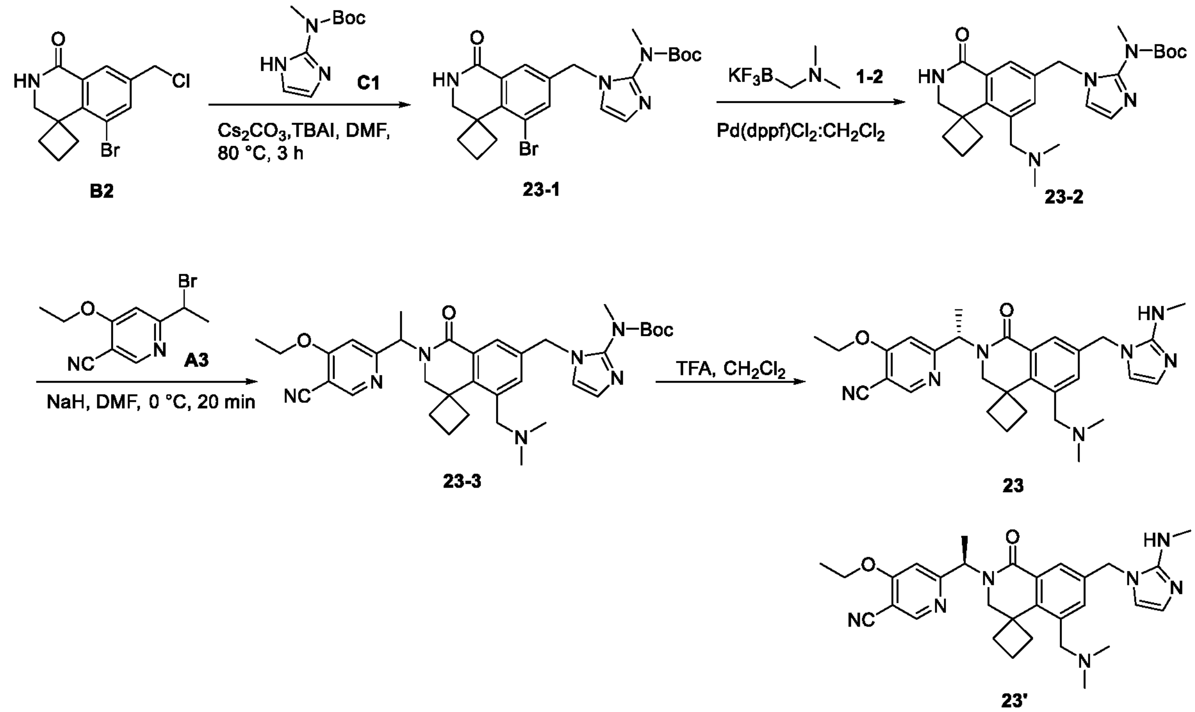


B2 23-1 23-2 23-3 23 23'

Step 1: cesium carbonate (445 mg, 1.369 mmol) and tetrabutylammonium iodide (253 mg, 0.685 mmol) were added into a solution of **B2** (215 mg, 0.675 mmol) in DMF (10 mL) at 0 °C and reacted at 0 °C for 20 mins, followed by adding a solution of tert-butyl (1H-imidazol-2-yl)(methyl)carbamate **C1** (270 mg, 1.369 mmol) in DMF(2 mL). The reaction mixture was reacted at 80 °C for 3 hours until the completion of reaction was monitored by LCMS, diluted with water (60 mL), and extracted with ethyl acetate (3 * 30 mL). The organic phase was combined, dried, filtered and concentrated. After column chromatography purification (dichloromethane: methanol=10:1), **23-1** (280 mg) was obtained.

LCMS: m/z: 477.1 [M+H]+.

Step 2: [1,1'-bis(diphenylphosphino)ferrocene]palladium (II) chloride dichloromethane complex (62 mg, 0.076 mmol) was added into a mixed solution of **23-1**(180 mg, 0.379 mmol) and potassium N,N-dimethyl-1-(trifluoro-14-boranyl)methylamine **1-2** (125 mg, 0.757 mmol), potassium carbonate (105 mg, 0.757 mmol) in dioxane (6 mL) and water (2 mL) under argon protection and reacted under microwave at 115 °C for 1.5 hours under argon protection until the completion of reaction was monitored by LCMS. The reaction liquid was diluted with water (30 mL), extracted with ethyl acetate (3 * 30 mL). The organic phase was combined, dried, filtered and concentrated. After column chromatography purification (dichloromethane: methanol=10:1), **23-2** (90 mg, yield: 52.3 %) was obtained.

LCMS: m/z: 454.3 [M+H]+.

Step 3: sodium hydride (60% dispersed in oil, 13 mg, 0.325 mmol) was added into a solution of **23-2** (33 mg,

0.072 mmol) in dry DMF (8 mL) at 0°C under nitrogen protection and stirred at 0°C for 30 mins, followed by adding dropwise a solution of **A3**(30 mg, 0.118 mmol) in DMF (2 mL). The reaction liquid was kept at 0°C and reacted for 20 mins. Then the reaction liquid was poured into a saturated aqueous solution of ammonium chloride and extracted with ethyl acetate (3 * 30 mL). The organic phase was combined, washed with water (3 * 50 mL) and saturated saline (2 * 50 mL) in sequence, dried with anhydrous sodium sulfate, filtered and concentrated to dryness. The residue was purified through column chromatography (dichloromethane/ methanol=30/1), **23-3** (25 mg, yield: 54.7%) was obtained.
LCMS: m/z 628.3 $[M+H]^+$

Step 4: trifluoroacetic acid (2.5 mL) was added dropwise into a solution of **23-3** (25 mg, 0.040 mmol) in dichloromethane (10 mL) and reacted at room temperature for 1 hour. The reaction liquid was concentrated to remove trifluoroacetic acid. The residue was diluted with dichloromethane (50 mL). The organic phase was washed with saturated sodium bicarbonate (50 mL) and saturated saline (50 mL) in sequence, dried with anhydrous sodium sulfate, filtered and concentrated to dryness. The residue was separated and purified through high performance liquid chromatography (SunFire Prep C18 OBD 10um, acetonitrile/water system) to obtain a racemic product, which was purified through SFC (column model: DAICELCHIRALCEL®AD; Mobile phase: redistilled grade EtOH and Supercritical $CO_2$; wavelength:214 nm; system: Waters SFC 150) to obtain a chiral Compound **23** (9 mg, yield:42.9%) and 23' (8.8 mg, 40%).
LCMS: m/z 528.2 $[M+H]^+$

$^1$H NMR (400MHZ- methanol-d4) δ: 8.66 (s, 1H), 7.78 (d, *J* = 1.6 Hz, 1H), 7.46 (d, *J* = 2.0 Hz, 1H), 7.22 (s, 1H), 6.97 (d, *J* = 2.4 Hz, 1H), 6.91 (d, *J* = 2.4 Hz, 1H), 5.98~5.93 (m, 1H), 5.08 (s, 2H), 4.35~4.27 (m, 2H), 3.87~3.79 (m, 2H), 3.74~3.62 (m, 2H), 2.98 (s, 3H), 2.87~2.84 (m, 1H), 2.54~2.51 (m, 1H), 2.31 (s, 6H), 2.01~2.00 (m, 3H), 1.70 (d, *J*=6.4 Hz, 3H), 1.61~1.57 (m, 1H), 1.49 (t, *J*=7.2 Hz, 3H).

The following compounds were synthesized by the same method or modified method using the corresponding intermediates with reference to the synthesis method for Compound 23 of Example 5.

The following Examples refer to free compounds or pharmaceutically acceptable salts thereof.

| No. | Starting intermediate and material, reference step | Compound structure | Compound name | Data analysis |
|---|---|---|---|---|
| Compound **18** | **23-2, A1** with reference to Step 3 to Step 4 of Example 5 | 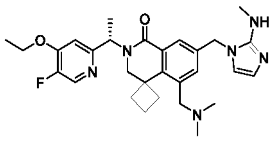 | (S)-5'-((dimethylamino) methyl)-2'-(1-(4-ethoxy-5-fluoropyridine-2-yl)ethyl)-7'-((2-(methylamino)-1H-imidazol-l-yl)methyl)-2', 3'-dihydro-1'H-spiro [cyclobutan-1,4'-isoquinoline]-1'-one | LCMS: m/z: 521.3 [M+H] +. $^1$H NMR (400 MHz, methanol-$d_4$): δ ppm 8.30 (d, *J* = 2.8 Hz, 1H), 7.94 (d, *J* = 1.6 Hz, 1H), 7.63 (d, *J* = 1.6 Hz, 1H), 7.20 (d, *J* = 6.8 Hz, 1H), 6.99-6.94 (m, 2H), 5.97 (t, *J* = 7.2 Hz, 1H), 5.15 (s, 2H), 4.84-4.66 (m, 2H), 4.26-4.16 (m, 2H), 3.74-3.63 (m, 2H), 3.00 (s, 3H), 2.94 (s, 6H), 2.62-2.54 (m, 1H), 2.21-1.98 (m, 4H), 1.67-1.61 (m, 4H), 1.43 (s, 3H). |

(continued)

| No. | Starting intermediate and material, reference step | Compound structure | Compound name | Data analysis |
|---|---|---|---|---|
| Compound **19** | **B3, 1-2, C1, A1** with reference to Step 1 to Step 4 of Example 5 | 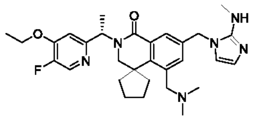 | (S)-5'-((dimethylamino) methyl)-2'-(1-(4-ethoxy-5-fluoropyridine-2-yl)ethyl)-7'-((2-(methylamino)-1H-imidazol-l-yl)methyl)-2', 3'-dihydro-1'H-spiro [cyclopentan-1,4'-isoquinoline]-1'-one | LCMS: m/z: 535.2 $[M+H]^+$ . 1H NMR (400 MHz, methanol-d4): δ 8.45 (brs, 1H), 8.26 (d, *J* = 3.2 Hz, 1H), 7.85 (s, 1H), 7.70 (s, 1H), 7.16 (d, *J* = 6.8 Hz, 1H), 6.97-6.92 (m, 2H), 5.98-5.93 (m, 1H), 5.10 (s, 2H), 4.27-4.14 (m, 2H), 3.88-3.66 (m, 2H), 3.27-3.19 (m, 2H), 2.99 (s, 3H), 2.35 (s, 6H), 2.19-2.12 (m, 1H), 1.94-1.81 (m, 2H), 1.76-1.64 (m, 3H), 1.59 (d, *J* = 7.2 Hz, 3H), 1.43 (t, *J* = 6.8 Hz, 3H), 1.37-1.33 (m, 2H). |
| Compound **122** | **B3, 1-2, C1, A3** with reference to Step 1 to Step 4 of Example 5 | 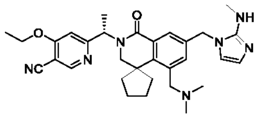 | (S)-6-(1-(5'-((dimethylamino) methyl)-7'-((2-(methylamino)-1H-imidazol-1-yl)methyl)-1'-oxo-l'H-spiro [cyclopentan-1,4'-isoquinoline]-2'(3'H)-yl) ethyl)-4-ethoxynicotinonitrile | LCMS: m/z: 542.3 $[M+H]^+$ . |
| Compound **d47** | **23-1, A3, D66** with reference to Step 2 to Step 4 of Example 5 | 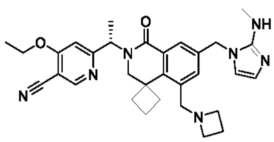 | (S)-6-(1-(5'-(azetidinyl-1-ylmethyl)-7'-((2-(methylamino)-1H-imidazol-1-yl)methyl)-1'-one-1'H-spiro[cyclobutan-1,4'-isoquinoline]-2'(3'H)-yl) ethyl)-4-ethoxynicotinonitrile | LCMS: m/z: 540.3 $[M+H]^+$ . |
| Compound **48** | **23-1, A3, D75** with reference to Step 2 to Step 4 of Example 5 | 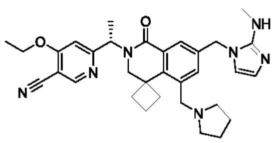 | (S)-4-ethoxy-6-(l-(7'-((2-(methylamino)-1H-imidazol-1-yl)methyl)-1'-one-5'-(pyrrolidin-l-ylmethyl)-l'H-spiro [cyclobutan-1,4'-isoquinoline]-2'(3'H)-yl) ethyl)nicotinonitrile | LCMS: m/z: 554.3 $[M+H]^+$ . |

(continued)

| No. | Starting intermediate and material, reference step | Compound structure | Compound name | Data analysis |
|---|---|---|---|---|
| Compound **49** | **23-1, A3, D69** with reference to Step 2 to Step 4 of Example 5 | 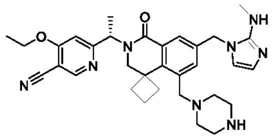 | (S)-4-ethoxy-6-(l-(7'-((2-(methylamino)-1H-imidazol-1-yl)methyl)-1'-one-5'-(piperazin-1-ylmethyl)-l'H-spiro [cyclobutan-1,4'-isoquinoline]-2'(3'H)-yl) ethyl)nicotinonitrile | LCMS: m/z: 569.3 $[M+H]^+$ . $^1H$ NMR (400 MHz, methanol-d4) δ ppm: 8.67 (s, 1H), 7.80 (d, *J* = 1.2 Hz, 1H), 7.40 (s, 1H), 7.22 (s, 1H), 6.97 - 6.84 (m, 2H), 5.99~5.94 (m, 1H), 5.06 (s, 2H), 4.36~4.26 (m, 2H), 3.95~3.62 (m, 4H), 3.18~3.16 (m, 4H), 2.96 (s, 3H), 2.89~2.86 (m, 1H), 2.67~2.65 (m, 4H), 2.54~2.46 (m, 1H), 2.04~2.01 (m, 3H), 1.70 (d, *J* = 7.2 Hz, 3H), 1.57~1.53 (m, 1H), 1.47 (t, *J* = 6.8 Hz, 3H). |
| Compound **72** | **23-1, A3, D59** with reference to Step 2 to Step 4 of Example 5 | 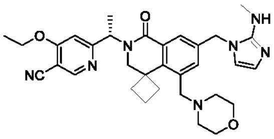 | (S)-4-ethoxy-6-(1-(7'-((2-(methylamino)-1H-imidazol-l-yl)methyl)-5'-(morpholinylmethyl)-1'-one-l'H-spiro[cyclobutyl-1,4'-isoquinoline]-2'(3'H)-yl) ethyl)nicotinonitrile | LCMS: m/z: 570.7 $[M+H]^+$ . $^1H$ NMR (400 MHz, methanol-d4): δ ppm 8.67(s, 1H), 7.78 (d, *J* = 2.4 Hz, 1H), 7.34 (d, *J* = 2.0 Hz, 1H), 7.22 (s, 1H), 6.82 (d, *J* = 2.0 Hz, 1H), 6.79 (d, *J* = 2.0 Hz, 1H), 5.99~5.94 (m, 1H), 5.01 (s, 2H), 4.35~4.26 (m, 2H), 3.85~3.61 (m, 8H), 2.92(s, 3H), 2.59~2.57 (m, 4H), 2.41 (s, 4H), 2.02~1.97 (m, 3H), 1.70 (d, *J* = 7.2 Hz, 3H), 1.54~1.51 (m, 4H), 1.46 (t, *J* = 7.2 Hz, 3H). |

(continued)

| No. | Starting intermediate and material, reference step | Compound structure | Compound name | Data analysis |
|---|---|---|---|---|
| Compound **73** | **23-1, A3, D78** with reference to Step 2 to Step 4 of Example 5 | 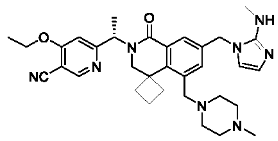 | (S)-4-ethoxy-6-(l-(7'-((2-(methylamino)-1H-imidazol-l-yl)methyl)-5'-((4-methylpiperazin-1-yl) methyl)-l'-one-l'H-spiro [cyclobutan-1,4'-isoquinoline]-2'(3'H)-yl) ethyl)nicotinonitrile | LCMS: m/z: 583.8 $[M+H]^+$. $^1H$ NMR (400 MHz, methanol-d4): δ ppm 8.67 (s, 1H), 8.40 (br, 1H), 7.78 (s, 1H), 7.39 (s, 1H), 7.22 (s, 1H), 6.94 (s, 1H), 6.89 (s, 1H), 5.99~5.94 (m, 1H), 5.06 (s, 2H), 4.36~4.26 (m, 2H), 3.91 (d, *J* = 12.4 Hz, 1H), 3.79~3.74 (m, 2H), 3.62 (d, *J* = 12.4 Hz, 1H), 2.98 -2.89 (m, 8H), 2.64~2.50 (m, 8H), 2.03~1.99 (m, 3H), 1.69 (d, *J* = 7.2 Hz, 3H), 1.54~1.50 (m, 1H), 1.47 (t, *J* =6.8 Hz, 3H). |
| Compound **74** | **23-1, A3, D72** with reference to Step 2 to Step 4 of Example 5 | 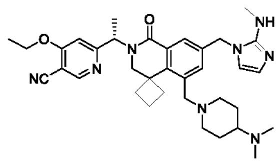 | (S)-6-(1-(5'-((4-(dimethylamino) pyridin-1-yl)methyl)-7'-((2-(methylamino)-1H-imidazol-1-yl)methyl)-1'-one- 1'H-spiro [cyclobutyl-1,4'-isoquinoline]-2'(3'H)-yl) ethyl)-4-ethoxynicotinonitrile | LCMS: m/z: 611.2 $[M+H]^+$ $^1H$ NMR (400 MHz, methanol-d4) δ ppm: 8.67 (s, 1H), 8.46 (s, 2H), 7.78 (s, 1H), 7.38 (s, 1H), 7.22 (s, 1H), 6.90 (d, *J* = 24.2 Hz, 2H), 6.00~5.95 (m, 1H), 5.06 (s,2H), 4.36 - 4.22 (m, 2H), 3.88 - 3.56 (m, 4H), 3.12~3.10 (m, 1H), 2.98~2.89 (m, 6H), 2.81 (s,6H), 2.56~2.47 (m, 1H), 2.15 - 1.98 (m, 7H), 1.70~1.58 (m, 5H), 1.51~1.45 (m, 4H). |

(continued)

| No. | Starting intermediate and material, reference step | Compound structure | Compound name | Data analysis |
|---|---|---|---|---|
| Compoun d **75** | **23-1, A3, D71** with reference to Step 2 to Step 4 of Example 5 | 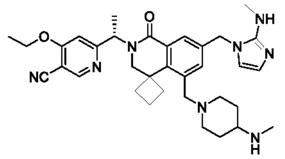 | (S)-4-ethoxy-6-(l-(7'-((2-(methylamino)-1H-imidazol-l-yl)methyl)-5'-((4-(methylamino) piperidin-1-yl)methyl)-1'-one-1'H-spiro[cyclobutan-1,4'-isoquinoline]-2'(3'H)-yl) ethyl)nicotinonitrile | LCMS: m/z: 597.3 $[M+H]^+$ . $^1H$ NMR (400 MHz, methanol-d4): δ ppm 8.67 (s, 1H), 8.49 (br, 2H), 7.78 (s, 1H), 7.36 (s, 1H), 7.22 (s, 1H), 6.91 (s, 1H), 6.86 (s, 1H), 6.00~5.97 (m, 1H), 5.06 (s, 2H), 4.36~4.26 (m, 2H), 3.86 (d, *J* =13.2 Hz, 1H), 3.73 (t, *J* =13.6 Hz, 2H), 3.62 (d, *J* =13.2 Hz, 1H), 3.03~3.28 (m, 7H), 2.67 (s, 3H), 2.55~2.52 (m, 1H), 2.15~1.98 (m, 7H), 1.69 (d, *J* = 7.2 Hz, 3H), 1.59~1.52 (m, 3H), 1.47 (t, *J* =6.8 Hz, 3H). |
| Compoun d 77 | **23-2, A13,** with reference to Step 2 to Step 4 of Example 5 | 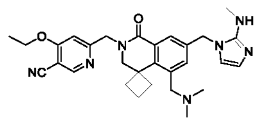 | 6-((5'-((dimethylamino) methyl)-7'-((2-(methylamino)-1H-imidazol-1-yl)methyl)-1'-one-l'H-spiro[cyclobutan-1,4'-isoquinoline]-2'(3'H)-yl) methyl)-4-ethoxynicotinonitrile | LCMS: m/z: 514.3 $[M+H]^+$. $^1H$ NMR (400 MHz, methanol-d4): δ ppm 8.62 (s, 1H), 8.44 (br, 2H), 7.79 (s, 1H), 7.47 (s, 1H), 7.22 (s, 1H), 6.95 (d, *J* =2.8 Hz, 1H), 6.89 (d, *J* =2.8 Hz, 1H), 5.07 (s, 2H), 4.89 (s, 2H), 4.32~4.27 (m, 2H), 3.81~3.61 (m, 4H), 2.97 (s, 3H), 2.80~2.73 (m, 2H), 2.33 (s, 6H), 2.14~1.96 (m, 4H), 1.47 (t, *J* =6.8 Hz, 3H). |

(continued)

| No. | Starting intermediate and material, reference step | Compound structure | Compound name | Data analysis |
|---|---|---|---|---|
| Compound **78** | **23-1, A1, D67** with reference to Step 2 to Step 4 of Example 5 | 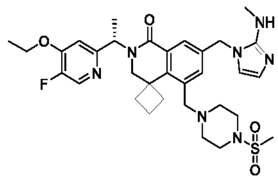 | (S)-2'-(l-(4-ethoxy-5-fluoropyridine-2-yl)ethyl)-7'-((2-(methylamino)-1H-imidazol-1-yl)methyl)-5'-((4-(methylsulfonyl) piperazin -1-yl)methyl)-2',3'-dihydro-1'H-spiro [cyclobutan-1,4'-isoquinoline]-1'-one | LCMS: m/z 640.2 $[M+H]^+$ $^1$H NMR (400 MHz, methanol-d4) δ ppm: 8.76 (d, *J* = 4.8 Hz, 1H), 8.09 (d, *J* = 1.2 Hz, 1H), 7.91 (d, *J* = 1.2 Hz, 1H), 7.76 (d, J = 7.2 Hz, 1H), 7.06 (d, *J* = 2.4 Hz, 1H), 6.99 (d, *J* = 2.4 Hz, 1H), 5.84 (q, *J* = 7.2 Hz, 1H), 5.22 (s, 2H), 4.97~4.85 (m, 2H), 4.63 ~ 4.57 (m, 2H), 3.98~3.44 (m, 10H), 3.00 (s, 3H), 2.97 (s, 3H), 2.61 - 2.12 (m, 6H), 1.90 (d, *J* = 7.2 Hz, 3H), 1.55 (t, *J* = 6.8 Hz, 3H). |
| Compound **79** | **23-1, A3, D61** with reference to Step 2 to Step 4 of Example 5 | 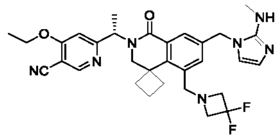 | (S)-6-(1-(5'-((3,3-difluoroazetidinyl-1-yl) methyl)-7'-((2-(methylamino)-1H-imidazol-1-yl)methyl)-1'-one-1'H-spiro [cyclobutan-1,4'-isoquinoline]-2'(3'H)-yl) ethyl)-4-ethoxy nicotinonitrile | LCMS: m/z: 576.5 $[M+H]^+$ . $^1$H NMR (400 MHz, methanol-d4): δ ppm 8.67 (s, 1H), 8.53 (br, 1H), 7.79 (d, *J* = 1.6 Hz, 1H), 7.37 (d, *J* = 2.0 Hz, 1H), 7.21 (s, 1H), 6.81~6.77 (m, 2H), 5.98~5.93 (m, 1H), 5.02 (s, 2H), 4.35~4.26 (m, 2H), 4.10~4.03 (m, 2H), 3.73~3.56 (m, 6H), 2.93 (s, 3H), 2.89~2.85 (m, 1H), 2.55~2.50 (m, 1H), 2.03~2.01 (m, 3H), 1.68 (d, *J* = 7.2 Hz, 3H), 1.60~1.56 (m, 1H), 1.46 (t, *J* =7.2 Hz, 3H). |

(continued)

| No. | Starting intermediate and material, reference step | Compound structure | Compound name | Data analysis |
|---|---|---|---|---|
| Compound **80** | **23-1, A3, D67** with reference to Step 2 to Step 4 of Example 5 | 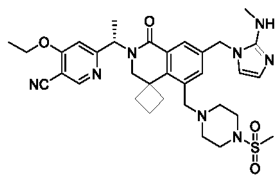 | (S)-4-ethoxy-6-(l-(7'-((2-(methylamino)-1H-imidazol-1-y l)methy l)-5 '-((4-(methylsulfonyl) piperazin -l-yl)methyl)-l'-one-l'H-spiro[cyclobutan-1,4'-isoquinoline]-2'(3'H)-yl) ethyl)nicotinonitrile | LCMS: m/z 647.3 [M+H]+ $^1$H NMR (400 MHz, methanol-d4) δ ppm: 8.90 (s, 0.3H), 8.87 (s, 0.7 H), 8.07 (s, 1H), 7.90 (s, 1H), 7.68 (s, 0.3H), 7.45 (s, 0.7H), 7.03 - 6.95 (m, 2H), 5.88~5.79 (m, 1H), 5.18 (s, 2H), 4.90~4.83 (m, 2H), 4.68- 4.43 (m, 2H), 3.98 - 3.43 (m, 10H), 3.00 - 2.93 (m, 6H), 2.63 - 1.96 (m, 6H), 1.92 (d, *J* = 7.2 Hz, 1H), 1.80 (d, *J* = 7.2 Hz, 2H), 1.59 (t, *J* = 7.2 Hz, 1H), 1.52 (t, *J* = 7.2 Hz, 2H). |
| Compound **81** | **23-1, A3, D70** with reference to Step 2 to Step 4 of Example 5 | 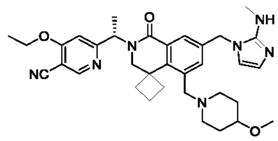 | (S)-4-ethoxy-6-(l-(5'-((4-methoxypyridin-1-yl) methyl)-7'-((2-(methylamino)-1H-imidazol-1-yl)methyl)-1'-one-l'H-spiro[cyclobutan-1,4'-isoquinoline]-2'(3'H)-yl) ethyl)nicotinonitrile | LCMS: m/z: 598.4 [M+H]+ $^1$H NMR (400 MHz, methanol-d4) δ ppm: 8.67 (s, 1H), 8.52 (s, 1H), 7.78 (d, *J* = 2.0 Hz, 1H), 7.34 (s, 1H), 7.21 (s, 1H), 6.82 (s, 1H), 6.79 (s, 1H), 5.97 (q, *J* = 7.2 Hz, 1H), 5.01 (s, 2H), 4.35 - 4.26 (m, 2H), 3.79 - 3.60 (m, 4H), 3.33 (s, 3H), 3.28~3.23 (m, 1H), 2.96~2.90 (m, 4H), 2.68 - 2.54 (m, 3H), 2.14 (t, *J* = 10.0 Hz, 2H), 2.01~1.87 (m, 5H), 1.70 (d, J = 7.2 Hz, 3H), 1.54 - 1.44 (m, 6H). |

(continued)

| No. | Starting intermediate and material, reference step | Compound structure | Compound name | Data analysis |
|---|---|---|---|---|
| Compound **82** | **23-1, A13, D61** with reference to Step 2 to Step 4 of Example 5 | 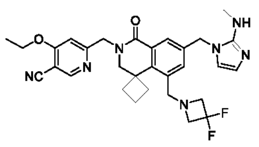 | 6-((5'-((3,3-difluoroazetidinyl-1-yl) methyl)-7'-((2-(methylamino)-1H-imidazol-1-yl)methyl)-1'-one-l'H-spiro[cyclobutan-1,4'-isoquinoline]-2'(3'H)-yl) methyl)-4-ethoxynicotinonitrile | LCMS: m/z: 562.3 $[M+H]^+$ . $^1H$ NMR (400 MHz, methanol-d4): δ ppm 8.62 (s, 1H), 8.53 (br, 1H), 7.81 (d, *J* = 2.0 Hz, 1H), 7.37 (d, *J* = 2.0 Hz, 1H), 7.21 (s, 1H), 6.76~6.75 (m, 2H), 5.01 (s, 2H), 4.88 (s, 2H), 4.32~4.27 (m, 2H), 4.09 (s, 2H), 3.80 (s, 2H), 3.60 (t, *J* = 12.0 Hz, 4H), 2.91 (s, 3H), 2.81~2.73 (m, 2H), 2.13~2.07 (m, 2H), 2.01~1.96 (m, 2H), 1.46 (t, *J* = 7.2 Hz, 3H). |
| Compound **83** | **23-1, A1, D78** with reference to Step 2 to Step 4 of Example 5 | 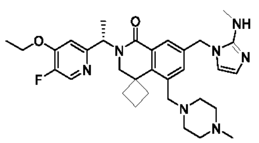 | (S)-2'-(l-(4-ethoxy-5-fluoropyridine-2-yl)ethyl)-7'-((2-(methylamino)-1H-imidazol-1-yl)methyl)-5'-((4-methylpiperazin-1-yl) methyl)-2',3'-dihydro-1'H-spiro [cyclobutan-1,4'-isoquinoline]-1'-one | LCMS: m/z: 576.5 $[M+H]^+$ . $^1H$ NMR (400 MHz, methanol-d4): δ ppm 8.47 (br, 2H), 8.29 (d, *J* = 3.2 Hz, 1H), 7.80 (d, *J* = 1.6 Hz, 1H), 7.38 (s, 1H), 7.19 (d, *J* = 6.8 Hz, 1H), 6.94 (d, *J* = 2.0 Hz, 1H), 6.89 (d, *J* = 2.0 Hz, 1H), 6.01~5.97 (m, 1H), 5.06 (s, 2H), 4.25~4.13 (m, 2H), 3.95~3.89 (m, 1H), 3.76~3.54 (m, 3H), 2.98 (s, 3H), 2.94~2.87 (m, 5H), 2.63 (s, 6H), 2.63~2.61 (m, 1H), 2.45~2.37 (m, 1H), 1.96~1.91 (m, 3H), 1.65 (d, *J* = 6.8 Hz, 3H), 1.45~1.33 (m, 4H). |

(continued)

| No. | Starting intermediate and material, reference step | Compound structure | Compound name | Data analysis |
|---|---|---|---|---|
| Compound **84** | **23-1, A1, D74** with reference to Step 2 to Step 4 of Example 5 | 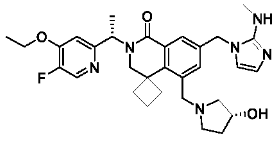 | 2'-((S)-l-(4-ethoxy-5-fluoropyridine-2-yl)ethyl)-5'-(((R)-3hydroxylpyrrolidin-l-yl)methyl)-7'-((2-(methylamino)-1H-imidazol-1-yl)methyl)-2',3'-dihydro-1'H-spiro [cyclobutyl-1,4'-isoquinoline]-1'-one | $^1$H NMR (400 MHz, methanol-d4): δ ppm 8.28 (d, *J* = 3.2 Hz, 1H), 7.81 (d, *J* = 3.2 Hz, 1H), 7.29 (s, 1H), 7.17 (d, *J* = 7.2 Hz, 1H), 6.61 (d, *J* = 8.0 Hz, 2H), 6.00~5.98 (m, 1H), 4.94 (s, 2H), 4.31~4.24 (m, 1H), 4.21~4.17 (m, 2H), 3.91~3.78 (m, 2H), 3.62~3.51 (m, 2H), 2.98~2.96 (m, 1H), 2.85 (s, 3H), 2.76~2.63 (m, 2H), 2.48~2.39 (m, 3H), 2.11~2.08 (m, 1H), 1.93~1.89 (m, 3H), 1.65~1.63 (m, 4H), 1.44~1.28 (m, 4H). |
| Compound **85** | **23-1, A1, D73** with reference to Step 2 to Step 4 of Example 5 | 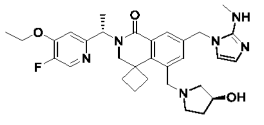 | 2'-((S)-l-(4-ethoxy-5-fluoropyridine-2-yl)ethyl)-5'-(((S)-3-hydroxylpyrrolidin-1-yl)methyl)-7'-((2-(methylamino)-1H-imidazol-1-yl)methyl)-2',3'-dihydro-1'H-spiro [cyclobutan-1,4'-isoquinoline]-1'-one | LCMS: m/z: 563.4 [M+H] +. $^1$H NMR (400 MHz, methanol-d4): δ ppm 8.28 (d, *J* = 3.2 Hz, 1H), 7.8 (d, *J* = 3.2 Hz, 1H), 7.29 (s, 1H), 7.16 (d, *J* = 6.8 Hz, 1H), 6.61 (m, 2H), 6.00~5.98 (m, 1H), 4.93 (s, 2H), 4.30 (s, 1H), 4.22~4.16 (m, 2H), 3.91~3.78 (m, 2H), 3.62~3.51 (m, 2H), 3.01~2.95 (m, 1H), 2.84 (s, 3H), 2.73~2.65 (m, 2H), 2.48~2.39 (m, 3H), 2.11~2.07 (m, 1H), 1.93~1.88 (m, 3H), 1.67~1.63 (m, 4H), 1.44~1.29 (m, 4H). |

(continued)

| No. | Starting intermediate and material, reference step | Compound structure | Compound name | Data analysis |
|---|---|---|---|---|
| Compound**86** | **23-1, A1, D68** with reference to Step 2 to Step 4 of Example 5 | 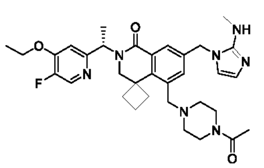 | (S)-5'-((4-acetylpiperazin-l-yl)methyl)-2'-(l-(4-ethoxy-5-fluoropyridine-2-yl)ethyl)-7'-((2-(methylamino)-1H-imidazol-1-yl)methyl)-2',3'-dihydro-1'H-spiro [cyclobutyl-1,4'-isoquinoline]-1'-one | LCMS: m/z: 604.4 $[M+H]^+$ . $^1$H NMR (400 MHz, methanol-d4): δ ppm 8.28 (d, *J* = 3.2 Hz, 1H),7.80 (d, *J* = 2.0 Hz, 1H), 7.37 (d, *J* = 2.0 Hz, 1H), 7.17 (d, *J* = 6.8Hz, 1H), 6.90 (d, *J* = 2.4 Hz, 1H), 6.85 (d, *J* = 2.4 Hz, 1H), 6.02~5.97 (m, 1H), 5.05 (s, 2H), 4.25~4.15 (m, 2H), 3.85 (d, *J* = 12.4 Hz, 1H), 3.73~3.65 (m, 2H), 3.57~3.49 (m, 5H), 3.00~2.93 (m, 4H), 2.48~2.36 (m, 5H), 2.08 (s, 3H), 1.97~1.89 (m, 3H), 1.65 (d, *J* = 7.2 Hz, 3H), 1.41 (t, *J* = 7.2 Hz, 4H). |
| Compound**87** | **23-1, A8, D78** with reference to Step 2 to Step 4 of Example 5 | 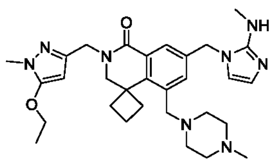 | 2'-((5-ethoxy-1-methyl-1H-pyrazole-3-yl)methyl)-7'-((2-(methylamino)-1H-imidazol-1-yl)methyl)-5'-((4-methylpiperazin-1-yl) methyl)-2',3'-dihydro-1'H-spiro[cyclobutan-1,4'-isoquinoline]-1'-one | LCMS: m/z: 547.2 [M+H]+ $^1$H NMR (400 MHz, methanol-d4) δ ppm: 7.82 (d, *J* = 2.0 Hz, 1H), 7.25 (d, J = 2.0 Hz, 1H), 6.62~6.59 (m, 2H), 5.63 (s, 1H), 4.93 (s, 2H), 4.61 (s, 2H), 4.10 (q, *J* = 7.2 Hz, 2H), 3.74 (s, 2H), 3.63 (s, 2H), 3.59 (s, 3H), 2.85 (s, 3H), 2.78~2.70 (m, 2H), 2.45~2.27 (m, 11H), 2.01~1.94 (m, 2H), 1.81~1.75 (m, 2H), 1.38 (t, *J* = 7.2 Hz, 3H). |

(continued)

| No. | Starting intermediate and material, reference step | Compound structure | Compound name | Data analysis |
|---|---|---|---|---|
| Compound **102** | **23-1, A3, D77** with reference to Step 2 to Step 4 of Example 5 | 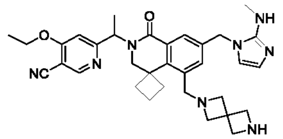 | 6-(1-(5'-((2,6-diazaspiro[3.3] heptan-2-yl)methyl)-7'-((2-(methylamino)-1H-imidazol-1-yl)methyl)-1'-oxo-l'H-spiro [cyclobutan-1,4'-isoquinoline]-2'(3'H-yl) ethyl)-4-ethoxy nicotinonitrile | LCMS: m/z: 581.2 $[M+H]^+$ , $^1$H NMR (400 MHz, methanol-d4) δ ppm: 8.66 (s, 2H), 7.78 (s, 1H),7.32 (s, 1H), 7.21 (s, 1H), 6.89 (s, 1H), 6.84 (s, 1H), 5.95 (q, *J* = 6.8 Hz, 1H), 5.05 (s, 2H), 4.37~4.24 (m, 2H), 4.16 (s, 4H), 3.95 ~3.85 (m, 2H), 3.73~3.59 (m, 2H), 3.39 (s, 4H), 2.97 (s, 3H), 2.83~2.77 (m, 1H), 2.53~2.46 (m, 1H), 2.03~1.97 (m, 3H), 1.70 (d, *J* =7.2 Hz, 3H), 1.59~1.53 (m, 1H), 1.47 (t, *J* = 7.2 Hz, 3H). |
| Compound **107** | **23-2, A6** with reference to Step 3 to Step 4 of Example 5 | 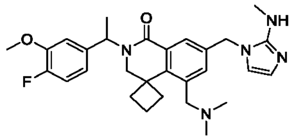 | 5'-((dimethylamino)methyl)-2'-(1-(4-fluoro-3-methoxyphenyl)ethyl)-7'-((2-(methylamino)-1H-imidazol-l-yl)methyl)-2',3'-dihydro-1'H-spiro [cyclobutan-1,4'-isoquinoline]-1'-one | LCMS: m/z: 506.3 $[M+H]^+$ . $^1$H NMR (400 MHz, methanol-d4) δ ppm: 7.85 (d, *J* = 2.0 Hz, 1H), 7.25 (d, *J* = 2.0 Hz, 1H), 7.14~7.03 (m, 3H), 6.61~6.58 (m, 2H), 6.08~6.03 (m, 1H), 4.94 (s, 2H), 3.84 (s, 3H), 3.63 (s, 2H), 3.45~3.42 (m, 1H), 3.30~3.28 (m, 1H), 2.98~2.91 (m, 1H), 2.84 (s, 3H), 2.42~2.34 (m, 1H), 2.19 (s, 6H), 1.93~1.84 (m, H), 1.79~1.72 (m, 1H), 1.63 (d, *J* = 7.2 Hz, 3H), 1.44~1.38 (m, 1H). |

(continued)

| No. | Starting intermediate and material, reference step | Compound structure | Compound name | Data analysis |
|---|---|---|---|---|
| Compound **113** | **23-2, A15** with reference to Step 3 to Step 4 of Example 5 | 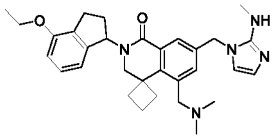 | 5'-((dimethylamino)methyl)-2'-(4-ethoxy-2,3-dihydro-1H-indene-1-yl)-7'-((2-(methylamino)-1H-imidazol-l-yl)methyl)-2',3'-dihydro-1'H-spiro [cyclobutan-1,4'-isoquinoline]-1'-one | LCMS: m/z: 514.3 [M+H]$^+$. $^1$H NMR (400 MHz, methanol-d4) δ ppm: 8.34 (s, 1H), 7.89 (s, 1H), 7.55 (s, 1H), 7.20 (t, *J* = 8.0 Hz, 1H), 6.99 (dd, *J* =2.8 Hz, 13.6 Hz, 2H), 6.87~6.79 (m, 2H), 6.32~6.28 (m, 1H), 5.12 (s, 2H), 4.13~3.99 (m, 4H), 3.40~3.36 (m, 1H), 3.22~3.19 (m, 1H), 3.14~3.06 (m, 1H), 3.01 (s, 3H), 2.94~2.86 (m, 1H), 2.77~2.70 (m, 1H), 2.56~2.44 (m, 8H), 2.15~2.10 (m, 1H), 2.01~1.89 (m, 3H), 1.85~1.75 (m, 1H), 1.42 (t, *J* = 7.2 Hz, 3H). |
| Compound **115** | **23-2, A19** with reference to Step 3 to Step 4 of Example 5 | 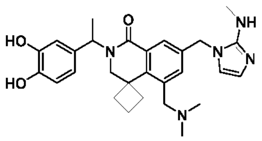 | 2'-(1-(3,4-dihydroxylphenyl) ethyl)-5'-((dimethylamino) methyl)-7'-((2-(methylamino)-1H-imidazol-l-yl)methyl)-2' ,3'-dihydro-1'H-spiro [cyclobutan-1,4'-isoquinoline]-1'-one | LCMS: m/z: 490.3 [M+H]$^+$ . $^1$H NMR (400 MHz, methanol-$d_4$): δ ppm 8.53 (brs, 1H), 7.81 (s, 1H), 7.43 (s, 1H), 6.96~6.78 (m, 5H), 5.98~5.93 (m, 1H), 5.08 (s, 2H), 3.76 (s, 2H), 3.40~3.37 (m, 2H), 2.99 (s, 3H), 2.91~2.84 (m, 1H), 2.35~2.17 (m, 8H), 1.90~1.73 (m, 3H), 1.56 (d, *J* =7.2 Hz, 3H). |
| Compound **119** | **23-2, A16** with reference to Step 3 to Step 4 of Example 5 | 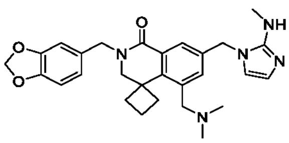 | 2'-(benzo[d][1,3]dioxolan-5-ylmethyl)-5'-((dimethylamino)methyl)-7'-((2-(methylamino)- 1H-imidazol-l-yl)methyl)-2',3'-dihydro-1'H-spiro [cyclobutan-1,4'-isoquinoline]-1'-one | LCMS: m/z: 488.3 [M+H]$^+$ . $^1$H NMR (400 MHz, methanol-$d_4$): δ ppm 8.47 (s, 1H), 7.80 (s, 1H), 7.41 (s, 1H), 6.93~6.79 (m, 5H), 5.93 (s, 2H), 5.07 (s, 2H), 4.67 (s, 2H), 3.73 (s, 2H), 3.58 (s, 2H), 2.98 (s, 3H), 2.73~2.66 (m, 2H), 2.24 (s, 6H), 1.97~1.94 (m, 2H), 1.82~1.77 (m, 2H). |

**Example 6: Synthesis of Compound 70**

2'-((S)-1-(4-ethoxy-5-fluoropyridine-2-yl)ethyl)-5'-((3-methyl-3,6-diazadicyclo[3.1.1]heptan-6-yl)methyl)-7'-((2-(methylamino)-1H-imidazol-1-yl)methyl)-2',3'-dihydro-1'H-spiro[cyclopropan-1,4'-isoquinoline]-1 '-one

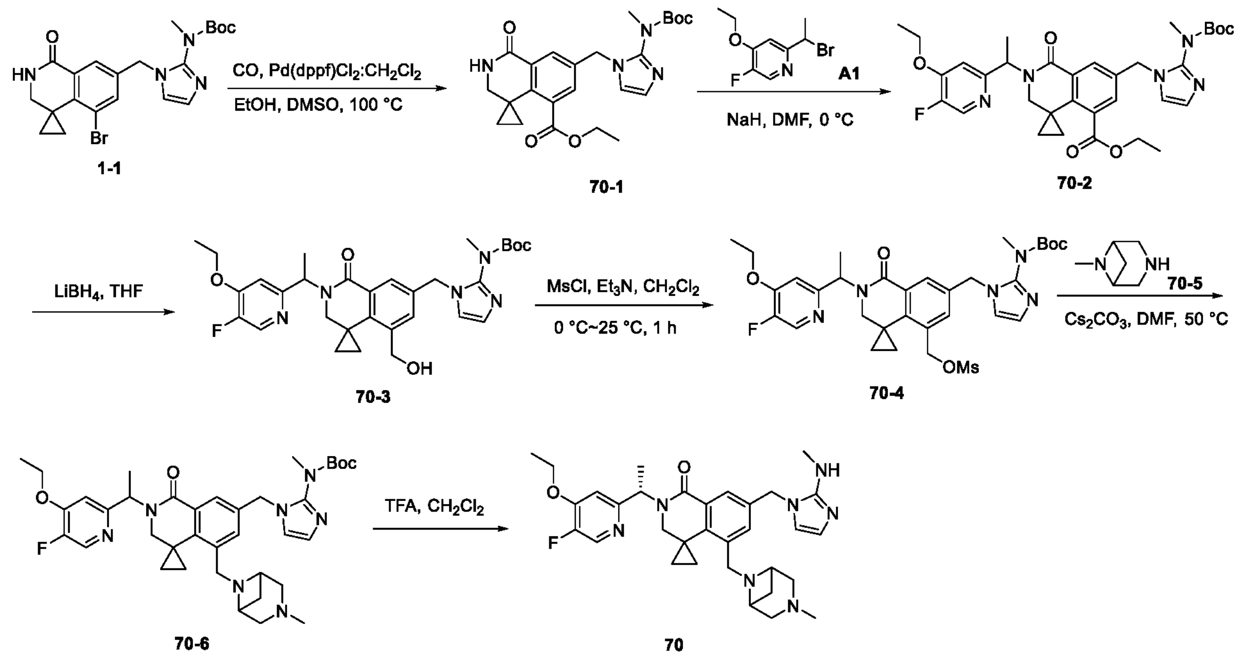


Step 1: N,N-diisopropylethanamine (3.0 g, 23.8 mmol) and [1,1'-bis(diphenylphosphino)ferrocene]palladium (II) chloride dichloromethane complex (389 mg, 0.48 mmol) were added into **1-1** (1.1 g, 2.38 mmol) in anhydrous ethanol (25 mL). After the air was replaced with CO three times, the reaction mixture was heated to 100 °C under 3 MPa pressure of CO and reacted overnight. The reaction liquid was concentrated to dryness. The residue was purified through column chromatography (dichloromethane/methanol=30/1) to obtain a light yellow solid of **70-1** (725 mg, yield: 66.9%)
LCMS: m/z 455.1 $[M+H]^+$

Step 2: sodium hydride (60% dispersed in oil, 200 mg, 5 mmol) was added into **70-1** (513 mg, 1.13 mmol) in dry DMF (15 mL) at 0 °C under nitrogen protection and kept reacting at 0°C for 30 mins. A solution of **A1**(330 mg, 1.33 mmol) in DMF (5 mL) was added dropwise and the reaction mixture was kept reacting at 0°C for 30 mins. The reaction liquid was poured into a saturated aqueous solution of ammonium chloride, extracted with ethyl acetate (3 * 100 mL). The organic phase was combined, washed with water (3 * 200 mL) and saturated saline (2 * 200 mL) in sequence, dried with anhydrous sodium sulfate, filtered and concentrated to dryness. The residue was purified through column chromatography (dichloromethane/methanol = 30/1) to obtain a light yellow solid of **70-2** (550 mg, yield:78.4%).
LCMS: m/z 622.2 $[M+H]^+$

Step 3: lithium borohydride (2M in tetrahydrofuran, 1.2 mL, 2.4 mmol) was added dropwise a solution of Compound **70-2** (500 mg, 0.80 mmol) in dry tetrahydrofuran (30 mL) at 0°C under nitrogen protection and kept reacting at 0°C for 5 mins, then heated to 25°C and kept reacting at this temperature overnight. The reaction liquid was poured into water, extracted with dichloromethane (3 * 80 mL). The organic phase was combined, washed with saturated saline (150 mL), dried with anhydrous sodium sulfate, filtered and concentrated to dryness. The residue was dissolved with methanol (50 mL), heated to 85°C and stirred overnight. The reaction liquid was concentrated under reduced pressure. The residue was purified through column chromatography (dichloromethane/methanol = 40/1) to obtained a white solid of **70-3** (360 mg, yield:77.3%).
LCMS: m/z 580.3 $[M+H]^+$

Step 4: at 0°C under nitrogen protection, triethylamine (104 mg, 1.03 mmol) was added into a solution of Compound **70-3** (50 mg, 0.086 mmol) in dichloromethane (5 mL), followed by adding dropwise a solution of methanesulfonyl chloride (80 mg, 0.70 mmol) in dichloromethane (4 mL). After the end of addition, the reaction mixture was kept reacting at 0°C for 5 mins, then heated to 25°C and kept reacting at this temperature for 1 hour. The reaction liquid was poured into water, extracted with dichloromethane (2 * 50 mL). The organic phase was combined, washed with saturated saline (100 mL), dried with anhydrous sodium sulfate, filtered and concentrated to dryness to obtain a colorless oil of

**70-4** (crude product, 60 mg).

Step 5: cesium carbonate (295 mg, 0.91 mmol) was added into a solution of Compound **70-4** (60 mg, 0.086 mmol) and hydrochloride of Intermediate **70-5** (50 mg, 0.27 mmol) in DMF (10 mL) under nitrogen protection, heated to 50°C and reacted overnight. The reaction liquid was poured into water, extracted with ethyl acetate (3 * 50 mL). The organic phase was combined, washed with saturated saline (100 mL), dried with anhydrous sodium sulfate, filtered and concentrated to dryness. The residue was purified through column chromatography (dichloromethane/methanol=10/1) purification to obtain a light yellow solid of **70-6** (50 mg, two step yield: 86.0%).

LCMS: m/z 674.3 $[M+H]^+$

Step 6: trifluoroacetic acid (2.5 mL) was added dropwise into a solution of **70-6** (50 mg, 0.074 mmol) in dichloromethane (10 mL) and reacted at room temperature for 1 hour. The reaction liquid was concentrated to remove trifluoroacetic acid. The residuewas diluted with dichloromethane (50 mL), washed with saturated sodium bicarbonate (50 mL) and saturated saline (50 mL) in sequence, dried with anhydrous sodium sulfate, filtered and concentrated to dryness. The residue was separated and purified through high performance liquid chromatography to obtain a light-yellow solid. The Compound was separated and purified through SFC to obtain 2'-((S)-1-(4-ethoxy-5-fluoropyridine-2-yl)ethyl)-5'-((3-methyl-3, 6-diazadicyclo[3 .1.1 ]heptan-6-yl)methyl)-7'-((2-(methylamino)-1H-imidazol-1-yl)methyl)-2',3'-dihydro-1'H-spiro[cyclopropan-1,4'-isoquinoline]-1'-one (Compound **70, 4** mg, yield: 9.4%).

LCMS: m/z 574.2 $[M+H]^+$

$^1H$ NMR(400 MHz, methanol-d4) δ ppm: 8.23 (d, *J* = 3.2 Hz, 1H), 7.81 (d, *J* = 1.6 Hz, 1H), 7.17 (d, *J* = 6.8 Hz, 1H), 7.10 (d, *J* = 1.6 Hz, 1H), 6.64~6.61 (m, 2H), 5.97~5.92 (m, 1H), 4.95 (s, 2H), 4.24~4.16 (m, 2H), 3.60 (s, 2H), 3.48~3.31 (m, 3H), 2.97 (d, *J* = 12.8 Hz, 1H), 2.87 (s, 4H), 2.84 (s, 3H), 2.44 (s, 3H), 2.40~2.39 (m, 1H), 1.94 (d, *J* = 8.0 Hz, 1H), 1.56~1.51 (m, 4H), 1.45 (t, *J* = 6.8 Hz, 3H), 1.12~1.07 (m, 1H), 0.93~0.87 (m, 1H), 0.49~0.46 (m, 1H).

$^{19}F$ NMR (376 MHz, methanol-d4) δ ppm: -154.04

### Example 7: Synthesis of Compound 41

2'-(1-(4-ethoxy-5-fluoropyridine-2-yl)ethyl)-5'-((methyl(2,2,2-trifluoroethyl)amino)methyl)-7'-((2-(methylamino)-1H-imidazol-1-yl)methyl)-2',3'-dihydro-1'H-spiro[cyclopropan-1,4'-isoquinoline]-1'-one

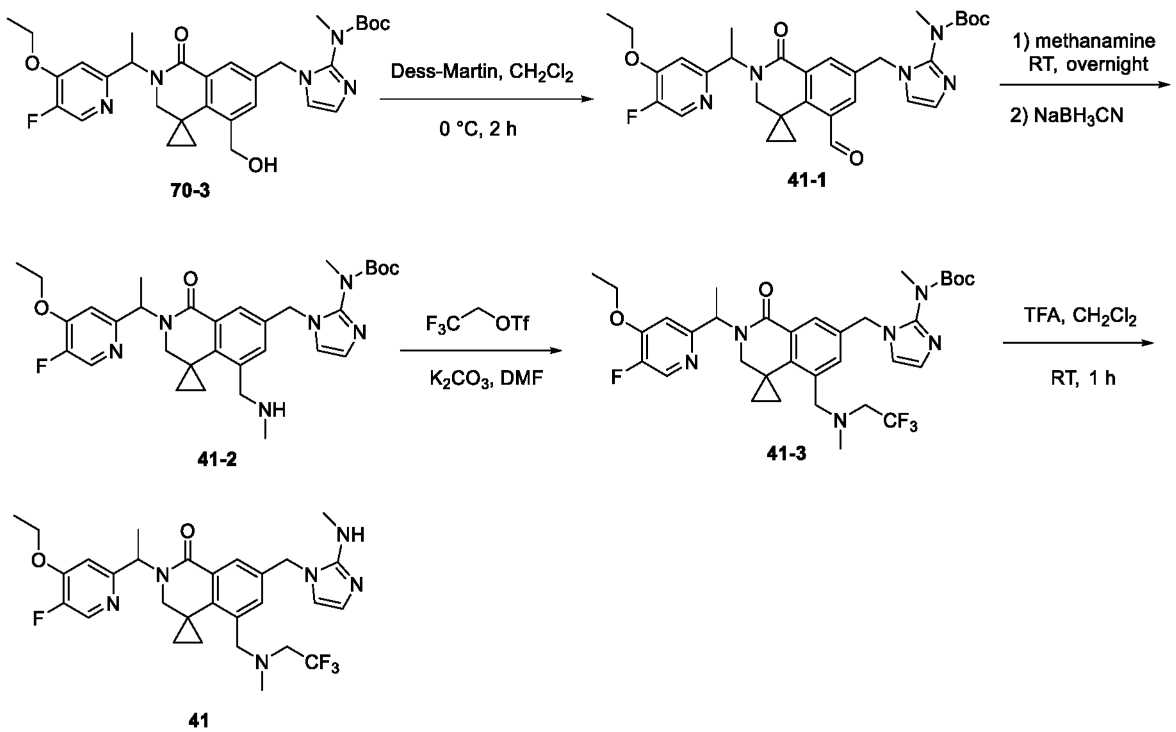


70-3, 41-1, 41-2, 41-3, 41

Step 1: Dess-Martin periodinane (190 mg, 0.45 mmol) was added into a solution of **70-3**(200 mg, 0.35 mmol) in dichloromethane (10 mL) at 0°C under nitrogen protection. After the end of addition, the reaction mixture was kept reacting at 0°C for 2 hours. The reaction liquid was poured into water, extracted with dichloromethane (2 * 60 mL). The

organic phase was combined, washed with saturated saline (100 mL), dried with anhydrous sodium sulfate and filtered. The filtrate was concentrated under reduced pressure to dryness, purified through column chromatography to obtained a white solid of **41-1** (200 mg, yield: 100%).

LCMS: m/z 578.3 $[M+H]^+$

Step 2: methylamine in tetrahydrofuran (2 mL, 4 mmol) was added into a solution of **41-1** (50 mg, 0.086 mmol) in dichloroethane (10 mL) under nitrogen protection and reacted at room temperature overnight. The reaction liquid was cooled to 0°C and added with sodium cyanoborohydride (10 mg, 0.17 mmol). The reaction mixture was heated to room temperature and reacted for 2 hours. The reaction liquid was poured into water, extracted with dichloromethane (3 * 100 mL). The organic phase was combined, washed with saturated saline (200 mL), dried with anhydrous sodium sulfate, filtered and concentrated. The residue was purified through column chromatography (dichloromethane/methanol=30/1) to obtain a light-yellow solid of **41-2** (50 mg, yield: 97.5%).

LCMS: m/z 593.3 $[M+H]^+$

Step 3: potassium carbonate (28 mg, 0.2 mmol) was added into a solution of **41-2** (30 mg, 0.051 mmol) and 2,2,2-trifluoroethyltrifluoromethane sulfonate (35 mg, 0.15 mmol) in DMF (10 mL) under nitrogen protection, heated to 50°C and reacted for 4 hours. The reaction liquid was poured into water and extracted with dichloromethane (3 * 50 mL). The organic phase was combined, washed with saturated saline (200 mL), dried with anhydrous sodium sulfate and filtered. The filtrate was concentrated under reduced pressure to dryness and purified through column chromatography (dichloromethane/methanol=20/1) to obtain a light yellow solid of **41-3** (5 mg, yield: 14.7%).

LCMS: m/z 675.4 $[M+H]^+$

Step 4: trifluoroacetic acid (1 mL) was added dropwise into a solution of **41-3** (5 mg, 0.007 mmol) in dichloromethane (5 mL) and reacted at room temperature for 1 hour. The reaction liquid was concentrated to remove trifluoroacetic acid. The residue was diluted with dichloromethane (50 mL), washed with saturated sodium bicarbonate (50 mL) and saturated saline (50 mL) in sequence, dried with anhydrous sodium sulfate and filtered. The filtrate was concentrated under reduced pressure to dryness. The residue was separated and purified through high performance liquid chromatography to obtain white dicarboxylic acid salt of Compound **41** (0.26 mg, yield: 5.3%).

LCMS: m/z 575.3 $[M+H]^+$

$^1H$ NMR (400 MHz, methanol-d4) δ ppm: 8.52 (br, 2H), 8.25 (d, *J*=3.2 Hz, 1H), 7.86 (d, *J*=2.4 Hz, 1H), 7.31 (s, 1H), 7.19 (d, *J*=6.8 Hz, 1H), 6.90-6.86 (m, 2H), 5.97-5.95 (m, 1H), 4.96 (s, 2H), 4.60 (s, 2H), 4.24~4.21 (m, 2H), 3.77~3.67 (m, 2H), 3.08~2.99 (m, 2H), 2.97 (s, 3H), 2.36 (s, 3H), 1.59~1.53 (m, 4H), 1.47 (t, *J*=6.8 Hz, 3H), 1.11~1.10 (m, 1H), 0.93~0.91 (m, 1H), 0.51-0.49 (m, 1H).

$^{19}F$ NMR (376 MHz, methanol-d4) δ ppm: -70.21, -153.98.

The following compounds were synthesized by the same method or modified method using the corresponding intermediates with reference to the synthesis method for Compound 41 of Example 7.

The following Examples refer to free compounds or pharmaceutically acceptable salts thereof.

| No. | Starting intermediate and material, reference step | Compound structure | Compound name | Data analysis |
|---|---|---|---|---|
| Compound **31** | **41-2, dibromoethanol,** with reference to Step 3 to Step 4 of Example 7 | 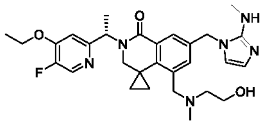 | (S)-2'-(l-(4-ethoxy-5-fluoropyridine-2-yl)ethyl)-5'-(((2-hydroxylethyl)(methyl)amino)met hyl)-7'-((2-(methylamino)-1H-imidazol-l-yl)methyl)-2',3'-dihydro-1'H-spiro[cyclopropan-1,4'-isoquinoline]-1 '-one | LCMS: m/z: 537.3 $[M+H]^+$ . |

(continued)

| No. | Starting intermediate and material, reference step | Compound structure | Compound name | Data analysis |
|---|---|---|---|---|
| Compound **42** | **41-1, 3,3,3-trifluoropropylamine,** with reference to Step 3 and Step 4 of Example 7 | 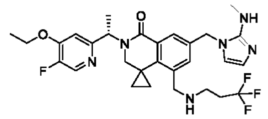 | (S)-2'-(l-(4-ethoxy-5-fluoropyridine-2-yl)ethyl)-7'-((2-(methylamino)-1H-imidazol-1-yl)methyl)-5'-(((3,3,3-trifluoropropyl) amino)methyl)-2',3'-dihydro-1'H-spiro [cyclopropyl-1,4'-isoquinoline]-1'-one | LCMS: m/z: 575.3 $[M+H]^+$ . |
| Compound **43** | **41-2, bromoacetonitrile**, with reference to Step 3, 4 of Example 7 | 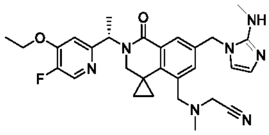 | (S)-2'-(1-(4-ethoxy-5-fluoropyridine-2-yl)ethyl)-7'-((2-(methylamino)- 1H-imidazol-1 - yl)methyl)-l'-one- 2',3'-dihydro-1 [cyclopropyl-1,4'-isoquinoline]-5'-yl)methyl) (methyl)amino)acetonit rile | LCMS: m/z: 532.3 $[M+H]^+$ . $^1$H NMR (400 MHz, methanol-d4) δ ppm: 8.23 (d, *J*=3.2 Hz, 1H), 7.87 (d, *J*=1.6 Hz, 1H), 7.20 (d, *J*=1.6 Hz, 1H), 7.16 (d, *J*=6.8 Hz, 1H), 6.66 (s, 1H), 6.62 (s, 1H), 5.94~5.92 (m, 1H), 4.97 (s, 2H), 4.21~4.18 (m, 2H), 3.65~3.44 (m, 5H), 2.97 (d, *J*=13.6 Hz, 1H), 2.94 (s, 3H), 2.29 (s, 3H), 1.56~1.49 (m, 4H), 1.43 (t, J=7.2 Hz, 3H), 1.10~1.08 (m, 1H), 0.85~0.82 (m, 1H), 0.47~0.45 (m, 1H). |
| Compound **44** | **41-1, bromoacetonitrile**, with reference to Step 2, 4 of Example 7 | 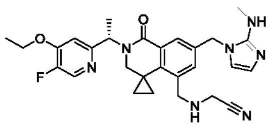 | (S)-2-(((2'-(1-(4-ethoxy-5-fluoropyridine-2-yl)ethyl)-7'-((2-(methylamino)-1H-imidazol-1-yl)methyl)-l'-one- 2',3'-dihydro-1'H-spiro [cyclopropyl-1,4'-isoquinoline]-5'-yl)methyl) amino)acetonitrile | LCMS: m/z: 518.2 $[M+H]^+$ . |
| Compound **45** | **41-2, 3,3,3-trifluorobromopropane,** with reference to Step 3 and Step 4 of Example 7 | 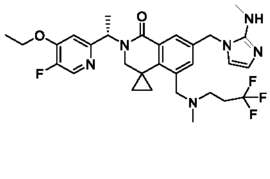 | (S)-2'-(l-(4-ethoxy-5-fluoropyridine-2-yl)ethyl)-5'-((methyl(3,3,3-trifluoropropyl)amino) methyl)-7'-((2-(methylamino)-1H-imidazol-1-yl)methyl)-2',3'-dihydro-1'H-spiro [cyclopropyl-1,4'-isoquinoline]-1'-one | LCMS: m/z: 589.2 $[M+H]^+$ . |

(continued)

| No. | Starting intermediate and material, reference step | Compound structure | Compound name | Data analysis |
|---|---|---|---|---|
| Compound **46** | **41-1 trifluorobromoethane,** with reference to Step 2, 4 of Example 7 | 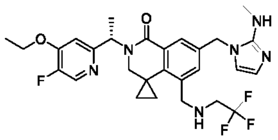 | (S)-2'-(l-(4-ethoxy-5-fluoropyridine-2-yl)ethyl)-7'-((2-(methylamino)-1H-imidazol-1-yl)methyl)-5'-(((2,2,2-trifluoroethyl) amino)methyl)-2',3'-dihydro-l'H-spiro [cyclopropyl-1,4'-isoquinoline]-1'-one | LCMS: m/z: 561.3 [M+H]+. |
| Compound **109** | **A3, 1-1** | 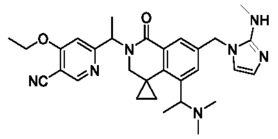 | 6-(1-(5'-(1-(dimethylamino) ethyl)-7'-((2-(methylamino)-1H-imidazol-1 - yl)methyl)-1'-oxo-1'H-spiro [cyclopropyl-1,4'-isoquinoline]-2'(3'H)-yl) ethyl)-4-ethoxynicotinonitrile | LCMS: m/z: 528.3 [M+H]+. |

**Example 8: Synthesis of Compound 88 and Compound 89**

5'-((dimethylamino)methyl)-2'-(-1-(4-ethoxy-5-fluoropyridine-2-yl)ethyl)-2,2-difluoro-7'-((2-(methylamino)-1H imidazol-1-yl)methyl)-2' , 3'-dihydro-1'H-spiro[cyclopropan-1,4'-isoquinoline]-1'-one

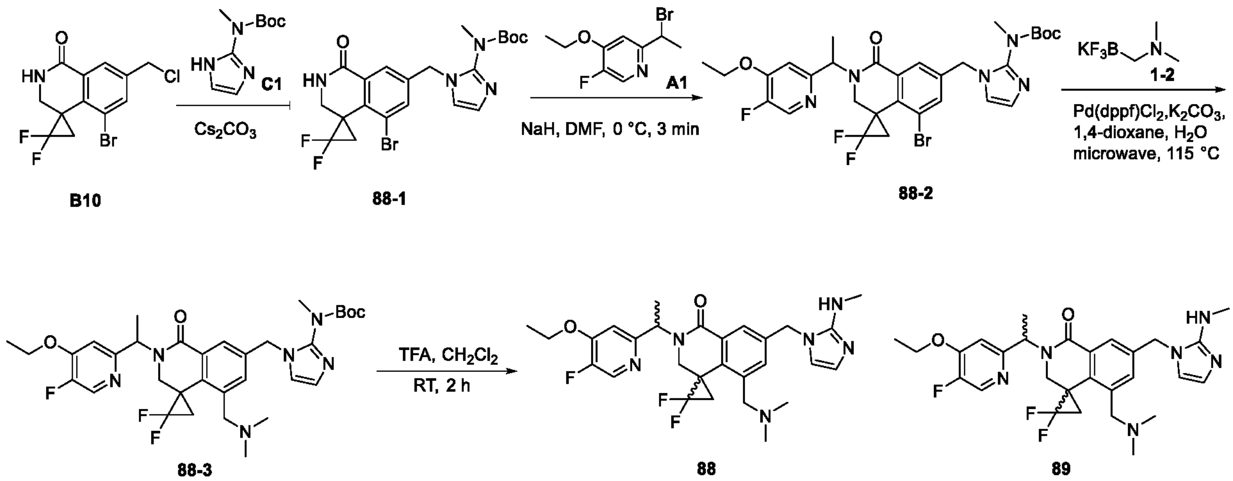

B10 88-1 88-2 88-3 88 89

Step 1: With reference to the synthesis procedure of **1-1,** Intermediate **88-1** was synthesized by one step from B10 as the starting Compound.

LCMS: m/z: 497.1[M+H]+.

Step 2: sodium hydride (60% dispersed in oil, 40 mg, 1.01 mmol) was added into a solution of **88-1** (125 mg, 0.25 mmol) in DMF (5 mL) at 0°C under argon protection and stirred at 0°C for 0.5 hours, followed by adding dropwise a solution of **A1** (187 mg, 0.75 mmol) in DMF (2 mL). After the end of addition, the reaction mixture was continued to react for 3 mins until the completion of reaction was shown by TLC. The reaction liquid was poured into ice water, extracted with ethyl acetate (20 mL) twice. The organic phase was combined, washed with water (30 mL) and a saturated

aqueous solution of NaCl (30 mL) and dried with anhydrous sodium sulfate and filtered. The filtrate was concentrated under reduced pressure. After column chromatography purification, Intermediate **88-2** (120 mg, yield: 71.8%) was obtained.

LCMS: m/z: 666.0 $[M+H]^+$ .

Step 3: potassium dimethylaminomethyl trifluoroborate (147 mg, 0.89 mmol), potassium carbonate (74 mg, 0.54 mmol) and [1,1'-bis(diphenylphosphino)ferrocene] palladium (II) chloride (26 mg, 0.04 mmol) were added into a solution of **88-2** (120 mg, 0.18 mmol) in 1,4-dioxane/water (10 mL/2.5 mL) and reacted at 115°C under microwave for 0.5 hours. The reaction liquid was directly concentrated and purified through column chromatography (dichloromethane: methanol = 40:1) to obtain **88-3** (65 mg, yield: 56.0%).

LCMS: m/z 643.2 $[M+H]^+$.

Step 4: trifluoroacetic acid (1 mL) was added dropwise a solution of **88-3** (65 mg, 0.10 mmol) in dichloromethane (4 mL) and reacted at room temperature for 3 hours. The reaction liquid was poured into ice water, extracted with dichloromethane (20 mL) and concentrated. After high performance liquid chromatography separation and purification, two diastereoisomers of 5'-((dimethylamino)methyl)-2'-(1-(4-ethoxy-5-fluoropyridine-2-yl)ethyl)-2,2-difluoro-7'-((2-(methylamino)-1H)imidazol-1-yl)methyl)-2' ,3'-dihydro-1'H-spiro[cyclopropan-1,4'-isoquinoline] -1'-one (Compound **88:** 6 mg, yield: 10.9% and Compound **89:** 7 mg, yield: 12.8%) were obtained.

Compound **88:**

LCMS: m/z 543.2 $[M+H]^+$.

$^1H$ NMR (400 MHz, methanol-d4): δ ppm 8.21 (d, *J* = 3.6 Hz, 1H), 7.89 (d, *J* = 1.6 Hz, 1H), 7.19~7.17 (m, 2H), 6.65~6.62 (m, 2H), 5.95~5.93 (m, 1H), 4.99 (s, 2H), 4.22~4.13 (m, 2H), 3.99-3.94 (m, 1H), 3.45-3.42 (m, 1H), 3.31~3.26 (m, 1H), 3.17~3.08 (m, 2H), 2.85 (s, 3H), 2.12 (s, 6H), 1.74~1.67 (m, 1H), 1.56 (d, *J* = 7.2 Hz, 3H), 1.41 (t, *J* = 7.2 Hz, 3H).

$^{19}F$ NMR (376 MHz, methanol-d4): δ ppm -137.71, -138.11, -142.16, -142.57, -154.24.

Compound **89:**

LCMS: m/z 543.2 $[M+H]^+$.

$^1H$ NMR (400 MHz, methanol-d4): δ ppm 8.19 (d, *J* = 3.2 Hz, 1H), 7.87 (d, *J* = 2.0 Hz, 1H), 7.15~7.08 (m, 2H), 6.63~6.61 (m, 2H), 5.95~5.90 (m, 1H), 4.98 (d, *J* = 2.0 Hz, 2H), 4.22~4.15 (m, 2H), 3.65~3.61 (m, 1H), 3.46~3.43 (m, 1H), 3.31~3.12 (m, 3H), 2.84 (s, 3H), 2.12 (s, 6H), 1.90~1.85 (m, 1H), 1.63 (d, *J* = 7.2 Hz, 3H), 1.42 (t, *J* = 7.2 Hz, 3H).

$^{19}F$ NMR (376 MHz, methanol-d4): δ ppm -137.52, -137.93, -140.86, -141.27, -154.27.

### Example 9: Synthesis of Compound 90 and Compound 91

**Compound 90:** (R)-5'-((dimethylamino)methyl)-2'-((R)-1-(4-ethoxy-5-fluoropyridine-2-yl)ethyl)-2,2-difluoro-7'-((2-(methylamino)-1H imidazol-1-yl)methyl)-2' , 3'-dihydro-1'H-spiro[cyclopropan-1,4'-isoquinoline] -1 '-one

Compound 91: (S)-5'-((dimethylamino)methyl)-2'-((S)-1-(4-ethoxy-5-fluoropyridine-2-yl)ethyl)-2,2-difluoro-7'-((2-(methylamino)-1H imidazol-1-yl)methyl)-2' , 3'-dihydro-1'H-spiro[cyclopropan-1,4'-isoquinoline] -1 '-one

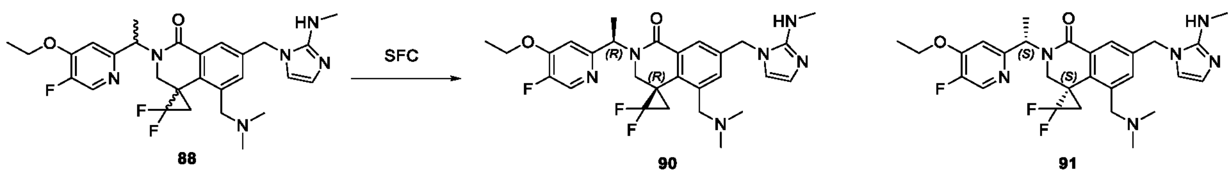


88 90 91

Compound **88** (300 mg) was separated and purified through SFC to obtain two compounds of Compound **90** (75.11 mg, yield:25.0%) and Compound **91** (78.88 mg, yield: 26.3%) as white solids. The absolute configuration of Compound 90 and Compound 91 can be obtained by XRD single crystal diffraction data, or it can be obtained by analyzing the common crystal structure of the compound and WDR5 protein.

**Compound 90:** (R)-5'-((dimethylamino)methyl)-2'-((R)-1-(4-ethoxy-5-fluoropyridine-2-yl)ethyl)-2,2-difluoro-7'-((2-(methylamino)-1H imidazol-1-yl)methyl)-2' , 3'-dihydro-1'H-spiro[cyclopropan-1,4'-isoquinoline] -1 '-one

LCMS: m/z 543.15 $[M+H]^+$.

$^1H$ NMR (400 MHz, DMSO-$d_6$): δ ppm 8.36 (d, *J* = 2.8 Hz, 1H), 7.80 (d, *J* = 1.6 Hz, 1H), 7.35~7.31 (m, 2H), 7.13 (d, *J* = 6.8 Hz, 1H), 6.97 (d, *J* = 2.0 Hz, 1H), 6.86 (d, *J* =1.2 Hz, 1H), 5.90~5.84 (m, 1H), 5.11 (s, 2H), 4.23~4.04 (m, 2H), 3.92~3.88 (m, 1H), 3.48~3.18 (m, 2H), 3.08-3.02 (m, 2H), 2.84 (d, *J* = 4.4 Hz, 3H), 2.08 (s, 6H), 1.96~1.91 (m, 1H), 1.48 (d, *J* = 7.2 Hz, 3H), 1.33 (t, *J* = 6.8 Hz, 3H).

$^{19}F$ NMR (376 MHz, DMSO-$d_6$): δ ppm -134.96, -135.35, -140.52, -140.91, -153.14.

**Compound 91:** (S)-5'-((dimethylamino)methyl)-2'-((S)-1-(4-ethoxy-5-fluoropyridine-2-yl)ethyl)-2,2-difluoro-7'-((2-(methylamino)-1H imidazol-1-yl)methyl)-2' , 3'-dihydro-1'H-spiro[cyclopropan-1,4'-isoquinoline] -1 '-one

LCMS: m/z 543.15 $[M+H]^+$.

$^1H$ NMR (400 MHz, DMSO-$d_6$): δ ppm 8.36 (d, *J* = 3.2 Hz, 1H), 7.80 (d, *J* = 1.6 Hz, 1H), 7.35~7.28 (m, 2H), 7.13 (d, *J* = 6.8 Hz, 1H), 6.96 (d, *J* = 1.6 Hz, 1H), 6.84 (d, *J* =1.6 Hz, 1H), 5.90~5.84 (m, 1H), 5.12 (s, 2H), 4.21~4.06 (m, 2H), 3.92~3.88 (m, 1H), 3.48~3.42 (m, 1H), 3.21~3.17 (m, 1H), 3.08~3.02 (m, 2H), 2.84 (d, *J* = 4.8 Hz, 3H), 2.08 (s, 6H), 1.95~1.91 (m, 1H), 1.48 (d, *J* = 7.2 Hz, 3H), 1.33 (t, *J* = 6.8 Hz, 3H).

$^{19}F$ NMR (376 MHz, DMSO-$d_6$): δ ppm -134.96, -135.35, -140.52, -140.91, -153.15.

The following compounds or salts thereof were synthesized by the method with reference to the synthesis method for Compounds 88 and 89 of Example 8.

| No. | Starting intermediate and material, reference step | Compound structure | Compound name | Data analysis |
|---|---|---|---|---|
| formate salt of Com pou nd **92** | 88-2, D78, Step 3, Step 4 of Example 8 | 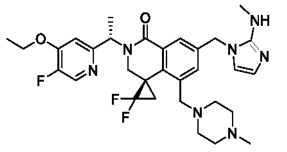 HCOOH 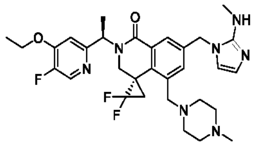 HCOOH | 2'-(-1-(4-ethoxy-5-fluoropyridine-2-yl)ethyl)-2,2-difluoro-7'-((2-(methylamino)-1H-imidazol-1yl)methyl)-5'-((4-methylpiperazin-1-yl) methyl)-2',3'-dihydro-1'H-spiro[cyclopropan-1,4'-isoquinoline]-1'-one formate salt | LCMS: m/z 598.3 $[M+H]^+$. $^1H$ NMR (400 MHz, methanol-d4): δ ppm 8.61 (brs, 2H), 8.22 (d, *J* = 2.8 Hz, 1H), 7.87 (s, 1H), 7.36 (s, 1H), 7.20 (d, *J* =7.6 Hz, 1H), 6.97~6.94 (m, 2H), 5.95~5.90 (m, 1H), 5.12 (s, 2H), 4.25~4.11 (m, 2H), 4.00~3.95 (m, 1H), 3.80~3.76 (m, 1H), 3.47~3.44 (m, 1H), 3.22~3.19 (m, 1H), 3.12~3.00 (m, 8H), 2.67 (s, 3H), 2.57 (s, 4H), 1.81~1.77 (m, 1H), 1.57 (d, *J* = 7.2 Hz, 3H), 1.42 (t, *J* = 7.2 Hz, 3H). |
| formate salt of Com pou nd **93** | The same as Compound 92 | 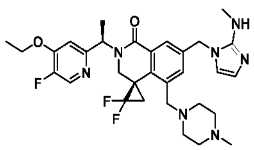 HCOOH , 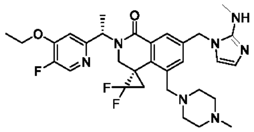 HCOOH | 2'-(-1-(4-ethoxy-5-fluoropyridine-2-yl)ethyl)-2,2-difluoro-7'-((2-(methylamino)-1H-imidazol-1yl)methyl)-5'-((4-methylpiperazin-1-yl) methyl)-2',3'-dihydro-1'H-spiro [cyclopropan-1,4'-isoquinoline]-1'-one formate salt | LCMS: m/z 598.3 $[M+H]^+$. $^1H$ NMR (400 MHz, methanol-d4): δ ppm 8.50 (brs, 2H), 8.18 (d, *J* = 3.2 Hz, 1H), 7.84 (s, 1H), 7.35 (s, 1H), 7.12 (d, *J* =6.8 Hz, 1H), 6.93~6.92 (m, 2H), 5.95~5.90 (m, 1H), 5.12 (s, 2H), 4.24~4.17 (m, 2H), 3.81~3.775 (m, 1H), 3.68~3.64 (m, 1H), 3.47~3.44 (m, 1H), 3.31~3.23 (m, 1H), 3.17~3.12 (m, 1H), 2.99 (s, 7H), 2.69 (s, 3H), 2.59 (s, 4H), 1.98~1.91 (m, 1H), 1.64 (d, *J* = 7.2 Hz, 3H), 1.43 (t, *J* = 6.8 Hz, 3H). |

(continued)

| No. | Starting intermediate and material, reference step | Compound structure | Compound name | Data analysis |
|---|---|---|---|---|
| formate salt of Com pou nd **94** | A3, B10, C1, 1-2, Step 1 to Step 4 of Example 8 | 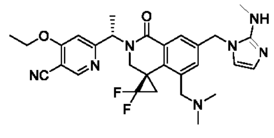 HCOOH , 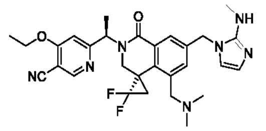 HCOOH | 6-(-1-(5'-((dimethylamino) methyl)-2,2-difluoro-7'-((2-(methylamino)-1H-imidazol-1-yl)methyl)-1'-one-1'H-spiro[cyclopropan-1,4'-isoquinoline]-2'(3'H)-yl) ethyl)-4--ethoxynicotinonitrile formate salt | LCMS: m/z 550.3 $[M+H]^+$. $^1H$ NMR (400 MHz, methanol-d4): δ ppm 8.52 (s, 1H), 8.35 (br, 2H), 7.75 (d, *J* = 1.6 Hz, 1H), 7.25 ((d, *J* = 1.6 Hz, 1H), 7.11 (s, 1H), 6.86~6.82 (m, 2H), 5.78~5.76 (m, 1H), 5.01 (s, 2H), 4.23~4.18 (m, 2H), 3.97~3.92 (m, 1H), 3.44~3.38 (m, 1H), 3.28~3.14 (m, 2H), 3.04~3.03 (m, 1H), 2.88 (s, 3 H), 2.07 (s, 6H), 1.76~1.69 (m, 1H), 1.52 (d, *J* = 7.2 Hz, 3H), 1.37~1.33 (m, 3H). |
| formate salt ofC omp oun d **95** | The same as Compound 94 | 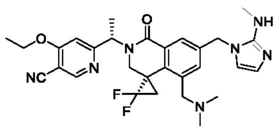 HCOOH , 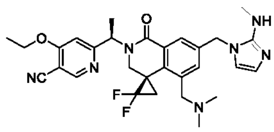 HCOOH | 6-(-1- (5'-((dimethylamino) methyl)-2,2-difluoro-7'-((2-(methylamino)-1H-imidazol-1-yl)methyl)-1'-one-1'H-spiro[cyclopropan-1,4'-isoquinoline]-2'(3'H)-yl) ethyl)-4--ethoxynicotinonitrileformate salt | LCMS: m/z 550.3 $[M+H]^+$. $^1H$ NMR (400 MHz, methanol-d4): δ ppm 8.58 (s, 1H), 8.45 (br, 2H), 7.84 (s, 1H), 7.40 (s, 1H), 7.20 (s, 1H), 6.99~6.96 (m, 2H), 5.96~5.90 (m, 1H), 5.14 (s, 2H), 4.36~4.31 (m, 2H), 3.92~3.87 (m, 1H), 3.62~3.59 (m, 1H), 3.50~3.47 (m, 1H), 3.36~3.33 (m, 1H), 3.19~3.14 (m, 1H), 3.01 (s, 3H), 2.25 (s, 6H), 2.02~2.00 (m, 1H), 1.69 (d, *J* = 7.2 Hz, 3H), 1.49 (t, *J* = 6.8 Hz, 3H). |

(continued)

| No. | Starting intermediate and material, reference step | Compound structure | Compound name | Data analysis |
|---|---|---|---|---|
| Compound **96** | Resolved from Compound 89 through SFC | 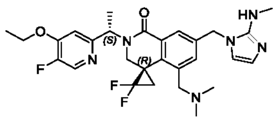 | (S)-5'-((dimethylamino) methyl)-2'-((R)-1-(4-ethoxy-5-fluoropyridine-2-yl)ethyl)-2,2-difluoro-7'-((2-(methylamino)-1H imidazol-l-yl)methyl)-2', 3'-dihydro-1'H-spiro [cyclopropan-1,4'-isoquinoline] -1'-one | LCMS: m/z 543.2 $[M+H]^+$. $^1H$ NMR (400 MHz, methanol-d4): δ ppm 8.19 (d, *J* = 3.2 Hz, 1H), 7.87 (d, *J* = 2.0 Hz, 1H), 7.15~7.08 (m, 2H), 6.63~6.61 (m, 2H), 5.95~5.90 (m, 1H), 4.98 (d, *J* = 2.0 Hz, 2H), 4.22~4.15 (m, 2H), 3.65~3.61 (m, 1H), 3.46~3.43 (m, 1H), 3.31~3.12 (m, 3H), 2.84 (s, 3H), 2.12 (s, 6H), 1.90~1.85 (m, 1H), 1.63 (d, *J* = 7.2 Hz, 3H), 1.42 (t, *J* = 7.2 Hz, 3H). $^{19}F$ NMR (376 MHz, methanol-d4): δ ppm -137.52, -137.93, - 140.86, -141.27, -154.27. |
| Compound **97** (Resolve d from **89)** | Resolved from Compound 89 through SFC | 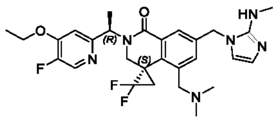 | (R)-5'-((dimethylamino) methyl)-2'-((S)-l-(4-ethoxy-5-fluoropyridine-2-yl)ethyl)-2,2-difluoro-7'-((2-(methylamino)-1H imidazol-1-yl)methyl)-2', 3'-dihydro-1'H-spiro [cyclopropan-1,4'-isoquinoline]-1'-one | |
| Compound **98** | Al, B11, C1, 1-2, Step 1 to Step 4 of Example 8 | 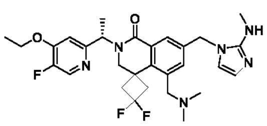 | (S)-5'-((dimethylamino) methyl)-2'-1-(4-ethoxy-5-fluoropyridine-2-yl)ethyl)-3,3-difluoro-7'-((2-(methylamino)-1H-imidazol-1-yl)methyl)-2',3'-dihydro-1'H-spiro [cyclobutan-1,4'-isoquinoline]-1'-one | LCMS: m/z 557.3 $[M+H]^+$. $^1H$ NMR (400 MHz, methanol-d4) δ ppm: 8.47(bs, 1H), 8.27 (d, *J* = 2.4 Hz, 1H), 7.88 (d, *J* = 1.2 Hz, 1H), 7.36 (s, 1H), 7.18 (d, *J* = 6.8 Hz, 1H), 6.98~6.92 (m, 2H), 5.97 (q, *J* = 6.8 Hz, 1H), 5.11 (s, 2H), 4.28~4.16 (m, 2H), 3.74~3.49 (m, 5H), 3.16~3.05 (m, 1H), 2.99 (s, 3H), 2.59~2.49 (m, 1H), 2.20~2.12 (m, 7H), 1.63 (d, *J* = 7.2 Hz, 3H), 1.44 (t, *J* = 7.2 Hz, 3H). |

(continued)

| No. | Starting intermediate and material, reference step | Compound structure | Compound name | Data analysis |
|---|---|---|---|---|
| Compound **99** | A3, B11, C1 1-2, Step 1 to Step 4 of Example 8 | 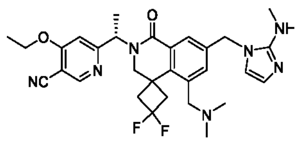 | (S)-6-(1-(5'-((dimethylamino) methyl)-3,3 - difluoro-7'-((2-(methylamino)-1H-imidazol-1 yl)methyl)-1'-oxo-1) 'H-spiro[cyclobutan-1,4'-isoquinoline] -2'(3'H) -yl) ethyl)-4-ethoxynicotinonitrile | LCMS: m/z 564.3 $[M+H]^+$. $^1H$ NMR (400 MHz, methanol-$d_4$): δ ppm 8.64 (s, 1H), 8.53 (s, 1H), 7.85 (d, $J$ = 2.0 Hz, 1H), 7.26 (d, $J$ = 2.0 Hz, 1H), 7.21 (s, 2H), 6.77~6.75 (m, 2H), 5.96~5.91 (m, 1H), 5.01 (s, 2H), 4.35~4.29 (m, 2H), 3.67~3.56 (m, 3H), 3.54~3.49 (m, 2H), 2.91 (s, 3H), 2.63~2.55 (m, 1H), 2.35~2.27 (m, 1H), 2. 71 (s, 6H), 1.65 (d, $J$ = 7.2 Hz, 3H), 1.47 (t, $J$ = 7.2 Hz, 3H). |

**Example 10: Synthesis of Compound 100:**

6-(1-(5'-((bis(methyl-d3)amino)methyl)-7'-((2-(methylamino)-1H-imidazol-1-yl)methyl)-1'-oxo-1'H-spiro [cyclobutan-1,4'-isoquinoline]-2'(3'H)-yl)ethyl)-4-ethoxynicotinonitrile

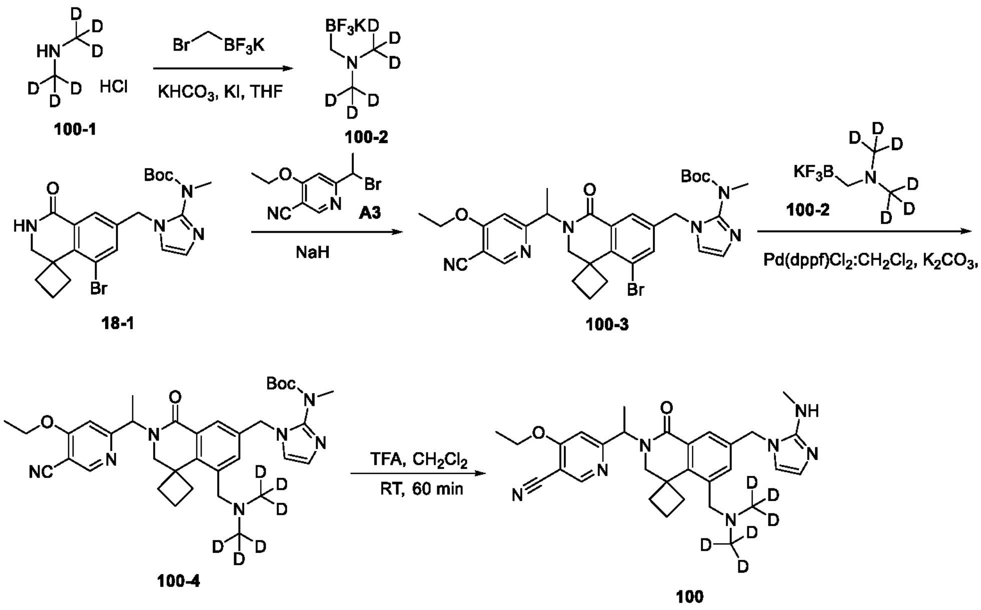

Step 1: potassium (bromomethyl) trifluoroborate (229 mg, 1.14 mmol), potassium bicarbonate (342 mg, 3.42 mmol) and potassium iodide (18 mg, 0.11 mmol) were added into a solution of dimethyl-d6-aminehydrochloride **100-1** (100 mg, 1.14 mmol) in tetrahydrofuran (40 mL) and reacted at 70°C for 4 hours. The reaction liquid was concentrated under reduced pressure, followed by adding acetone (200 mL), heated to 40°C, stirred for 15 mins and filtered. The filtrate was concentrated under reduced pressure to obtain **100-2** (180 mg, yield: 92.3%).

Step 2: Intermediate **100-3** was synthesized through one step with reference to the synthesis of Intermediate **23-1.**

Step 3: **100-2** (96 mg, 0.62 mmol), potassium carbonate (129 mg, 0.93 mmol) and [1,1'-bis(diphenylphosphino)ferrocene]palladium (II) chloride dichloromethane complex (49 mg, 0.06 mmol) were added into a solution of **100-3** (200 mg, 0.31 mmol) in dioxane (3 mL) and water (1 mL) and reacted at under argon protection at 115°C under microwave for 70 mins. The reaction was quenched with water and extracted with ethyl acetate (3 * 20 mL). The organic phase was combined, dried, filtered and concentrated. After column chromatography purification, **100-4** (170 mg, yield: 86.7 %) was obtained.

LCMS: m/z: 634.3 [M+H]+

Step 4: trifluoroacetic acid (2 mL) was added dropwise into a solution of **100-4** (170 mg, 0.27 mmol) in dichloromethane (10 mL) at 0°C and reacted at room temperature for 60 mins. The reaction mixture was concentrated under reduced pressure, added with a saturated sodium bicarbonate aqueous solution and extracted with dichloromethane (3 * 20 mL). The organic phase was combined, dried, filtered and concentrated. After high performance liquid chromatography separation and purification, a dicarboxylic acid salt of Compound **100** (40.5 mg, yield: 24.1%) is obtained.

LCMS: m/z: 534.3 $[M+H]^+$

1H NMR (400 MHz, methanol-d4) δ ppm: 8.66 (s, 1H), 8.44 (s, 2H), 7.84 (s, 1H), 7.54 (s, 1H), 7.23 (s, 1H), 6.97 (d, *J* = 2.4 Hz, 1H), 6.92 (d, *J* = 2.4 Hz, 1H), 5.95 (q, *J* = 7.2 Hz, 1H), 5.10 (s, 2H), 4.36 ~ 4.27 (m, 2H), 4.07 (s, 2H), 3.76~3.64 (m, 2H), 2.99 (s, 3H), 2.82~2.77(m, 1H), 2.50~2.43 (m, 1H), 2.10 - 2.02 (m, 3H), 1.71 - 1.61 (m, 4H), 1.47 (t, *J* = 6.8 Hz, 3H).

**Example 11: Synthesis of Compound 101**

6-(1-(5'-((dimethylamino)methyl)-7'-((2-(methylamino)-1H-imidazol-1-yl)methyl)-1'-oxo-1'H-spiro[cyclobutane)-1,4'-isoquinoline]-2'(3'H)-yl)ethyl-1-d)-4-ethoxynicotinonitrile

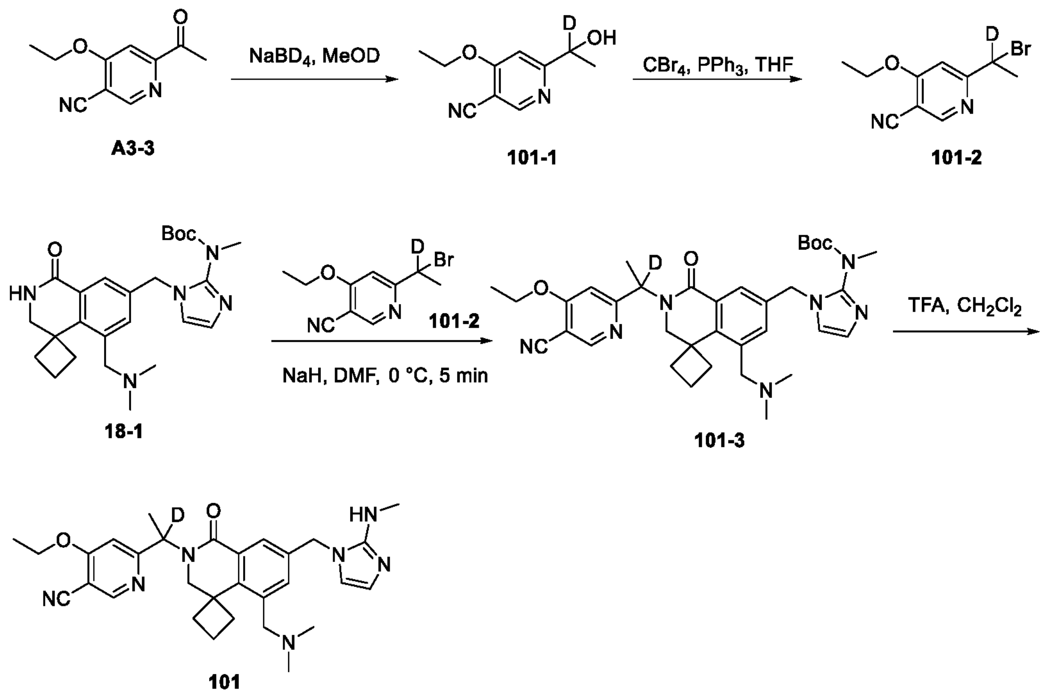


Step 1: sodium borodeuteride (107 mg, 2.55 mmol) was added into a solution **of A3-3** (485 mg, 2.55 mmol) in methanol-d4 (5 mL) at 0 °C and reacted at 0°C for 1 hour. The reaction was quenched with ice water, diluted with ethyl

acetate (100 mL), washed with saturated saline (3 * 20 mL), dried with anhydrous sodium sulfate and filtered. The filtrate was concentrated under reduced pressure to dryness. After column chromatography (petroleum ether/ethyl acetate =1/1) purification, a light white solid of **101-1** (350 mg, yield: 71.1%) was obtained.

LCMS: m/z: 194 $[M+H]^+$.

Step 2: carbon tetrabromide (463 mg, 1.40 mmol) was added into a solution of **101-1**(180 mg, 0.93 mmol) and triphenylphosphine (486 mg, 1.87 mmol) in tetrahydrofuran (10 mL) at 0 °C under argon protection, reacted at 0°C for 5 mins, then brought to room temperature and reacted under stirring for 16 hours. The reaction liquid was filtered. The filtrate was concentrated under reduced pressure to dryness. After column chromatography (petroleum ether/ethyl acetate =10/1) purification, a light yellow solid of **101-2** (160 mg, yield: 67.1 %) was obtained.

LCMS: m/z: 256 $[M+H]^+$ .

Step 3: sodium hydride (60% dispersed in oil, 25 mg, 0.66 mmol) was added into a solution of Intermediate **18-1** (100 mg, 0.22 mmol) in DMF (10 mL) at 0°C. After the completion of the addition, the reaction mixture was reacted for 20 mins at 0°C, followed by adding dropwise a solution of **101-2** (84 mg, 0.33 mmol) in DMF, and stirred at 0°C for 5 mins. The reaction liquid was added with ice water and extracted with ethyl acetate (3 * 30 mL). The organic phase was washed with saturated saline, dried, filtered and concentrated to dryness. After column chromatography (dichloromethane/methanol=20/1) purification, a white solid of **101-3** (100 mg, yield: 72.4%) was obtained.

LCMS: m/z: 629 $[M+H]^+$ .

Step 4: trifluoroacetic acid (2 mL) was added dropwise into a solution of **101-3** (100 mg, 0.16 mmol) in dichloromethane (4 mL) at 0°C and reacted at room temperature for 1.5 hours. The reaction liquid was diluted with dichloromethane (10 mL), added with a saturated aqueous solution of sodium bicarbonate and stirred at room temperature. The water phase and the organic phase were separated. The water phase was extracted with dichloromethane (3 * 20 mL). The organic phase was combined, washed with saturated saline, dried, filtered, and concentrated under reduced pressure to dryness. After high performance liquid chromatography separation and purification, a dicarboxylic acid salt of Compound **101** (30.91 mg, yield:31.3 %) was obtained.

LCMS: m/z: 529 $[M+H]^+$ .

$^1$H NMR (400 MHz, methanol-d4) δ ppm: 8.67 (s, 1H), 8.36 (s, 2H), 7.79 (d, *J* = 2.0 Hz, 1H), 7.46 (d, *J* = 2.0 Hz, 1H), 7.22 (s, 1H), 6.96 (d, *J* = 2.4 Hz, 1H), 6.91 (d, *J* = 2.4 Hz, 1H), 5.07 (s, 2H), 4.35~4.27 (m, 2H), 3.86~3.62 (m, 4H), 2.98 (s, 3H), 2.87~2.81 (m, 1H), 2.56~2.46 (m, 1H), 2.30 (s, 6H), 2.05~1.99 (m, 3H), 1.68 (s, 3H), 1.60~1.56 (m, 1H), 1.47 (t, *J* = 6.8 Hz, 3H).

**Example 12: Synthesis of Compound 111**

6-(1-(5'-((dimethylamino)methyl)-7'-((2-(methylamino)-1H-imidazol-1-yl)methyl)-1'-oxo-1'H-spiro[cyclobutan-1,4'-isoquinoline]-2'(3'H)-yl)ethyl)-4-(ethoxy-d5)nicotinonitrile

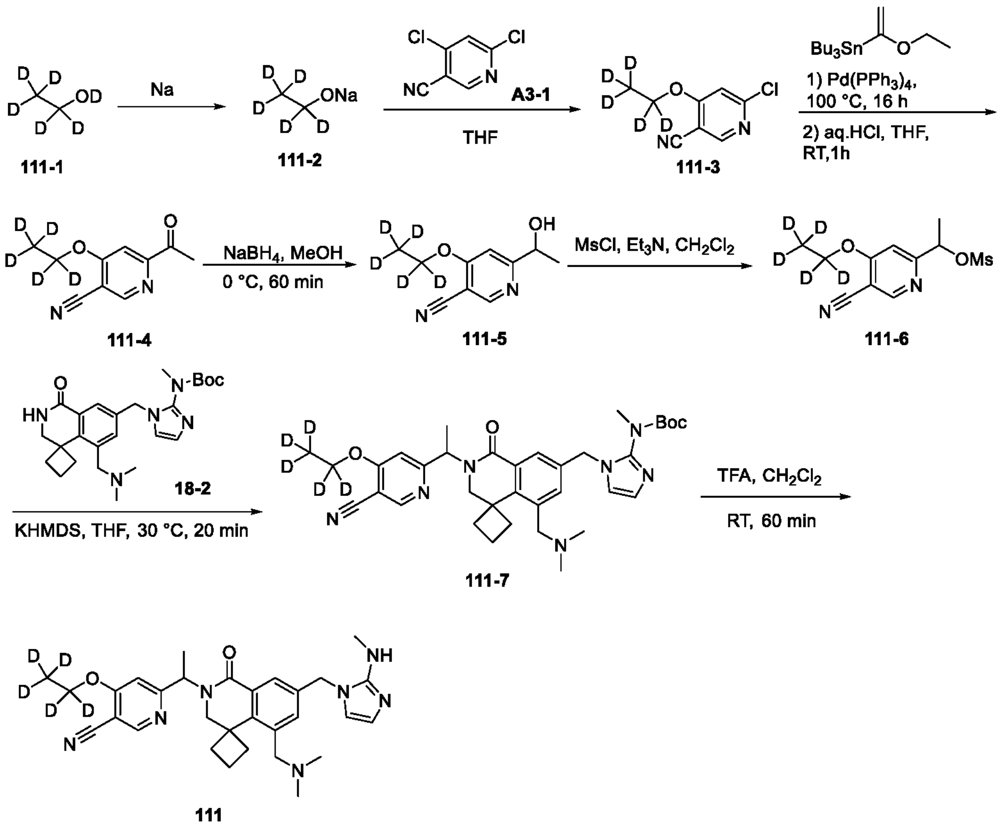


Step 1: metal sodium (210 mg, 9.13 mmol) was added into a solution of **111-1** (5 mL) at 0°C. After the completion of addition, the reaction mixture was reacted at 0°C for 30 mins, then heated to 80°C and reacted for 3 hours until metal sodium completely disappeared. The reaction liquid was cooled to room temperature to obtain a solution of **111-2** (4 mL, 9.13 mmol).

Step 2: a solution of **111-2** in $CD_3CD_2OD$ (4 mL, 9.13 mmol) was added into a solution of **A3-1** (1.5 g, 8.72 mmol) in tetrahydrofuran at 0°C. After the completion of addition, the reaction mixture was reacted at 0 °C for 30 mins and reacted at room temperature for 2 hours. The reaction liquid was added with water and extracted with ethyl acetate (3 * 100 mL). The organic phase was washed with saturated saline, dried, filtered and concentrated to dryness. After column chromatography (petroleum ether/ethyl acetate =2/1) purification, a white solid of **111-3** (1.1 g, yield:67.6%) was obtained.

LCMS: m/z: 188 $[M+H]^+$.

Step 3: tetrakis (triphenylphosphine)palladium (0.73 g, 0.64 mmol) was added into a solution of **111-3** (600 mg, 3.20 mmol) and tributyl (1-ethoxyvinyl) stannane (1.7 g, 4.79 mmol) in 1,4-dioxane (30 mL) under argon protection, heated to 100°C and reacted for 16 hours. The reaction liquid was concentrated under reduced pressure to remove solvents, diluted with ethyl acetate (50 mL), added with a saturated aqueous solution of potassium fluoride and stirred at room temperature for 1 hour. The water phase and the organic phase were separated. The water phase was extracted with ethyl acetate (50 mL). The organic phase was combined, washed with saturated saline, dried and filtered. The filtrate was concentrated under reduced pressure to dryness. The residue was dissolved in tetrahydrofuran (50 mL), added with aqueous HCl (2M, 5 mL) and reacted at room temperature for 1 hour. The reaction liquid was diluted with ethyl acetate (100 mL), washed with saturated sodium bicarbonate and saturated saline in sequence, dried, filtered, and concentrated under reduced pressure to dryness. After column chromatography (petroleum ether/ethyl acetate =10/1) purification, a white solid of **111-4** (580 mg, yield: 92.9 %) was obtained.

LCMS: m/z: 196 $[M+H]^+$.

Step 4: sodium borohydride (110 mg, 2.97 mmol) was added into a solution of **111-4** (580 mg, 2.97 mmol) in methanol (5 mL) at 0°C and reacted at 0°C for 1 hour until the completion of reaction was monitored by LCMS. The reaction was quenched with ice water, diluted with ethyl acetate (50 mL), washed with saturated saline (3 * 20 mL), dried with anhydrous sodium sulfate and filtered. The filtrate was concentrated under reduced pressure to dryness. After column chromatography (petroleum ether/ethyl acetate =1/1) purification, a light yellow solid of **111-5** (540 mg, yield:92.3%) was obtained.

LCMS: m/z: 198 $[M+H]^+$.

Step 5: methanesulfonyl chloride (174 mg, 1.52 mmol) was added into a solution of **111-5** (100 mg, 0.51 mmol) and triethylamine (255 mg, 2.52 mmol) in dichloromethane (10 mL) at 0°C and reacted for 60 mins at room temperature. The reaction liquid was diluted with ethyl acetate (50 mL), washed with saturated saline (20 mL), dried with anhydrous sodium sulfate and filtered. The filtrate was concentrated under reduced pressure to dryness. After column chromatography (petroleum ether/ethyl acetate =2/1) purification, a light yellow solid of **111-6** (130 mg, yield:93.5%) was obtained.
LCMS: m/z: 276.1 $[M+H]^+$.
Step 6: a solution of KHMDS (1M, 0.26 mL, 0.26 mmol) in tetrahydrofuran was added dropwise into a solution of Intermediate **18-2** (80 mg, 0.18 mmol) in anhydrous tetrahydrofuran (10 mL) at -70°C under argon protection. After the end of addition, the reaction mixture was raised to -30°C ~-20°C and kept reacting for 60 mins, cooled to -70°C and added dropwise with a solution of **111-6** (130 mg, 0.47 mmol) in anhydrous tetrahydrofuran (5 mL). After the end of addition, the reaction mixture was raised to 30°C and kept reacting for 20 mins. The reaction liquid was poured into ice water and extracted with ethyl acetate (3 * 50 mL). The organic phase was combined, washed with saturated saline (100 mL), dried, filtered and concentrated. The residue was purified through column chromatography (dichloromethane/methanol=40/1) to obtain a light yellow solid of **111-7** (30 mg, yield: 26.9%).
LCMS: m/z: 633.5$[M+H]^+$
Step 6: trifluoroacetic acid (1.5 mL) was added dropwise into a solution of **111-7** (30 mg, 0.05 mmol) in dichloromethane (7.5 mL) and reacted for 60 mins at room temperature. The reaction liquid was poured slowly into a saturated solution of sodium bicarbonate (20 mL) and extracted with dichloromethane (3 * 30 mL). The organic phase was combined, washed with saturated saline (100 mL), dried with anhydrous sodium sulfate and filtered. The filtrate was concentrated under reduced pressure. The residue was separated and purified through high performance liquid chromatography to obtain Compound 111(10 mg, yield: 40.0%).
LCMS: m/z: 533.5 $[M+H]^+$
$^1$H NMR (400 MHz, methanol-d4): δ ppm 8.67 (s, 1H), 7.80 (d, *J* = 2.0 Hz, 1H), 7.26 (d, *J* = 2.0 Hz, 1H), 7.20 (s, 1H), 6.60 (d, *J* = 1.6 Hz, 1H), 6.58 (d, *J* = 1.6 Hz, 1H), 5.99~5.94 (m, 1H), 4.93 (s, 2H), 3.70~3.59 (m, 4H), 2.91~2.89 (m, 1H), 2.83 (s, 3H), 2.55~2.52 (m, 1H), 2.20 (s, 6H), 2.00~1.95 (m, 3H), 1.69 (d, *J* = 7.2 Hz, 3H), 1.51~1.54 (m, 1H).

**Example 13: Synthesis of Compound 50**

(S)-5'-((dimethylamino)methyl)-2'-(1-(4-ethoxy-5-fluoropyridine-2-yl)ethyl)-7'-((2-methyl-1H-imidazol-1-yl)methyl)-2',3'-dihydro-1'H-spiro[cyclopropan-1,4'-isoquinoline]-one

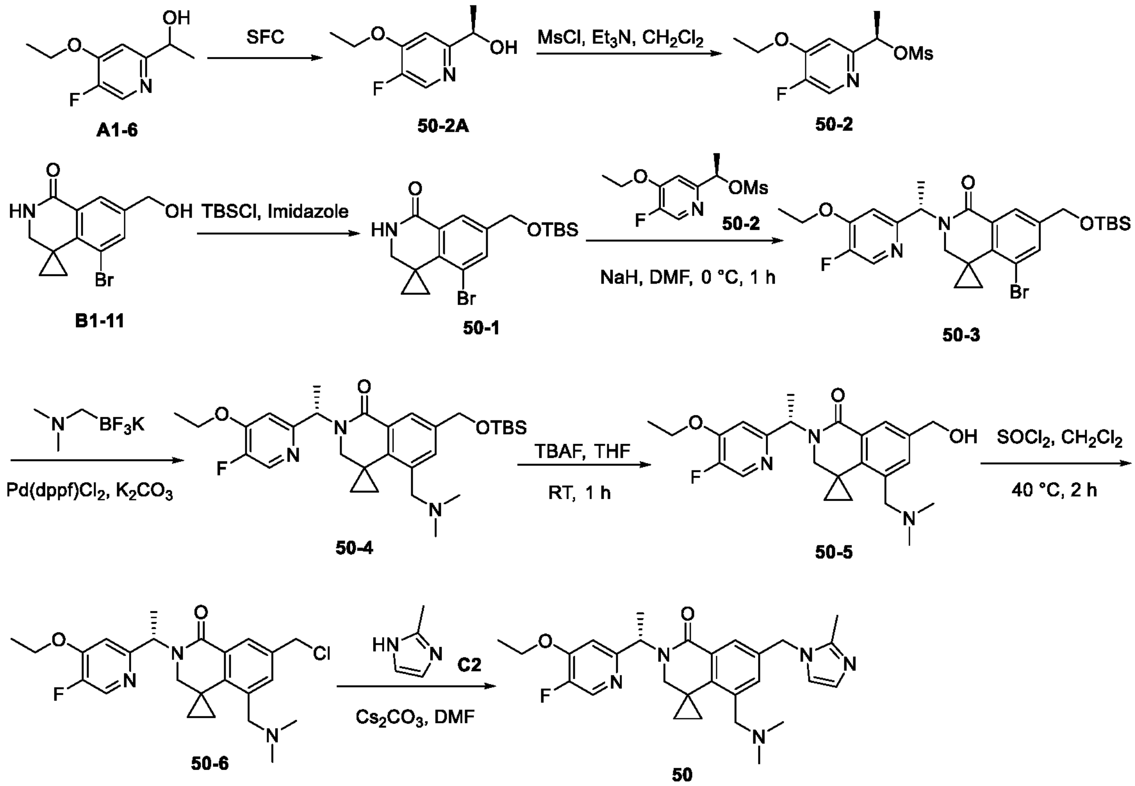


Step 1: **A1-6** was purified through SFC to obtain a light yellow solid of (R)-1-(5-fluoro-4-ethoxypyridine2-yl)ethan-1-ol (**50-2A**, 24.3 g, yield:47.6%, ee%:100%).

$^1$H NMR (400 MHz, chloroform-d): δ ppm 8.29 (d, *J* = 3.2 Hz, 1H), 7.26 (d, *J* = 7.2 Hz, 1H), 5.41 (d, *J* = 4.4 Hz, 1H), 4.69~4.63 (m, 1H), 4.25~4.17 (m, 2H), 1.39~1.33 (m, 6H).

Step 2: methanesulfonyl chloride (13.9 g, 121.5 mmol) was added dropwise into a solution of **50-2A** (15.0 g, 81.0 mmol) and triethylamine (24.6 g, 243.0 mmol) in dichloromethane (150 mL) at 0 °C under argon protection and reacted at room temperature for 2 hours. The reaction liquid was poured into ice water (500 mL) and extracted with dichloromethane (3 * 300 mL). The organic phase was combined, dried with anhydrous sodium sulfate and filtered. The filtrate was concentrated under reduced pressure to dryness. After silica gel chromatography (0 to 40% gradient, ethyl acetate: petroleum ether) purification, a yellow oil of **50-2** (16.8 g, yield: 78.8%) was obtained.

$^1$H NMR (400 MHz, chloroform-d): δ ppm 8.29 (d, *J* = 2.8 Hz, 1H), 7.07 (d, *J* = 6.8 Hz, 1H), 5.73 (q, *J* = 6.8 Hz, 1H), 4.24 (q, *J* = 6.8 Hz, 2H), 2.96 (s, 3H), 1.74 (d, *J* = 6.8 Hz, 3H), 1.50 (t, *J* = 7.2 Hz, 3H).

$^{19}$F NMR (376 MHz, chloroform-d): δ ppm -150.27.

Step 3: a solution of TBSCl (5.3 g, 35.1 mmol) in DMF (10 mL) was added dropwise into a solution of **B1-11** (3.3 g, 11.7 mmol) and imidazole (3.2 g, 46.8 mmol) in DMF (60 mL) under argon protection and reacted at room temperature for 2 hours. The reaction liquid was poured into water and extracted with ethyl acetate (3 * 150 mL). The organic phase was combined, washed with water (3 * 250 mL), saturated saline (2 * 250 mL) in sequence, dried, filtered and concentrated. The residue was purified through column chromatography (petroleum ether/ethyl acetate =15/1) to obtain a white solid of **50-1** (4.5 g, yield: 97.8%).

$^1$H NMR (400 MHz, chloroform-d): δ ppm 8.01 (d, *J*=1.6 Hz, 1H), 7.66~7.65 (m, 1H), 6.36 (s, 1H), 4.70 (s, 2H), 3.22 (d, *J*=2.8Hz, 2H), 1.97~1.94 (m, 2H), 0.94 (s, 9H), 0.93 ~ 0.91 (m, 2H), 0.10 (s, 6H).
LCMS: m/z: 398.0[M+H]$^+$

Step 4: sodium hydride (60% dispersed in oil, 925 mg, 23.2 mmol) was added into a solution of 50-1 (1.2 g, 3.03 mmol) in anhydrous DMF (35 mL) at 0°C under argon protection and kept reacting at the temperature for 30 mins. The reaction mixture was added dropwise with a solution of (R)-1-(4-ethoxy-5-fluoropyridine-2-yl) ethylmethanesulfonate **50-2** (1.53 g, 5.81 mmol) in DMF (5 mL) and kept reacting at the temperature for 1 hour. The reaction liquid was poured into ice water, extracted with ethyl acetate (3 * 80 mL). The organic phase was combined, washed with water (3 * 150 mL) and saturated saline (2 * 150 mL) in sequence, dried, filtered and concentrated. The residue was purified with column chromatography (dichloromethane/methanol=40/1) to obtain a light yellow solid of **50-3** (734 mg, yield: 43.5%).
LCMS: m/z=563.2[M+H]$^+$

Step 5: **50-3** (900 mg, 1.6 mmol) and potassium dimethylaminomethyl trifluoroborate (527 mg, 3.2 mmol) were

dissolved in dioxane (9 mL) and water (3 mL) and potassium carbonate (663 mg, 4.8 mmol) was added. The reaction mixture was purged with argon and added with [1,1'-bis(diphenylphosphino)ferrocene]palladium (II) chloride (117 mg, 0.16 mmol). Then the reaction mixture was purged with argon and reacted under microwave at 120°C for 2 hours. The reaction liquid was concentrated. The residue was purified through column chromatography (dichloromethane/methanol=40/1) to obtain a light yellow oil **50-4** (594 mg, yield: 68.7%).

LCMS: m/z=542.7[M+H]$^+$

Step 6: TBAF (1M in tetrahydrofuran, 2.5 mL, 2.50 mmol) was added into a solution of **50-4** (660 mg, 1.22 mmol) in tetrahydrofuran (30 mL) at 0 °C under argon protection and reacted at room temperature for 1 hour. The reaction liquid was diluted with ethyl acetate (500 mL). The organic phase was washed with water (5 * 300 mL) and saturated saline (250 mL), dried, filtered and concentrated. The residue was purified through column chromatography (dichloromethane/methanol=10/1) to obtain a light yellow solid of **50-5** (450 mg, yield: 86.3%).

LCMS: m/z=428.2[M+H]$^+$

Step 7: thionyl chloride (104 mg, 0.87 mmol) was added into a solution of **50-5** (53 mg, 0.12 mmol) in dichloromethane (20 mL) at 0°C.The atmosphere was vacuumed and replaced with argon three times. After the end of addition, the reaction mixture was raised to 40 °C and reacted for 2 hours. The reaction liquid was concentrated to dryness to obtain **50-6** (54 mg, yield: 97.7 %).

LCMS: m/z=446.1[M+H]$^+$

Step 8: a solution of **50-6** (54 mg, 0.12 mmol) in DMF (5 mL) and cesium carbonate (119 mg, 0.36 mmol) were added into a solution of 2-methylimidazole (**C2**, 12 mg, 0.15 mmol) in DMF (10 mL) at 0 °C. The atmosphere was vacuumed and replaced with argon three times. The reaction mixture was reacted at room temperature for 2 hours. The reaction liquid was poured slowly into ice-water (10 mL) and extracted with ethyl acetate (3 * 30 mL). The organic phase was combined, washed with saturated saline (2 * 30 mL), dried with anhydrous sodium sulfate and filtered. The filtrate was concentrated under reduced pressure. The residue was separated and purified through high performance liquid chromatography to obtain white Compound **50** (12 mg, yield: 20.3%).

LCMS: m/z: 492.2 [M+H]$^+$

$^1$H NMR (400 MHz, methanol-d4): δ ppm 8.23 (d, *J* = 3.2 Hz, 1H), 7.80 (d, *J* = 2.0 Hz, 1H), 7.16 (d, *J* = 6.8 Hz, 1H), 7.12 (d, *J* = 2.0 Hz, 1H), 7.06 (d, *J* = 1.2 Hz, 1H), 6.86 (d, *J* = 1.2 Hz, 1H), 5.96~5.91 (m, 1H), 5.19 (s, 2H), 4.23~4.15 (m, 2H), 3.44 (d, *J* = 12.4 Hz, 1H), 3.36 (d, *J* = 13.2 Hz, 1H), 3.44 (d, *J* = 12.4 Hz, 1H), 2.96 (d, *J* = 13.2 Hz, 1H), 2.30 (s, 3H), 2.11 (s, 6H), 1.60~1.54 (m, 4H), 1.42 (t, *J* = 7.2 Hz, 3H), 1.10~1.05 (m, 1H), 0.88~0.83 (m, 1H), 0.44~0.40 (m, 1H).

$^{19}$F NMR (376 MHz, methanol-d4): δ ppm -154.04.

The following compounds were synthesized by the same method or modified method using the corresponding intermediates and synthesis route with reference to the synthesis method for Compound 50 of Example 13. If the final product needed deprotecting the protective N-Boc group, the following conditions were optional for deprotection: 1) reacted with a solution of 4 N HCl/1,4-dioxane/methanol (1/1) at room temperature or stirred under heating until the completion of reaction, the reaction liquid was concentrated and separated by preparative HPLC to obtain the target product; 2) reacted with a solution of trifluoroacetic acid/dichloromethane (1/10) at room temperature until the completion of reaction, the reaction liquid was concentrated and separated by preparative HPLC to obtain the target product.

The following Examples refer to free compounds or pharmaceutically acceptable salts thereof.

| No. | Starting Intermediate and material; reference step | Compound structure | Chinese name | Data analysis |
|---|---|---|---|---|
| Compound **51** | 50-6, C3, Step 8 of Example 13 | 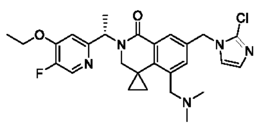 | (S)-7'-((2-chloro-1H-imidazol-1-yl)methyl)-5'-((dimethylamino)methyl)-2'-(1-(4-ethoxy-5-fluoropyridine-2-yl)ethyl)-2',3'-dihydro-1'H-spiro[cyclopropan-1,4'-isoquinoline]-1'-one | LCMS: m/z: 512.2 [M+H]$^+$. $^1$H NMR (400 MHz, methanol-$d_4$): δ ppm 8.23 (d, *J* = 3.2 Hz, 1H), 7.88 (s, 1H), 7.27 (s, 1H), 7.21 (s, 1H), 7.16 (d, *J* = 6.8 Hz, 1H), 6.96 (s, 1H), 5.97~5.91 (m, 1H), 5.24 (s, 2H), 4.23~4.16 (m, 2H), 3.46 (d, *J* = 12.8 Hz, 1H), 3.36~3.30 (m, 2H), 2.96 (d, *J* = 13.2 Hz, 1H), 2.13 (s, 6H), 1.61~1.54 (m, 4H), 1.42 (t, *J* = 6.8 Hz, 3H), 1.10~1.05 (m, 1H), 0.90~0.85 (m, 1H), 0.46~0.41 (m, 1H). |

(continued)

| No. | Starting Intermediate and material; reference step | Compound structure | Chinese name | Data analysis |
|---|---|---|---|---|
| Compound **103** | 50-6, C4, Step 8 of Example 13 | 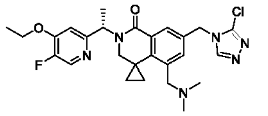 | 7'-((3-chloro-4H-1,2,4-triazole-4-yl)methyl)-5'-((dimethylamino) methyl)-2'-(1-(4-ethoxy-5-fluoropyridine) -2-yl) ethyl)-2',3'-dihydro-1'H-spiro [cyclopropan-1,4'-isoquinoline]-1'-one | LCMS: m/z: 513.2 [M+H]$^+$. $^1$H NMR (400 MHz, methanol-$d_4$): δ ppm 8.52 (s, 1H), 8.23 (d, *J* = 3.2 Hz, 1H), 7.95 (d, *J* = 2.0 Hz, 1H), 7.35 (d, *J* = 2.0 Hz, 1H), 7.16 (d, *J* = 6.8 Hz, 1H), 5.97~5.92 (m, 1H), 5.38 (s, 2H), 4.23~4.15 (m, 2H), 3.47 (d, *J* = 12.8 Hz, 1H), 3.35~3.27 (m, 2H), 2.97 (d, *J* = 12.8 Hz, 1H), 2.14 (s, 6H), 1.61~1.54 (m, 4H), 1.42 (t, *J* = 6.8 Hz, 3H), 1.11~1.06 (m, 1H), 0.90~0.85 (m, 1H), 0.47~0.42 (m, 1H). |
| Compound **104** | 50-6, C5, Step 8 of Example 13 | 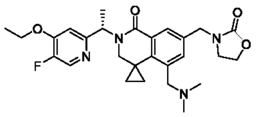 | 3-((5'-((dimethylamino) methyl)-2'-(1-(4-ethoxy-5-fluoropyridine-2-yl) ethyl)-1'-oxo-2',3'-dihydro-1'H-spiro [cyclopropan-1,4'-isoquinoline]-7'-yl) methyl)oxazol-2-one | LCMS: m/z: 497.3 [M+H]$^+$ . $^1$H NMR (400 MHz, methanol-$d_4$): δ ppm 8.24 (d, *J* = 3.2 Hz, 1H), 7.94 (d, *J* = 2.0 Hz, 1H), 7.30 (d, *J* = 1.6 Hz, 1H), 7.18 (d, *J* = 7.2 Hz, 1H), 5.99~5.94 (m, 1H), 4.44 (s, 2H), 4.37~4.32 (m, 2H), 4.24~4.17 (m, 2H), 3.53~3.44 (m, 3H), 3.37~3.34 (m, 2H), 2.98 (d, *J* = 12.8 Hz, 1H), 2.15 (s, 6H), 1.62~1.55 (m, 4H), 1.43 (t, *J* = 7.2Hz,3H), 1.11~1.06 (m, 1H), 0.90~0.85 (m, 1H), 0.46~0.41 (m, 1H). |
| Compound **105** | 50-6, C6, Step 8 of Example 13 | 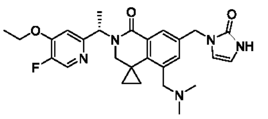 | (S)-5'-((dimethylamino) methyl)-2'-(1-(4-ethoxy-5-fluoropyridine-2-yl) ethyl)-7'-((2-oxo-2,3-dihydro-1H-imidazole -1-yl)methyl)-2',3'-dihydro-1'H-spiro [cyclopropan-1,4'-isoquinoline]-1'-one | LCMS: m/z: 494.4 [M+H]$^+$ . $^1$H NMR (400 MHz, methanol-$d_4$): δ ppm 8.23 (d, *J* = 3.2 Hz, 1H), 7.92 (d, *J* = 2.0 Hz, 1H), 7.25 (d, *J* = 2.0 Hz, 1H), 7.16 (d, *J* = 7.2 Hz, 1H), 6.42~6.39 (m, 2H), 5.97~5.92 (m, 1H), 4.81 (s, 2H), 4.23~4.14 (m, 2H), 3.45 (d, *J* = 12.4 Hz, 1H), 3.35 (d, *J* = 13.2 Hz, 1H), 3.29 (d, *J* = 13.2 Hz, 1H), 2.95 (d, *J* = 13.2 Hz, 1H), 2.12 (s, 6H), 1.61~1.54 (m, 4H), 1.42 (t, *J* = 6.8 Hz, 3H), 1.09~1.02 (m, 1H), 0.88~0.82 (m, 1H), 0.44~0.39 (m, 1H). |

(continued)

| No. | Starting Intermediate and material; reference step | Compound structure | Chinese name | Data analysis |
|---|---|---|---|---|
| Compound **106** | 50-6, C7, Step 8 of Example 13 | 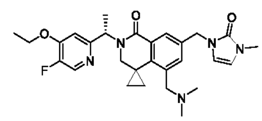 | (S)-5'-((dimethylamino) methyl)-2'-(1-(4-ethoxy-5-fluoropyridine-2-yl) ethyl)-7'-((3-methyl-2-oxo-2,3-dihydro -1H-imidazol-1-yl)methyl)-2',3'-dihydro-1'H-spiro [cyclopropan-1,4'-isoquinoline]-1'-one | LCMS: m/z: 508.2 $[M+H]^+$ . $^1H$ NMR (400 MHz, methanol-$d_4$): δ ppm 8.23 (d, *J* = 3.2 Hz, 1H), 7.89 (d, *J* = 2.0 Hz, 1H), 7.25 (d, *J* = 2.0 Hz, 1H), 7.16 (d, *J* = 6.8 Hz, 1H), 6.47~6.45 (m, 2H), 5.97~5.92 (m, 1H), 4.82 (s, 2H), 4.23~4.15 (m, 2H), 3.45 (d, *J* = 12.4 Hz, 1H), 3.35 (d, *J* = 13.2 Hz, 1H), 3.29-3.27 (m, 4H), 2.95 (d, *J* = 12.4 Hz, 1H), 2.13 (s, 6H), 1.60~1.54 (m, 4H), 1.42 (t, *J* = 6.8 Hz, 3H), 1.09~1.04 (m, 1H), 0.88~0.82 (m, 1H), 0.44~0.39 (m, 1H). |
| Compound **108** | B2-8, A3, C8, Step 3 to Step 8 of Example 13 | 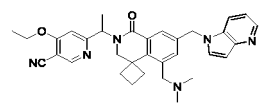 | 6-(1-(7'-((1Hpyrrol[3,2-b]pyridine-1-yl)methyl)-5'-((dimethylamino) methyl)-1'-oxo-1'H-spiro[cyclobutan-1,4'-isoquinoline]-2'(3'H)-yl) ethyl)-4-ethoxynicotinonitrile | LCMS: m/z: 549.4 $[M+H]^+$ . $^1H$ NMR (400 MHz, methanol-d4) δ ppm: 8.64 (s, 1H), 8.32~8.31 (m, 2H), 7.89~7.87 (m, 2H), 7.73 (d, *J* = 3.2 Hz, 1H), 7.42 (s, 1H), 7.19~7.16 (m, 2H), 6.68 (d, *J* = 3.2 Hz, 1H), 5.92 (q, *J* =7.2 Hz, 1H), 5.49 (s, 2H), 4.31 -4.23 (m, 2H), 4.02 (s, 2H), 3.71~3.59 (m, 2H), 2.77~2.70 (m, 1H), 2.41~2.36 (m, 7H), 2.06~1.98 (m, 3H), 1.67 (d, *J* = 7.2 Hz, 2H), 1.62~1.56 (m, 2H), 1.44 (t, *J* = 6.8 Hz, 3H). |
| Compound **114** | 50-6, C9, Step 8 of Example 13 | 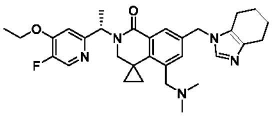 | (S)-5'-((dimethylamino) methyl)-2'-(1-(4-ethoxy-5-fluoropyridine-2-yl) ethyl)-7'-((4,5,6,7-tetrahydro-1H-benzo[d] imidazol-1-yl)methyl)-2',3'-dihydro-1'H-spiro [cyclopropan-1,4'-isoquinoline]-1'-one | LCMS: m/z: 532.4 $[M+H]^+$ . $^1H$ NMR (400 MHz, methanol-$d_4$): δ ppm 8.46 (s, 1 H), 8.22(d, *J* = 3.2 Hz, 1H), 7.81(s, 1H), 7.69(s, 1H), 7.15(d, *J* = 7.2 Hz, 1H), 7.12(s, 1H), 5.97~5.91 (m, 1H), 5.16 (s, 2H), 4.29~4.18 (m, 2H), 3.47~3.30 (m, 3H), 2.97~2.94 (m, 1H), 2.53(s, 2H), 2.37(s, 2H), 2. 13(s, 6H), 1.76(s, 4H), 1.60~1.54 (m, 3H), 1.42(1, *J* = 7.2 Hz, 3H), 1.08~1.06 (m, 1H), 0.89~0.86 (m, 2H), 0.45~0.43 (m, 1H). |

(continued)

| No. | Starting Intermediate and material; reference step | Compound structure | Chinese name | Data analysis |
|---|---|---|---|---|
| Compound **117** | A13, B2, 1-2, C2, Step 3 to Step 8 of Example 13 | 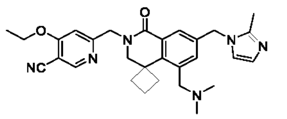 | 6-((5'-((dimethylamino)methyl)-7'-((2-methyl-1H-imidazol-1-yl)methyl)-1'-oxo-1'H-spiro[cyclobutan-1,4'-isoquinoline]-2'-3'H-yl)methyl)-4-ethoxynicotinonitrile | LCMS: m/z: 499.2 $[M+H]^+$ . $^1H$ NMR (400 MHz, methanol-d4) δ ppm: 8.62 (s, 1H), 8.33 (br, 1H), 7.82 (d, *J* = 1.6 Hz, 1H), 7.51 (d, *J* = 1.6 Hz, 1H), 7.33 (d, *J* = 0.8 Hz, 1H), 7.23~7.22 (m, 2H), 5.33 (s, 2H), 4.89 (s, 2H), 4.30 (q, *J* = 6.8 Hz, 2H), 3.95 (s, 2H), 3.82 (s, 2H), 2.76~2.69 (m, 2H), 2.51 (s, 3H), 2.38 (s, 6H), 2.15~1.99 (m, 4H), 1.47 (t, *J* = 6.8 Hz, 3H). |

**Example 14: Synthesis of Compound 118**

4-ethoxy-6-(1-(7'-((2-(methylamino)-1H-imidazol-1-yl)methyl)-5'-((methylamino)methyl)-1'-oxo-1'H-spiro[cyclobutan-1,4'-isoquinoline]-2'(3'H)-yl)ethyl)nicotinonitrile

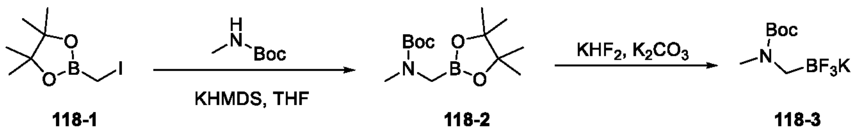


118-1 118-2 118-3

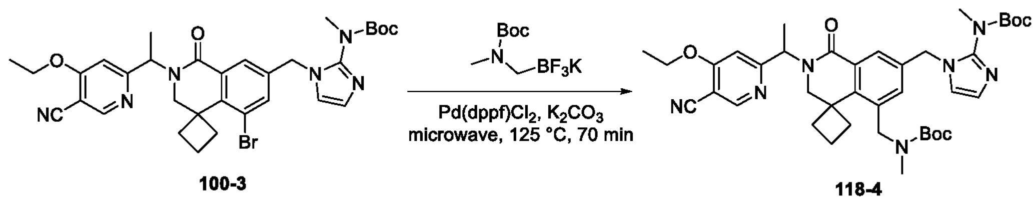


100-3 118-4

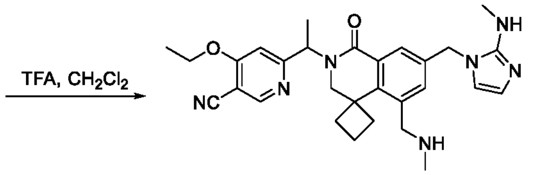


118

Step 1: a solution of potassium bis(trimethylsilyl)amide (1 mol/L, 5.4 mL, 5.40 mmol) in tetrahydrofuran was added dropwise into a solution of tert-butyl methyl carbamate (700 mg, 5.34 mmol) in tetrahydrofuran (10 mL) at -60 °C under argon protection and stirred at -60 °C for 30 min. A solution of **118-1** (1.43 g, 5.34 mmol) in tetrahydrofuran was added dropwise at -60 °C, stirred for 10 mins, then stirred at room temperature overnight. The reaction was quenched with a saturated aqueous solution of ammonium chloride, extracted with ethyl acetate and concentrated to obtain red oil of **118-2** (700 mg, yield: 48.4%).

LCMS: m/z: 272 $[M+H]^+$.

Step 2: potassium hydrogen fluoride (1.04 g, 13.41 mmol) was added into a solution of **118-2** (700 mg, 2.58 mmol) and potassium carbonate (0.37 g, 2.68 mmol) in acetone/water (15 mL /5 mL) at 0 °C and reacted at room temperature for 2 hours. The reaction liquid was concentrated under reduced pressure to remove solvents. The residue was dissolved with acetone (50 mL) and filtered. The filtrate was concentrated. The crude product was added with ethyl ether, stirred for 10 mins and filtered. The filter cake was washed with ethyl ether to obtain a white solid of **118-3** (650

mg, yield: 100%).

Step 3: **118-3** (290 mg, 1.16 mmol), **100-3** (150 mg, 0.23 mmol), [1,1'-bis(diphenylphosphino)ferrocene]palladium (II) chloride (170 mg, 0.23 mmol) and potassium carbonate (320 mg, 2.31 mmol) were reacted in a solution of dioxane/water (6 mL/2 mL) under microwave at 125 °C for 70 mins. The reaction liquid was added with ethyl acetate and water. The organic phase was washed with saturated saline, dried with anhydrous sodium sulfate and filtered. The filtrate was concentrated under reduced pressure to dryness. After column chromatography (dichloromethane/methanol=50/1) purification and C18(water/methanol=7/3) purification, a yellow solid of **118-4** (65 mg, yield:39.6%) was obtained.

LCMS: m/z: 714 $[M+H]^+$.

Step 4: trifluoroacetic acid (1 mL) was added into a solution of **118-4** (65 mg, 0.09 mmol) in dichloromethane (2 mL) at 0 °C and reacted at ambient temperature for 1 hour. The reaction liquid was diluted with dichloromethane (10 mL), added with a saturated aqueous solution of sodium bicarbonate and stirred at room temperature for 10 mins. The water phase and the organic phase were separated. The water phase was extracted with dichloromethane (3 * 20 mL). The organic phase was combined, washed with saturated saline, dried with anhydrous sodium sulfate and filtered. The filtrate was concentrated under reduced pressure to dryness. After high performance liquid chromatography purification, a white formate salt of Compound **118** (4.65 mg, yield: 9.1%) was obtained.

LCMS: m/z: 514 $[M+H]^+$ .

$^1H$ NMR (400 MHz, methanol-d4): δ ppm 8.67 (s, 1H), 8.49 (br, 1H), 7.79 (d, *J* = 2.0 Hz, 1H), 7.39 (d, *J* = 1.6 Hz, 1H), 7.20 (s, 1H), 6.66 (s, 1H), 6.64 (s, 1H), 5.95 (q, *J* = 6.8 Hz, 1H), 4.96 (s, 2H), 4.34~4.26 (m, 2H), 4.03~3.96 (m, 2H), 3.71~3.60 (m, 2H), 2.86 (s, 3H), 2.70~2.66 (m, 1H), 2.48 (s, 3H), 2.44~2.36 (m, 1H), 2.11~2.02 (m, 3H), 1.70~1.61 (m, 4H), 1.46 (t, *J* = 6.8 Hz, 3H).

**Example 15: Synthesis of Compound 110**

**(S)-2'-(1-(4-ethoxy-5-fluoropyridine-2-yl)ethyl)-N, N-dimethyl-7'-(2-(methylamino)-1H-imidazol-1-yl)methyl)-1'-oxo-2',3'-dihydro-1'H-spiro[cyclopropan-1,4'-isoquinoline]-5'-formamide**

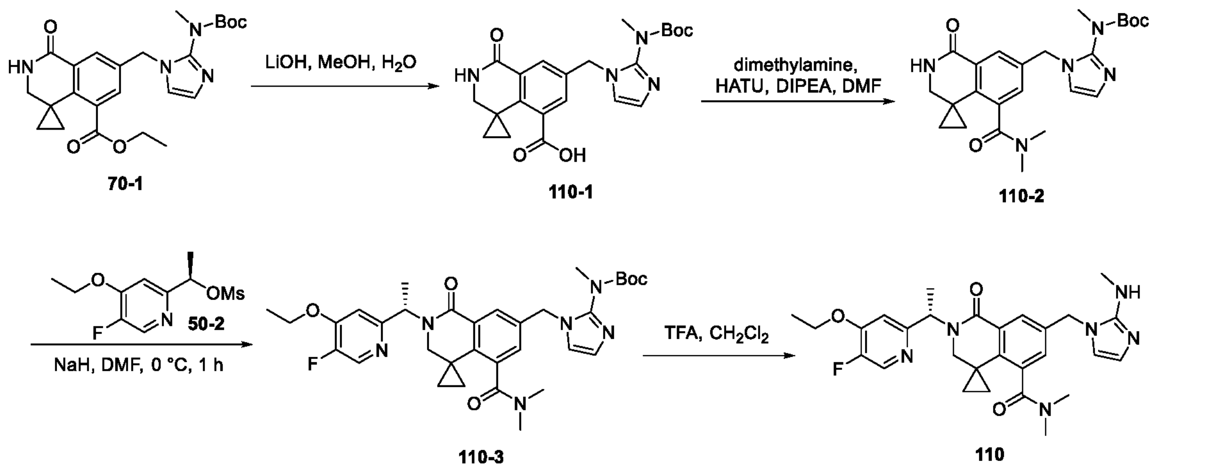


70-1 110-1 110-2 110-3 110

Step 1: lithium hydroxide (22 mg, 0.91 mmol) was added into a solution of **70-1** in methanol (10 mL) and water (10 mL), heated to 65 °C and reacted overnight. The reaction liquid was concentrated to remove methanol. The residue was adjusted to pH 5-6 with potassium bisulfate and concentrated under reduced pressure to remove water. The residue was dissolved in dichloromethane/methanol (10/1, 100 mL), filtered and concentrated. The residue was purified through column chromatography (dichloromethane/methanol=5/1) to obtain an off-white solid of **110-1** (120 mg, yield:62.2%).

Step 2: a solution of dimethylamine in tetrahydrofuran (2M, 2 mL, 4 mmol) and HATU (200 mg, 0.52 mmol) was added into a solution of **110-1**(110 mg, 0.26 mmol) and N,N-diisopropylethanamine (100 mg, 0.78 mmol) in DMF(10 mL) and reacted at room temperature for 2 hours. The reaction liquid was concentrated. The residue was purified through column chromatography (dichloromethane/methanol=20/1) to obtain a light yellow solid of **110-2** (50 mg, yield: 42.4%). LCMS: m/z: 454.2$[M+H]^+$

Step 3: sodium hydride (60% dispersed in oil, 18 mg, 0.45 mmol) was added into **110-2** (50 mg, 0.11 mmol) in anhydrous DMF (10 mL) at 0 °C under argon protection and kept reacting at this temperature for 30 mins after the end of addition. A solution of **50-2** (56 mg, 0.22 mmol) in anhydrous DMF (3 mL) was added dropwise. After the end of

addition, the reaction mixture was kept reacting at this temperature for 1 hour. The reaction liquid was quenched with a saturated aqueous solution of ammonium chloride and extracted with ethyl acetate (3 * 50 mL). The organic phase was combined, washed with water (3 * 100 mL) and saturated saline (2 * 100 mL), dried, filtered and concentrated. The residue was purified through column chromatography (dichloromethane/methanol=10/1) to obtain a light yellow solid of **110-3** (30 mg, yield:44.1%).

LCMS: m/z: 621.5[M+H]$^+$

Step 4: trifluoroacetic acid (2 mL) was added into a solution of **110-3** (30 mg, 0.05 mmol) in dichloromethane (10 mL). After the end of addition, the reaction mixture was reacted at room temperature for 1 hour. The reaction liquid was poured into a saturated aqueous solution of sodium bicarbonate and extracted with dichloromethane/methanol (20/1, 3 * 50 mL). The organic phase was combined, washed with saturated saline (100 mL), dried, filtered and concentrated. The residue was purified through high performance liquid chromatography to obtain Compound **110** (16 mg, yield:64.0 %).

LCMS: m/z: 521.3[M+H]$^+$

$^1$H NMR (400 MHz, methanol-d4): δ ppm 8.24~8.21 (m, 1H), 7.95~7.94 (m, 1H), 7.16 (d, *J* = 6.8 Hz, 1H), 7.01-7.00 (m, 1H), 6.65 (d, *J* = 1.6 Hz, 1H), 6.60 (d, *J* = 1.6 Hz, 1H), 5.99~5.94 (m, 1H), 4.99 (s, 2H), 4.24~4.15 (m, 2H), 3.66 (d, *J* = 12.4 Hz, 0.5H), 3.30 (d, *J* = 16.8 Hz, 0.5H), 3.15 (d, *J* = 13.2 Hz, 0.5H), 3.06 (d, *J* = 2.0 Hz, 3H), 2.90 (d, *J* = 12.8 Hz, 0.5H), 2.84 (d, *J* = 1.2 Hz, 4.5H), 2.78 (s, 1.5H), 1.56 (t, *J* = 6.8 Hz, 3H), 1.45~1.40 (m, 3H), 1.25~1.20 (m, 0.6H), 1.03~0.98 (m, 1H), 0.94~0.91 (m, 0.4H), 0.84~0.79 (m, 0.5H), 0.76~0.72 (m, 0.5H), 0.65~0.62 (m, 0.5H), 0.42~0.37 (m, 0.5H).

$^{19}$F NMR (376 MHz, methanol-d4): δ ppm -153.96, -154.14.

**Example 16: Synthesis of Compound 120, 121**

**Compound 120:**(S)-4-ethoxy-6-(1-(5'-(methoxymethyl)-7'-((2-(methylamino)-1H-imidazol-1-yl)methyl)-1'-oxo-1'H-spiro[cyclobutan-1,4'-isoquinoline]-2'(3'H)-yl)ethyl)nicotinonitrile

**Compound 121:**(R)-4-ethoxy-6-(1-(5'-(methoxymethyl)-7'-((2-(methylamino)-1H-imidazol-1-yl)methyl)-1'-oxo-1'H-spiro[cyclobutan-1,4'-isoquinoline]-2'(3'H)-yl)ethyl)nicotinonitrile

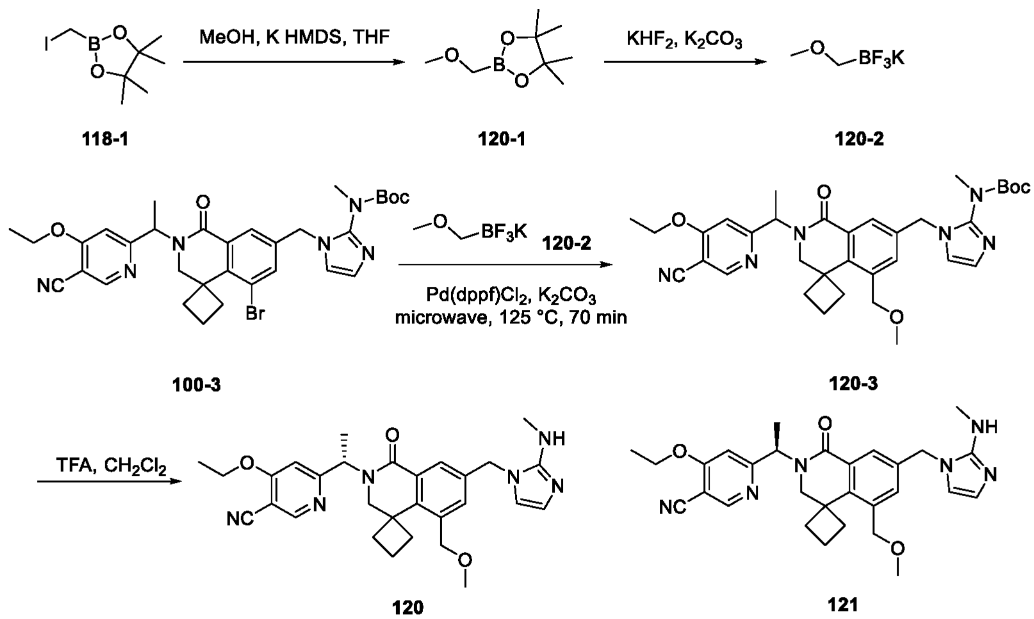


118-1 120-1 120-2

100-3 120-3

120 121

Step 1: a solution of KHMDS (1 mol/L, 9.4 mL, 9.40 mmol) in tetrahydrofuran was added into a solution of methanol (0.3 g, 9.38 mmol) in tetrahydrofuran (10 mL) at -60 °C under argon protection, stirred at -60 °C for 30 mins, added dropwise with a solution of **118-1** (2.5 g, 9.38 mmol) in tetrahydrofuran at -60°C, stirred at -60 °C for 10 mins, then reacted at room temperature overnight. The reaction was quenched with a saturated aqueous solution of ammonium chloride. The reaction liquid was extracted with ethyl acetate (50 mL * 2), dried with anhydrous sodium sulfate and filtered. The filtrate was concentrated under reduced pressure to obtain a colorless oil of **120-1** (1.6 g, yield:99.7%).

Step 2: potassium hydrogen fluoride (3.6 g, 46.51 mmol) was added into a solution of **120-1** (1.6 g, 9.30 mmol) and potassium carbonate (1.3 g, 9.30 mmol) in acetone/water (30/10 mL) at 0 °C and reacted at room temperature for 2 hours. The reaction liquid was concentrated under reduced pressure to remove solvents. The residue was dissolved with acetone (50 mL) and filtered. The filtrate was concentrated. The crude product was added with ethyl ether, stirred for 10 mins and filtered. The filter cake was washed with ethyl ether to obtain a white solid of **120-2** (600 mg, yield:42.5%).

Step 3: **120-2** (93 mg, 0.62 mmol), **100-3** (80 mg, 0.12 mmol), [1,1'-bis(diphenylphosphino)ferrocene]palladium (II) chloride (27 mg, 0.04 mmol) and potassium carbonate (85 mg, 0.62 mmol) were reacted in a solution of dioxane/water (6 mL/2 mL) under microwave at 115 °C for 70 mins. The reaction liquid was added with ethyl acetate (50 mL) and water (20 mL). The organic phase was washed with saturated saline and dried with anhydrous sodium sulfate. After column chromatography (dichloromethane/methanol=50/1) purification and C18 (water/methanol=65/35) purification, a yellow solid of **120-3** (70 mg, yield:92.5 %) was obtained.

LCMS: m/z: 615 $[M+H]^+$.

Step 4: TFA (1 mL) was added into a solution of **120-3** (70 mg, 0.11 mmol) in dichloromethane (2 mL) at 0 °C and reacted at ambient temperature for 1 hour. The reaction liquid was diluted with dichloromethane (10 mL), added with a saturated aqueous solution of sodium bicarbonate and stirred at room temperature for 10 mins. The water phase and the organic phase were separated. The water phase was extracted with dichloromethane (3 * 20 mL). The organic phase was combined, washed with saturated saline, dried with anhydrous sodium sulfate and filtered. The filtrate was concentrated under reduced pressure to dryness. The residue was purified through high performance liquid chromatography, then purified with SFC to obtain Compound **120** (2 mg, yield:3.4%) and Compound **121** (4 mg, yield: 6.8%).

Compound 120:

LCMS: m/z: 515.1 $[M+H]^+$ .

$^1H$ NMR (400 MHz, methanol-d4): δ ppm 8.67 (s, 1H), 7.82 (d, *J* = 1.6 Hz, 1H), 7.35 (d, *J* = 2.0 Hz, 1H), 7.21 (s, 1H), 6.64~6.62 (m, 2H), 5.96 (q, *J* = 7.2 Hz, 1H), 4.96 (s, 2H), 4.70~4.64 (m, 2H), 4.33~4.27 (m, 2H), 3.72~3.60 (m, 2H), 3.41 (s, 3H), 2.86 (s, 3H), 2.79~2.74 (m, 1H), 2.47~2.39 (m, 1H), 2.04~1.98 (m, 3H), 1.69 (d, *J* = 7.2 Hz, 3H), 1.59~1.56 (m, 1H), 1.46 (t, *J* = 6.8 Hz, 3H).

Compound 121:

LCMS: m/z: 515.1 $[M+H]^+$ .

$^1H$ NMR (400 MHz, methanol-$d_4$): δ ppm 8.67 (s, 1H), 7.82 (d, *J* = 2.0 Hz, 1H), 7.35 (d, *J* = 2.0 Hz, 1H), 7.21 (s, 1H), 6.63~6.61 (m, 2H), 5.96 (q, *J* = 7.2 Hz, 1H), 4.95 (s, 2H), 4.70~4.64 (m, 2H), 4.35~4.24 (m, 2H), 3.72~3.60 (m, 2H), 3.41 (s, 3H), 2.85 (s, 3H), 2.80~2.73 (m, 1H), 2.47~2.39 (m, 1H), 2.05~1.98 (m, 3H), 1.69 (d, *J* = 7.2 Hz, 3H), 1.60~1.57 (m, 1H), 1.46 (t, *J* = 7.2 Hz, 3H).

The biological function of the compound according to the present disclosure has been demonstrated in WDR5-MLL PPI activity and cell level testing. For example, in the WDR5-MLL PPI inhibitory activity test, the compound according to the present disclosure can achieve strong inhibitory activity (IC50<100nM, up to 10nM or less). At the cellular level, the compound according to the present disclosure also exhibits good activity to inhibit the proliferation of cancer cells, and good proliferation inhibitory activity on MV4-11 cell lines (IC50<200 nM, up to 10 nM or less). Moreover, the compound according to the present disclosure has weak inhibitory activity on Jurkat cells, indicating that it has good selectivity (more than 300 times). Further, the compound according to the present disclosure has good pharmacokinetic properties and has a good effect in terms of tissue distribution, half-life, in vivo clearance and oral bioavailability.

## Test Example 1: WDR5-MLL PPI inhibitory activity test

Effect of the compounds on WDR5 (WD Repeat Domain 5) and MLL1 (mixed-lineage leukemia 1) peptides at molecular level.

The effect of the compound on the interaction of WDR5 protein (22-334aa) with MLL1 polypeptide (3672-3773aa) was detected by HTRF (Homogeneous Time-Resolved Fluorescence) technology. When the N-terminal His-tagged WDR5 recognized by the Anti-6His-Eu cryptate donor interacts with the biotin-tagged MLL1 polypeptide recognized by the streptavidin-APC receptor, the donor and acceptor are pulled close enough to produce fluorescence resonance energy transfer (FRET). When the compound inhibits the interaction of WDR5 with MLL1 polypeptide, FRET no longer occurs in the donor and acceptor. Therefore, the decrease in FRET signal can reflect the compound's inhibition on the interaction of WDR5 with MLL1 polypeptide.

The compound to be tested was diluted 3-fold with DMSO. A total of 10 concentration gradients were set with 2 replicates at each concentration. Then the compound was diluted with reaction buffer (25mM HEPES pH=7.5, 100mM NaCl, 0.05% Tween 20) to control the final concentration of DMSO at 0.5%. In a 384-well plate, 1 μL of compound and

3 μL of 42 nMWDR5 protein were added, centrifuged at 1000 rpm for 1 min, incubated at room temperature for 60 min, followed by adding 3 μL of 333 nM MLL1 polypeptide with incubation at room temperature for 10 min, and followed by adding 3 μL of a mixed liquid of Anti-6His-Eu cryptate and streptavidin-APC with centrifugation at 1000 rpm for 1 min and incubation at room temperature protected from light for 60 min, using a multifunction microplate reader (EnVision® 2105) to read the signal. The following controls were set up per plate: control group (1 μL with 5% DMSO reaction buffer, 3 μL of 42 nM WDR5, 3 μL of MLL1 polypeptide, 3 μL of Anti-6His-Eu cryptate and streptavidin-APC). Blank group (1 μL reaction buffer with 5% DMSO, 3 μL reaction buffer, 3 μL MLL1 polypeptide, 3 μL Anti-6His-Eu cryptate and streptavidin-APC). The test data was calculated according to the signal ratio of two beams of fluorescence at different wavelengths: (665nm signal/620nm signal) * 10000. The calculation formula of the inhibition rate is: inhibition rate (%) = 100 - (experimental group - blank group)/(control group - blank group) * 100. The IC50 value was calculated using XLfit software. The results are shown in Table 1.

Table 1

| Compound No. | WDR5-MLL PPI inhibitory activity | Compound No. | WDR5-MLL PPI inhibitory activity | Compound No. | WDR5-MLL PPI inhibitory activity |
|---|---|---|---|---|---|
| Compound 1 | A | Compound 64 | A | Compound 89 | A |
| Compound 2 | A | Compound 65 | A | Compound 90 | A |
| Compound 3 | A | Compound 66 | A | Compound 91 | A |
| Compound 4 | A | Compound 67 | A | Compound 92 | A |
| Compound 5 | A | Compound 68 | A | Compound 93 | A |
| Compound 7 | A | Compound 69 | A | Compound 94 | A |
| Compound 8 | A | Compound 70 | A | Compound 95 | A |
| Compound 10 | A | Compound 71 | A | Compound 96 | A |
| Compound 14 | A | Compound 72 | A | Compound 97 | A |
| Compound 18 | A | Compound 73 | A | Compound 98 | A |
| Compound 19 | A | Compound 74 | A | Compound 99 | A |
| Compound 19 | A | Compound 75 | A | Compound 100 | A |
| Compound 23 | A | Compound 76 | B | Compound 101 | A |
| Compound 37 | A | Compound 77 | A | Compound 102 | B |
| Compound 41 | B | Compound 78 | A | Compound 107 | A |
| Compound 43 | A | Compound 79 | A | Compound 108 | B |
| Compound 49 | A | Compound 80 | A | Compound 110 | A |

(continued)

| Compound No. | WDR5-MLL PPI inhibitory activity | Compound No. | WDR5-MLL PPI inhibitory activity | Compound No. | WDR5-MLL PPI inhibitory activity |
|---|---|---|---|---|---|
| Compound 55 | A | Compound 81 | A | Compound 111 | A |
| Compound 57 | A | Compound 82 | A | Compound 113 | A |
| Compound 58 | A | Compound 83 | A | Compound 114 | A |
| Compound 59 | A | Compound 84 | A | Compound 115 | B |
| Compound 60 | A | Compound 85 | A | Compound 117 | A |
| Compound 61 | A | Compound 86 | A | Compound 119 | B |
| Compound 62 | A | Compound 87 | A | | |
| Compound 63 | A | Compound 88 | A | | |

where the letter A represents that IC50 is less than 20 nM; the letter B represents that IC50 is from 20 nM to 100 nM; and the letter C represents that IC50 is greater than 100 nM.

**Test Example 2: MV4-11 cell (ATCC: CRL-9591) proliferation inhibition experiment**

ATP in living cells was quantitatively detected by CellTiter-Glo (Promega-G7573) Luminescence Cell Viability Assay Kit to determine the effect of the compounds to be tested on cell proliferation.

Step 1: MV4-11 cells were seeded in 96-well plates (Greiner-655098) at a density of 1,500 cells per well with a volume of 140 μL per well; Cell culture medium components included RPMI1640 (Gibco-A10491-01) +10% FBS (Gibco-10099-141C) +1% Penicillin-Streptomycin (5,000 U/mL, Gibco-15070-063).

Step 2: On the day of cell seeding, the cells were treated with the compound to be tested. The compound to be tested was diluted with a 3-fold gradient, and a total of 9 concentration gradients were set. DMSO (Sigma, D2650) with 0.5% final concentration or a DMSO solution containing the compound to be tested was added per well with 2 or 3 replicate wells at each concentration point. The plates were incubated at 37°C in a 5% $CO_2$ incubator (Thermo Scientific Steri-Cycle 371) for 6 days.

Step 3: The cell viability in the control and treatment groups was detected with CellTiter-Glo Luminescent Cell Viability Assay Kit. 50 μL of CellTiter-Glo was added per well, mixed well, and incubated for 15 min at room temperature protected from light. According to the kit instructions, the chemiluminescence signal was read using a multifunctional microplate reader (EnVision, PerkinElmer).

The inhibition percentage (%) of the compound at each concentration point was calculated by the following formula:

Inhibition Percentage (%) = (1- signal value of the group treated with the compound /signal value of the group treated with DMSO) * 100.

The value of cell proliferation semi-inhibitory concentration $IC_{50}$ was calculated using GraphPad Prism software, and the results are shown in Table 2.

Table 2

| Compound No. | cell proliferation inhibitory activity | Compound No. | cell proliferation inhibitory activity | Compound No. | cell proliferation inhibitory activity |
|---|---|---|---|---|---|
| Compound 1 | B | Compound 63 | B | Compound 85 | A |
| Compound 2 | B | Compound 64 | B | Compound 87 | A |
| Compound 3 | A | Compound 66 | A | Compound 88 | A |
| Compound 4 | A | Compound 67 | A | Compound 89 | A |
| Compound 5 | A | Compound 68 | B | Compound 91 | A |
| Compound 8 | B | Compound 70 | A | Compound 92 | B |
| Compound 10 | B | Compound 71 | A | Compound 93 | A |
| Compound 14 | B | Compound 72 | A | Compound 94 | A |
| Compound 18 | B | Compound 73 | A | Compound 95 | A |
| Compound 19 | A | Compound 77 | A | Compound 96 | A |
| Compound 23 | A | Compound 78 | A | Compound 98 | B |
| Compound 37 | A | Compound 79 | A | Compound 99 | A |
| Compound 41 | B | Compound 80 | A | Compound 100 | A |
| Compound 55 | B | Compound 81 | A | Compound 101 | A |
| Compound 57 | A | Compound 82 | A | Compound 107 | A |
| Compound 59 | A | Compound 83 | A | Compound 111 | A |
| Compound 60 | A | Compound 84 | A | | |
| Compound 61 | B | | | | |

wherein the letter A represents that $IC_{50}$ is less than 50 nM; the letter B represents that $IC_{50}$ is from 50 nM to 200 nM; and the letter C represents that $IC_{50}$ is greater than 200 nM.

**Test example 3: Jurkat (ATCC: TIB-152) cell proliferation inhibition experiment**

ATP in living cells was quantitatively detected by CellTiter-Glo (Promega-G7573) Luminescence Cell Viability Assay Kit to determine the effect of the compounds to be tested on cell proliferation.

Step 1: Jurkat cells were seeded in 96-well plates (Greiner-655098) at a density of 1,500 cells per well with a volume of 140 μL per well; Cell culture medium components included RPMI1640 (Gibco-A10491-01) +10% FBS (Gibco-10099-141C) +1% Penicillin-Streptomycin (5,000 U/mL, Gibco-15070-063).

Step 2: On the day of cell seeding, the cells were treated with the compound to be tested. The compound to be tested was diluted with a 3-fold gradient, and a total of 9 concentration gradients were set. DMSO (Sigma, D2650) with 0.5% final concentration or a DMSO solution containing the compound to be tested was added per well with 2 or 3 replicate wells at each concentration point. The plates were incubated at 37°C in a 5% $CO_2$ incubator (Thermo Scientific Steri-Cycle 371) for 6 days.

Step 3: The cell viability in the control and treatment groups was detected with CellTiter-Glo Luminescent Cell Viability Assay Kit. 50 μL of CellTiter-Glo was added per well, mixed well, and incubated for 15 min at room temperature protected from light. According to the kit instructions, the chemiluminescence signal was read using a multifunctional microplate reader (EnVision, PerkinElmer).

The inhibition percentage (%) of the compound at each concentration point was calculated by the following formula:

Inhibition Percentage (%) = (1- signal value of the group treated with the compound /signal value of the group treated with DMSO) * 100.

The value of cell proliferation semi-inhibitory concentration $IC_{50}$ was calculated using GraphPad Prism software, and the results are shown in Table 3. The ratio of Jurkat cell proliferation inhibitory activity $IC_{50}$ to MV4-11 cell proliferation inhibitory activity $IC_{50}$ may be further calculated to obtain selectivity. The compound of the present disclosure has weak inhibitory activity on Jurkat cells and selectivity of more than 300 times, indicating that the compound has good selectivity.

| Compound No. | Jurkat cell proliferation inhibitory activity $IC_{50}$(uM) | MV4-11 cell proliferation inhibitory activity $IC_{50}$(uM) | selectivity (time) |
|---|---|---|---|
| Compound 1 | 6.8 | 0.019 | 357 |
| Compound 23 | 1.4 | 0.004 | 350 |
| Compound 91 | 3.1 | 0.01 | 310 |
| Compound 94 | 1.4 | 0.003 | 466 |
| Compound 99 | 1.2 | 0.004 | 300 |
| Compound 100 | 1.6 | 0.005 | 320 |

**Test example 4: Compound pharmacokinetic experiment**

The compound according to the present disclosure was tested for pharmacokinetic determination. The present application adopted the following method to determine the pharmacokinetic parameters of the compound of the present application.

Healthy male adult mice used in the study were administered a single gavage of 5-100 mg/kg per group of animals. Fasting from 10 hours before administration to 4 hours after administration was performed. Blood was collected after different time points after administration and the plasma content of the compound was determined (LC-MS/MS). The relationship between plasma concentration and time was analyzed with specialized software (winnonlin) to calculate the pharmacokinetic parameters of the compound. The compound of the present disclosure has good properties in terms of bioavailability, tissue distribution, half-life and in vivo clearance.

All documents referred to in the present disclosure are cited herein as references, just as each document is individually cited as a reference. The preferred embodiments of the present disclosure are described in detail above. However, the present disclosure is not limited to the specific details in the above embodiments. Within the scope of the technical concept of the present disclosure, a variety of simple variants of the technical solution of the present disclosure may be made and these simple variants are within the scope of protection of the present disclosure.

It should also be noted that the specific technical features described in the above embodiments, without contradiction, may be combined in any suitable way. In order to avoid unnecessary duplication, the present disclosure for various possible combinations will not be otherwise described. Further, various embodiments of the present disclosure may also be arbitrarily combined, as long as it does not contradict the ideas of the present disclosure, it should also be regarded as the content disclosed in the present disclosure.

**Claims**

1. A compound of Formula I, or a pharmaceutically acceptable salt thereof, or an enantiomer, a diastereoisomer, a tautomer, an isotope substitute, a solvate, a polymorph, a prodrug or metabolite thereof:

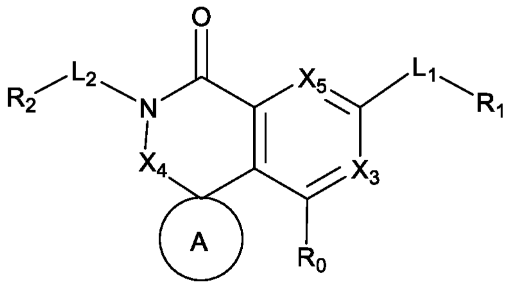

Formula I

wherein

ring A is a substituted or unsubstituted 3-8 membered saturated or unsaturated carboncycle or a substituted or unsubstituted 3-8 membered saturated or unsaturated heterocycle, wherein said heterocycle contains 1-2 hetero atoms selected from N, O and S;
$X_3$ is selected from $CR_5$ and N;
$X_4$ is selected from optionally substituted $-(CH_2)_n-$, -C(=O)- and $NR_6$, wherein n is 1, 2 or 3;
$X_5$ is selected from N and $CR_5$;
$L_1$ is selected from a bond, optionally substituted C1-C6 alkylene, optionally substituted C1-C6 alkylene-O-, optionally substituted C1-C6 alkylene-NH-, optionally substituted C1-C6 alkylene-S- and optionally substituted C2-C4 alkenylene;
$L_2$ is selected from a bond, optionally substituted C1-C6 alkylene, optionally substituted C1-C6 alkylene-O-, optionally substituted C1-C6 alkylene-NH-, optionally substituted C1-C6 alkylene-S- and optionally substituted C2-C4 alkenylene;
$R_0$ is selected from H, C1-C6 alkyl, optionally substituted C1-C6 alkoxy, optionally substituted C1-C6 haloalkyl, $-C(O)NR_3R_4$, $-CH_2OR_3$, and C1-C6 alkyl substituted with $-NR_3R_4$;
$R_1$ is selected from optionally substituted cycloalkyl, optionally substituted aryl, optionally substituted heteroaryl, optionally substituted heterocyclyl, optionally substituted amino and guanidyl;
$R_2$ is selected from optionally substituted cycloalkyl, optionally substituted aryl, optionally substituted heteroaryl and optionally substituted heterocyclyl;
$R_3$ and $R_4$ are each independently selected from H, optionally substituted C1-C6 alkyl, optionally substituted C1-C6 alkoxy, optionally substituted C1-C6 haloalkyl, optionally substituted 4-10 membered heterocyclyl and optionally substituted 5-10 membered heteroaryl; or $R_3$ and $R_4$ form optionally substituted 4-10 membered heterocyclyl or 5-10 membered heteroaryl with the nitrogen atom to which they are attached;
$R_5$ is selected from H, halogen, C1-C6 alkyl, halogenated C1-C6 alkyl and C1-C6 alkoxy;
$R_6$ is selected from H, C1-C6 alkyl, halogenated C1-C6 alkyl and C1-C6 alkoxy.

2. The compound of Formula I according to claim 1, or a pharmaceutically acceptable salt thereof, or an enantiomer, a diastereoisomer, a tautomer, an isotope substitute, a solvate, a polymorph, a prodrug or metabolite thereof, wherein ring A is C3-C8 cycloalkyl or 3-7 membered heterocyclyl.

3. The compound of Formula I according to claim 1 or 2, or a pharmaceutically acceptable salt thereof, or an enantiomer, a diastereoisomer, a tautomer, an isotope substitute, a solvate, a polymorph, a prodrug or metabolite thereof, wherein ring A is selected from cyclopropyl, cyclobutyl, cyclopentyl, cyclohexyl, aziridinyl, azetidinyl, pyrrolidinyl, piperidinyl, piperazinyl, oxiranyl, oxetanyl, tetrahydrofuranyl, tetrahydropyranyl and morpholinyl.

4. The compound of Formula I according to any one of claims 1-3, or a pharmaceutically acceptable salt thereof, or an enantiomer, a diastereoisomer, a tautomer, an isotope substitute, a solvate, a polymorph, a prodrug or metabolite thereof, wherein ring A is substituted with 1, 2 or 3 substituents selected from halogen, hydroxyl, cyano, C1-C6 alkyl, halogenated C1-C6 alkyl, hydroxyl-substituted C1-C6 alkyl, C1-C6 alkoxy, halogenated C1-C6 alkoxy, $-NR_aR_b$, carbonyl substituted with $-NR_aR_b$, C1-C6 alkyl substituted with $-NR_aR_b$, C1-C6 alkylcarbonyl, C1-C6 alkoxycarbonyl,

optionally substituted 6-14 membered aryl, optionally substituted 5-10 membered heteroaryl, optionally substituted 4-10 membered heterocyclyl, optionally substituted C3-C8 cycloalkyl, C1-C6 alkyl substituted with optionally substituted 6-14 membered aryl, C1-C6 alkyl substituted with optionally substituted 5-10 membered heteroaryl, C1-C6 alkyl substituted with optionally substituted 4-10 membered heterocyclyl, and C1-C6 alkyl substituted with optionally substituted C3-C8 cycloalky; wherein when said 6-14 membered aryl, 5-10 membered heteroaryl, 4-10 membered heterocyclyl and C3-C8 cycloalkyl are substituted, the substituent is 1, 2 or 3 substituents selected from halogen, C1-C6 alkyl, halogenated C1-C6 alkyl, C1-C6 alkoxy, halogenated C1-C6 alkoxy, $-NR_aR_b$, carbonyl substituted with $-NR_aR_b$, C1-C6 alkyl substituted with $-NR_aR_b$, C1-C6 alkylcarbonyl and C1-C6 alkoxycarbonyl; wherein said $R_a$ and $R_b$ are each independently selected from H and C1-C6 alkyl.

**5.** The compound of Formula I according to any one of claims 1-4, or a pharmaceutically acceptable salt thereof, or an enantiomer, a diastereoisomer, a tautomer, an isotope substitute, a solvate, a polymorph, a prodrug or metabolite thereof, wherein ring A is cyclopropyl, cyclobutyl, cyclopentyl, azetidinyl, oxetanyl, pyrrolidinyl or tetrahydrofuranyl.

**6.** The compound of Formula I according to any one of claims 1-5, or a pharmaceutically acceptable salt thereof, or an enantiomer, a diastereoisomer, a tautomer, an isotope substitute, a solvate, a polymorph, a prodrug or metabolite thereof, wherein ring A is optionally substituted with 1, 2 or 3 substituents selected from F, Cl, Br, $CF_3$, $CH_3$, -OH, $-OCH_3$, $-NH_2$, $-NHCH_3$ and - $NH_2(CH_3)_2$.

**7.** The compound of Formula I according to any one of claims 1-6, or a pharmaceutically acceptable salt thereof, or an enantiomer, a diastereoisomer, a tautomer, an isotope substitute, a solvate, a polymorph, a prodrug or metabolite thereof, wherein $R_0$ is C1-C6 alkyl substituted with $-NR_3R_4$; wherein $R_3$ and $R_4$ are each independently selected from H, C1-C6 alkyl optionally substituted with 1, 2, 3, 4, 5 or 6 substituents selected from halogen, cyano, C1-C6 alkoxy, halogenated C1-C6 alkoxy, $-NR_aR_b$ and hydroxyl, or $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

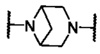 or 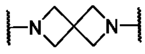

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

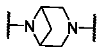 or 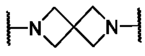

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, sulfo, C1-C6 alkylsulfonyl, C1-C6 alkoxysulfonyl, C1-C6 alkylcarbonyl, hydroxyl and halogenated C1-C6 alkylcarbonyl, wherein $R_a$ and $R_b$ are each independently H or C1-C4 alkyl.

**8.** The compound of Formula I according to any one of claims 1-7, or a pharmaceutically acceptable salt thereof, or an enantiomer, a diastereoisomer, a tautomer, an isotope substitute, a solvate, a polymorph, a prodrug or metabolite thereof, wherein $R_0$ is C1-C6 alkyl substituted with $-NR_3R_4$, wherein $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

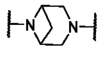 or 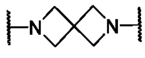

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

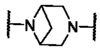 or 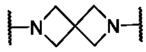

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, sulfo, C1-C6 alkylsulfonyl, C1-C6 alkoxysulfonyl, C1-C6 alkylcarbonyl, halogenated C1-C6 alkylcarbonyl, C1-C6 alkoxycarbonyl, halogenated C1-C6 alkoxycarbonyl, $-NR_aR_b$ and hydroxyl, wherein $R_a$ and $R_b$ are each independently H or C1-C4 alkyl.

**9.** The compound of Formula I according to any one of claims 1-8, or a pharmaceutically acceptable salt thereof, or an enantiomer, a diastereoisomer, a tautomer, an isotope substitute, a solvate, a polymorph, a prodrug or metabolite thereof, wherein $R_0$ is C1-C6 alkyl substituted with $-NR_3R_4$, wherein $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

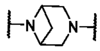 or 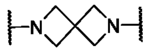

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

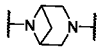 or 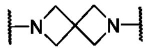

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, $-NR_aR_b$ and hydroxyl; wherein $R_a$ and $R_b$ are each independently H or C1-C4 alkyl.

**10.** The compound of Formula I according to any one of claims 1-9, or a pharmaceutically acceptable salt thereof, or an enantiomer, a diastereoisomer, a tautomer, an isotope substitute, a solvate, a polymorph, a prodrug or metabolite thereof, wherein
$X_3$ and/or $X_5$ is $CR_5$.

**11.** The compound of Formula I according to any one of claims 1-10, or a pharmaceutically acceptable salt thereof, or an enantiomer, a diastereoisomer, a tautomer, an isotope substitute, a solvate, a polymorph, a prodrug or metabolite thereof, wherein $R_5$ is selected from H and C1-C4 alkyl.

**12.** The compound of Formula I according to any one of claims 1-11, or a pharmaceutically acceptable salt thereof, or an enantiomer, a diastereoisomer, a tautomer, an isotope substitute, a solvate, a polymorph, a prodrug or metabolite thereof, wherein $X_4$ is $-(CH_2)_n-$, n is 1 or 2; wherein said $-(CH_2)_n-$ is optionally substituted with 1 or 2 substituents selected from halogen, hydroxyl, amino, C1-C6 alkyl and halogenated C1-C6 alkyl.

**13.** The compound of Formula I according to any one of claims 1-12, or a pharmaceutically acceptable salt thereof, or an enantiomer, a diastereoisomer, a tautomer, an isotope substitute, a solvate, a polymorph, a prodrug or metabolite thereof, wherein Li is selected from C1-C6 alkylene, C1-C6 alkylene-O-, C1-C6 alkylene-NH-, C1-C6 alkylene-S- and C2-C4 alkenylene, wherein said C1-C6 alkylene, C1-C6 alkylene-O-, C1-C6 alkylene-NH-, C1-C6 alkylene-S- and C2-C4 alkenylene are optionally substituted with 1, 2 or 3 substituents selected from halogen, C1-C4 alkyl and hydroxyl.

**14.** The compound of Formula I according to any one of claims 1-13, or a pharmaceutically acceptable salt thereof, or an enantiomer, a diastereoisomer, a tautomer, an isotope substitute, a solvate, a polymorph, a prodrug or metabolite thereof, wherein $L_1$ is selected from C1-C6 alkylene or C1-C6 alkylene-NH-, wherein said C1-C6 alkylene or C1-C6 alkylene-NH- are optionally substituted with 1, 2 or 3 substituents selected from halogen, C1-C4 alkyl and hydroxyl.

**15.** The compound of Formula I according to any one of claims 1-14, or a pharmaceutically acceptable salt thereof, or an enantiomer, a diastereoisomer, a tautomer, an isotope substitute, a solvate, a polymorph, a prodrug or metabolite thereof, wherein $R_1$ is selected from optionally substituted C3-C8 cycloalkyl, optionally substituted 6-14 membered

aryl, optionally substituted 5-10 membered heteroaryl and optionally substituted 4-10 membered heterocyclyl, wherein said cycloalkyl, aryl, heteroaryl and heterocyclyl are each optionally substituted with 1, 2 or 3 substituents selected from optionally substituted amino, C1-C6 alkyl, halogen, hydroxyl, C1-C6 alkoxy, C1-C6 haloalkyl and cyano; wherein said optionally substituted amino is $-NR_aR_b$, wherein $R_a$ and $R_b$ are each independently H or C1-C6 alkyl.

**16.** The compound of Formula I according to any one of claims 1-15, or a pharmaceutically acceptable salt thereof, or an enantiomer, a diastereoisomer, a tautomer, an isotope substitute, a solvate, a polymorph, a prodrug or metabolite thereof, wherein $R_1$ is selected from optionally substituted phenyl, naphthyl, pyridyl, pyrrolyl, pyrazolyl, imidazolyl, imidazolonyl, triazolyl, pyrimidinyl, pyrazinyl, triazinyl, indolyl, isoindolyl, indazolyl, benzimidazolyl, pyridinopyrrolyl, imidazopyridinyl, benzotriazolyl, oxazolone, oxazolidinone, benzoxazolyl, benzisoxazolyl, quinolyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, decahydroisoquinolyl, imidazolinyl, imidazolidinyl, quinazinyl, thiazolidinyl, isothiazolidinyl, isoxazolidinyl, dihydroindolyl, octahydroindolyl, octahydroisoindolyl, pyrrolidinyl, pyrazolindinyl, phthalimidinyl,

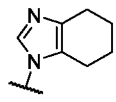 and 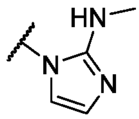 .

**17.** The compound of Formula I according to any one of claims 1-16, or a pharmaceutically acceptable salt thereof, or an enantiomer, a diastereoisomer, a tautomer, an isotope substitute, a solvate, a polymorph, a prodrug or metabolite thereof, wherein said $L_1$ is C1-C4 alkylene or C 1-C4 alkylene-NH-, $R_1$ is pyridyl, pyrrolyl, pyrazolyl, imidazolyl, triazolyl, pyrimidinyl, pyrazinyl, triazinyl,

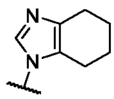 or 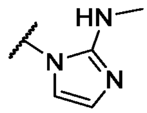 ,

wherein said pyridyl, pyrrolyl, pyrazolyl, imidazolyl, triazolyl, pyrimidinyl, pyrazinyl, triazinyl,

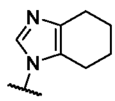 or 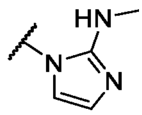

is optionally substituted with 1 or 2 substituents selected from halogen, C1-C4 alkyl, cyano and $-NR_aR_b$, wherein $R_a$ and $R_b$ are each independently H or C1-C4 alkyl.

**18.** The compound of Formula I according to any one of claims 1-17, or a pharmaceutically acceptable salt thereof, or an enantiomer, a diastereoisomer, a tautomer, an isotope substitute, a solvate, a polymorph, a prodrug or metabolite thereof, wherein $L_2$ is selected from a bond, C3-C5 cycloalkyl and C1-C6 alkylene, wherein said C3-C5 cycloalkyl, and C1-C6 alkylene are optionally substituted with 1, 2 or 3 substituents selected from halogen, C1-C3 alkyl, C3-C5 cycloalkyl and hydroxyl.

**19.** The compound of Formula I according to any one of claims 1-18, or a pharmaceutically acceptable salt thereof, or an enantiomer, a diastereoisomer, a tautomer, an isotope substitute, a solvate, a polymorph, a prodrug or metabolite thereof, wherein $R_2$ is optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl and 5-10 membered heteroaryl; wherein $R_a$ and $R_b$ are each independently H or C1-C6 alkyl; wherein said heteroaryl is preferably a heteroaryl containing 1 or 2 nitrogen ring atoms, preferably selected from pyrazolyl, imidazolyl, pyridyl, pyrazinyl and piperazinyl.

**20.** The compound of Formula I according to any one of claims 1-19, or a pharmaceutically acceptable salt thereof, or an enantiomer, a diastereoisomer, a tautomer, an isotope substitute, a solvate, a polymorph, a prodrug or metabolite thereof, wherein $R_2$ is selected from 6-14 membered aryl, 5-10 membered heteroaryl and 4-10 membered heterocyclyl, said 6-14 membered aryl, 5-10 membered heteroaryl and 4-10 membered heterocyclyl is optionally substituted

with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl and 5-10 membered heteroaryl.

**21.** The compound of Formula I according to any one of claims 1-20, or a pharmaceutically acceptable salt thereof, or an enantiomer, a diastereoisomer, a tautomer, an isotope substitute, a solvate, a polymorph, a prodrug or metabolite thereof, wherein $R_2$ is selected from phenyl, naphthyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl, azetidinyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, benzodihydrofuranyl, decahydroisoquinolyl, imidazolinyl, imidazolidinyl, 6,7-dihydro-5H-cyclopenta[c]pyridine, quinazinyl, thiazolidinyl, isothiazolidinyl, isoxazolidinyl, dihydroindolyl, octahydroindolyl, octahydroisoindolyl, pyrrolidinyl, pyrazolindinyl,

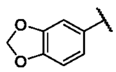 and 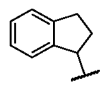,

wherein said phenyl, naphthyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl, azetidinyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, benzodihydrofuranyl, decahydroisoquinolyl, imidazolinyl, imidazolidinyl, 6,7-dihydro-SH-cyclopenta[c]pyridine, quinazinyl, thiazolidinyl, isothiazolidinyl, isoxazolidinyl, dihydroindolyl, octahydroindolyl, octahydroisoindolyl, pyrrolidinyl, pyrazolindinyl,

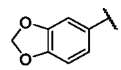

and

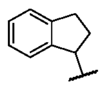

are optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl and 5-10 membered heteroaryl.

**22.** The compound of Formula I according to any one of claims 1-21, or a pharmaceutically acceptable salt thereof, or an enantiomer, a diastereoisomer, a tautomer, an isotope substitute, a solvate, a polymorph, a prodrug or metabolite thereof, wherein $L_2$ is C1-C4 alkylene, $R_2$ is 6-14 membered aryl, 5-10 membered nitrogen-containing heteroaryl or 4-10 membered heterocyclyl, wherein said 6-14 membered aryl, 5-10 membered nitrogen-containing heteroaryl or 4-10 membered heterocyclyl is optionally substituted with 1 or 2 substituents selected from C1-C6 alkoxy, halogen, C1-C6 alkyl, cyano, C3-C8 cycloalkyl-O- and halogenated C1-C6 alkoxy; $R_2$ is preferably phenyl, pyridyl, pyrazolyl, pyrrolopyridyl, dihydrofuropyridinyl, pyrido 1,3-dioxolyl, indolyl,

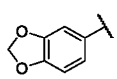 or 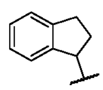,

wherein said phenyl, pyridyl, pyrazolyl, pyrrolopyridyl, dihydrofuropyridinyl, pyrido 1,3-dioxolyl,

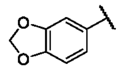 or 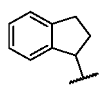

is optionally substituted with 1 or 2 substituents selected from C1-C6 alkoxy, halogen, C1-C6 alkyl, cyano, C3-C8 cycloalkyl-O-and halogenated C1-C6 alkoxy.

**23.** The compound of Formula I according to claim 1, or a pharmaceutically acceptable salt thereof, or an enantiomer, a diastereoisomer, a tautomer, an isotope substitute, a solvate, a polymorph, a prodrug or metabolite thereof, wherein

ring A is C3-C6 cycloalkyl or 4-8 membered heterocyclyl, wherein said cycloalkyl or heterocyclyl is optionally substituted with 1, 2 or 3 substituents selected from halogen, C1-C4 alkyl, halogenated C1-C4 alkyl, C1-C4 alkoxy, halogenated C1-C4 alkoxy;
$X_3$ and $X_5$ are both CH;
$X_4$ is $CH_2$;
$L_1$ is C1-C4 alkylene;
$R_1$ is 5 or 6 membered nitrogen-containing heteroaryl optionally substituted with 1 or 2 substituents selected from halogen, C1-C4 alkyl and $-NR_aR_b$, wherein said 5 or 6 membered nitrogen-containing heteroaryl is preferably pyridyl, pyrrolyl, pyrazolyl, imidazolyl, triazolyl, pyrimidinyl, pyrazinyl, triazinyl or

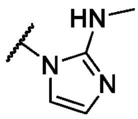
;

wherein $R_a$ and $R_b$ are each independently H or C1-C4 alkyl;
$L_2$ is C1-C4 alkylene;
$R_2$ is phenyl, 5-10 membered nitrogen-containing heteroaryl or 4-10 membered heterocyclyl, preferably pyridyl, pyrimidinyl, pyrazolyl, pyrrolyl, imidazolyl, pyrazinyl, pyrrolopyridyl, dihydrofuropyridinyl, pyrido 1,3-dioxolyl or indolyl, wherein said phenyl, 5-10 membered nitrogen-containing heteroaryl and 4-10 membered heterocyclyl are each optionally substituted with 1 or 2 substituents selected from C1-C6 alkoxy, halogen, C1-C6 alkyl, cyano, C3-C8 cycloalkyl-O- and halogenated C1-C6 alkoxy;
$R_0$ is C1-C4 alkyl substituted with $-NR_3R_4$; wherein $R_3$ and $R_4$ are each independently selected from H, C1-C6 alkyl optionally substituted with 1-6 substituents selected from hydroxyl, cyano and halogen, and a 4-8 membered heterocyclyl optionally substituted with 1, 2 or 3 substituents selected from halogen, C1-C6 alkyl, halogenated C1-C6 alkyl, C1-C6 alkoxy, halogenated C1-C6 alkoxy, $-NR_aR_b$ and hydroxyl, wherein $R_a$ and $R_b$ are each independently H or C1-C4 alkyl; or $R_3$ and $R_4$ form a 4-8 membered heterocyclyl optionally substituted with 1, 2 or 3 substituents selected from halogen, C1-C6 alkyl, C3-C6 cycloalkyl, halogenated C1-C6 alkyl, C1-C6 alkoxy, halogenated C1-C6 alkoxy, $-NR_aR_b$ and hydroxyl with the nitrogen atom to which they are attached, wherein $R_a$ and $R_b$ are each independently H or C1-C4 alkyl; preferably, said 4-8 membered heterocyclyl is each independently selected from azetidinyl, pyrrolidinyl, piperidinyl, piperazinyl, morpholinyl,

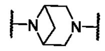 or 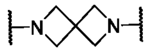.

**24.** The compound of Formula I according to claim 1, or a pharmaceutically acceptable salt thereof, or an enantiomer, a diastereoisomer, a tautomer, an isotope substitute, a solvate, a polymorph, a prodrug or metabolite thereof, wherein

ring A is C3-C6 cycloalkyl, wherein said cycloalkyl is optionally substituted with 1, 2 or 3 substituents selected from halogen, C1-C4 alkyl and halogenated C1-C4 alkyl;
$X_3$ and $X_5$ are both CH;
$X_4$ is $CH_2$;
$L_1$ is C1-C4 alkylene;
$R_1$ is 5 or 6 membered nitrogen-containing heteroaryl optionally substituted with 1 or 2 substituents selected from C1-C4 alkyl and $-NR_aR_b$, preferably pyridyl, pyrrolyl, pyrazolyl, imidazolyl, triazolyl, pyrimidinyl, pyrazinyl, triazinyl or

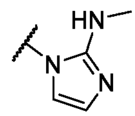
;

wherein $R_a$ and $R_b$ are each independently H or C1-C4 alkyl;

$L_2$ is C1-C4 alkylene;

$R_2$ is 5-10 membered nitrogen-containing heteroaryl or 4-10 membered heterocyclyl, preferably pyridyl, pyrimidinyl, pyrazolyl, pyrrolyl, imidazolyl, pyrazinyl, pyrrolopyridyl, dihydrofuropyridinyl, pyrido 1,3-dioxolyl or indolyl, wherein said 5-10 membered nitrogen-containing heteroaryl or 4-10 membered heterocyclyl is each optionally substituted with 1 or 2 substituents selected from C1-C6 alkoxy, C1-C6 alkyl, C3-C8 cycloalkyl-O-, halogenated C1-C6 alkoxy, halo and cyano;

$R_0$ is C1-C4 alkyl substituted with $-NR_3R_4$, wherein $R_3$ and $R_4$ are each independently selected from H and C1-C6 alkyl optionally substituted with 1, 2, 3, 4, 5 or 6 substituents selected from hydroxyl, cyano and halogen, or $R_3$ and $R_4$ form a 4-8 membered heterocyclyl optionally substituted with 1, 2 or 3 substituents selected from halogen, hydroxyl, C1-C4 alkoxy, C3-C6 alkoxy and C1-C4 alkyl with the nitrogen atom to which they are attached, preferably, said 4-8 membered heterocyclyl is selected from azetidinyl, pyrrolidinyl, piperidinyl, piperazinyl, morpholinyl,

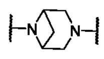 or 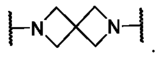.

**25.** A compound of Formula II, or a pharmaceutically acceptable salt thereof, or an enantiomer, a diastereoisomer, a tautomer, an isotope substitute, a solvate, a polymorph, a prodrug or metabolite thereof:

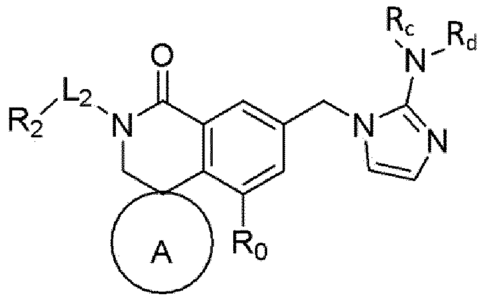

**II**

wherein

ring A is a substituted or unsubstituted 3-8 membered saturated or unsaturated carboncycle or a substituted or unsubstituted 3-8 membered saturated or unsaturated heterocycle, wherein said heterocycle contains 1-2 hetero atoms selected from N, O and S;

$L_2$ is selected from a bond, optionally substituted C1-C6 alkylene, optionally substituted C1-C6 alkylene-O-, optionally substituted C1-C6 alkylene-NH-, optionally substituted C1-C6 alkylene-S- and optionally substituted C2-C4 alkenylene;

$R_0$ is selected from H, C1-C6 alkyl, optionally substituted C1-C6 alkoxy, optionally substituted C1-C6 haloalkyl, $-C(O)NR_3R_4$, $-CH_2OR_3$, C1-C6 alkyl substituted with $-NR_3R_4$,;

$R_2$ is selected from optionally substituted cycloalkyl, optionally substituted aryl, optionally substituted heteroaryl and optionally substituted heterocyclyl;

$R_3$ and $R_4$ are each independently selected from H, optionally substituted C1-C6 alkyl, optionally substituted C1-C6 alkoxy, optionally substituted C1-C6 haloalkyl, optionally substituted 4-10 membered heterocyclyl and optionally substituted 5-10 membered heteroaryl; or $R_3$ and $R_4$ form optionally substituted 4-10 membered heterocyclyl or 5-10 membered heteroaryl with the nitrogen atom to which they are attached; and

$R_c$ and $R_d$ are each independently H or C1-C4 alkyl.

**26.** The compound of Formula II according to claim 25, or a pharmaceutically acceptable salt thereof, or an enantiomer, a diastereoisomer, a tautomer, an isotope substitute, a solvate, a polymorph, a prodrug or metabolite thereof, wherein ring A is C3-C8 cycloalkyl or 3-7 membered heterocyclyl.

**27.** The compound of Formula II according to claim 25 or 26, or a pharmaceutically acceptable salt thereof, or an enantiomer, a diastereoisomer, a tautomer, an isotope substitute, a solvate, a polymorph, a prodrug or metabolite thereof, wherein ring A is selected from cyclopropyl, cyclobutyl, cyclopentyl, cyclohexyl, aziridinyl, azetidinyl, pyrrolidinyl, piperidinyl, piperazinyl, oxiranyl, oxetanyl, tetrahydrofuranyl, tetrahydropyranyl and morpholinyl.

**28.** The compound of Formula II according to any one of claims 25-27, or a pharmaceutically acceptable salt thereof, or an enantiomer, a diastereoisomer, a tautomer, an isotope substitute, a solvate, a polymorph, a prodrug or metabolite thereof, wherein ring A is substituted with 1, 2 or 3 substituents selected from halogen, hydroxyl, cyano, C1-C6 alkyl, halogenated C1-C6 alkyl, hydroxyl-substituted C1-C6 alkyl, C1-C6 alkoxy, halogenated C1-C6 alkoxy, $-NR_aR_b$, C1-C6 alkylcarbonyl and C1-C6 alkoxycarbonyl, wherein said $R_a$ and $R_b$ are each independently selected from H and C1-C6 alkyl.

**29.** The compound of Formula II according to any one of claims 25-28, or a pharmaceutically acceptable salt thereof, or an enantiomer, a diastereoisomer, a tautomer, an isotope substitute, a solvate, a polymorph, a prodrug or metabolite thereof, wherein ring A is cyclopropyl, cyclobutyl, cyclopentyl, cyclohexyl, azetidinyl, oxetanyl, pyrrolidinyl, tetrahydrofuranyl or tetrahydropyranyl.

**30.** The compound of Formula II according to any one of claims 25-29, or a pharmaceutically acceptable salt thereof, or an enantiomer, a diastereoisomer, a tautomer, an isotope substitute, a solvate, a polymorph, a prodrug or metabolite thereof, wherein ring A is optionally substituted with 1, 2 or 3 substituents selected from F, Cl, Br, $CF_3$, $CH_3$, -OH, $-OCH_3$, $-NH_2$, $-NHCH_3$, and - $NH_2(CH_3)_2$.

**31.** The compound of Formula II according to any one of claims 25-30, or a pharmaceutically acceptable salt thereof, or an enantiomer, a diastereoisomer, a tautomer, an isotope substitute, a solvate, a polymorph, a prodrug or metabolite thereof, wherein $R_0$ is C1-C6 alkyl substituted with $-NR_3R_4$; wherein $R_3$ and $R_4$ are each independently selected from H, C1-C6 alkyl optionally substituted with 1, 2, 3, 4, 5 or 6 substituents selected from halogen, cyano, C1-C6 alkoxy, halogenated C1-C6 alkoxy, $-NR_aR_b$ and hydroxyl, or $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

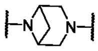 or 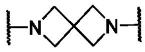

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

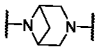 or 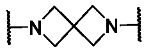

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, sulfo, C1-C6 alkylsulfonyl, C1-C6 alkoxysulfonyl, C1-C6 alkylcarbonyl, halogenated C1-C6 alkylcarbonyl, C1-C6 alkoxycarbonyl, halogenated C1-C6 alkoxycarbonyl, $-NR_aR_b$ and hydroxyl, wherein $R_a$ and $R_b$ are each independently H or C1-C4 alkyl.

**32.** The compound of Formula II according to any one of claims 25-31, or a pharmaceutically acceptable salt thereof, or an enantiomer, a diastereoisomer, a tautomer, an isotope substitute, a solvate, a polymorph, a prodrug or metabolite thereof, wherein $R_0$ is C1-C6 alkyl substituted with $-NR_3R_4$, wherein $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

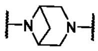 or 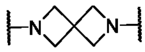

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

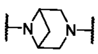 or 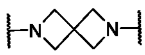

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, sulfo, C1-C6 alkylsulfonyl, C1-C6 alkoxysulfonyl, C1-C6 alkylcarbonyl, halogenated C1-C6 alkylcarbonyl, C1-C6 alkoxycarbonyl, halogenated C1-C6 alkoxycarbonyl, $-NR_aR_b$ and hydroxyl; wherein $R_a$ and $R_b$ are each independently H or C1-C4 alkyl.

**33.** The compound of Formula II according to any one of claims 25-32, or a pharmaceutically acceptable salt thereof, or an enantiomer, a diastereoisomer, a tautomer, an isotope substitute, a solvate, a polymorph, a prodrug or metabolite thereof, wherein $R_0$ is C1-C6 alkyl substituted with $-NR_3R_4$, wherein $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

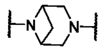 and 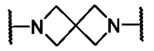

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

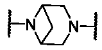 and 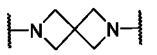

are each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, $-NR_aR_b$ and hydroxyl; wherein $R_a$ and $R_b$ are each independently H or C1-C4 alkyl.

**34.** The compound of Formula II according to any one of claims 25-33, or a pharmaceutically acceptable salt thereof, or an enantiomer, a diastereoisomer, a tautomer, an isotope substitute, a solvate, a polymorph, a prodrug or metabolite thereof, wherein $R_c$ and $R_d$ are each independently H or methyl.

**35.** The compound of Formula II according to any one of claims 25-34, or a pharmaceutically acceptable salt thereof, or an enantiomer, a diastereoisomer, a tautomer, an isotope substitute, a solvate, a polymorph, a prodrug or metabolite thereof, wherein $L_2$ is selected from a bond and C1-C6 alkylene, wherein said C1-C6 alkylene is optionally substituted with 1, 2 or 3 substituents selected from halogen, C1-C3 alkyl, C3-C5 cycloalkyl and hydroxyl.

**36.** The compound of Formula II according to any one of claims 25-35, or a pharmaceutically acceptable salt thereof, or an enantiomer, a diastereoisomer, a tautomer, an isotope substitute, a solvate, a polymorph, a prodrug or metabolite thereof, wherein $R_2$ is optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano; wherein $R_a$ and $R_b$ are each independently H or C1-C6 alkyl; and wherein said heteroaryl is preferably a heteroaryl containing 1 or 2 nitrogen ring atoms, preferably selected from pyrazolyl, imidazolyl, pyridyl, pyrazinyl and piperazinyl.

**37.** The compound of Formula II according to any one of claims 25-36, or a pharmaceutically acceptable salt thereof, or an enantiomer, a diastereoisomer, a tautomer, an isotope substitute, a solvate, a polymorph, a prodrug or metabolite thereof, wherein $R_2$ is selected from 6-14 membered aryl, 5-10 membered heteroaryl and 4-10 membered heterocyclyl, wherein said 6-14 membered aryl, 5-10 membered heteroaryl and 4-10 membered heterocyclyl are optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano.

**38.** The compound of Formula II according to any one of claims 25-37, or a pharmaceutically acceptable salt thereof, or an enantiomer, a diastereoisomer, a tautomer, an isotope substitute, a solvate, a polymorph, a prodrug or metabolite thereof, wherein $R_2$ is selected from phenyl, naphthyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl, azetidinyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, benzodihydrofuranyl, decahydroisoquinolyl, imidazolinyl, imidazolidinyl, 6,7-dihydro-5H-cyclopenta[c]pyridine, quinazinyl,

thiazolidinyl, isothiazolidinyl, isoxazolidinyl, dihydroindolyl, octahydroindolyl, octahydroisoindolyl, pyrrolidinyl, pyrazolindinyl,

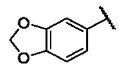 and 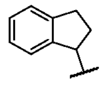,

wherein said phenyl, naphthyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl, azetidinyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, benzodihydrofuranyl, decahydroisoquinolyl, imidazolinyl, imidazolidinyl, 6,7-dihydro-SH-cyclopenta[c]pyridine, quinazinyl, thiazolidinyl, isothiazolidinyl, isoxazolidinyl, dihydroindolyl, octahydroindolyl, octahydroisoindolyl, pyrrolidinyl, pyrazolindinyl,

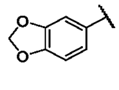

and

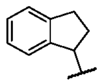

are optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano.

**39.** The compound of Formula II according to any one of claims 25-38, or a pharmaceutically acceptable salt thereof, or an enantiomer, a diastereoisomer, a tautomer, an isotope substitute, a solvate, a polymorph, a prodrug or metabolite thereof, wherein $L_2$ is C1-C4 alkylene, $R_2$ is phenyl, pyridyl, pyrazolyl, pyrrolopyridyl, dihydrofuropyridinyl, pyrido 1,3-dioxolyl or indolyl, wherein said phenyl, pyridyl, pyrazolyl, pyrrolopyridyl, dihydrofuropyridinyl, pyrido 1,3-dioxolyl or indolyl is substituted with 1 or 2 substituents selected from C1-C6 alkoxy, halogen, C1-C6 alkyl, cyano, C3-C8 cycloalkyl-O- and halogenated C1-C6 alkoxy.

**40.** The compound of Formula II according to claim 25, or a pharmaceutically acceptable salt thereof, or an enantiomer, a diastereoisomer, a tautomer, an isotope substitute, a solvate, a polymorph, a prodrug or metabolite thereof, wherein

ring A is C3-C6 cycloalkyl optionally substituted with substituents selected from F, Cl, hydroxyl or C1-C4 alkyl or C4-C6 heterocyclyl containing one nitrogen or one O atom, preferably cyclopropyl, cyclobutyl, oxetanyl or azetidinyl optionally substituted with F or C1-C4 alkyl;
$R_0$ is H or C1-C4 alkyl substituted with $-NR_3R_4$, wherein $R_3$ and $R_4$ are each independently selected from H, 4-6 membered heterocyclyl and C1-C6 alkyl optionally substituted with 1, 2, 3, 4, 5 or 6 substituents selected from hydroxyl, cyano and halogen, or $R_3$ and $R_4$ form a 4-6 membered heterocyclyl optionally substituted with 1, 2 or 3 substituents selected from halogen, hydroxyl, C1-C4 alkoxy, C3-C6 cycloalkyl and C1-C4 alkyl with the nitrogen atom to which they are attached; wherein said 4-6 membered heterocyclyl is preferably only nitrogen-containing heterocyclyl or oxygen and nitrogen-containing heterocyclyl, preferably selected from azetidinyl, pyrrolidinyl, piperidinyl, piperazinyl, 2-oxopiperazinyl, morpholinyl,

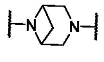 or 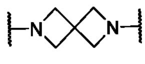;

$L_2$ is C1-C4 alkylene;
$R_2$ is 6-14 membered aryl, 5-10 membered nitrogen-containing heteroaryl or 4-10 membered heterocyclyl, preferably phenyl, pyridyl, pyrimidinyl, pyrazolyl, pyrrolyl, imidazolyl, pyrazinyl, indolyl, pyrrolopyridyl, dihydrofuropyridinyl, pyrido 1,3-dioxolyl,

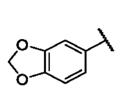 and 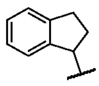,

wherein said 6-14 membered aryl, 5-10 membered nitrogen-containing heteroaryl or 4-10 membered heterocyclyl is each optionally substituted with 1 or 2 substituents selected from C1-C6 alkoxy, C1-C4 alkyl, halogen, C3-C6 cycloalkyl-oxy, halogenated C1-C4 alkoxy and cyano;
$R_c$ and $R_d$ are each independently H or C1-C4 alkyl.

**41.** The compound of Formula II according to claim 25, or a pharmaceutically acceptable salt thereof, or an enantiomer, a diastereoisomer, a tautomer, an isotope substitute, a solvate, a polymorph, a prodrug or metabolite thereof, wherein

ring A is C3-C6 cycloalkyl or 4-8 membered heterocyclyl, wherein said cycloalkyl or heterocyclyl is optionally substituted with 1, 2 or 3 substituents selected from halogen, C1-C4 alkyl, halogenated C1-C4 alkyl, C1-C4 alkoxy, halogenated C1-C4 alkoxy;
$L_2$ is C1-C4 alkylene;
$R_2$ is phenyl, 5-10 membered nitrogen-containing heteroaryl or 4-10 membered heterocyclyl, preferably pyridyl, pyrimidinyl, pyrazolyl, pyrrolyl, imidazolyl, pyrazinyl, pyrrolopyridyl, dihydrofuropyridinyl or pyrido 1,3-dioxolyl or indolyl, wherein said phenyl, 5-10 membered nitrogen-containing heteroaryl and 4-10 membered heterocyclyl are each optionally substituted with 1 or 2 substituents selected from C1-C6 alkoxy, halogen, C1-C6 alkyl, cyano, C3-C8 cycloalkyl-O- and halogenated C1-C6 alkoxy;
$R_0$ is C1-C4 alkyl substituted with $-NR_3R_4$; wherein $R_3$ and $R_4$ are each independently selected from H, C1-C6 alkyl optionally substituted with 1-6 substituents selected from hydroxyl, cyano and halogen, and a 4-8 membered heterocyclyl optionally substituted with 1, 2 or 3 substituents selected from halogen, C1-C6 alkyl, halogenated C1-C6 alkyl, C1-C6 alkoxy, halogenated C1-C6 alkoxy, $-NR_aR_b$ and hydroxyl, wherein $R_a$ and $R_b$ are each independently H or C1-C4 alkyl; or $R_3$ and $R_4$ form a 4-8 membered heterocyclyl optionally substituted with 1, 2 or 3 substituents selected from halogen, C1-C6 alkyl, C3-C6 cycloalkyl, halogenated C1-C6 alkyl, C1-C6 alkoxy, halogenated C1-C6 alkoxy, $-NR_aR_b$ and hydroxyl with the nitrogen atom to which they are attached, wherein $R_a$ and $R_b$ are each independently H or C1-C4 alkyl; preferably, said 4-8 membered heterocyclyl is each independently selected from the group consisting of azetidinyl, pyrrolidinyl, piperidinyl, piperazinyl, 2-oxopiperazinyl, morpholinyl,

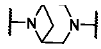 or 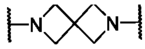;

and
$R_c$ and $R_d$ are each independently H or C1-C4 alkyl.

**42.** The compound of Formula II according to claim 25, or a pharmaceutically acceptable salt thereof, or an enantiomer, a diastereoisomer, a tautomer, an isotope substitute, a solvate, a polymorph, a prodrug or metabolite thereof, wherein

ring A is C3-C6 cycloalkyl, wherein said cycloalkyl is optionally substituted with 1, 2 or 3 substituents selected from halogen, C1-C4 alkyl and halogenated C1-C4 alkyl;
$L_2$ is C1-C4 alkylene;
$R_2$ is 5-10 membered nitrogen-containing heteroaryl or 4-10 membered heterocyclyl, preferably pyridyl, pyrimidinyl, pyrazolyl, pyrrolyl, imidazolyl, pyrazinyl, pyrrolopyridyl, dihydrofuropyridinyl or pyrido 1,3-dioxolyl or indolyl, wherein said 5-10 membered nitrogen-containing heteroaryl or 4-10 membered heterocyclyl is each optionally substituted with 1 or 2 substituents selected from C1-C6 alkoxy, C1-C6 alkyl, C3-C8 cycloalkyl-O-, halogenated C1-C6 alkoxy, halogen and cyano;
$R_0$ is C1-C4 alkyl substituted with $-NR_3R_4$, wherein $R_3$ and $R_4$ are each independently selected from H and C1-C6 alkyl optionally substituted with 1, 2, 3, 4, 5 or 6 substituents selected from hydroxyl, cyano and halogen, or $R_3$ and $R_4$ form a 4-8 membered heterocyclyl optionally substituted with 1, 2 or 3 substituents selected from halogen, hydroxyl, C1-C4 alkoxy and C1-C4 alky with the nitrogen atom to which they are attached, preferably, said 4-8 membered heterocyclyl is selected from the group consisting of azetidinyl, pyrrolidinyl, piperidinyl, piperazinyl, 2-oxopiperazinyl, morpholinyl,

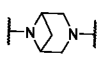 or 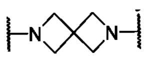;

and

$R_c$ and $R_d$ are each independently H or C1-C4 alkyl.

**43.** A compound of Formula III, or a pharmaceutically acceptable salt thereof, or an enantiomer, a diastereoisomer, a tautomer, an isotope substitute, a solvate, a polymorph, a prodrug or metabolite thereof:

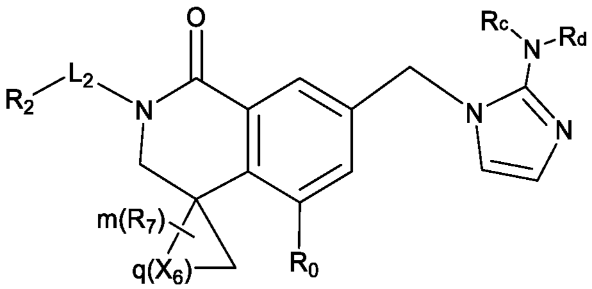

**III**

wherein

$L_2$ is selected from a bond, optionally substituted C1-C6 alkylene, optionally substituted C1-C6 alkylene-O-, optionally substituted C1-C6 alkylene-NH-, optionally substituted C1-C6 alkylene-S- and optionally substituted C2-C4 alkenylene;
$R_0$ is selected from H, C1-C6 alkyl, optionally substituted C1-C6 alkoxy, optionally substituted C1-C6 haloalkyl, $-C(O)NR_3R_4$, $-CH_2OR_3$ and C1-C6 alkyl substituted with $-NR_3R_4$;
$R_2$ is selected from optionally substituted cycloalkyl, optionally substituted aryl, optionally substituted heteroaryl and optionally substituted heterocyclyl;
$R_3$ and $R_4$ are each independently selected from H, optionally substituted C1-C6 alkyl, optionally substituted C1-C6 alkoxy, optionally substituted C1-C6 haloalkyl, optionally substituted 4-10 membered heterocyclyl and optionally substituted 5-10 membered heteroaryl; or $R_3$ and $R_4$ form an optionally substituted 4-10 membered heterocyclyl or 5-10 membered heteroaryl with the nitrogen atom to which they are attached;
$X_6$ are each independently selected from the group consisting of C, O, N, S,
q is an integer of 1, 2 or 3,
$R_7$ is selected from F, Cl, cyano, hydroxyl, C1-C6 alkyl, fluorinated or chlorinated C1-C6 alkyl, hydroxyl-substituted C1-C6 alkyl, C1-C6 alkoxy, fluorinated or chlorinated C1-C6 alkoxy, hydroxyl-substituted C1-C6 alkyl and $-NR_aR_b$; wherein said $R_a$ and $R_b$ are each independently selected from H and C1-C6 alkyl,
m is an integer of 0, 1, 2 or 3; and
$R_c$ and $R_d$ are each independently H or C1-C4 alkyl.

**44.** The compound of Formula III according to claim 43, or a pharmaceutically acceptable salt thereof, or an enantiomer, a diastereoisomer, a tautomer, an isotope substitute, a solvate, a polymorph, a prodrug or metabolite thereof, wherein $R_0$ is C1-C6 alkyl substituted with $-NR_3R_4$; wherein $R_3$ and $R_4$ are each independently selected from H, C1-C6 alkyl optionally substituted with 1, 2, 3, 4, 5 or 6 substituents selected from halogen, cyano, C1-C6 alkoxy, halogenated C1-C6 alkoxy, $-NR_aR_b$ and hydroxyl, or $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

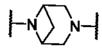 or 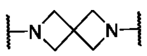

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

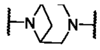 or 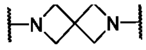

is each optionally substituted with 1, 2 or 3 substituents selected from halogen, cyano, C1-C6 alkyl, halogenated C1-C6 alkyl, C3-C6 cycloalkyl, C1-C6 alkoxy, halogenated C1-C6 alkoxy, sulfo, C1-C6 alkylsulfonyl, C1-C6 alkoxysulfonyl, C1-C6 alkylcarbonyl, halogenated C1-C6 alkylcarbonyl, C1-C6 alkoxycarbonyl, halogenated C1-C6 alkoxycarbonyl, $-NR_aR_b$ and hydroxyl, wherein $R_a$ and $R_b$ are each independently H or C1-C4 alkyl.

**45.** The compound of Formula III according to any one of claims 43-44, or a pharmaceutically acceptable salt thereof, or an enantiomer, a diastereoisomer, a tautomer, an isotope substitute, a solvate, a polymorph, a prodrug or metabolite thereof, wherein $R_0$ is C1-C6 alkyl substituted with $-NR_3R_4$, and wherein $R_3$ and $R_4$ form azetidinyl, pyrrolidinyl, piperidinyl, piperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

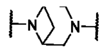 or 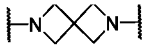

with the nitrogen atom to which they are attached, wherein said azetidinyl, pyrrolidinyl, piperidinyl, piperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

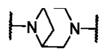 or 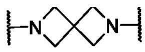

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, sulfo, C1-C6 alkylsulfonyl, C1-C6 alkoxysulfonyl, C1-C6 alkylcarbonyl, halogenated C1-C6 alkylcarbonyl, C1-C6 alkoxycarbonyl, halogenated C1-C6 alkoxycarbonyl, $-NR_aR_b$ and hydroxyl; wherein $R_a$ and $R_b$ are each independently H or C1-C4 alkyl.

**46.** The compound of Formula III according to any one of claims 43-45, or a pharmaceutically acceptable salt thereof, or an enantiomer, a diastereoisomer, a tautomer, an isotope substitute, a solvate, a polymorph, a prodrug or metabolite thereof, wherein $R_0$ is C1-C6 alkyl substituted with $-NR_3R_4$, wherein $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

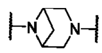 or 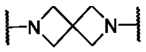

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

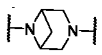 or 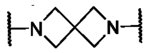

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, $-NR_aR_b$ and hydroxyl; wherein $R_a$ and $R_b$ are each independently H or C1-C4 alkyl.

**47.** The compound of Formula III according to any one of claims 43-46, or a pharmaceutically acceptable salt thereof, or an enantiomer, a diastereoisomer, a tautomer, an isotope substitute, a solvate, a polymorph, a prodrug or metabolite thereof, wherein $R_c$ and $R_d$ are each independently H or methyl.

**48.** The compound of Formula III according to any one of claims 43-47, or a pharmaceutically acceptable salt thereof, or an enantiomer, a diastereoisomer, a tautomer, an isotope substitute, a solvate, a polymorph, a prodrug or metabolite thereof, wherein $L_2$ is selected from a bond and C1-C6 alkylene, wherein said C1-C6 alkylene is optionally substituted with 1, 2 or 3 substituents selected from halogen, C1-C3 alkyl, C3-C5 cycloalkyl and hydroxyl.

**49.** The compound of Formula III according to any one of claims 43-48, or a pharmaceutically acceptable salt thereof, or an enantiomer, a diastereoisomer, a tautomer, an isotope substitute, a solvate, a polymorph, a prodrug or metabolite thereof, wherein $R_2$ is optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano; wherein $R_a$ and $R_b$ are each independently H or C1-C6 alkyl; and wherein said heteroaryl is preferably a heteroaryl containing 1 or 2 nitrogen ring atoms, preferably selected from pyrazolyl, imidazolyl, pyridyl, pyrazinyl and piperazinyl.

**50.** The compound of Formula III according to any one of claims 43-49, or a pharmaceutically acceptable salt thereof, or an enantiomer, a diastereoisomer, a tautomer, an isotope substitute, a solvate, a polymorph, a prodrug or metabolite thereof, wherein $R_2$ is selected from 6-14 membered aryl, 5-10 membered heteroaryl and 4-10 membered heterocyclyl, wherein said 6-14 membered aryl, 5-10 membered heteroaryl and 4-10 membered heterocyclyl are optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano.

**51.** The compound of Formula III according to any one of claims 43-50, or a pharmaceutically acceptable salt thereof, or an enantiomer, a diastereoisomer, a tautomer, an isotope substitute, a solvate, a polymorph, a prodrug or metabolite thereof, wherein $R_2$ is selected from phenyl, naphthyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl, azetidinyl, pyrrolidinyl, morpholinyl, piperazinyl, homopiperazinyl, piperidinyl, pyranyl, dihydrofuropyridinyl, pyrido 1,3-dioxolyl, 2-oxopiperazinyl, tetrahydropyranyl, tetrahydrofuranyl, oxazinyl, tetrahydroisoquinolyl, benzodihydrofuranyl, decahydroisoquinolyl, imidazolinyl, imidazolidinyl, 6,7-dihydro-5H-cyclopenta[c]pyridine, quinazinyl, thiazolidinyl, isothiazolidinyl, isoxazolidinyl, dihydroindolyl, octahydroindolyl, octahydroisoindolyl, pyrrolidinyl, pyrazolindinyl,

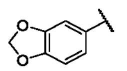 and 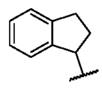,

wherein said phenyl, naphthyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl, azetidinyl, pyrrolidinyl, morpholinyl, piperazinyl, homopiperazinyl, piperidinyl, pyranyl, dihydrofuropyridinyl, pyrido 1,3-dioxolyl, 2-oxopiperazinyl, tetrahydropyranyl, tetrahydrofuranyl, oxazinyl, tetrahydroisoquinolyl, benzodihydrofuranyl, decahydroisoquinolyl, imidazolinyl, imidazolidinyl, 6,7-dihydro-5H-cyclopenta[c]pyridine, quinazinyl, thiazolidinyl, isothiazolidinyl, isoxazolidinyl, dihydroindolyl, octahydroindolyl, octahydroisoindolyl, pyrrolidinyl, pyrazolindinyl,

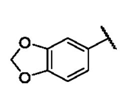 and 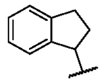

are optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano.

**52.** The compound of Formula III according to any one of claims 43-51, or a pharmaceutically acceptable salt thereof, or an enantiomer, a diastereoisomer, a tautomer, an isotope substitute, a solvate, a polymorph, a prodrug or metabolite thereof, wherein $L_2$ is C1-C4 alkylene, $R_2$ is 6-14 membered aryl, 5-10 membered nitrogen-containing heteroaryl or 4-10 membered heterocyclyl, preferably phenyl, pyridyl, pyrazolyl, pyrrolopyridyl, dihydrofuropyridinyl, pyrido 1,3-dioxolyl or indolyl, wherein said 6-14 membered aryl, 5-10 membered nitrogen-containing heteroaryl or 4-10 membered heterocyclyl may be optionally substituted with 1 or 2 substituents selected from C1-C6 alkoxy, halogen, C1-C6 alkyl, cyano, C3-C8 cycloalkyl-O- and halogenated C1-C6 alkoxy.

**53.** The compound of Formula III according to claim 43, or a pharmaceutically acceptable salt thereof, or an enantiomer, a diastereoisomer, a tautomer, an isotope substitute, a solvate, a polymorph, a prodrug or metabolite thereof, wherein

$R_0$ is H or C1-C4 alkyl substituted with $-NR_3R_4$, wherein $R_3$ and $R_4$ are each independently selected from H, 4-6 membered heterocyclyl and C1-C6 alkyl optionally substituted with 1, 2, 3, 4, 5 or 6 substituents selected from hydroxyl, cyano and halogen, or $R_3$ and $R_4$ form a 4-6 membered heterocyclyl optionally substituted with 1, 2 or 3 substituents selected from halogen, hydroxyl, C1-C4 alkoxy and C1-C4 alkyl with the nitrogen atom to which they are attached; wherein said 4-6 membered heterocyclyl is preferably an oxygen and/or nitrogen-containing heterocyclyl, preferably selected from the group consisting of azetidinyl, pyrrolidinyl, piperidinyl, piperazinyl, 2-oxopiperazinyl, morpholinyl,

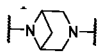 or 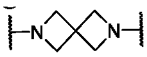;

$L_2$ is C1-C4 alkylene;

$R_2$ is 6-14 membered aryl, 5-10 membered nitrogen-containing heteroaryl or 4-10 membered heterocyclyl, preferably phenyl, pyridyl, pyrimidinyl, pyrazolyl, pyrrolyl, imidazolyl, pyrazinyl, indolyl, pyrrolopyridyl, dihydrofuropyridinyl or pyrido 1,3-dioxolyl, wherein said 6-14 membered aryl, 5-10 membered nitrogen-containing heteroaryl or 4-10 membered heterocyclyl is each optionally substituted with 1 or 2 substituents selected from C1-C6 alkoxy, C1-C4 alkyl, halogen, C3-C6 cycloalkyl-oxy, halogenated C1-C4 alkoxy and cyano;

$R_c$ and $R_d$ are each independently H or C1-C4 alkyl;

$X_6$ is C or O;

q is 1 or 2; and

$R_7$ is selected from the group consisting of F, Cl, cyano, hydroxyl, C1-C6 alkyl, fluorinated or chlorinated C1-C6 alkyl, hydroxyl-substituted C1-C6 alkyl, C1-C6 alkoxy, fluorinated or chlorinated C1-C6 alkoxy and hydroxyl-substituted C1-C6 alkyl.

**54.** The compound of Formula III according to claim 43, or a pharmaceutically acceptable salt thereof, or an enantiomer, a diastereoisomer, a tautomer, an isotope substitute, a solvate, a polymorph, a prodrug or metabolite thereof, wherein

$L_2$ is C1-C4 alkylene;

$R_2$ is phenyl, 5-10 membered nitrogen-containing heteroaryl or 4-10 membered heterocyclyl, preferably pyridyl, pyrimidinyl, pyrazolyl, pyrrolyl, imidazolyl, pyrazinyl, pyrrolopyridyl, dihydrofuropyridinyl or pyrido 1,3-dioxolyl or indolyl, wherein said phenyl, 5-10 membered nitrogen-containing heteroaryl and 4-10 membered heterocyclyl are each optionally substituted with 1 or 2 substituents selected from C1-C6 alkoxy, halogen, C1-C6 alkyl, cyano, C3-C8 cycloalkyl-O- and halogenated C1-C6 alkoxy;

$R_0$ is C1-C4 alkyl substituted with $-NR_3R_4$; wherein $R_3$ and $R_4$ are each independently selected from H, C1-C6 alkyl optionally substituted with 1-6 substituents selected from hydroxyl, cyano and halogen, and 4-8 membered heterocyclyl optionally substituted with 1, 2 or 3 substituents selected from halogen, C1-C6 alkyl, halogenated C1-C6 alkyl, C1-C6 alkoxy, halogenated C1-C6 alkoxy, $-NR_aR_b$ and hydroxyl, wherein $R_a$ and $R_b$ are each independently H or C1-C4 alkyl; or $R_3$ and $R_4$ form a 4-8 membered heterocyclyl optionally substituted with 1, 2 or 3 substituents selected from halogen, C1-C6 alkyl, halogenated C1-C6 alkyl, C1-C6 alkoxy, halogenated C1-C6 alkoxy, $-NR_aR_b$ and hydroxyl with the nitrogen atom to which they are attached, wherein $R_a$ and $R_b$ are each independently H or C1-C4 alkyl; preferably, said 4-8 membered heterocyclyl is each independently selected from azetidinyl, pyrrolidinyl, piperidinyl, piperazinyl, 2-oxopiperazinyl, morpholinyl,

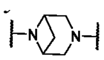 or 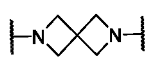;

$R_c$ and $R_d$ are each independently H or C1-C4 alkyl;

$X_6$ is C or O;

q is 1 or 2; and

$R_7$ is selected from F, Cl, cyano, hydroxyl, C1-C6 alkyl, fluorinated or chlorinated C1-C6 alkyl, hydroxyl-substituted C1-C6 alkyl, C1-C6 alkoxy, fluorinated or chlorinated C1-C6 alkoxy and hydroxyl-substituted C1-C6 alkyl.

**55.** The compound of Formula III according to claim 43, or a pharmaceutically acceptable salt thereof, or an enantiomer, a diastereoisomer, a tautomer, an isotope substitute, a solvate, a polymorph, a prodrug or metabolite thereof, wherein

$L_2$ is C1-C4 alkylene;

$R_2$ is 5-10 membered nitrogen-containing heteroaryl or 4-10 membered heterocyclyl, preferably pyridyl, pyrimidinyl, pyrazolyl, pyrrolyl, imidazolyl, pyrazinyl, pyrrolopyridyl, dihydrofuropyridinyl or pyrido 1,3-dioxolyl or indolyl, wherein said 5-10 membered nitrogen-containing heteroaryl or 4-10 membered heterocyclyl is each optionally substituted with 1 or 2 substituents selected from C1-C6 alkoxy, C1-C6 alkyl, C3-C8 cycloalkyl-O-, halogenated C1-C6 alkoxy, halo and cyano;

$R_0$ is C1-C4 alkyl substituted with $-NR_3R_4$, wherein $R_3$ and $R_4$ are each independently selected from H and C1-C6 alkyl optionally substituted with 1, 2, 3, 4, 5 or 6 substituents selected from hydroxyl, cyano and halogen, or $R_3$ and $R_4$ form a 4-8 membered heterocyclyl optionally substituted with 1, 2 or 3 substituents selected from halogen, hydroxyl and C1-C4 alkyl with the nitrogen atom to which they are attached, preferably, said 4-8 membered heterocyclyl is selected from the group consisting of azetidinyl, pyrrolidinyl, piperidinyl, piperazinyl, morpholinyl,

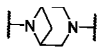 or 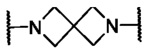;

$R_c$ and $R_d$ are each independently H or C1-C4 alkyl;

$X_6$ is C or O;

q is 1 or 2; and

$R_7$ is selected from F, Cl, cyano, hydroxyl, C1-C6 alkyl, fluorinated or chlorinated C1-C6 alkyl, hydroxyl-substituted C1-C6 alkyl, C1-C6 alkoxy, fluorinated or chlorinated C1-C6 alkoxy and hydroxyl-substituted C1-C6 alkyl.

**56.** A compound of Formula I, or a pharmaceutically acceptable salt thereof, or an enantiomer, a diastereoisomer, a tautomer, an isotope substitute, a solvate, a polymorph, a prodrug or metabolite thereof:

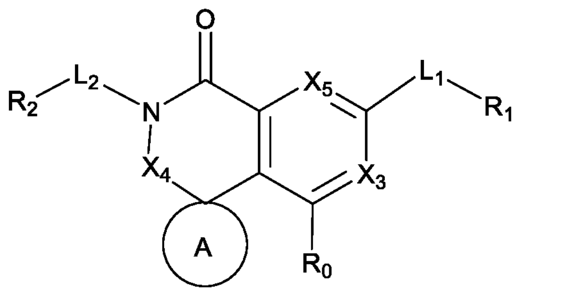

Formula I

wherein,

ring A is a substituted or unsubstituted 3-8 membered saturated carboncycle or a substituted or unsubstituted 3-8 membered saturated heterocycle, wherein said heterocycle contains 1-2 hetero atoms selected from N, O and S;

$X_3$ is selected from $CR_5$ and N;

$X_4$ is selected from optionally substituted $-(CH_2)_n-$, -C(=O)- and $NR_6$, wherein n is 1, 2 or 3;

$X_5$ is selected from N and $CR_5$;

$L_1$ is selected from a bond, optionally substituted C1-C6 alkylene, optionally substituted C1-C6 alkylene-O-, optionally substituted C1-C6 alkylene-NH-, optionally substituted C1-C6 alkylene-S- and optionally substituted C2-C4 alkenylene;

$L_2$ is selected from a bond, optionally substituted C1-C6 alkylene, optionally substituted C1-C6 alkylene-O-, optionally substituted C1-C6 alkylene-NH-, optionally substituted C1-C6 alkylene-S- and optionally substituted C2-C4 alkenylene;

$R_0$ is selected from optionally substituted C1-C6 haloalkyl, $-C(O)NR_3R_4$, $-CH_2OR_3$ and C1-C6 alkyl substituted with $-NR_3R_4$ ;

$R_1$ is selected from optionally substituted cycloalkyl, optionally substituted aryl, optionally substituted heteroaryl, optionally substituted heterocyclyl, optionally substituted amino and guanidyl;

$R_2$ is selected from optionally substituted cycloalkyl, optionally substituted aryl, optionally substituted heteroaryl and optionally substituted heterocyclyl;

$R_3$ and $R_4$ are each independently selected from H, optionally substituted C1-C6 alkyl, optionally substituted C1-C6 alkoxy, optionally substituted C1-C6 haloalkyl, optionally substituted 4-10 membered heterocyclyl and

optionally substituted 5-10 membered heteroaryl; or $R_3$ and $R_4$ form optionally substituted 4-10 membered heterocyclyl or 5-10 membered heteroaryl with the nitrogen atom to which they are attached;
$R_5$ is selected from H, halogen, C1-C6 alkyl, halogenated C1-C6 alkyl and C1-C6 alkoxy; and
$R_6$ is selected from H, C1-C6 alkyl, halogenated C1-C6 alkyl and C1-C6 alkoxy.

**57.** The compound of Formula I according to claim 56, or a pharmaceutically acceptable salt thereof, or an enantiomer, a diastereoisomer, a tautomer, an isotope substitute, a solvate, a polymorph, a prodrug or metabolite thereof, wherein ring A is C3-C8 cycloalkyl or 3-7 membered heterocyclyl.

**58.** The compound of Formula I according to claim 56 or 57, or a pharmaceutically acceptable salt thereof, or an enantiomer, a diastereoisomer, a tautomer, an isotope substitute, a solvate, a polymorph, a prodrug or metabolite thereof, wherein ring A is selected from cyclopropyl, cyclobutyl, cyclopentyl, cyclohexyl, aziridinyl, azetidinyl, pyrrolidinyl, piperidinyl, piperazinyl, oxiranyl, oxetanyl, tetrahydrofuranyl, tetrahydropyranyl and morpholinyl.

**59.** The compound of Formula I according to any one of claims 56-58, or a pharmaceutically acceptable salt thereof, or an enantiomer, a diastereoisomer, a tautomer, an isotope substitute, a solvate, a polymorph, a prodrug or metabolite thereof, wherein ring A is substituted with 1, 2 or 3 substituents selected from halogen, hydroxyl, cyano, C1-C6 alkyl, halogenated C1-C6 alkyl, hydroxyl-substituted C1-C6 alkyl, C1-C6 alkoxy, halogenated C1-C6 alkoxy and $-NR_aR_b$; wherein said $R_a$ and $R_b$ are each independently selected from H and C1-C6 alkyl.

**60.** The compound of Formula I according to any one of claims 56-59, or a pharmaceutically acceptable salt thereof, or an enantiomer, a diastereoisomer, a tautomer, an isotope substitute, a solvate, a polymorph, a prodrug or metabolite thereof, wherein ring A is cyclopropyl, cyclobutyl, cyclopentyl, azetidinyl, oxetanyl, pyrrolidinyl or tetrahydrofuranyl,

**61.** The compound of Formula I according to any one of claims 56-60, or a pharmaceutically acceptable salt thereof, or an enantiomer, a diastereoisomer, a tautomer, an isotope substitute, a solvate, a polymorph, a prodrug or metabolite thereof, wherein ring A is optionally substituted with 1, 2 or 3 substituents selected from F, Cl, Br, $CF_3$, $CH_3$, -OH, $-OCH_3$, $-NH_2$, $-NHCH_3$ and$-NH_2(CH_3)_2$.

**62.** The compound of Formula I according to any one of claims 56-61, or a pharmaceutically acceptable salt thereof, or an enantiomer, a diastereoisomer, a tautomer, an isotope substitute, a solvate, a polymorph, a prodrug or metabolite thereof, wherein $R_0$ is C1-C6 alkyl substituted with $-NR_3R_4$; wherein $R_3$ and $R_4$ are each independently selected from H, C1-C6 alkyl optionally substituted with 1, 2, 3, 4, 5 or 6 substituents selected from halogen, cyano, C1-C6 alkoxy, halogenated C1-C6 alkoxy, $-NR_aR_b$ and hydroxyl, or $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

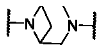 or 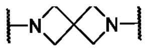

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

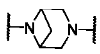 or 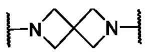

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, sulfo, C1-C6 alkylsulfonyl, C1-C6 alkoxysulfonyl, C1-C6 alkylcarbonyl, halogenated C1-C6 alkylcarbonyl, C1-C6 alkoxycarbonyl, halogenated C1-C6 alkoxycarbonyl, $-NR_aR_b$ and hydroxyl, wherein $R_a$ and $R_b$ are each independently H or C1-C4 alkyl.

**63.** The compound of Formula I according to any one of claims 56-62, or a pharmaceutically acceptable salt thereof, or an enantiomer, a diastereoisomer, a tautomer, an isotope substitute, a solvate, a polymorph, a prodrug or metabolite thereof, wherein $R_0$ is C1-C6 alkyl substituted with $-NR_3R_4$, and wherein $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl,

pyrazinyl, triazinyl, indolyl, isoindolyl, indazolyl, benzopyrrolyl, benzimidazolyl, benzotriazolyl, benzoxazolyl, benzisoxazolyl, quinolyl,

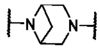 or 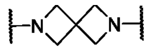

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

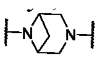 or 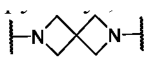

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, sulfo, C1-C6 alkylsulfonyl, C1-C6 alkoxysulfonyl, C1-C6 alkylcarbonyl, halogenated C1-C6 alkylcarbonyl, C1-C6 alkoxycarbonyl, halogenated C1-C6 alkoxycarbonyl, $-NR_aR_b$ and hydroxyl; wherein $R_a$ and $R_b$ are each independently H or C1-C4 alkyl.

**64.** The compound of Formula I according to any one of claims 56-63, or a pharmaceutically acceptable salt thereof, or an enantiomer, a diastereoisomer, a tautomer, an isotope substitute, a solvate, a polymorph, a prodrug or metabolite thereof, wherein $R_0$ is C1-C6 alkyl substituted with $-NR_3R_4$, wherein $R_3$ and $R_4$ form azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

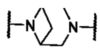 or 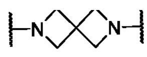

with the nitrogen atom to which they are attached, wherein said azetidinyl, pyrrolidinyl, piperidinyl, piperazinyl, 2-oxopiperazinyl, morpholinyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl,

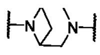 or 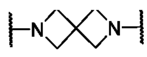

is each optionally substituted with 1, 2 or 3 substituents selected from halogen, cyano, C1-C6 alkyl, C3-C6 cycloalkyl, halogenated C1-C6 alkyl, C1-C6 alkoxy, halogenated C1-C6 alkoxy, $-NR_aR_b$ and hydroxyl; wherein $R_a$ and $R_b$ are each independently H or C1-C4 alkyl.

**65.** The compound of Formula I according to any one of claims 56-64, or a pharmaceutically acceptable salt thereof, or an enantiomer, a diastereoisomer, a tautomer, an isotope substitute, a solvate, a polymorph, a prodrug or metabolite thereof, wherein
$X_3$ and/or $X_5$ is $CR_5$.

**66.** The compound of Formula I according to any one of claims 56-65, or a pharmaceutically acceptable salt thereof, or an enantiomer, a diastereoisomer, a tautomer, an isotope substitute, a solvate, a polymorph, a prodrug or metabolite thereof, wherein $R_5$ is selected from H and C1-C4 alkyl.

**67.** The compound of Formula I according to any one of claims 56-66, or a pharmaceutically acceptable salt thereof, or an enantiomer, a diastereoisomer, a tautomer, an isotope substitute, a solvate, a polymorph, a prodrug or metabolite thereof, wherein $X_4$ is $-(CH_2)_n-$, n is 1 or 2; and wherein said-$(CH_2)_n$- is optionally substituted with 1 or 2 substituents selected from halogen, hydroxyl, amino, C1-C6 alkyl and halogenated C1-C6 alkyl.

**68.** The compound of Formula I according to any one of claims 56-67, or a pharmaceutically acceptable salt thereof, or an enantiomer, a diastereoisomer, a tautomer, an isotope substitute, a solvate, a polymorph, a prodrug or metabolite thereof, wherein $X_3$ and $X_5$ are $CR_5$; and $X_4$ is optionally substituted $-(CH_2)-$.

**69.** The compound of Formula I according to any one of claims 56-68, or a pharmaceutically acceptable salt thereof, or an enantiomer, a diastereoisomer, a tautomer, an isotope substitute, a solvate, a polymorph, a prodrug or metabolite thereof, wherein $L_1$ is selected from C1-C6 alkylene, C1-C6 alkylene-O-, C1-C6 alkylene-NH-, C1-C6 alkylene-S- and C2-C4 alkenylene, wherein said C1-C6 alkylene, C1-C6 alkylene-O-, C1-C6 alkylene-NH-, C1-C6 alkylene-S- and C2-C4 alkenylene are optionally substituted with 1, 2 or 3 substituents selected from halogen, C1-C4 alkyl and hydroxyl.

**70.** The compound of Formula I according to any one of claims 56-69, or a pharmaceutically acceptable salt thereof, or an enantiomer, a diastereoisomer, a tautomer, an isotope substitute, a solvate, a polymorph, a prodrug or metabolite thereof, wherein $L_1$ is selected from C1-C6 alkylene and C1-C6 alkylene-NH-, wherein said C1-C6 alkylene and C1-C6 alkylene-NH- are optionally substituted with 1, 2 or 3 substituents selected from halogen, C1-C4 alkyl and hydroxyl.

**71.** The compound of Formula I according to any one of claims 56-70, or a pharmaceutically acceptable salt thereof, or an enantiomer, a diastereoisomer, a tautomer, an isotope substitute, a solvate, a polymorph, a prodrug or metabolite thereof, wherein $R_1$ is selected from optionally substituted C3-C8 cycloalkyl, optionally substituted 6-14 membered aryl, optionally substituted 5-10 membered heteroaryl and optionally substituted 4-10 membered heterocyclyl, wherein said cycloalkyl, aryl, heteroaryl and heterocyclyl are each optionally substituted with 1, 2 or 3 substituents selected from optionally substituted amino, C1-C6 alkyl, halogen, hydroxyl, C1-C6 alkoxy, C1-C6 haloalkyl and cyano; wherein said optionally substituted amino is $-NR_aR_b$, wherein $R_a$ and $R_b$ are each independently H or C1-C6 alkyl.

**72.** The compound of Formula I according to any one of claims 56-71, or a pharmaceutically acceptable salt thereof, or an enantiomer, a diastereoisomer, a tautomer, an isotope substitute, a solvate, a polymorph, a prodrug or metabolite thereof, wherein $R_1$ is selected from optionally substituted phenyl, naphthyl pyridyl, pyrrolyl, pyrazolyl, imidazolyl, triazolyl, pyrimidinyl, pyrazinyl, triazinyl, imidazopyridinyl, indolyl, isoindolyl, indazolyl, benzimidazolyl, benzotriazolyl, benzoxazolyl, benzisoxazolyl, quinolyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, decahydroisoquinolyl, imidazolinyl, imidazolidinyl, quinazinyl, thiazolidinyl, isothiazolidinyl, isoxazolidinyl, dihydroindolyl, octahydroindolyl, octahydroisoindolyl, pyrrolidinyl, pyrazolindinyl, phthalimidinyl,

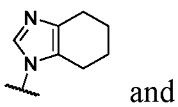 and 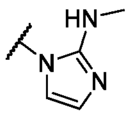.

**73.** The compound of Formula I according to any one of claims 56-72, or a pharmaceutically acceptable salt thereof, or an enantiomer, a diastereoisomer, a tautomer, an isotope substitute, a solvate, a polymorph, a prodrug or metabolite thereof, wherein said $L_1$ is C1-C4 alkylene or C1-C4 alkylene-NH-, $R_1$ is pyridyl, pyrrolyl, pyrazolyl, imidazolyl, triazolyl, pyrimidinyl, pyrazinyl, triazinyl or

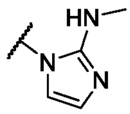,

wherein said pyridyl, pyrrolyl, pyrazolyl, imidazolyl, triazolyl, pyrimidinyl, pyrazinyl, triazinyl or

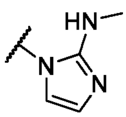

is optionally substituted with 1 or 2 substituents selected from halogen, C1-C4 alkyl and $-NR_aR_b$, wherein $R_a$ and $R_b$ are each independently H or C1-C4 alkyl.

**74.** The compound of Formula I according to any one of claims 56-73, or a pharmaceutically acceptable salt thereof, or an enantiomer, a diastereoisomer, a tautomer, an isotope substitute, a solvate, a polymorph, a prodrug or me-

tabolite thereof, wherein $L_2$ is selected from a bond and C1-C6 alkylene, wherein said C1-C6 alkylene is optionally substituted with 1, 2 or 3 substituents selected from halogen, C1-C3 alkyl, C3-C5 cycloalkyl and hydroxyl.

**75.** The compound of Formula I according to any one of claims 56-74, or a pharmaceutically acceptable salt thereof, or an enantiomer, a diastereoisomer, a tautomer, an isotope substitute, a solvate, a polymorph, a prodrug or metabolite thereof, wherein $R_2$ is optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano; wherein $R_a$ and $R_b$ are each independently H or C1-C6 alkyl; and wherein said heteroaryl is preferably a heteroaryl containing 1 or 2 nitrogen ring atoms, preferably selected from pyrazolyl, imidazolyl, pyridyl, pyrazinyl and piperazinyl.

**76.** The compound of Formula I according to any one of claims 56-75, or a pharmaceutically acceptable salt thereof, or an enantiomer, a diastereoisomer, a tautomer, an isotope substitute, a solvate, a polymorph, a prodrug or metabolite thereof, wherein $R_2$ is selected from 6-14 membered aryl, 5-10 membered heteroaryl and 4-10 membered heterocyclyl, wherein said 6-14 membered aryl, 5-10 membered heteroaryl and 4-10 membered heterocyclyl may be optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano.

**77.** The compound of Formula I according to any one of claims 56-76, or a pharmaceutically acceptable salt thereof, or an enantiomer, a diastereoisomer, a tautomer, an isotope substitute, a solvate, a polymorph, a prodrug or metabolite thereof, wherein $R_2$ is selected from phenyl, naphthyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl, azetidinyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, benzodihydrofuranyl, decahydroisoquinolyl, imidazolinyl, imidazolidinyl, 6,7-dihydro-5H-cyclopenta[c]pyridine, quinazinyl, thiazolidinyl, isothiazolidinyl, isoxazolidinyl, dihydroindolyl, octahydroindolyl, octahydroisoindolyl, pyrrolidinyl, pyrazolindinyl,

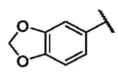 and 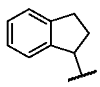,

wherein said phenyl, naphthyl, pyridyl, pyrrolyl, pyrazolyl, imidazolyl, triazolyl, pyrimidinyl, pyrazinyl, triazinyl, indolyl, isoindolyl, indazolyl, benzopyrrolyl, benzimidazolyl, benzotriazolyl, benzoxazolyl, benzisoxazolyl, quinolyl, azetidinyl, pyrrolidinyl, morpholinyl, piperazinyl, homopiperazinyl, piperidinyl, pyranyl, dihydrofuropyridinyl, pyrido 1,3-dioxolyl, tetrahydropyranyl, tetrahydrofuranyl, oxazinyl, tetrahydroisoquinolyl, benzodihydrofuranyl, decahydroisoquinolyl, imidazolinyl, imidazolidinyl, 6,7-dihydro-SH-cyclopenta[c]pyridine, quinazinyl, thiazolidinyl, isothiazolidinyl, isoxazolidinyl, dihydroindolyl, octahydroindolyl, octahydroisoindolyl, pyrrolidinyl, pyrazolindinyl,

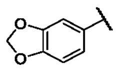

and

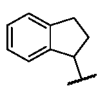

are optionally substituted with 1, 2 or 3 substituents selected from $-NR_aR_b$, cyano, C1-C6 alkyl, halogen, hydroxyl, C1-C6 alkoxy, halogenated C1-C6 alkyl, halogenated C1-C6 alkoxy, C3-C8 cycloalkyl, C3-C8 cycloalkyl-O-, 6-14 membered aryl, 5-10 membered heteroaryl and cyano.

**78.** The compound of Formula I according to any one of claims 56-77, or a pharmaceutically acceptable salt thereof, or an enantiomer, a diastereoisomer, a tautomer, an isotope substitute, a solvate, a polymorph, a prodrug or metabolite thereof, wherein $L_2$ is C1-C4 alkylene, $R_2$ is 6-14 membered aryl, 5-10 membered nitrogen-containing

heteroaryl or 4-10 membered heterocyclyl, preferably phenyl, pyridyl, pyrazolyl, pyrrolopyridyl, dihydrofuropyridinyl, pyrido 1,3-dioxolyl or indolyl, wherein said 6-14 membered aryl, 5-10 membered nitrogen-containing heteroaryl or 4-10 membered heterocyclyl may be optionally substituted with 1 or 2 substituents selected from C1-C6 alkoxy, halogen, C1-C6 alkyl, cyano, C3-C8 cycloalkyl-O- and halogenated C1-C6 alkoxy.

**79.** The compound of Formula I according to claim 56, or a pharmaceutically acceptable salt thereof, or an enantiomer, a diastereoisomer, a tautomer, an isotope substitute, a solvate, a polymorph, a prodrug or metabolite thereof, wherein

ring A is C3-C6 cycloalkyl or 4-8 membered heterocyclyl, wherein said cycloalkyl or heterocyclyl is optionally substituted with 1, 2 or 3 substituents selected from halogen, C1-C4 alkyl, halogenated C1-C4 alkyl, C1-C4 alkoxy and halogenated C1-C4 alkoxy;
$X_3$ and $X_5$ are both CH;
$X_4$ is $CH_2$;
$L_1$ is C1-C4 alkylene;
$R_1$ is

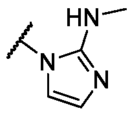
;

$L_2$ is C1-C4 alkylene;
$R_2$ is phenyl, 5-10 membered nitrogen-containing heteroaryl or 4-10 membered heterocyclyl, preferably pyridyl, pyrimidinyl, pyrazolyl, pyrrolyl, imidazolyl, pyrazinyl, pyrrolopyridyl, dihydrofuropyridinyl or pyrido 1,3-dioxolyl or indolyl, wherein said phenyl, 5-10 membered nitrogen-containing heteroaryl and 4-10 membered heterocyclyl are each optionally substituted with 1 or 2 substituents selected from C1-C6 alkoxy, halogen, C1-C6 alkyl, cyano, C3-C8 cycloalkyl-O- and halogenated C1-C6 alkoxy;
$R_0$ is C1-C4 alkyl substituted with $-NR_3R_4$; wherein $R_3$ and $R_4$ are each independently selected from H, C1-C6 alkyl optionally substituted with 1-6 substituents selected from hydroxyl, cyano and halo and a 4-8 membered heterocyclyl optionally substituted with 1, 2 or 3 substituents selected from halogen, C1-C6 alkyl, C3-C6 cycloalkyl, halogenated C1-C6 alkyl, C1-C6 alkoxy, halogenated C1-C6 alkoxy, $-NR_aR_b$ and hydroxyl, wherein $R_a$ and $R_b$ are each independently H or C1-C4 alkyl; or $R_3$ and $R_4$ form a 4-8 membered heterocyclyl optionally substituted with 1, 2 or 3 substitutents selected from halogen, C1-C6 alkyl, halogenated C1-C6 alkyl, C1-C6 alkoxy, halogenated C1-C6 alkoxy, $-NR_aR_b$ and hydroxyl with the nitrogen atom to which they are attached, wherein $R_a$ and $R_b$ are each independently H or C1-C4 alkyl; preferably, said 4-8 membered heterocyclyl is each independently selected from the group consisting of azetidinyl, pyrrolidinyl, piperidinyl, piperazinyl, morpholinyl,

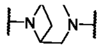
or
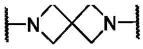
.

**80.** The compound of Formula I according to claim 56, or a pharmaceutically acceptable salt thereof, or an enantiomer, a diastereoisomer, a tautomer, an isotope substitute, a solvate, a polymorph, a prodrug or metabolite thereof, wherein

ring A is C3-C6 cycloalkyl, wherein said cycloalkyl is optionally substituted with 1, 2 or 3 substituents selected from halogen, C1-C4 alkyl and halogenated C1-C4 alkyl;
$X_3$ and $X_5$ are both CH;
$X_4$ is $CH_2$;
$L_1$ is C1-C4 alkylene;
$R_1$ is

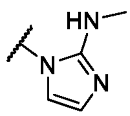
;

$L_2$ is C1-C4 alkylene;
$R_2$ is 5-10 membered nitrogen-containing heteroaryl or 4-10 membered heterocyclyl, preferably pyridyl, pyrimidinyl, pyrazolyl, pyrrolyl, imidazolyl, pyrazinyl, pyrrolopyridyl, dihydrofuropyridinyl or pyrido 1,3-dioxolyl or indolyl, wherein said 5-10 membered nitrogen-containing heteroaryl or 4-10 membered heterocyclyl is each optionally substituted with 1 or 2 substituents selected from C1-C6 alkoxy, C1-C6 alkyl, C3-C8 cycloalkyl-O-, halogenated C1-C6 alkoxy, halo and cyano;

$R_0$ is C1-C4 alkyl substituted with $-NR_3R_4$, wherein $R_3$ and $R_4$ are each independently selected from H and C1-C6 alkyl optionally substituted with 1, 2, 3, 4, 5 or 6 substituents selected from hydroxyl, cyano and halogen, or $R_3$ and $R_4$ form a 4-8 membered heterocyclyl optionally substituted with 1, 2 or 3 substituents selected from halogen, hydroxyl, C1-C4 alkoxy and C1-C4 alky with the nitrogen atom to which they are attached, preferably, said 4-8 membered heterocyclyl is selected from the group consisting of azetidinyl, pyrrolidinyl, piperidinyl, piperazinyl, morpholinyl,

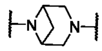 or 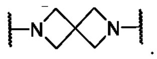.

**81.** The following compound, or a pharmaceutically acceptable salt thereof, or an enantiomer, a diastereoisomer, a tautomer, an isotope substitute, a solvate, a polymorph, a prodrug or metabolite thereof, wherein the compound is selected from a group consisting of:

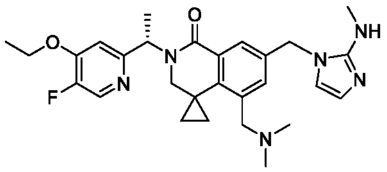, 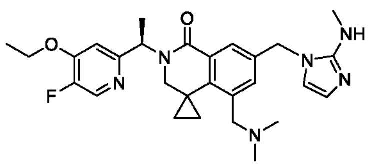, 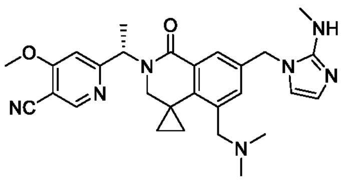,

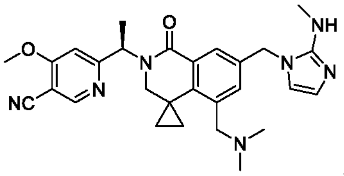, 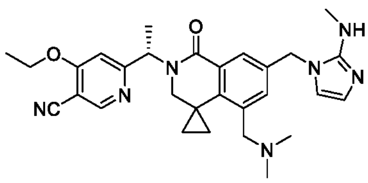, 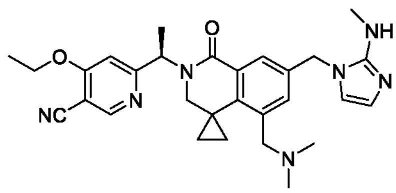,

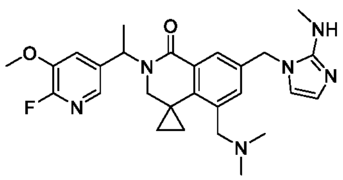, 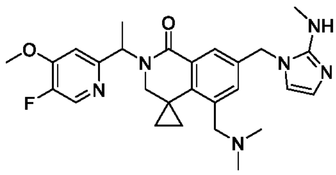, 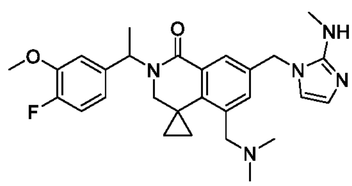,

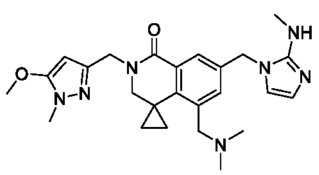, 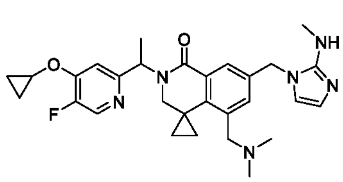, 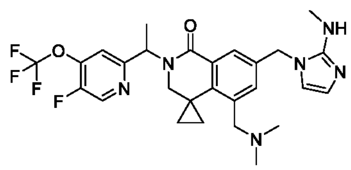,

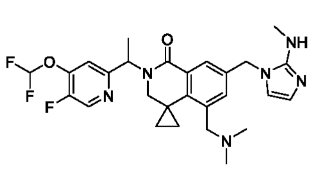, 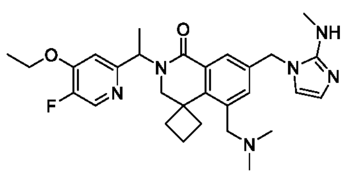, 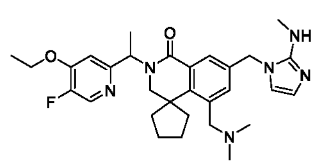,

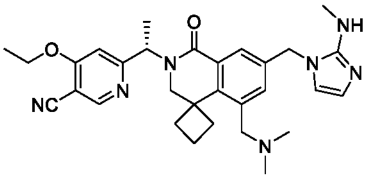,
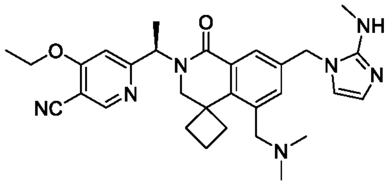,
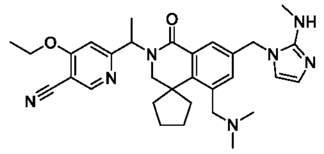,

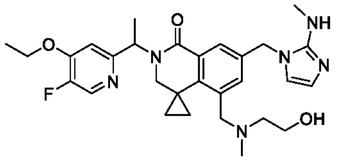,
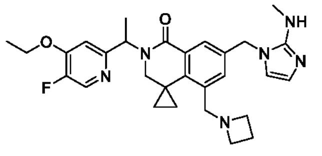,
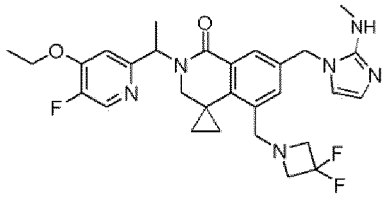,

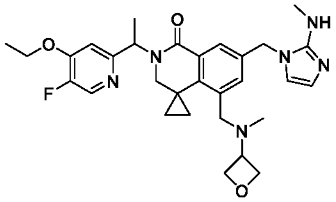,
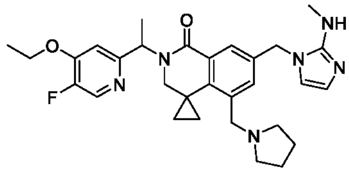,
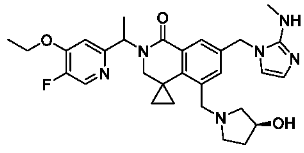,

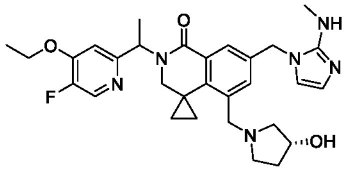,
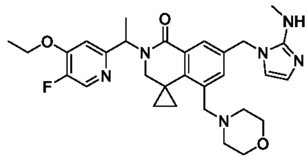,
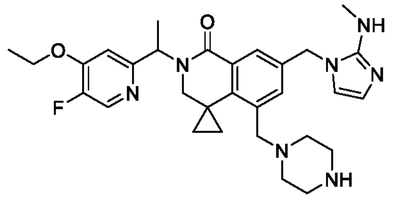,

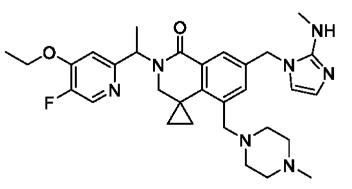,
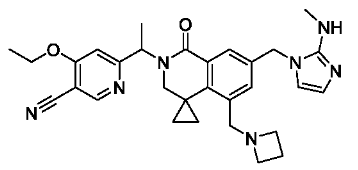,
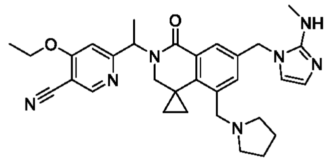,

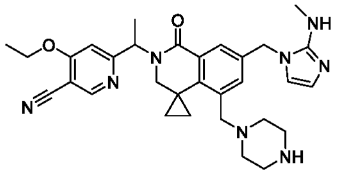,
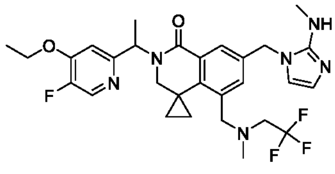,
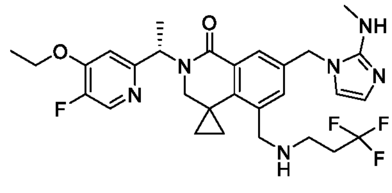,

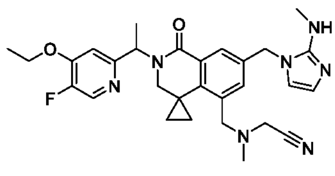 ,
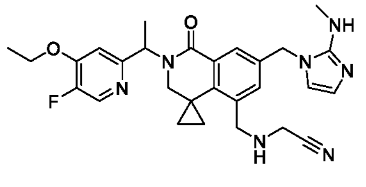 ,
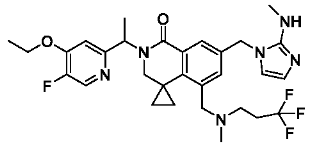 ,

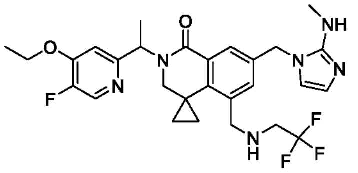 ,
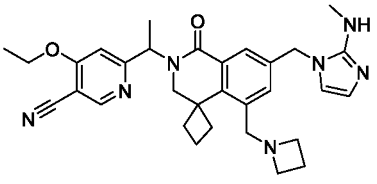 ,
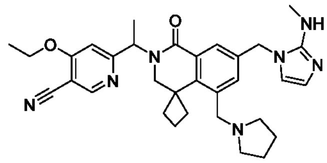 ,

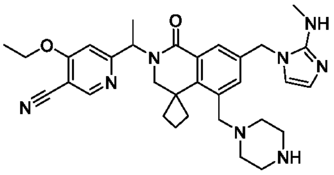 ,
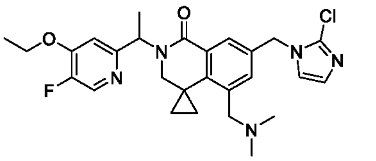 ,
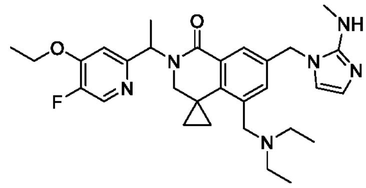 ,

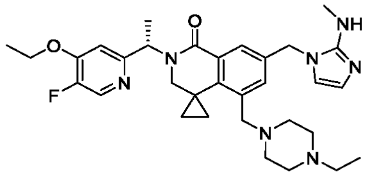 ,
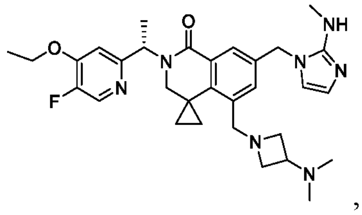 ,
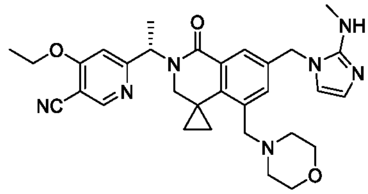 ,

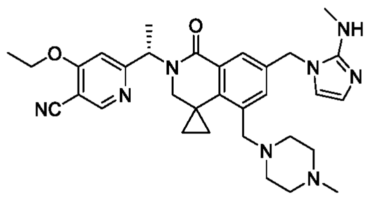 ,
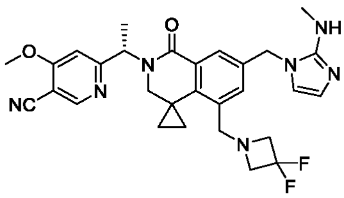 ,
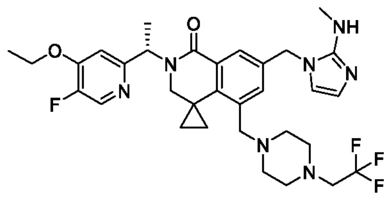 ,

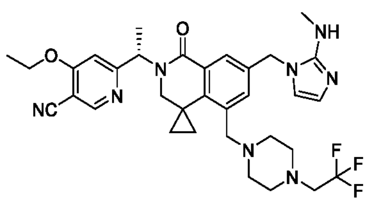 ,
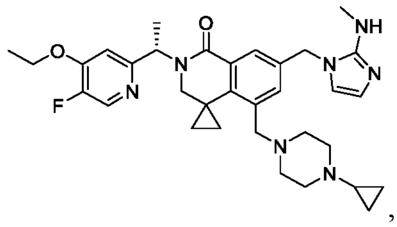 ,
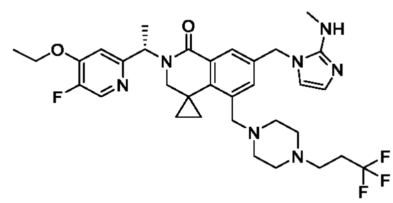 ,

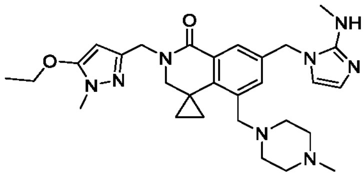,
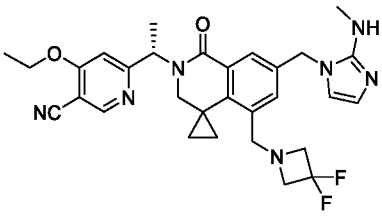,
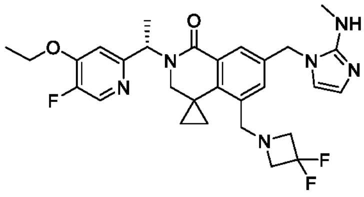,

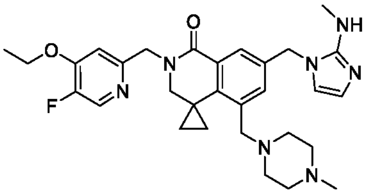,
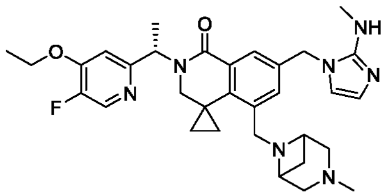,
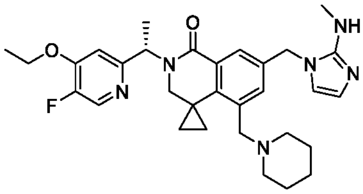,

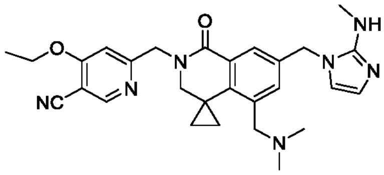,
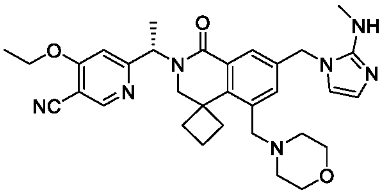,
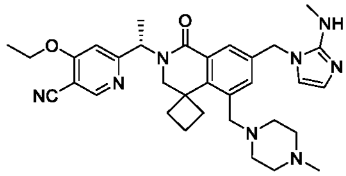,

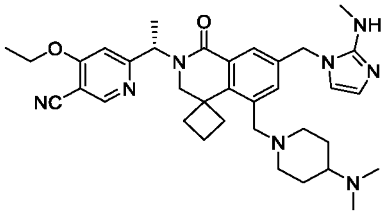,
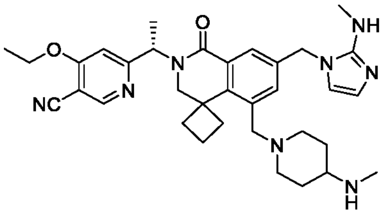,
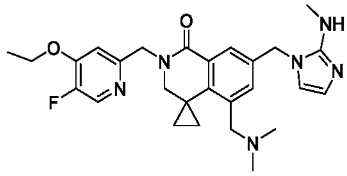,

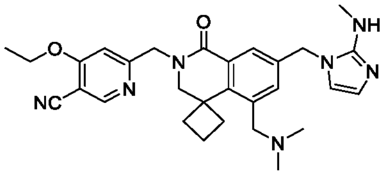,
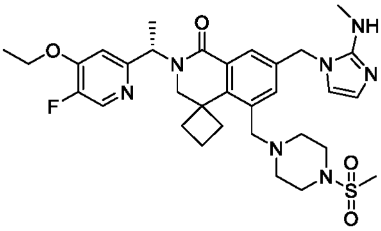,
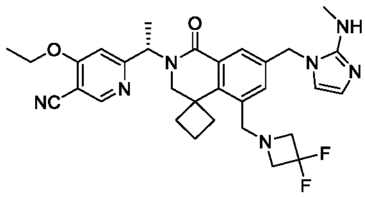,

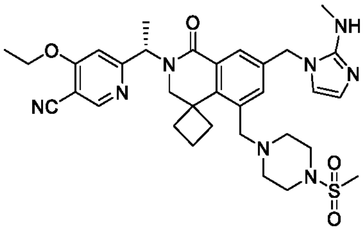,
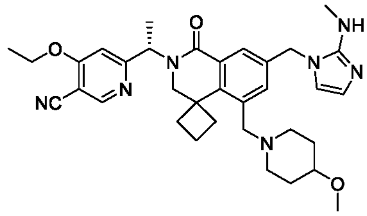,
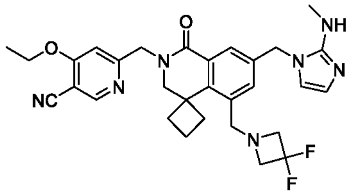,

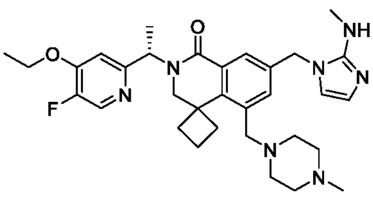 , 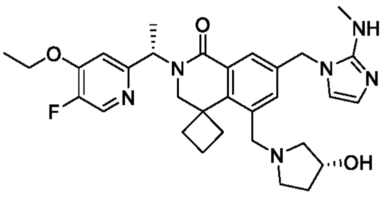 , 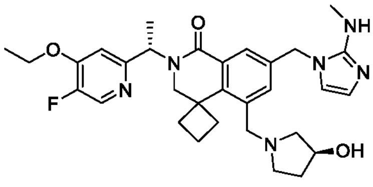 ,

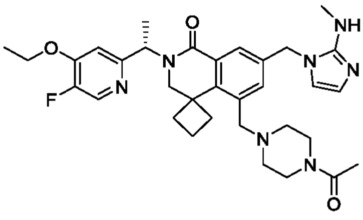 , 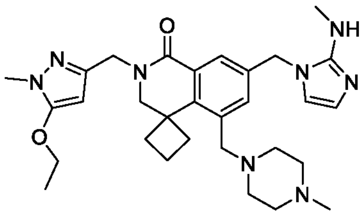 , 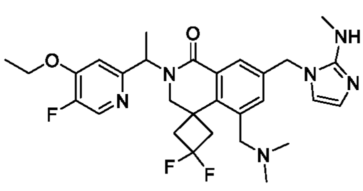 ,

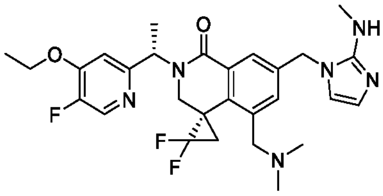 , 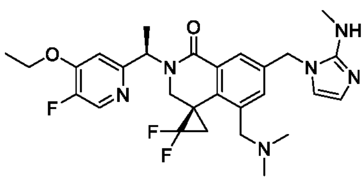 , 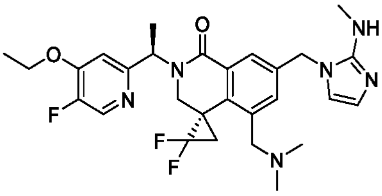 ,

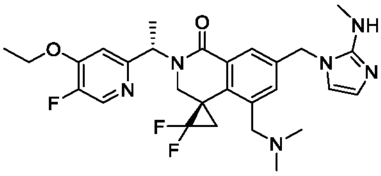 , 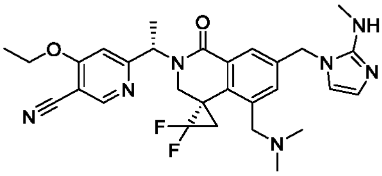 , 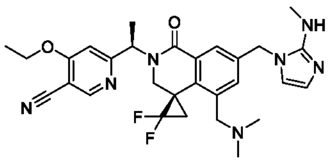 ,

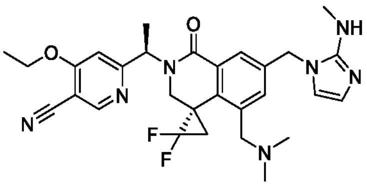 , 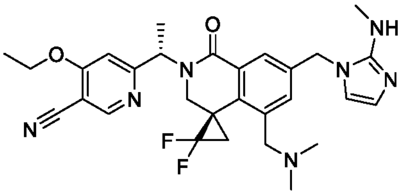 , 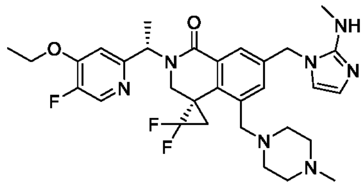 ,

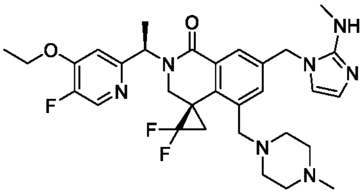 , 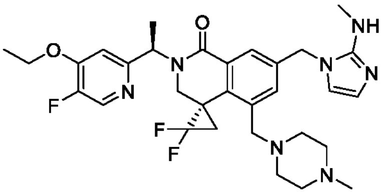 , 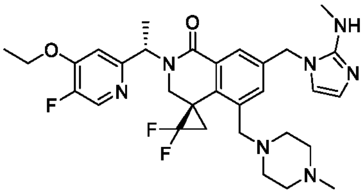 ,

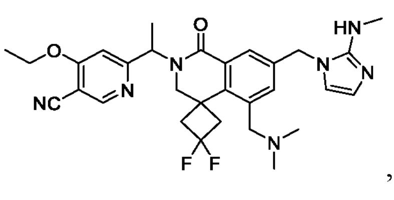 , 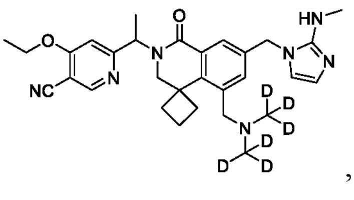 , 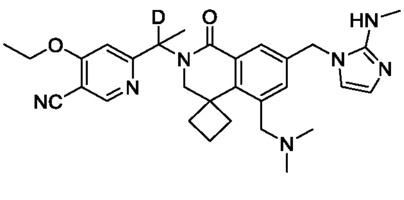 ,

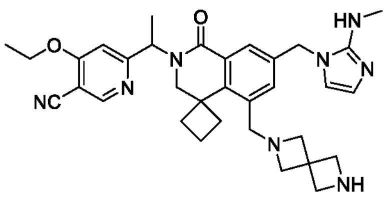, 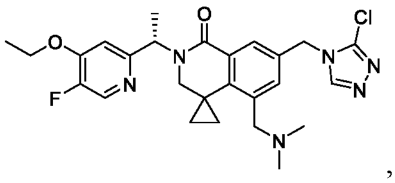, 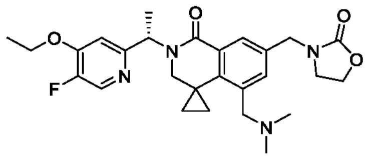,

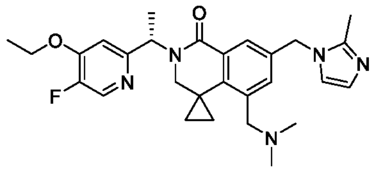, 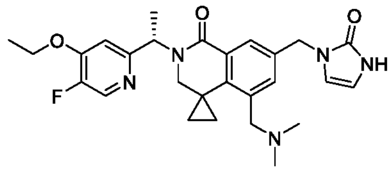, 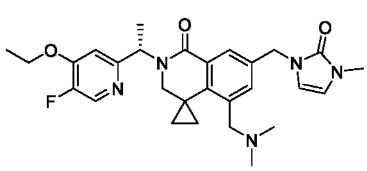,

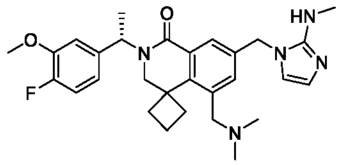, 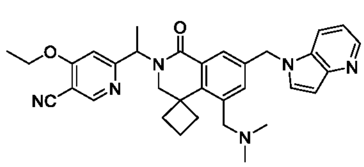, 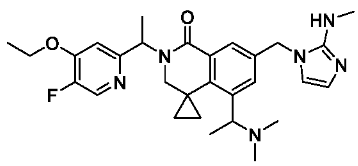,

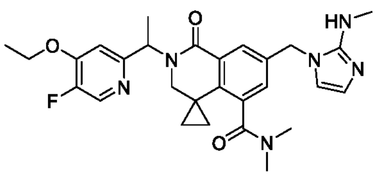, 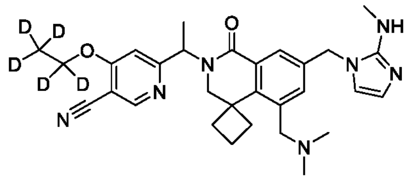, 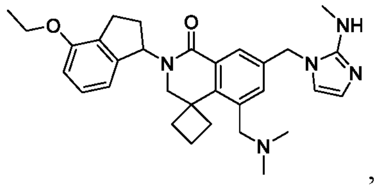,

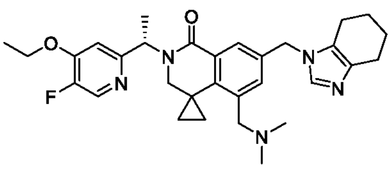, 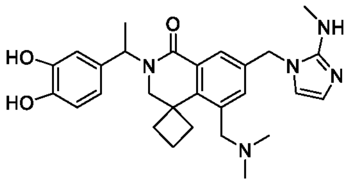, 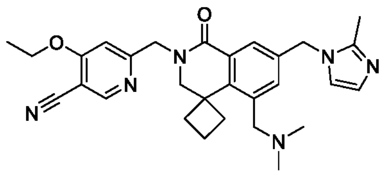,

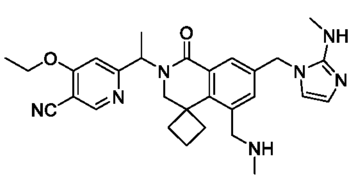, 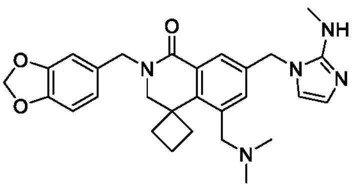, 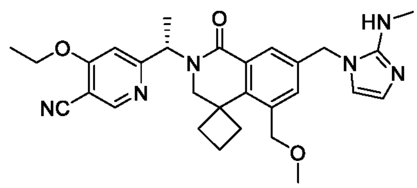

and

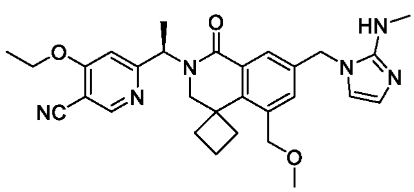.

**82.** A pharmaceutical comprising the compound according to any one of claims 1-81, or a pharmaceutically acceptable salt thereof, or an enantiomer, a diastereoisomer, a tautomer, an isotope substitute, a solvate, a polymorph, a

prodrug or metabolite thereof, and a pharmaceutically acceptable carrier or excipient.

**83.** Use of the compound according to any one of claims 1-81, or a pharmaceutically acceptable salt thereof, or an enantiomer, a diastereoisomer, a tautomer, an isotope substitute, a solvate, a polymorph, a prodrug or metabolite thereof in the manufacture of drugs for the treatment or prevention of WDR5-mediated diseases.

**84.** Use according to claim 83, wherein said WDR5-mediated disease comprises Kabuki syndrome as well as various solid tumors and hematological tumors.

**85.** Use according to claim 83, wherein said WDR5-mediated disease comprises Noonan syndrome, leopard syndrome, neuroblastoma, sarcoma, melanoma, articular chondroma, cholangioma, leukemia, breast cancer, gastrointestinal stromal tumor, histiocytic lymphoma, lung cancer, esophageal cancer, pancreatic cancer, breast cancer, prostate cancer, liver cancer, skin cancer, epithelial cell carcinoma, cervical cancer, ovarian cancer, bowel cancer, nasopharyngeal cancer, brain cancer, bone cancer, kidney cancer, oral cancer, head cancer, neuroblastoma, squamous cell carcinoma of the head and neck, anaplastic large cell lymphoma or glioblastoma, wherein leukemia is preferably juvenile myeloid monocytic leukemia, human myeloid monocytic leukemia, and lung cancer is preferably non-small cell lung cancer, small cell lung cancer, lung squamous cell carcinoma and lung adenocarcinoma.

**INTERNATIONAL SEARCH REPORT**

International application No. **PCT/CN2021/137254**

**A. CLASSIFICATION OF SUBJECT MATTER**

C07D 401/14(2006.01)i; C07D 413/14(2006.01)i; C07D 215/12(2006.01)i; A61K 31/47(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D 401/- C07D 413/- C07D 215/- A61K 31/- A61P 35/-

Documentation searched other than minimum documentation to the extent that such documents are included in the fields searched

Electronic data base consulted during the international search (name of data base and, where practicable, search terms used)

CNKI, CNABS, DWPI, VEN, REGISTRY, CAPLUS: 二氢异喹啉酮, 环烷基, 癌症, 增殖, WDR5, isoquinolinone, cycloalky +, cancer, tumor, proliferation, structure search

**C. DOCUMENTS CONSIDERED TO BE RELEVANT**

| Category* | Citation of document, with indication, where appropriate, of the relevant passages | Relevant to claim No. |
|---|---|---|
| X | WO 2020086857 A1 (VANDERBILT UNIVERSITY) 30 April 2020 (2020-04-30)<br>claims 1-12, embodiments 1-257, paragraph 00618 | 1-85 |
| X | REGISTRY. "《STN International》"<br>*CAS RN 1627175-04-5,* 26 September 2014 (2014-09-26),<br>pp. 2-3 | 1-3, 5, 10-12, 18 |
| A | Matth¨aus Getlik, et al. "Structure-Based Optimization of a Small Molecule Antagonist of the Interaction Between WD Repeat-Containing Protein 5 (WDR5) and Mixed-Lineage Leukemia 1 (MLL1)"<br>*J. Med. Chem.,* Vol. 59, No. 6, 09 March 2016 (2016-03-09),<br>pp. 2478-2496 | 1-85 |
| A | US 2015361067 A1 (PFIZER) 17 December 2015 (2015-12-17)<br>claims 1-20 | 1-85 |
| PA | WO 2021026672 A1 (NOVARTIS AG et al.) 18 February 2021 (2021-02-18)<br>entire document | 1-85 |

☐ Further documents are listed in the continuation of Box C.   ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **22 February 2022** | **09 March 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/137254**

| Patent document cited in search report | | | Publication date (day/month/year) | Patent family member(s) | | | Publication date (day/month/year) |
|---|---|---|---|---|---|---|---|
| WO | 2020086857 | A1 | 30 April 2020 | EP | 3870173 | A1 | 01 September 2021 |
| | | | | WO | 2020086857 | A8 | 12 November 2020 |
| US | 2015361067 | A1 | 17 December 2015 | TW | 201609697 | A | 16 March 2016 |
| | | | | TW | I561516 | B | 11 December 2016 |
| | | | | US | 2019040047 | A1 | 07 February 2019 |
| | | | | US | 10570121 | B2 | 25 February 2020 |
| | | | | AP | 201609605 | A0 | 31 December 2016 |
| | | | | AP | 2016009605 | A0 | 31 December 2016 |
| | | | | UY | 36170 | A | 29 January 2016 |
| | | | | SV | 2016005333 | A | 06 March 2017 |
| | | | | LT | 3157915 | T | 25 April 2019 |
| | | | | NZ | 726108 | A | 26 June 2020 |
| | | | | ME | 03419 | B | 20 January 2020 |
| | | | | PH | 12016502378 | A1 | 20 February 2017 |
| | | | | IL | 248991 | D0 | 31 January 2017 |
| | | | | IL | 248991 | A | 27 February 2020 |
| | | | | CU | 20160180 | A7 | 03 March 2017 |
| | | | | CU | 24408 | B1 | 03 May 2019 |
| | | | | MD | 20160136 | A2 | 31 May 2017 |
| | | | | JP | 6152495 | B1 | 21 June 2017 |
| | | | | JP | 2017519013 | A | 13 July 2017 |
| | | | | KR | 20170016493 | A | 13 February 2017 |
| | | | | KR | 101877187 | B1 | 10 July 2018 |
| | | | | ES | 2721031 | T3 | 26 July 2019 |
| | | | | CR | 20200484 | A | 26 April 2021 |
| | | | | CA | 2894298 | A1 | 17 December 2015 |
| | | | | CA | 2894298 | C | 18 April 2017 |
| | | | | US | 2016376254 | A1 | 29 December 2016 |
| | | | | SG | 11201609386 Y | A | 27 January 2017 |
| | | | | EP | 3157915 | A1 | 26 April 2017 |
| | | | | EP | 3157915 | B1 | 27 February 2019 |
| | | | | HR | P20190604 | T1 | 31 May 2019 |
| | | | | CN | 107207464 | A | 26 September 2017 |
| | | | | TN | 2016000529 | A1 | 04 April 2018 |
| | | | | US | 2017298048 | A1 | 19 October 2017 |
| | | | | EP | 3521285 | A1 | 07 August 2019 |
| | | | | PL | 3157915 | T3 | 31 July 2019 |
| | | | | SI | 3157915 | T1 | 31 May 2019 |
| | | | | US | 9481666 | B2 | 01 November 2016 |
| | | | | UA | 118380 | C2 | 10 January 2019 |
| | | | | WO | 2015193765 | A1 | 23 December 2015 |
| | | | | DK | 3157915 | T3 | 23 April 2019 |
| | | | | CR | 20160574 | A | 23 February 2017 |
| | | | | EA | 201692114 | A1 | 30 June 2017 |
| | | | | EA | 031892 | B1 | 29 March 2019 |
| | | | | HU | E042964 | T2 | 29 July 2019 |
| | | | | MA | 40225 | A | 27 February 2019 |
| | | | | MA | 40225 | B1 | 31 May 2019 |
| | | | | GE | P20186933 | B | 10 December 2018 |
| | | | | AU | 2015275826 | A1 | 24 November 2016 |

Form PCT/ISA/210 (patent family annex) (January 2015)

**INTERNATIONAL SEARCH REPORT**
**Information on patent family members**

International application No.
**PCT/CN2021/137254**

| Patent document cited in search report | Publication date (day/month/year) | Patent family member(s) | Publication date (day/month/year) |
| --- | --- | --- | --- |
| | | AU 2015275826 B2 | 16 May 2019 |
| WO 2021026672 A1 | 18 February 2021 | WO 2021028806 A1 | 18 February 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **CAREY ; SUNDBERG.** ADVANCED ORGANIC CHEMISTRY 4TH ED. Plenum Press, 2000, vol. A, B
- **GREENE, T. W. ; P. G. M. WUTS.** Protective Groups in Organi Synthesis. Wiley, 1999
- Chiral Separations, Methods and Protocols, Methods in Molecular Biology. 2004, vol. 243
- **A.M. STALCUP.** Annu. Rev. Anal. Chem. Chiral Separations, 2010, vol. 3, 341-63
- VOGEL'S ENCYCLOPEDIA OF PRACTICAL ORGANIC CHEMISTRY. Longman Scientific and Technical Ltd. Essex, 1991, 809-816
- **HELLER.** *Acc. Chem. Res.,* 1990, vol. 23, 128
- **GOODMAN ; GILMAN.** The Pharmacological Basis of Therapeutics. Pergamon
- Remington's, Pharmaceutical Sciences. Mack Publishing Co,
- *Tetrahedron Letters,* 2008, vol. 49 (44), 6368-6370